# EUROPEAN PATENT APPLICATION

(11) **EP 4 806 891 A1**
(43) Date of publication of application: **16.09.2026**
(21) Application number: 24888120.3
(22) Date of filing: 11.11.2024
(51) Int. Cl.: C07D 401/14, C07D 413/14, C07D 471/04, A61K 31/4709, A61K 31/538, A61K 31/5383, A61P 37/02, A61P 37/06

(54) **PYRROLIDINE DERIVATIVE, PHARMACEUTICAL COMPOSITION AND USE THEREOF IN MEDICINE**

(30) Priority: 10.11.2023 CN 202311506695; 18.11.2023 CN 202311547323; 06.12.2023 CN 202311666503; 13.12.2023 CN 202311712258; 06.02.2024 CN 202410167414; 25.04.2024 CN 202410503385; 26.04.2024 CN 202410512255; 29.05.2024 CN 202410681244; 05.06.2024 CN 202410720190; 16.08.2024 CN 202411127894
(71) Applicant: Kangbaida (Sichuan) Biotechnology Co., Ltd., Chengdu, Sichuan 610000 (CN)
(72) Inventor: YUAN, Yonghai, Chengdu, Sichuan 610000 (CN); WEI, Yonggang, Chengdu, Sichuan 610000 (CN); JIA, Zhilong, Chengdu, Sichuan 610000 (CN)
(74) Representative: Murgitroyd & Company
(86) International application number: PCT/CN2024/131222
(87) International publication number: WO 2025/098509

(57) **Abstract**

The present invention relates to a pyrrolidine derivative represented by general formula (I), a pharmaceutical composition and a use thereof in medicine.

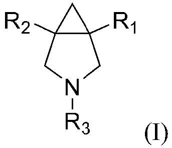

## Description

### TECHNICAL FIELD

The present disclosure relates to a pyrrolidine derivative, a pharmaceutical composition, and a use thereof in medicine.

### BACKGROUND

Toll-like receptors (TLRs) are a class of pattern recognition receptors widely distributed in various tissues. They monitor and recognize different pathogen-associated molecular patterns (PAMPs) and damage-associated molecular patterns (DAMPs), playing important roles in both innate and adaptive immunity.

TLRs belong to type I transmembrane proteins. To date, 13 members of the TLR family have been discovered, 10 of which exist in humans. TLR1, TLR2, TLR4, TLR5, TLR6, TLR10, and TLR11 are located on the cell membrane and can recognize microbial lipids, lipoproteins, and other substances; while TLR3, TLR7, TLR8, and TLR9 are located in intracellular vesicular structures (such as lysosomes, endosomes, and endoplasmic reticulum) and recognize microbial nucleic acids.

TLR7 and TLR8 are the most similar in sequence and function. Numerous studies have shown that activation of TLR7/8 can trigger type I interferon responses and various inflammatory reactions. In autoimmune disorders such as systemic lupus erythematosus (SLE), abnormal and persistent activation of TLR7/8 leads to worsening of the disease state. Therefore, developing compounds with selective and potent inhibitory activity that suppress excessively activated immune responses by antagonizing TLR7/8 is expected to become a new approach for the treatment of autoimmune diseases.

### SUMMARY

The objective of the present disclosure is to provide novel pyrrolidine derivatives or stereoisomers, pharmaceutical compositions thereof, and uses thereof in the manufacture of medicaments for autoimmune diseases.

One or more embodiments of the present disclosure provide a compound represented by general formula (I), or all stereoisomers, pharmaceutically acceptable salts, or deuterated derivatives thereof: 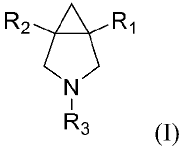 wherein:
R₂ is C₁₋₆ alkyl or C₃₋₁₀ cycloalkyl, wherein the C₁₋₆ alkyl or C₃₋₁₀ cycloalkyl is optionally further substituted with one or more halogens;
R₃ is 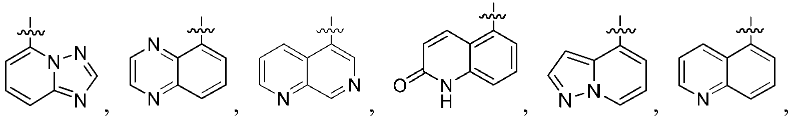 or 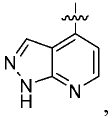 and R₃ is optionally further substituted with one or more substituents selected from the group consisting of C₁₋₃ haloalkyl, D, C₁₋₃ alkyl, halogen, -C₁₋₃ alkoxy, -O-C₁₋₃ haloalkyl, cyano, and -NO₂;
R₁ is 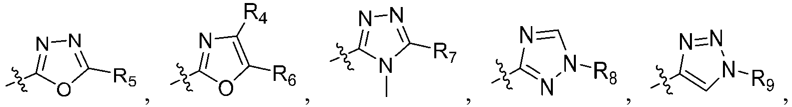 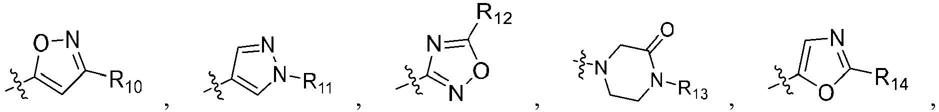 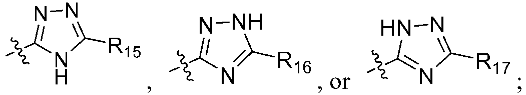
and when R₃ is 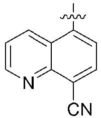 and R₁ is 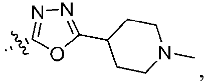 then R₂ is not -CF₃;
R₄ is H, C₁₋₆ alkyl, C₃₋₁₀ cycloalkyl, or halogen;
R₅, R₆, R₇, R₈, R₉, R₁₀, R₁₁, R₁₂, R₁₃, R₁₄, R₁₅, R₁₆, and R₁₇ are each independently H, C₁₋₆ alkyl, halogen, 7- to 10-membered bridged or spiro ring, 3- to 10-membered monocyclic heterocycle, 4- to 7-membered monocyclic carbocycle, 2- to 6-membered heteroalkyl, alkynyl, -NH₂, -C₁₋₃ alkyl-NH₂, -O-3- to 10-membered monocyclic heterocycle, -C₁₋₃ alkoxy-3- to 10-membered monocyclic heterocycle, -NH-3- to 10-membered monocyclic heterocycle, or -C₁₋₃ alkyl-3- to 10-membered monocyclic heterocycle;
R₅, R₆, R₇, R₈, R₉, R₁₀, R₁₁, R₁₂, R₁₃, R₁₄, R₁₅, R₁₆, and R₁₇ are each independently optionally further substituted with one or more substituted or unsubstituted -NH-CHO, C₁₋₆ alkyl, 2- to 6-membered heteroalkyl, -C₁₋₃ alkyl-CO-NH₂, =O, halogen, 3- to 10-membered cycloalkyl, hydroxyl, or -NH₂.

In the compound, or all stereoisomers, pharmaceutically acceptable salts, or deuterated derivatives thereof provided by one or more embodiments of the present disclosure, wherein:
the substituted R₅, R₆, R₇, R₈, R₉, R₁₀, R₁₁, R₁₂, R₁₃, R₁₄, R₁₅, R₁₆, or R₁₇ is further substituted with one or more substituents selected from the group consisting of 3- to 10-membered cycloalkyl, 3- to 10-membered heterocycloalkyl, C₁₋₆ alkyl, halogen, and -N-(CH₃)₂.

In the compound, or all stereoisomers, pharmaceutically acceptable salts, or deuterated derivatives thereof provided by one or more embodiments of the present disclosure, wherein:
R₁ is 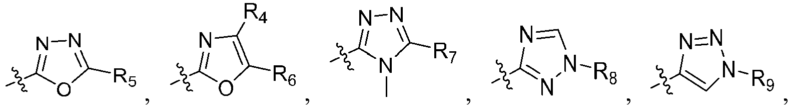 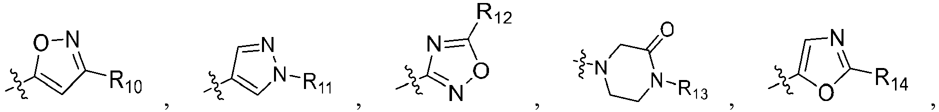 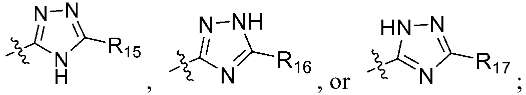
R₄ is H, C₁₋₆ alkyl, or halogen;
R₅, R₆, R₇, R₈, R₉, R₁₀, R₁₁, R₁₂, R₁₃, R₁₄, R₁₅, R₁₆, and R₁₇ are each independently C₁₋₆ alkyl, 2- to 6-membered heteroalkyl, alkynyl, -NH₂, -C₁₋₃ alkyl-NH₂, -O-3- to 10-membered monocyclic heterocycle, -C₁₋₃ alkoxy-3- to 10-membered monocyclic heterocycle, -NH-3- to 10-membered monocyclic heterocycle, or -C₁₋₃ alkyl-3- to 10-membered monocyclic heterocycle;
R₅, R₆, R₇, R₈, R₉, R₁₀, R₁₁, R₁₂, R₁₃, R₁₄, R₁₅, R₁₆, and R₁₇ are each independently optionally further substituted with one or more substituted or unsubstituted -NH-CHO, C₁₋₆ alkyl, 2- to 6-membered heteroalkyl, -C₁₋₃ alkyl-CO-NH₂, =O, halogen, 3- to 10-membered cycloalkyl, hydroxyl, or -NH₂;
the substituted R₅, R₆, R₇, R₈, R₉, R₁₀, R₁₁, R₁₂, R₁₃, R₁₄, R₁₅, R₁₆, or R₁₇ is optionally further substituted with one or more substituents selected from the group consisting of 3- to 10-membered cycloalkyl, 3- to 10-membered heterocycloalkyl, C₁₋₆ alkyl, halogen, and -N-(CH₃)₂.

In the compound, or all stereoisomers, pharmaceutically acceptable salts, or deuterated derivatives thereof provided by one or more embodiments of the present disclosure, wherein:
R₅ and R₇ are each independently C₁₋₆ alkyl, 2- to 6-membered heteroalkyl, alkynyl, - NH₂, -C₁₋₃ alkyl-NH₂, -O-3- to 10-membered monocyclic heterocycle, -C₁₋₃ alkoxy-3- to 10-membered monocyclic heterocycle, -NH-3- to 10-membered monocyclic heterocycle, or -C₁₋₃ alkyl-3- to 10-membered monocyclic heterocycle;
R₅ and R₇ are each independently optionally further substituted with one or more substituted or unsubstituted C₁₋₆ alkyl, 2- to 6-membered heteroalkyl, or -NH₂;
the substituted R₅ or R₇ is optionally further substituted with one or more substituents selected from the group consisting of 3- to 10-membered heterocycloalkyl, C₁₋₆ alkyl, and -N-(CH₃)₂.

In the compound, or all stereoisomers, pharmaceutically acceptable salts, or deuterated derivatives thereof provided by one or more embodiments of the present disclosure, wherein:
R₁ is 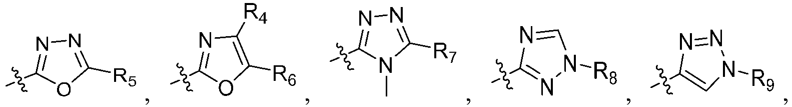 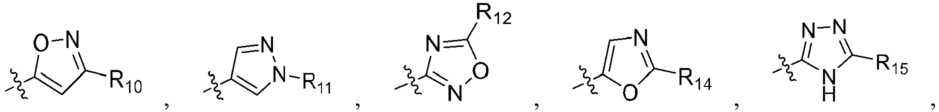 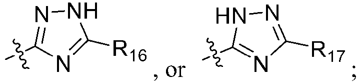
R₄ is H or halogen;
R₅, R₆, R₇, R₈, R₉, R₁₀, R₁₁, R₁₂, R₁₄, R₁₅, R₁₆, and R₁₇ are each independently 7- to 8-membered bridged ring, 7- to 9-membered spiro heterocycle, 4- to 6-membered monocyclic heterocycle, or 6-membered monocyclic carbocycle.
R₅, R₆, R₇, R₈, R₉, R₁₀, R₁₁, R₁₂, R₁₄, R₁₅, R₁₆, and R₁₇ are each independently optionally further substituted with one or more substituted or unsubstituted -NH-CHO, C₁₋₆ alkyl, hydroxyl, -C₁₋₃ alkyl-CO-NH₂, =O, halogen, or -NH₂;
the substituted R₅, R₆, R₇, R₈, R₉, R₁₀, R₁₁, R₁₂, R₁₄, R₁₅, R₁₆, or R₁₇ is further substituted with one or more substituents selected from the group consisting of 3- to 5-membered cycloalkyl, 5- to 6-membered heterocycloalkyl, and C₁₋₆ alkyl.

In the compound, or all stereoisomers, pharmaceutically acceptable salts, or deuterated derivatives thereof provided by one or more embodiments of the present disclosure, wherein:
R₅ is 4- to 6-membered non-aromatic nitrogen-containing monocyclic heterocycle, 6-membered non-aromatic monocyclic carbocycle, 8-membered bridged carbocycle, 7- to 8-membered nitrogen-containing bridged heterocycle, or 7- to 9-membered nitrogen-containing spiro heterocycle;
the 4- to 6-membered non-aromatic nitrogen-containing monocyclic heterocycle is optionally further substituted with one or more substituents selected from the group consisting of halogen, C₁₋₃ alkyl, -C₁₋₃ alkyl-CO-NH₂, =O, -NH₂, hydroxyl, and cyclopropyl-substituted C₁₋₃ alkyl;
the 8-membered bridged carbocycle is optionally further substituted with one or more substituents selected from the group consisting of -NH₂ and -NH-CHO, wherein the -NH₂ or - NH-CHO is optionally further substituted with C₁₋₃ alkyl or 6-membered nitrogen-containing heterocyclyl;
the 7- to 8-membered nitrogen-containing bridged heterocycle is optionally further substituted with one or more C₁₋₃ alkyl;
the 7- to 9-membered nitrogen-containing spiro heterocycle is optionally further substituted with one or more substituents selected from the group consisting of -C₁₋₃ alkyl-CO-NH₂, C₁₋₃ alkyl, and cyclopropyl-substituted C₁₋₃ alkyl;
the 6-membered non-aromatic monocyclic carbocycle is optionally further substituted with one or more -NH₂ or N-(CH₃)₂;
R₆, R₇, R₈, R₉, R₁₀, R₁₁, R₁₂, R₁₄, R₁₅, and R₁₆ are 6-membered non-aromatic nitrogen-containing monocyclic heterocycle, wherein the 6-membered non-aromatic nitrogen-containing monocyclic heterocycle is optionally further substituted with one or more C₁₋₃ alkyl;

In the compound, or all stereoisomers, pharmaceutically acceptable salts, or deuterated derivatives thereof provided by one or more embodiments of the present disclosure, the compound is one of the following structures:

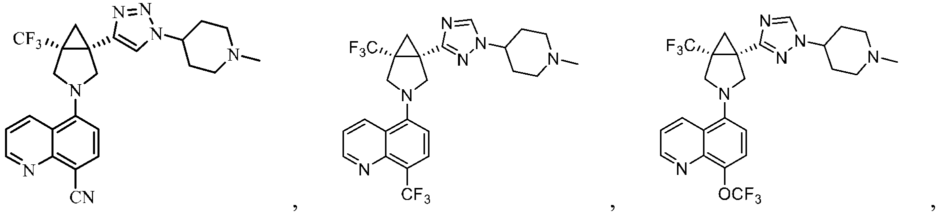

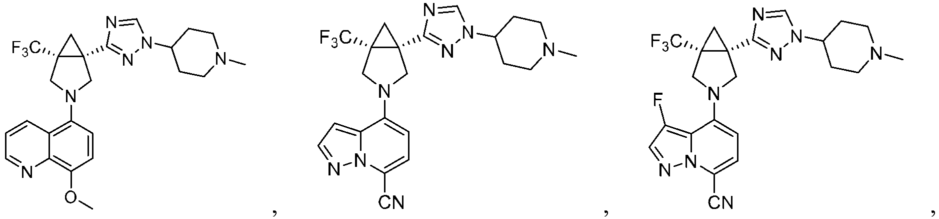

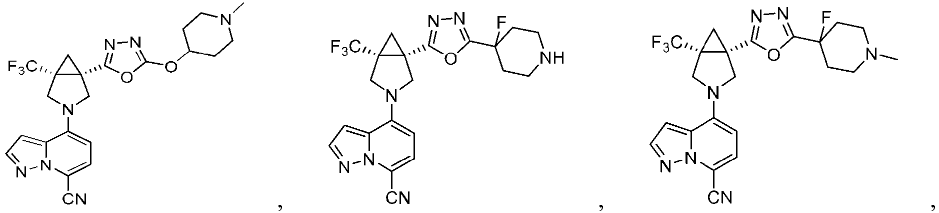

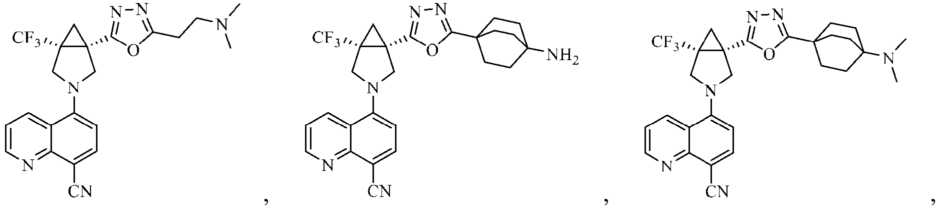

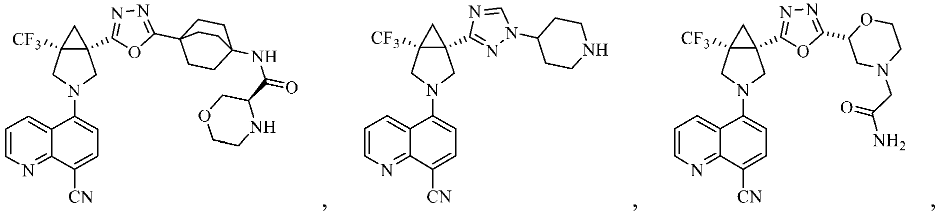

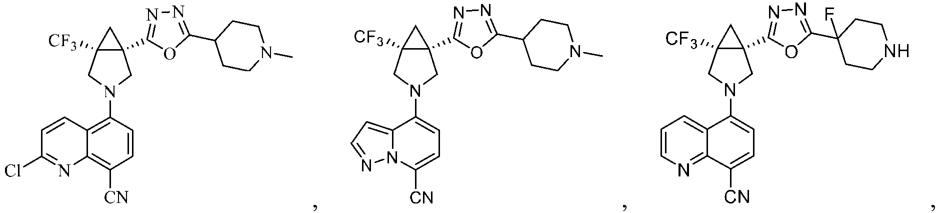

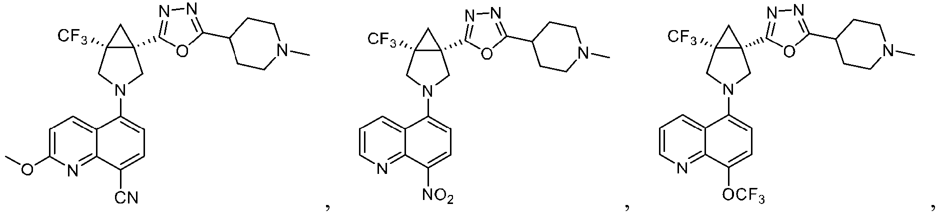

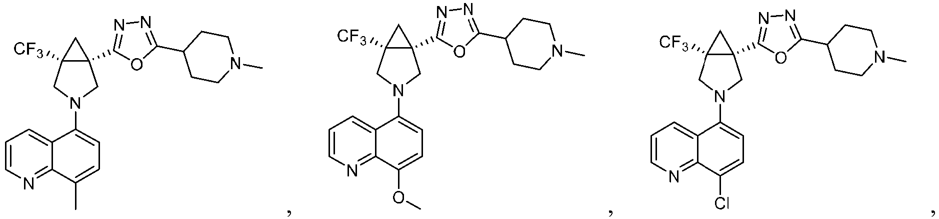

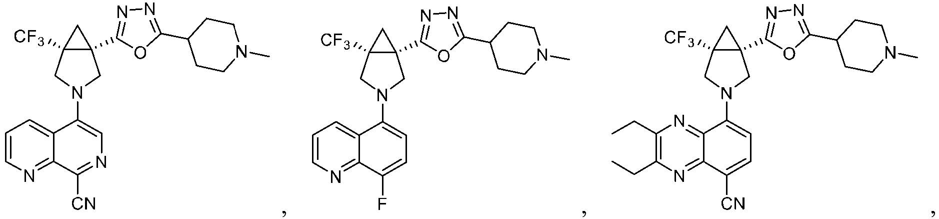

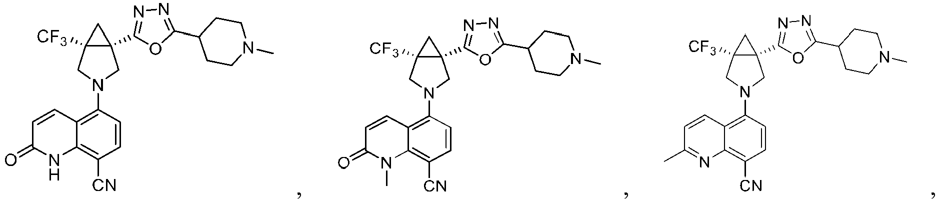

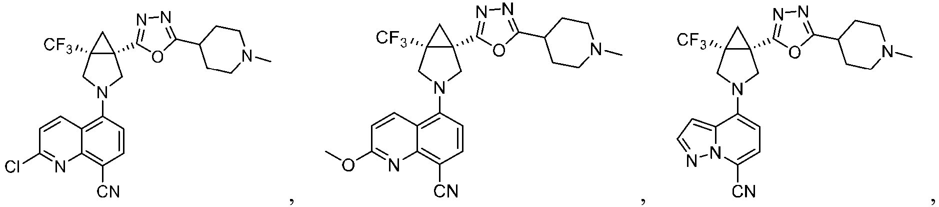

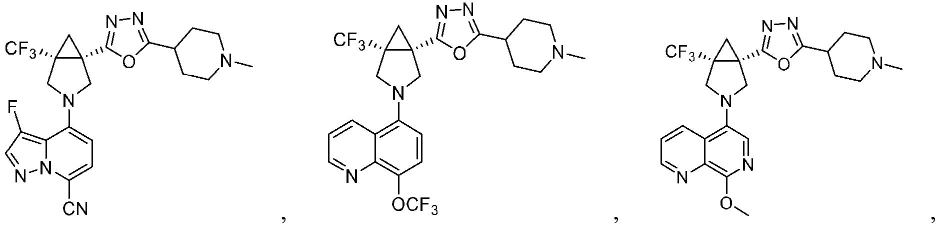

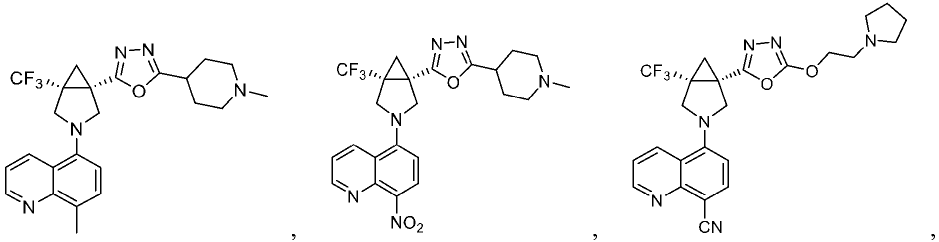

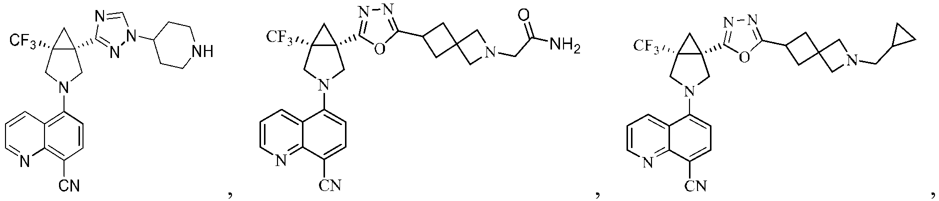

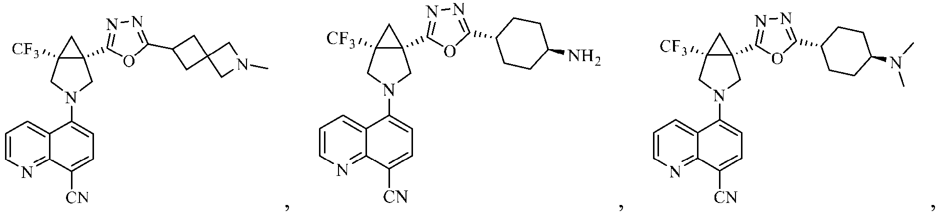

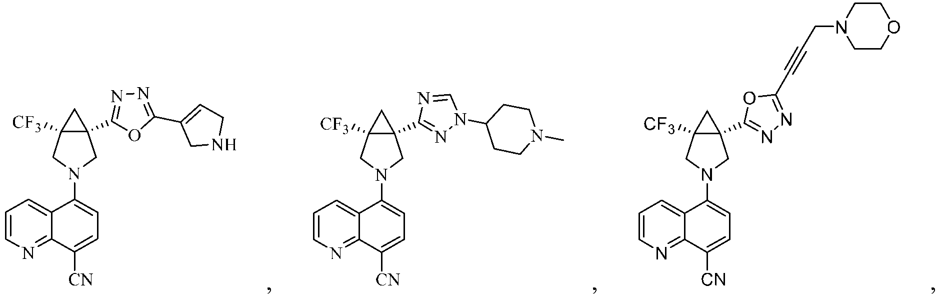

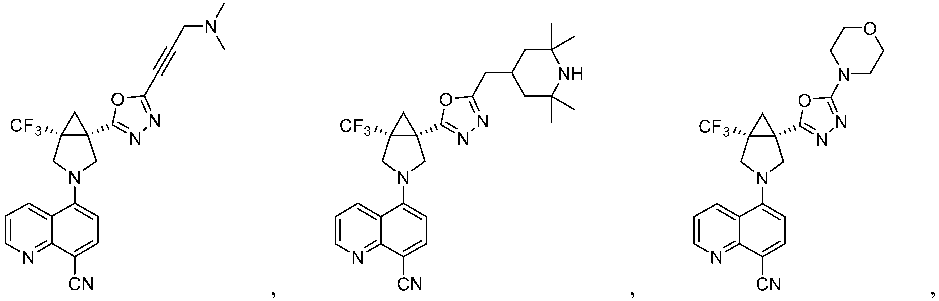

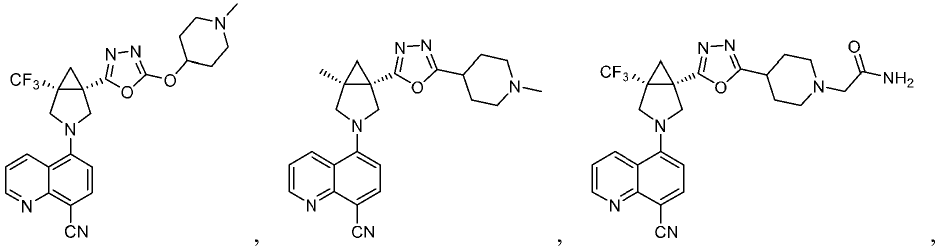

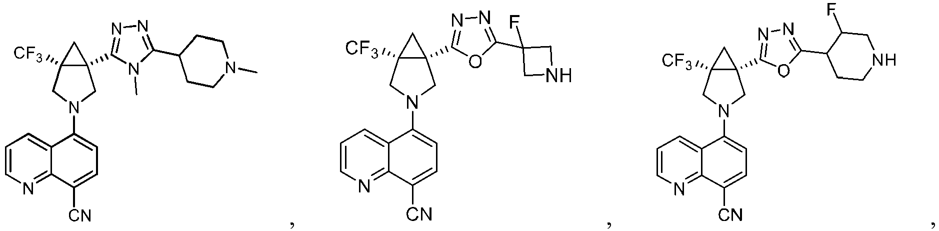

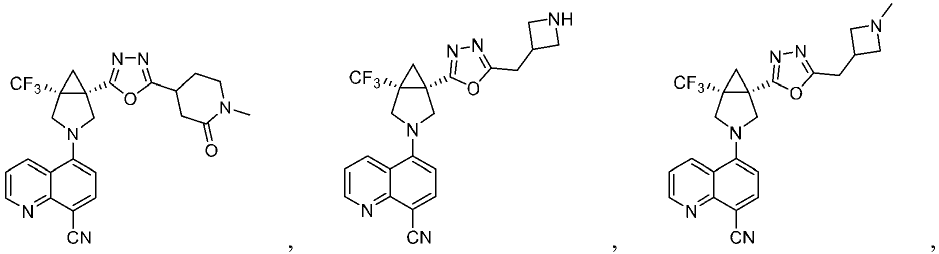

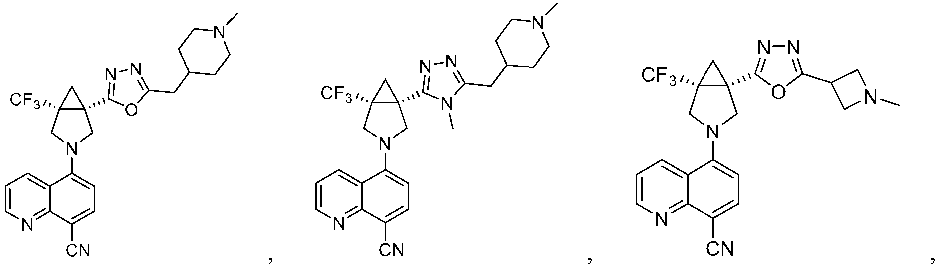

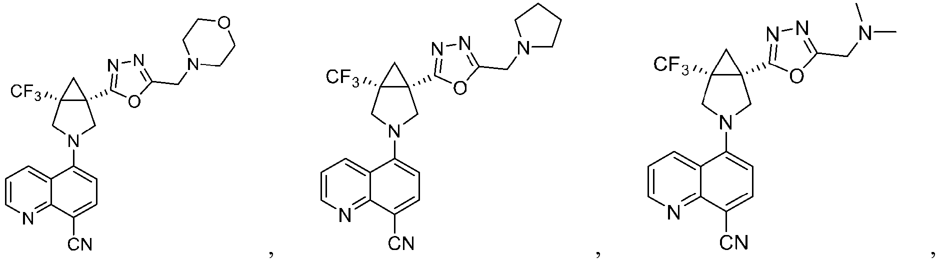

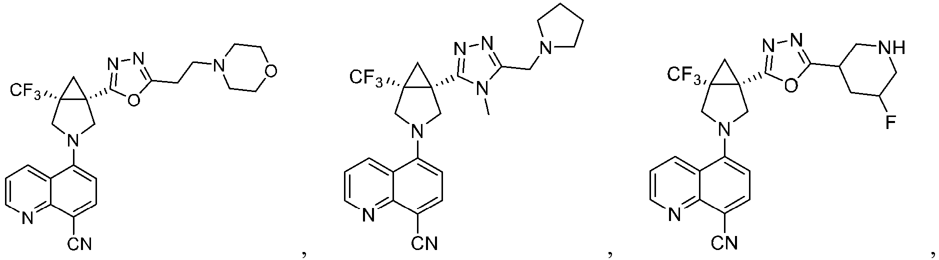

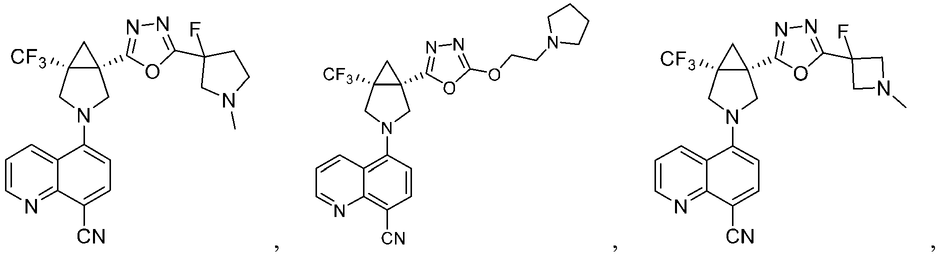

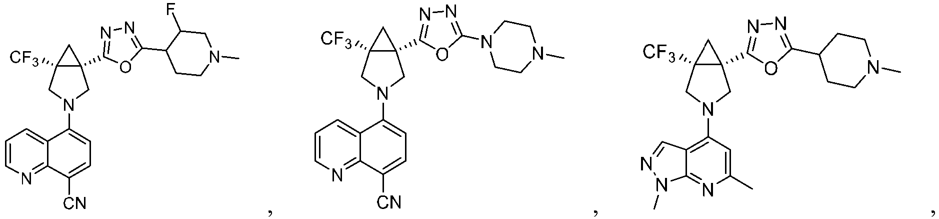

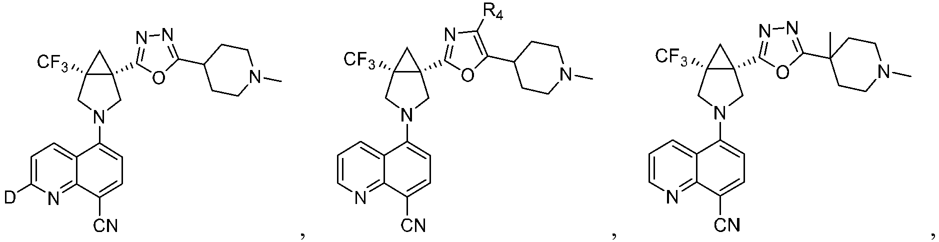

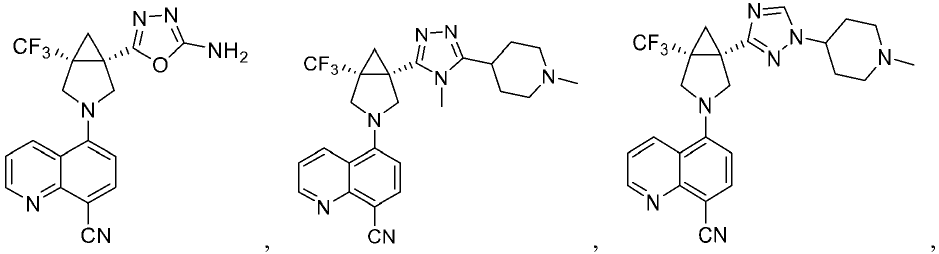

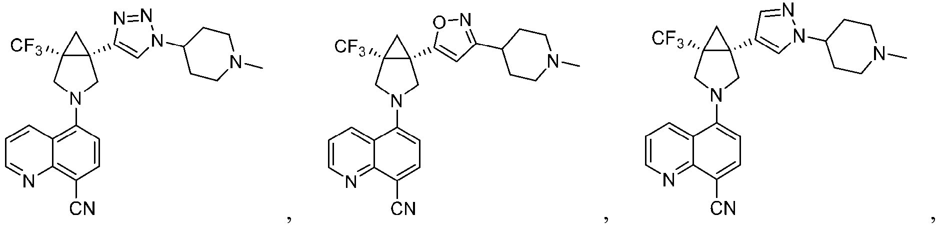

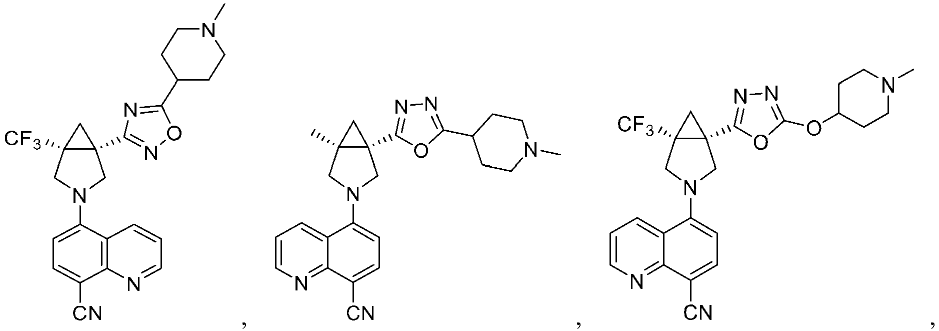

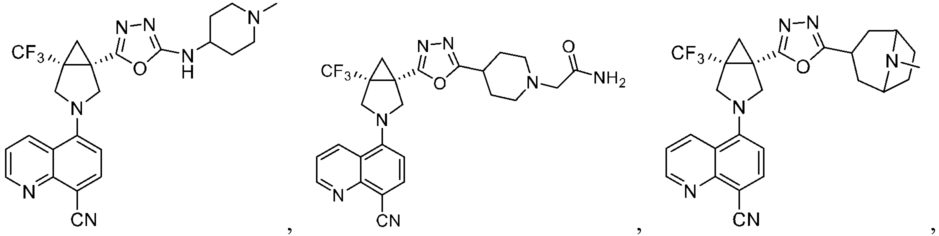

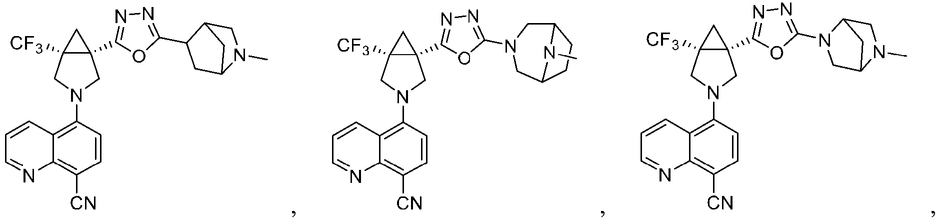

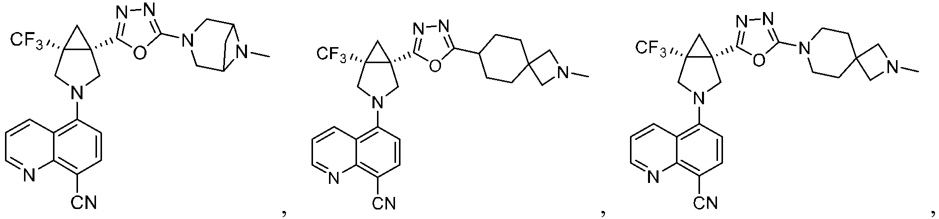

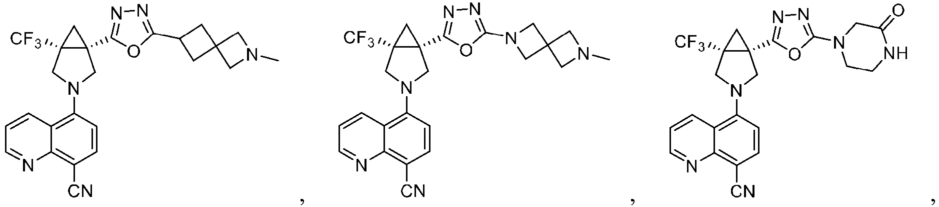

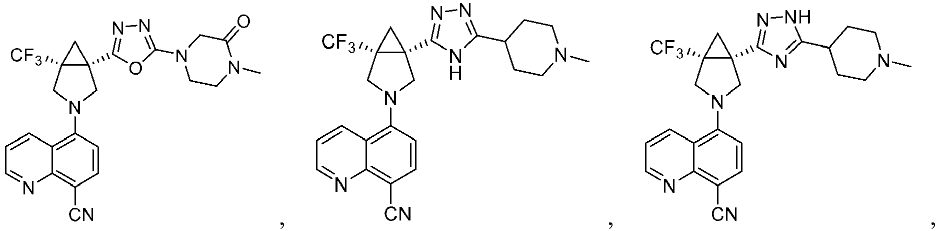

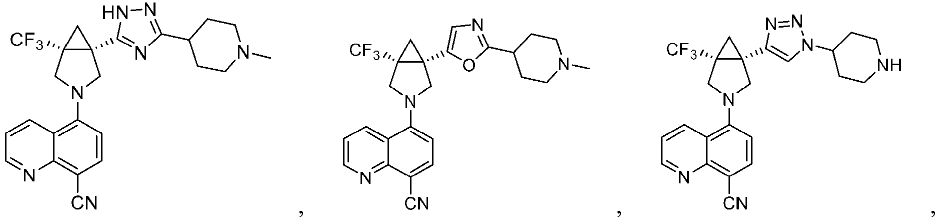

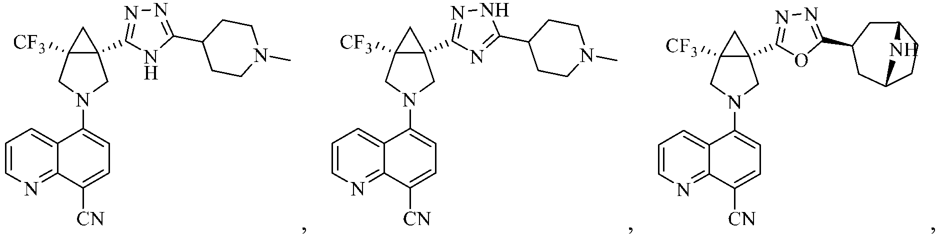

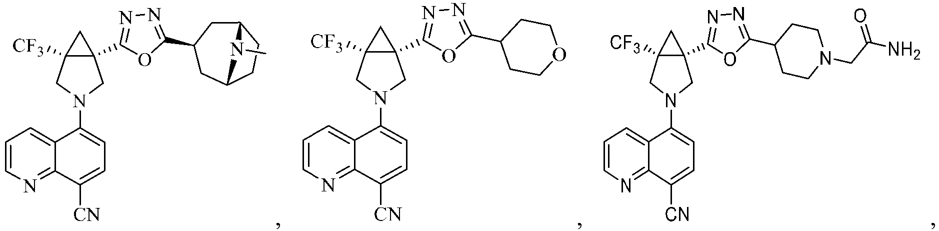

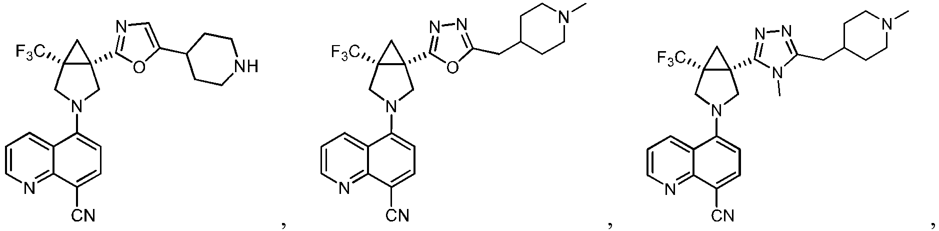

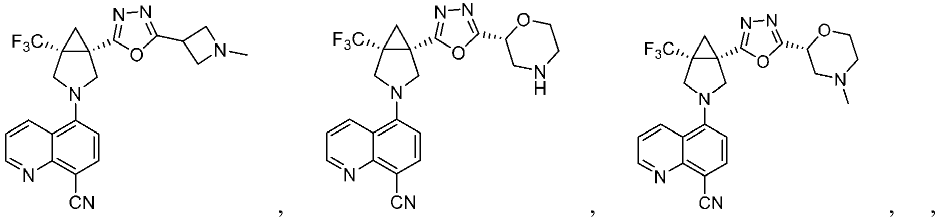

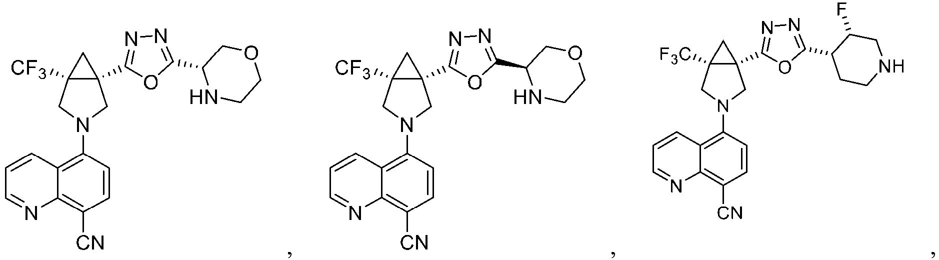

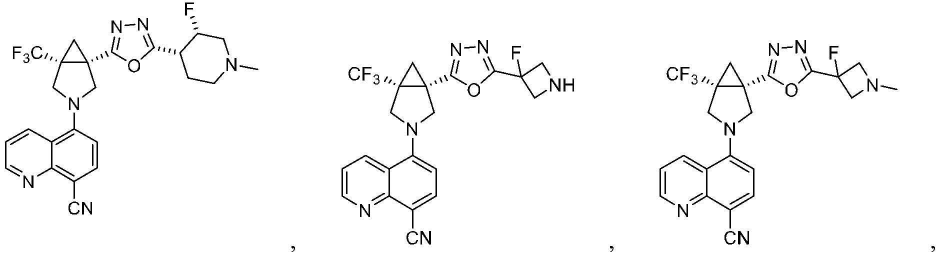

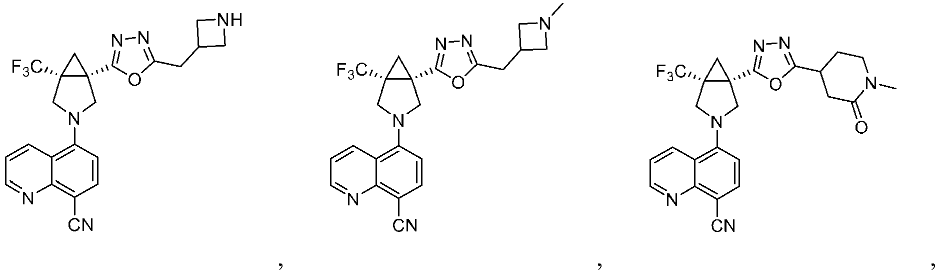

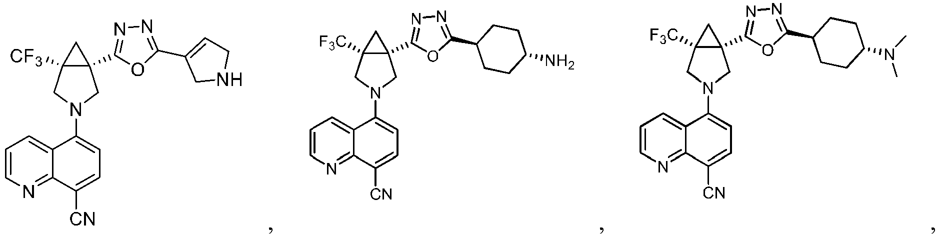

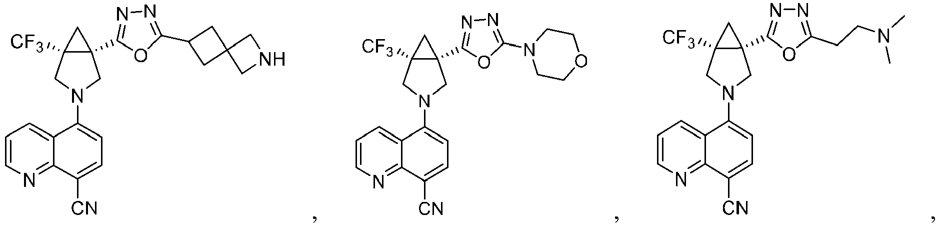

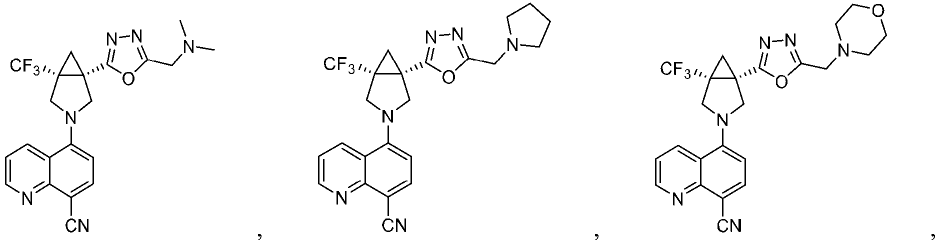

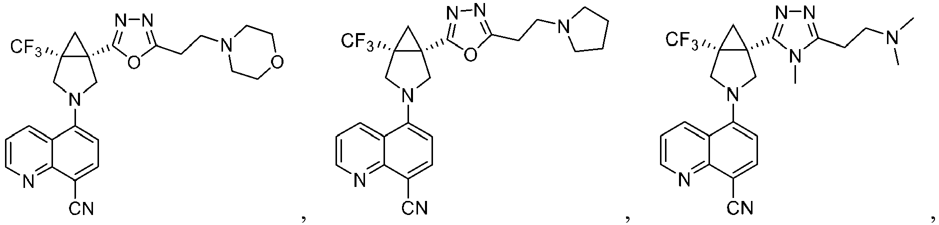

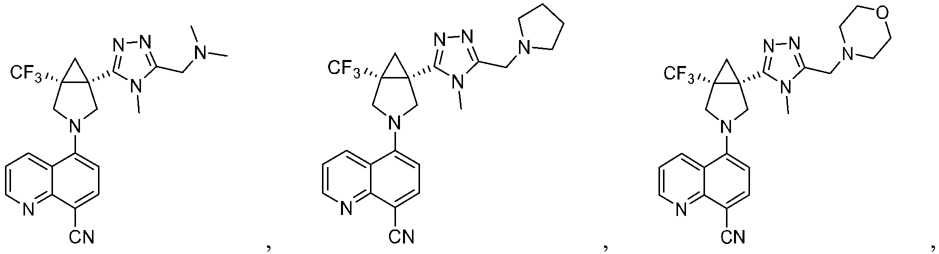

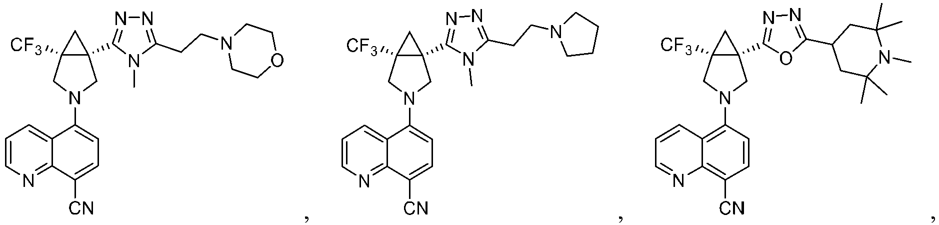

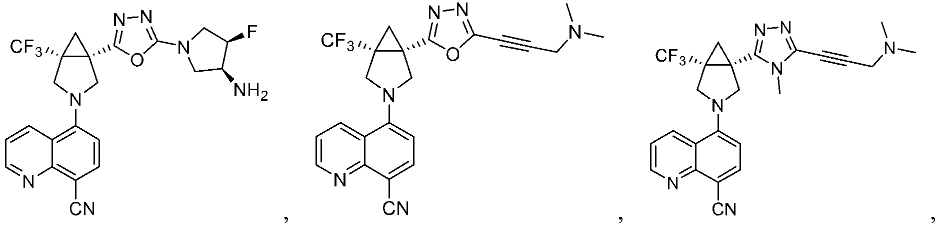

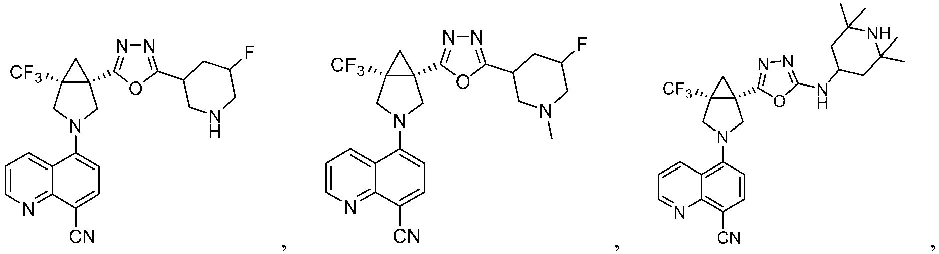

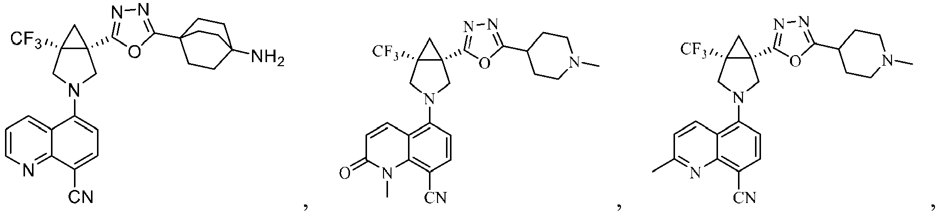

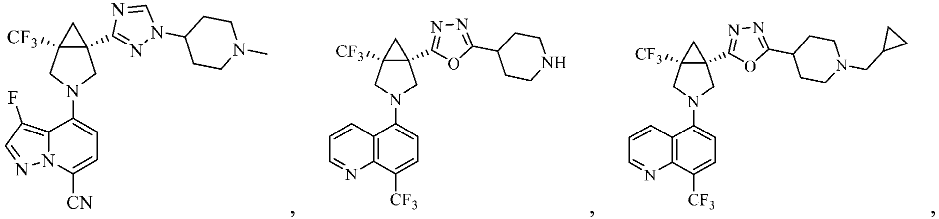

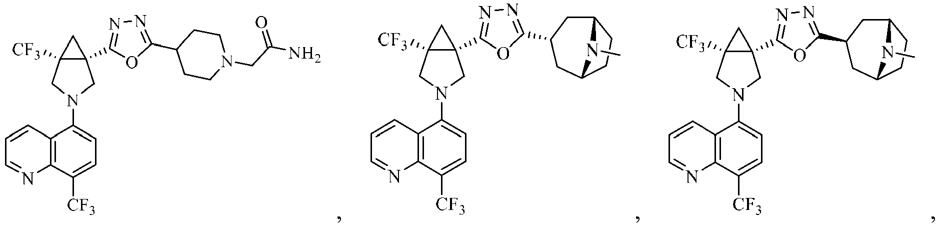

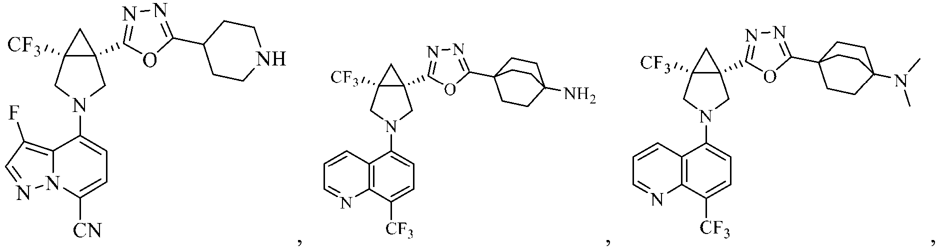

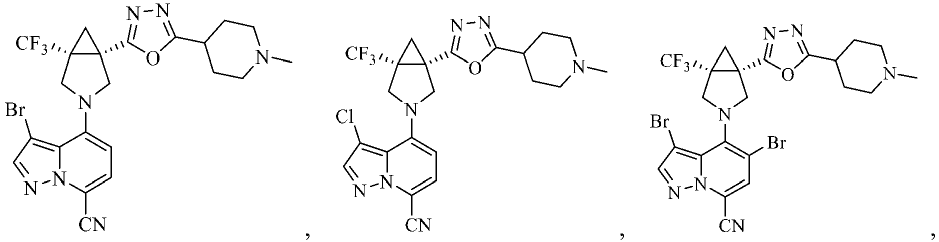

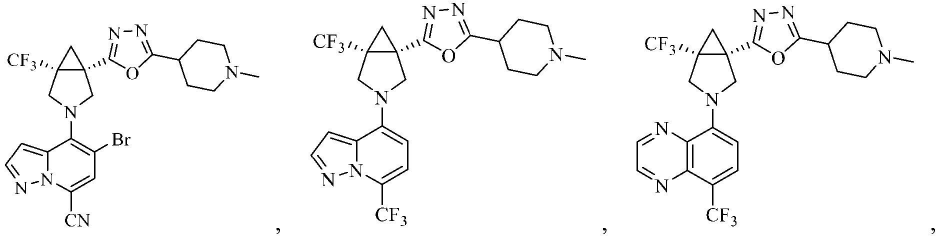

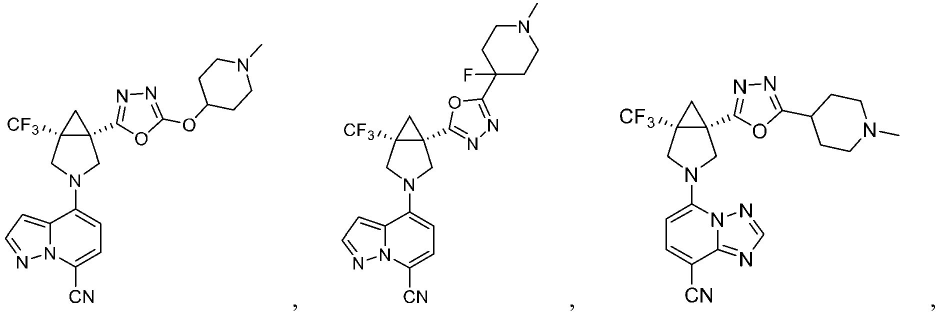

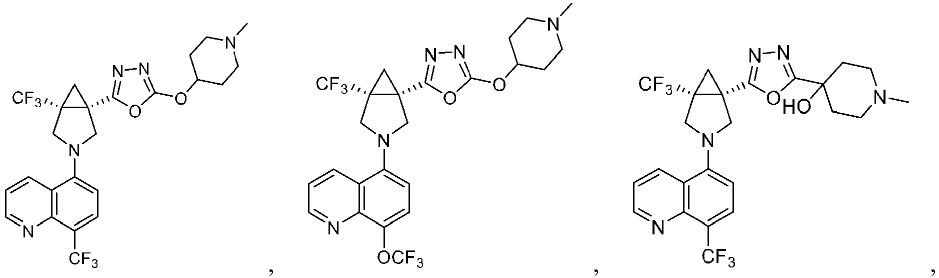

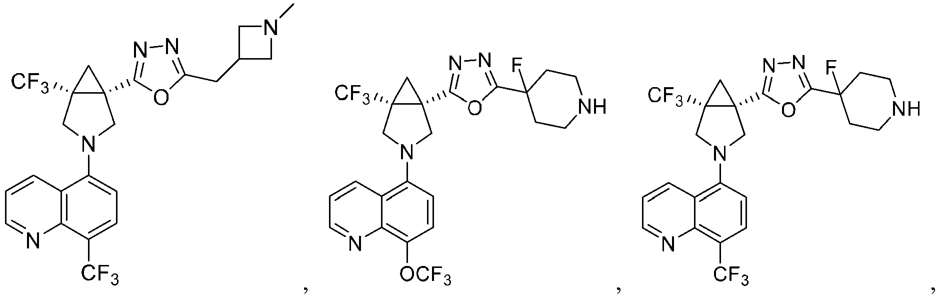

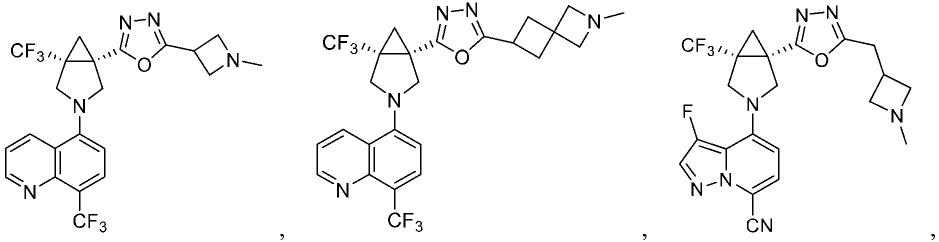

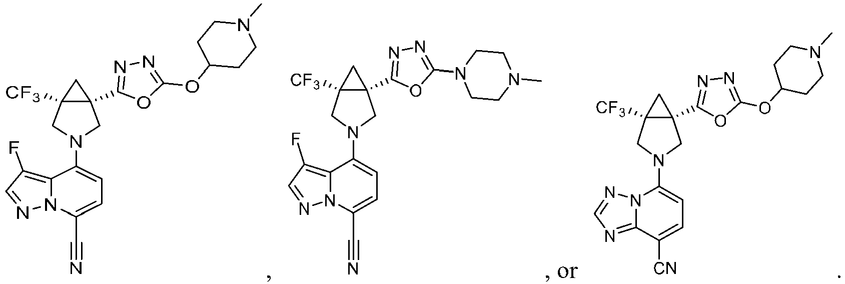

One or more embodiments of the present disclosure provide a compound represented by general formula (I-1), or all stereoisomers, pharmaceutically acceptable salts, or deuterated derivatives thereof: 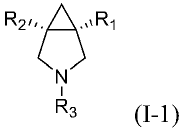 wherein:
R₂ is C₁₋₆ alkyl, wherein the alkyl is optionally further substituted with one or more halogens;
when R₃ is 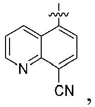 R₁ is 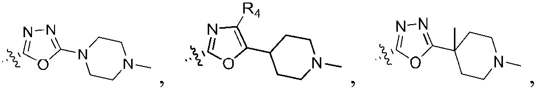 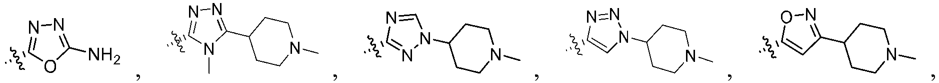 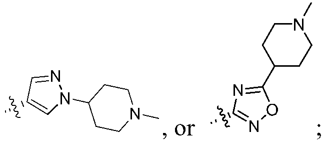
when R₃ is 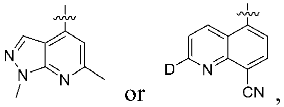 R₁ is 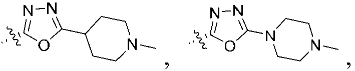 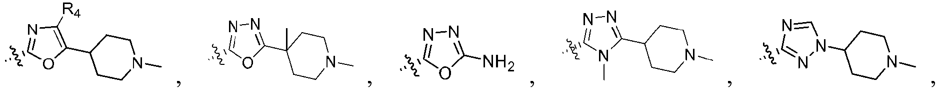 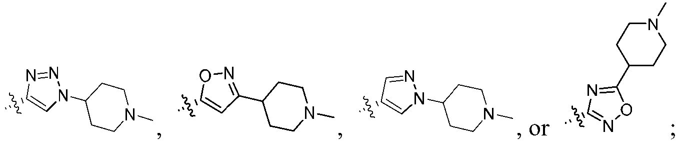
R₄ is C₁₋₆ alkyl or halogen.

One or more embodiments of the present disclosure provide a compound represented by general formula (I-2), or all stereoisomers, pharmaceutically acceptable salts, or deuterated derivatives thereof: 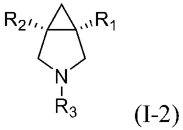 wherein:
R₂ is C₁₋₆ alkyl, wherein the alkyl is optionally further substituted with one or more halogens;
R₃ is 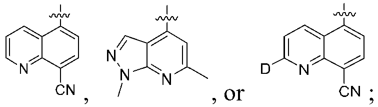
R₁ is 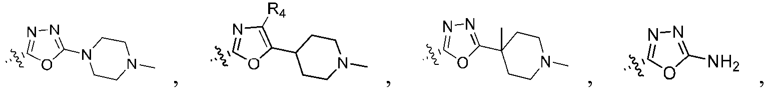 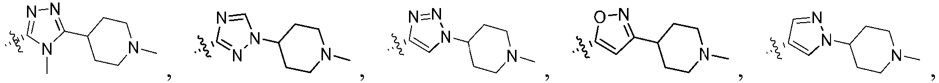 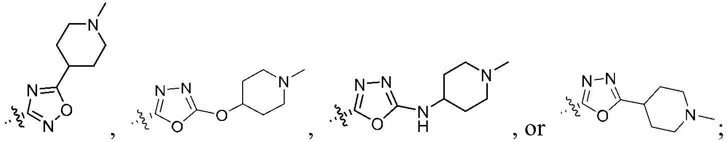
and when R₃ is 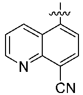 and R₁ is 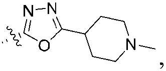 then R₂ is not -CF₃;
R₄ is C₁₋₆ alkyl or halogen.

One or more embodiments of the present disclosure provide a compound represented by general formula (I-3), or all stereoisomers, pharmaceutically acceptable salts, or deuterated derivatives thereof: 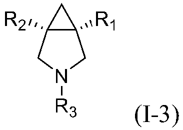 wherein:
R₂ is C₁₋₆ alkyl, wherein the alkyl is optionally further substituted with one or more halogens;
R₃ is 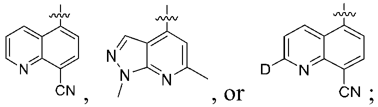
R₁ is 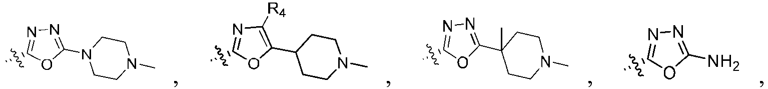 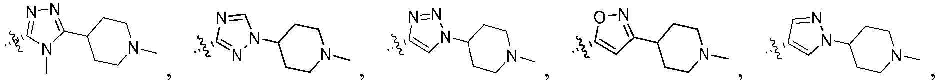 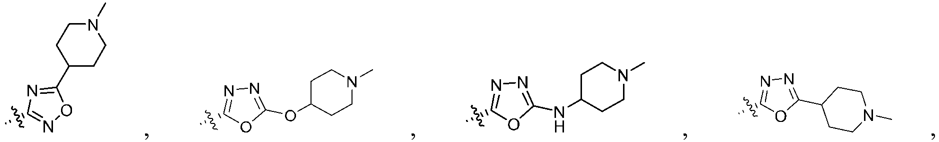 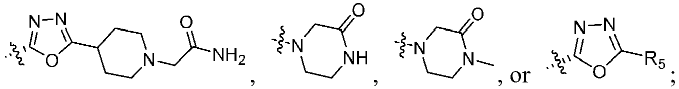
and when R₃ is 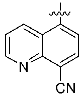 and R₁ is 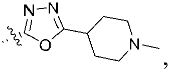 then R₂ is not -CF₃;
R₄ is C₁₋₆ alkyl or halogen;
R₅ is 7- to 10-membered bridged or spiro ring, and R₅ contains at least one N.

In one or more embodiments, R₅ is: 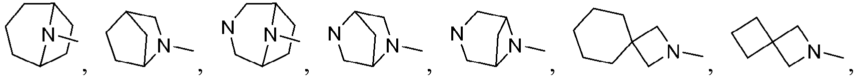 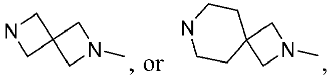 and the connection site of R₅ is at any connectable position thereon.

One or more embodiments of the present disclosure provide a compound represented by general formula (I-4), or all stereoisomers, pharmaceutically acceptable salts, or deuterated derivatives thereof: 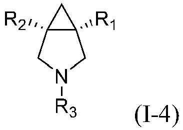 wherein:
R₂ is C₁₋₆ alkyl, wherein the alkyl is optionally further substituted with one or more halogens;
R₃ is 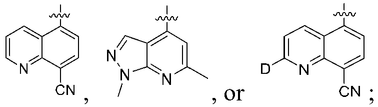
R₁ is 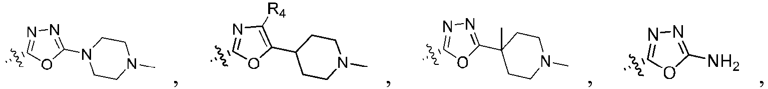 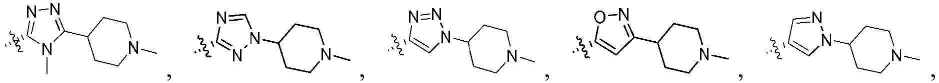 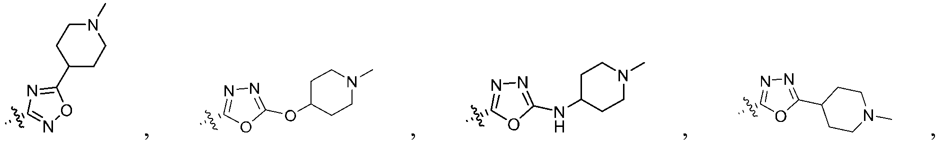 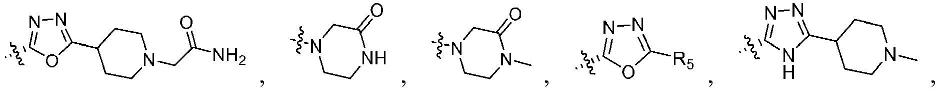 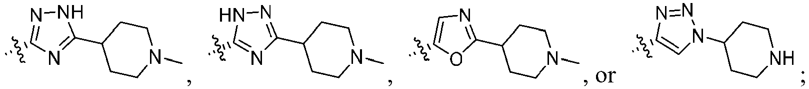
and when R₃ is 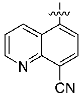 and R₁ is 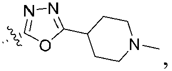 then R₂ is not -CF₃;
R₄ is C₁₋₆ alkyl or halogen;
R₅ is 7- to 10-membered bridged or spiro ring, and R₅ contains at least one N.

In one or more embodiments, R₅ is: 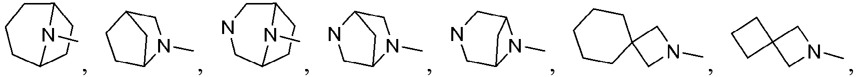 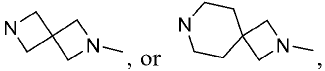 and the connection site of R₅ is at any connectable position thereon.

One or more embodiments of the present disclosure provide a compound represented by general formula (I-5), or all stereoisomers, pharmaceutically acceptable salts, or deuterated derivatives thereof: 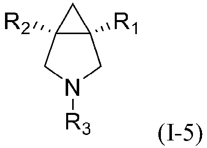 wherein:
R₂ is C₁₋₆ alkyl or C₃₋₁₀ cycloalkyl, wherein the C₁₋₆ alkyl or C₃₋₁₀ cycloalkyl is optionally further substituted with one or more halogens;
R₃ is 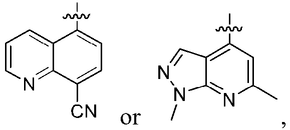 and R₃ is optionally substituted with one or more D;
R₁ is 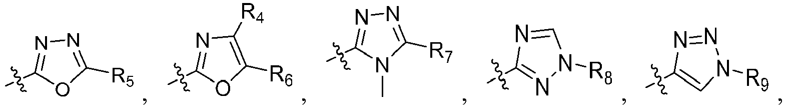 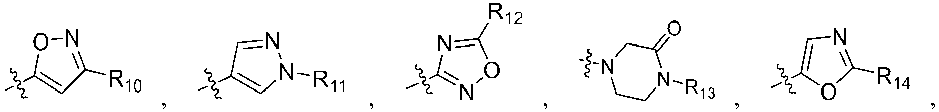 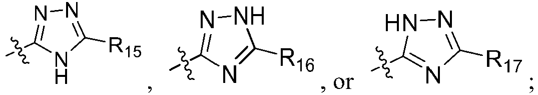
and when R₃ is 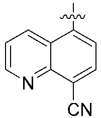 and R₁ is 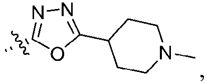 then R₂ is not -CF₃;
R₄ is H, C₁₋₆ alkyl, C₃₋₁₀ cycloalkyl, or halogen;
R₅, R₆, R₇, R₈, R₉, R₁₀, R₁₁, R₁₂, R₁₃, R₁₄, R₁₅, R₁₆, and R₁₇ are each independently H, C₁₋₆ alkyl, halogen, 7- to 10-membered bridged or spiro ring, 3- to 10-membered monocyclic heterocycle, 4- to 7-membered monocyclic carbocycle, 2- to 6-membered heteroalkyl, alkynyl, -NH₂, -O-3- to 10-membered monocyclic heterocycle, -NH-3- to 10-membered monocyclic heterocycle, or -C₁₋₃ alkyl-3- to 10-membered monocyclic heterocycle;
R₅, R₆, R₇, R₈, R₉, R₁₀, R₁₁, R₁₂, R₁₃, R₁₄, R₁₅, R₁₆, and R₁₇ are each independently optionally further substituted with one or more substituted or unsubstituted C₁₋₆ alkyl, 2- to 6-membered heteroalkyl, -C₁₋₃ alkyl-CO-NH₂, =O, halogen, 3- to 10-membered cycloalkyl, or - NH₂.

One or more embodiments of the present disclosure provide a compound represented by general formula (I-6), or all stereoisomers, pharmaceutically acceptable salts, or deuterated derivatives thereof: 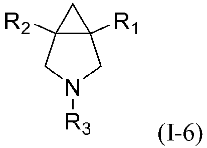 wherein:
R₂ is C₁₋₆ alkyl or C₃₋₁₀ cycloalkyl, wherein the C₁₋₆ alkyl or C₃₋₁₀ cycloalkyl is optionally further substituted with one or more halogens;
R₃ is 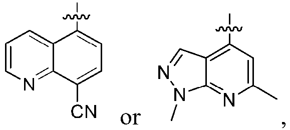 and R₃ is optionally substituted with one or more D;
R₁ is 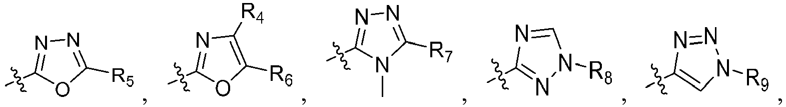 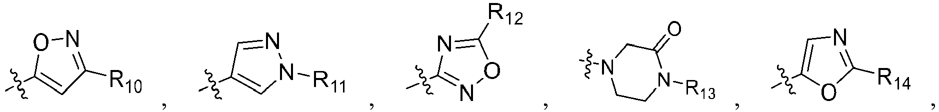 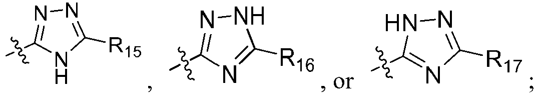
and when R₃ is 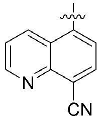 and R₁ is 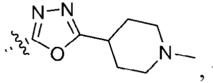 then R₂ is not -CF₃;
R₄ is H, C₁₋₆ alkyl, C₃₋₁₀ cycloalkyl, or halogen;
R₅, R₆, R₇, R₈, R₉, R₁₀, R₁₁, R₁₂, R₁₃, R₁₄, R₁₅, R₁₆, and R₁₇ are each independently H, C₁₋₆ alkyl, halogen, 7- to 10-membered bridged or spiro ring, 3- to 10-membered monocyclic heterocycle, 4- to 7-membered monocyclic carbocycle, 2- to 6-membered heteroalkyl, alkynyl, -NH₂, -C₁₋₃ alkyl-NH₂, -O-3- to 10-membered monocyclic heterocycle, -C₁₋₃ alkoxy-3- to 10-membered monocyclic heterocycle, -NH-3- to 10-membered monocyclic heterocycle, or -C₁₋₃ alkyl-3- to 10-membered monocyclic heterocycle;
R₅, R₆, R₇, R₈, R₉, R₁₀, R₁₁, R₁₂, R₁₃, R₁₄, R₁₅, R₁₆, and R₁₇ are each independently optionally further substituted with one or more substituted or unsubstituted C₁₋₆ alkyl, 2- to 6-membered heteroalkyl, -C₁₋₃ alkyl-CO-NH₂, =O, halogen, 3- to 10-membered cycloalkyl, or - NH₂.

In one or more embodiments, the substituted R₅, R₆, R₇, R₈, R₉, R₁₀, R₁₁, R₁₂, R₁₃, R₁₄, R₁₅, R₁₆, or R₁₇ is further substituted with one or more substituents selected from the group consisting of 3- to 10-membered heterocycloalkyl, C₁₋₆ alkyl, halogen, and -N-(CH₃)₂.

One or more embodiments of the present disclosure provide a compound represented by general formula (I-7), or all stereoisomers, pharmaceutically acceptable salts, or deuterated derivatives thereof: 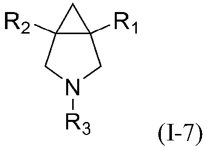 wherein:
R₂ is C₁₋₆ alkyl or C₃₋₁₀ cycloalkyl, wherein the C₁₋₆ alkyl or C₃₋₁₀ cycloalkyl is optionally further substituted with one or more halogens;
R₃ is 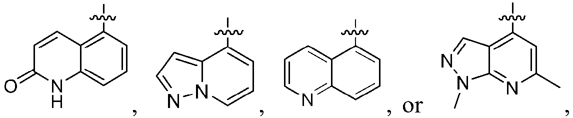 and R₃ is optionally further substituted with one or more substituents selected from the group consisting of D, C₁₋₃ alkyl, halogen, -C₁₋₃ alkoxy, -O-C₁₋₃ haloalkyl, cyano, and -NO₂;
R₁ is 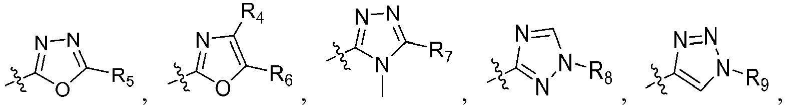 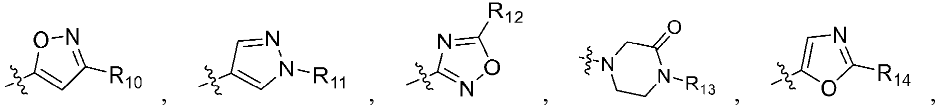 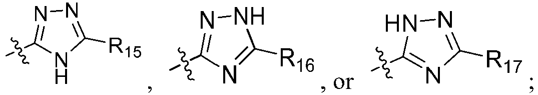
and when R₃ is 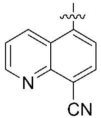 and R₁ is 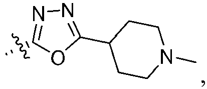 then R₂ is not -CF₃;
R₄ is H, C₁₋₆ alkyl, C₃₋₁₀ cycloalkyl, or halogen;
R₅, R₆, R₇, R₈, R₉, R₁₀, R₁₁, R₁₂, R₁₃, R₁₄, R₁₅, R₁₆, and R₁₇ are each independently H, C₁₋₆ alkyl, halogen, 7- to 10-membered bridged or spiro ring, 3- to 10-membered monocyclic heterocycle, 4- to 7-membered monocyclic carbocycle, 2- to 6-membered heteroalkyl, alkynyl, -NH₂, -C₁₋₃ alkyl-NH₂, -O-3- to 10-membered monocyclic heterocycle, -C₁₋₃ alkoxy-3- to 10-membered monocyclic heterocycle, -NH-3- to 10-membered monocyclic heterocycle, or -C₁₋₃ alkyl-3- to 10-membered monocyclic heterocycle;
R₅, R₆, R₇, R₈, R₉, R₁₀, R₁₁, R₁₂, R₁₃, R₁₄, R₁₅, R₁₆, and R₁₇ are each independently optionally further substituted with one or more substituted or unsubstituted C₁₋₆ alkyl, 2- to 6-membered heteroalkyl, -C₁₋₃ alkyl-CO-NH₂, =O, halogen, 3- to 10-membered cycloalkyl, or - NH₂.

In one or more embodiments, the substituted R₅, R₆, R₇, R₈, R₉, R₁₀, R₁₁, R₁₂, R₁₃, R₁₄, R₁₅, R₁₆, or R₁₇ is further substituted with one or more substituents selected from the group consisting of 3- to 10-membered cycloalkyl, 3- to 10-membered heterocycloalkyl, C₁₋₆ alkyl, halogen, and -N-(CH₃)₂.

One or more embodiments of the present disclosure provide a compound represented by general formula (I-8), or all stereoisomers, pharmaceutically acceptable salts, or deuterated derivatives thereof: 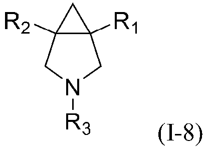 wherein:
R₂ is C₁₋₆ alkyl or C₃₋₁₀ cycloalkyl, wherein the C₁₋₆ alkyl or C₃₋₁₀ cycloalkyl is optionally further substituted with one or more halogens;
R₃ is 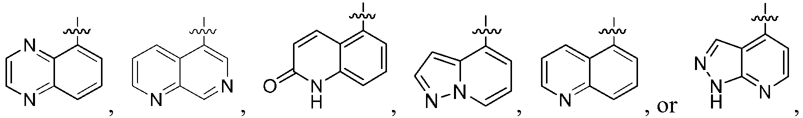 and R₃ is optionally further substituted with one or more substituents selected from the group consisting of C₁₋₃ haloalkyl, D, C₁₋₃ alkyl, halogen, -C₁₋₃ alkoxy, -O-C₁₋₃ haloalkyl, cyano, and -NO₂;
R₁ is 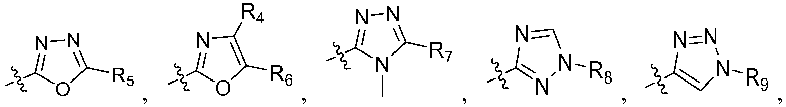 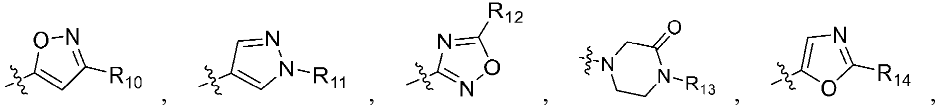 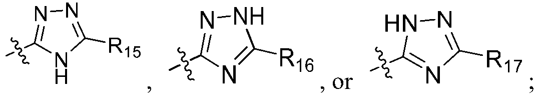
and when R₃ is 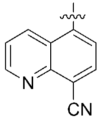 and R₁ is 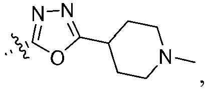 then R₂ is not -CF₃;
R₄ is H, C₁₋₆ alkyl, C₃₋₁₀ cycloalkyl, or halogen;
R₅, R₆, R₇, R₈, R₉, R₁₀, R₁₁, R₁₂, R₁₃, R₁₄, R₁₅, R₁₆, and R₁₇ are each independently H, C₁₋₆ alkyl, halogen, 7- to 10-membered bridged or spiro ring, 3- to 10-membered monocyclic heterocycle, 4- to 7-membered monocyclic carbocycle, 2- to 6-membered heteroalkyl, alkynyl, -NH₂, -C₁₋₃ alkyl-NH₂, -O-3- to 10-membered monocyclic heterocycle, -C₁₋₃ alkoxy-3- to 10-membered monocyclic heterocycle, -NH-3- to 10-membered monocyclic heterocycle, or -C₁₋₃ alkyl-3- to 10-membered monocyclic heterocycle;
R₅, R₆, R₇, R₈, R₉, R₁₀, R₁₁, R₁₂, R₁₃, R₁₄, R₁₅, R₁₆, and R₁₇ are each independently optionally further substituted with one or more substituted or unsubstituted -NH-CHO, C₁₋₆ alkyl, 2- to 6-membered heteroalkyl, -C₁₋₃ alkyl-CO-NH₂, =O, halogen, 3- to 10-membered cycloalkyl, or -NH₂.

In one or more embodiments, the substituted R₅, R₆, R₇, R₈, R₉, R₁₀, R₁₁, R₁₂, R₁₃, R₁₄, R₁₅, R₁₆, or R₁₇ is further substituted with one or more substituents selected from the group consisting of 3- to 10-membered cycloalkyl, 3- to 10-membered heterocycloalkyl, C₁₋₆ alkyl, halogen, and -N-(CH₃)₂.

One or more embodiments of the present disclosure provide a compound represented by general formula (I-9), or all stereoisomers, pharmaceutically acceptable salts, or deuterated derivatives thereof: 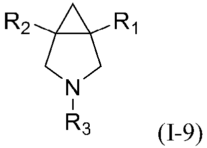 wherein:
R₂ is C₁₋₆ alkyl or C₃₋₁₀ cycloalkyl, wherein the C₁₋₆ alkyl or C₃₋₁₀ cycloalkyl is optionally further substituted with one or more halogens;
R₃ is 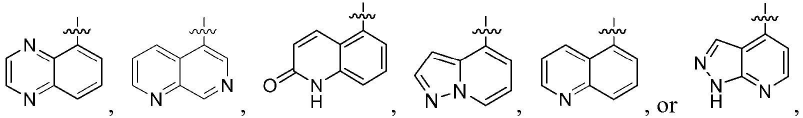 and R₃ is optionally further substituted with one or more substituents selected from the group consisting of C₁₋₃ haloalkyl, D, C₁₋₃ alkyl, halogen, -C₁₋₃ alkoxy, -O-C₁₋₃ haloalkyl, cyano, and -NO₂;
R₁ is 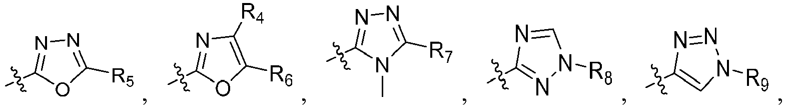 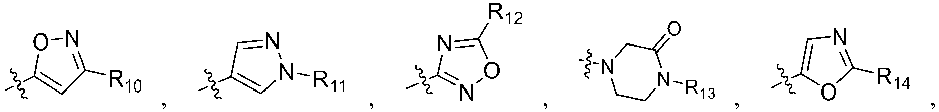 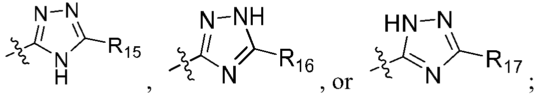
and when R₃ is 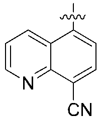 and R₁ is 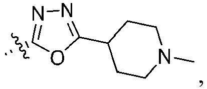 then R₂ is not -CF₃;
R₄ is H, C₁₋₆ alkyl, C₃₋₁₀ cycloalkyl, or halogen;
R₅, R₆, R₇, R₈, R₉, R₁₀, R₁₁, R₁₂, R₁₃, R₁₄, R₁₅, R₁₆, and R₁₇ are each independently H, C₁₋₆ alkyl, halogen, 7- to 10-membered bridged or spiro ring, 3- to 10-membered monocyclic heterocycle, 4- to 7-membered monocyclic carbocycle, 2- to 6-membered heteroalkyl, alkynyl, -NH₂, -C₁₋₃ alkyl-NH₂, -O-3- to 10-membered monocyclic heterocycle, -C₁₋₃ alkoxy-3- to 10-membered monocyclic heterocycle, -NH-3- to 10-membered monocyclic heterocycle, or -C₁₋₃ alkyl-3- to 10-membered monocyclic heterocycle;
R₅, R₆, R₇, R₈, R₉, R₁₀, R₁₁, R₁₂, R₁₃, R₁₄, R₁₅, R₁₆, and R₁₇ are each independently optionally further substituted with one or more substituted or unsubstituted -NH-CHO, C₁₋₆ alkyl, 2- to 6-membered heteroalkyl, -C₁₋₃ alkyl-CO-NH₂, =O, halogen, 3- to 10-membered cycloalkyl, or -NH₂.

In one or more embodiments, the substituted R₅, R₆, R₇, R₈, R₉, R₁₀, R₁₁, R₁₂, R₁₃, R₁₄, R₁₅, R₁₆, or R₁₇ is further substituted with one or more substituents selected from the group consisting of 3- to 10-membered cycloalkyl, 3- to 10-membered heterocycloalkyl, C₁₋₆ alkyl, halogen, and -N-(CH₃)₂.

One or more embodiments of the present disclosure provide a compound represented by general formula (I-10), or all stereoisomers, pharmaceutically acceptable salts, or deuterated derivatives thereof: 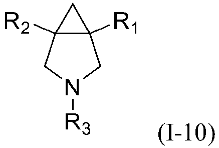 wherein:
R₂ is C₁₋₆ alkyl or C₃₋₁₀ cycloalkyl, wherein the C₁₋₆ alkyl or C₃₋₁₀ cycloalkyl is optionally further substituted with one or more halogens;
R₃ is 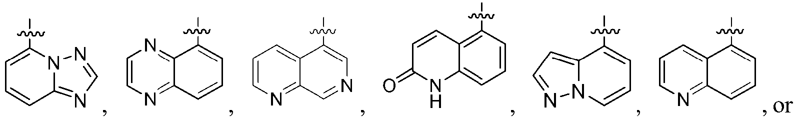 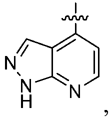 and R₃ is optionally further substituted with one or more substituents selected from the group consisting of C₁₋₃ haloalkyl, D, C₁₋₃ alkyl, halogen, -C₁₋₃ alkoxy, -O-C₁₋₃ haloalkyl, cyano, and -NO₂;
R₁ is 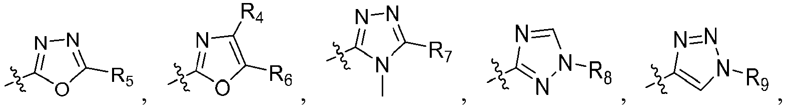 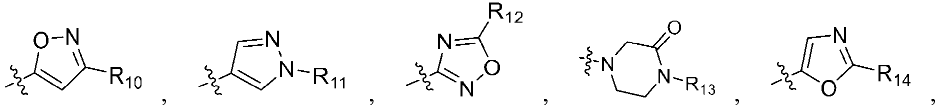 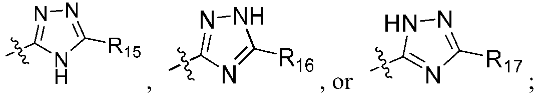
and when R₃ is 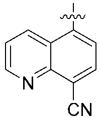 and R₁ is 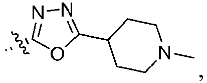 then R₂ is not -CF₃;
R₄ is H, C₁₋₆ alkyl, C₃₋₁₀ cycloalkyl, or halogen;
R₅, R₆, R₇, R₈, R₉, R₁₀, R₁₁, R₁₂, R₁₃, R₁₄, R₁₅, R₁₆, and R₁₇ are each independently H, C₁₋₆ alkyl, halogen, 7- to 10-membered bridged or spiro ring, 3- to 10-membered monocyclic heterocycle, 4- to 7-membered monocyclic carbocycle, 2- to 6-membered heteroalkyl, alkynyl, -NH₂, -C₁₋₃ alkyl-NH₂, -O-3- to 10-membered monocyclic heterocycle, -C₁₋₃ alkoxy-3- to 10-membered monocyclic heterocycle, -NH-3- to 10-membered monocyclic heterocycle, or -C₁₋₃ alkyl-3- to 10-membered monocyclic heterocycle;
R₅, R₆, R₇, R₈, R₉, R₁₀, R₁₁, R₁₂, R₁₃, R₁₄, R₁₅, R₁₆, and R₁₇ are each independently optionally further substituted with one or more substituted or unsubstituted -NH-CHO, C₁₋₆ alkyl, 2- to 6-membered heteroalkyl, -C₁₋₃ alkyl-CO-NH₂, =O, halogen, 3- to 10-membered cycloalkyl, hydroxyl, or -NH₂.

In one or more embodiments, the substituted R₅, R₆, R₇, R₈, R₉, R₁₀, R₁₁, R₁₂, R₁₃, R₁₄, R₁₅, R₁₆, or R₁₇ is further substituted with one or more substituents selected from the group consisting of 3- to 10-membered cycloalkyl, 3- to 10-membered heterocycloalkyl, C₁₋₆ alkyl, halogen, and -N-(CH₃)₂.

One or more embodiments of the present disclosure provide a compound represented by general formula (II), or all stereoisomers, pharmaceutically acceptable salts, or deuterated derivatives thereof: 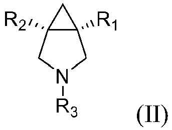 wherein:
R₂ is C₁₋₆ alkyl, wherein the alkyl is optionally further substituted with one or more halogens;
R₃ is 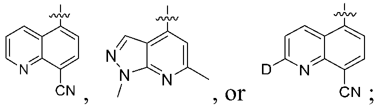
R₁ is 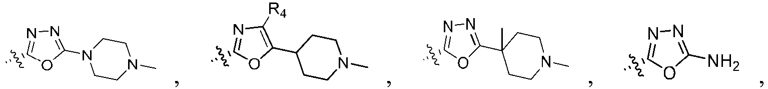 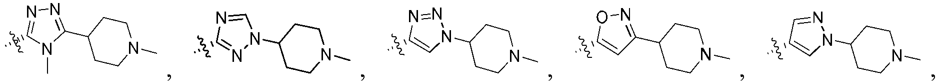 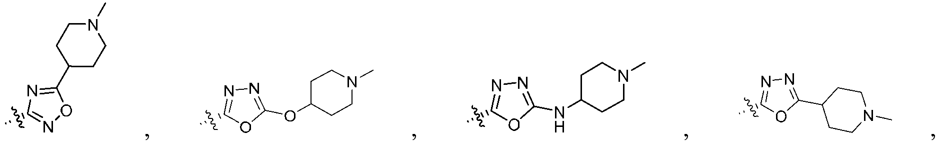 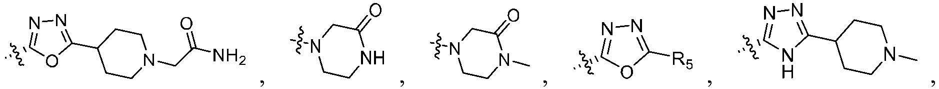 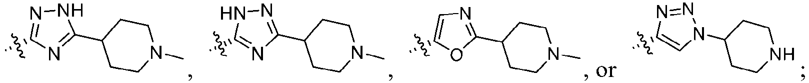
and when R₃ is 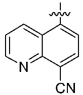 and R₁ is 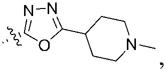 then R₂ is not -CF₃;
R₄ is C₁₋₆ alkyl or halogen;
R₅ is 7- to 10-membered bridged or spiro ring, and R₅ contains at least one N.
R₅ is: 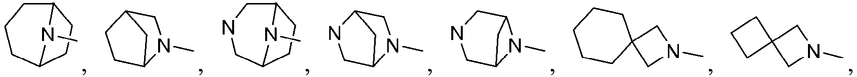 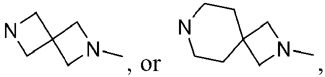 and the connection site of R₅ is at any connectable position thereon.

One or more embodiments of the present disclosure provide a pharmaceutical composition, comprising:
any one of the aforementioned compounds, or stereoisomers, pharmaceutically acceptable salts, or deuterated derivatives thereof;
optionally one or more other active ingredients; and
a pharmaceutically acceptable carrier and/or excipient.

One or more embodiments of the present disclosure provide a use of the compound, or the stereoisomer, pharmaceutically acceptable salt, or deuterated derivative thereof of the present disclosure, or the aforementioned pharmaceutical composition of the present disclosure, in the manufacture of a medicament for treating and/or preventing an autoimmune disease.

One or more embodiments of the present disclosure provide the compound, or the stereoisomer, pharmaceutically acceptable salt, or deuterated derivative thereof of the present disclosure, or the aforementioned pharmaceutical composition of the present disclosure, for use as a medicament.

One or more embodiments of the present disclosure provide the compound, or the stereoisomer, pharmaceutically acceptable salt, or deuterated derivative thereof of the present disclosure, or the aforementioned pharmaceutical composition of the present disclosure, for use in treating and/or preventing an autoimmune disease.

One or more embodiments of the present disclosure provide a method of treating and/or preventing an autoimmune disease, comprising administering to a subject in need thereof a therapeutically and/or prophylactically effective amount of the compound, or the stereoisomer, pharmaceutically acceptable salt, or deuterated derivative thereof of the present disclosure, or the aforementioned pharmaceutical composition of the present disclosure.

Unless stated otherwise, the terms used in the specification and claims have the following meanings.

The carbon, hydrogen, oxygen, sulfur, nitrogen, or F, Cl, Br, I involved in the groups and compounds of the present disclosure include their isotopic forms, and the carbon, hydrogen, oxygen, sulfur, or nitrogen in the groups and compounds of the present disclosure may optionally be further replaced by one or more of their corresponding isotopes, wherein the isotopes of carbon include ¹²C, ¹³C, and ¹⁴C; the isotopes of hydrogen include protium (H), deuterium (D, also called heavy hydrogen), and tritium (T, also called superheavy hydrogen); the isotopes of oxygen include ¹⁶O, ¹⁷O, and ¹⁸O; the isotopes of sulfur include ³²S, ³³S, ³⁴S, and ³⁶S; the isotopes of nitrogen include ¹⁴N and ¹⁵N; the isotopes of fluorine include ¹⁷F and ¹⁹F; the isotopes of chlorine include ³⁵Cl and ³⁷Cl; and the isotopes of bromine include ⁷⁹Br and ⁸¹Br.

"Alkyl" refers to a straight or branched saturated aliphatic hydrocarbon group having 1 to 20 carbon atoms, preferably an alkyl group having 1 to 8 (for example, 1, 2, 3, 4, 5, 6, 7, or 8) carbon atoms, more preferably an alkyl group having 1 to 6 carbon atoms, and further preferably an alkyl group having 1 to 4 carbon atoms. Non-limiting examples include methyl, ethyl, *n*-propyl, isopropyl, *n*-butyl, *sec*-butyl, neobutyl, *tert*-butyl, *n*-pentyl, isopentyl, neopentyl, *n*-hexyl, and various branched isomers thereof; when the alkyl is substituted, it may optionally be further substituted with one or more substituents. "Alkoxy" refers to a group formed by substituting at least one carbon atom in an alkyl group with an oxygen atom. Non-limiting examples include methoxy, ethoxy, *n*-propoxy, isopropoxy, *n*-butoxy, *sec*-butoxy, *tert*-butoxy, *n-*pentoxy, *n*-hexoxy, cyclopropoxy, and cyclobutoxy. The definition of the alkyl is the same as the definition of "alkyl" described above.

"Alkenyl" refers to a straight or branched unsaturated aliphatic hydrocarbon group having 2 to 20 carbon atoms and containing 1 to 10 (for example, 1, 2, 3, 4, 5, 6, 7, 8, 9, or 10) carbon-carbon double bonds, preferably an alkenyl group having 2 to 12 (for example, 2, 3, 4, 5, 6, 7, 8, 9, 10, 11, or 12) carbon atoms, more preferably an alkenyl group having 2 to 8 carbon atoms, and further preferably an alkenyl group having 2 to 6 carbon atoms. Non-limiting examples include vinyl, prop-2-enyl, but-2-enyl, but-2-enyl, pent-2-enyl, pent-4-enyl, hex-2-enyl, hex-3-enyl, hept-2-enyl, hept-3-enyl, hept-4-enyl, oct-3-enyl, non-3-enyl, dec-4-enyl, and undec-3-enyl. The alkenyl group may optionally be further substituted with one or more substituents.

"Alkynyl" refers to a straight or branched unsaturated aliphatic hydrocarbon group containing 1 to 10 (for example, 1, 2, 3, 4, 5, 6, 7, 8, 9, or 10) carbon-carbon triple bonds and consisting of 2 to 20 carbon atoms, preferably an alkynyl group having 2 to 12 (for example, 2, 3, 4, 5, 6, 7, 8, 9, 10, 11, or 12) carbon atoms, more preferably an alkynyl group having 2 to 8 carbon atoms, and further preferably an alkynyl group having 2 to 6 carbon atoms. Non-limiting examples include ethynyl, propyn-1-yl, propyn-2-yl, butyn-1-yl, butyn-2-yl, butyn-3-yl, 3,3-dimethylbutyn-2-yl, pentyn-1-yl, pentyn-2-yl, hexyn-1-yl, 1-heptyn-1-yl, heptyn-3-yl, heptyn-4-yl, octyn-3-yl, nonyn-3-yl, decyn-4-yl, undecyn-3-yl, and dodecyn-4-yl. The alkynyl group may optionally be further substituted with one or more substituents.

"Aryl" refers to a substituted or unsubstituted aromatic ring, which may be a 5- to 8-membered (for example, 5, 6, 7, or 8-membered) monocyclic, a 5- to 12-membered (for example, 5, 6, 7, 8, 9, 10, 11, or 12-membered) bicyclic, or a 10- to 15-membered (for example, 10, 11, 12, 13, 14, or 15-membered) tricyclic system, which may be a bridged ring or a spiro ring, non-limiting examples including phenyl and naphthyl. The aryl group may optionally be further substituted with one or more substituents.

"Heteroaryl" refers to a substituted or unsubstituted aromatic ring, which may be a 3-to 8-membered (for example, 3, 4, 5, 6, 7, or 8-membered) monocyclic, a 5- to 12-membered (for example, 5, 6, 7, 8, 9, 10, 11, or 12-membered) bicyclic, or a 10- to 15-membered (for example, 10, 11, 12, 13, 14, or 15-membered) tricyclic system, and contains 1 to 6 (for example, 1, 2, 3, 4, 5, or 6) heteroatoms selected from the group consisting of N, O, and S, preferably a 5- to 8-membered heteroaryl, wherein 1 to 4 (for example, 1, 2, 3, or 4) selectively substituted N or S in the heteroaryl ring may be oxidized to various oxidation states. The heteroaryl may be attached via a heteroatom or a carbon atom, and the heteroaryl may be a bridged ring or a spiro ring. Non-limiting examples include pyridinyl, furanyl, thienyl, pyranyl, pyrrolyl, pyrimidinyl, pyrazinyl, pyridazinyl, imidazolyl, piperidinyl, benzimidazolyl, benzopyridinyl, and pyrrolopyridinyl. The heteroaryl group is optionally further substituted with one or more substituents.

"Carbocyclyl" or "carbocycle" refers to a saturated or unsaturated aromatic ring or non-aromatic ring. When it is an aromatic ring, its definition is the same as that of "aryl" above; when it is a non-aromatic ring, it may be a 3- to 10-membered (for example, 3, 4, 5, 6, 7, 8, 9, or 10-membered) monocyclic, 4- to 12-membered (for example, 4, 5, 6, 7, 8, 9, 10, 11, or 12-membered) bicyclic, or 10- to 15-membered (for example, 10, 11, 12, 13, 14, or 15-membered) tricyclic system, which may be a bridged ring or a spiro ring. Non-limiting examples include cyclopropyl, cyclobutyl, cyclopentyl, 1-cyclopenten-1-yl, 1-cyclopenten-2-yl, 1-cyclopenten-3-yl, cyclohexyl, 1-cyclohexen-2-yl, 1-cyclohexen-3-yl, cyclohexenyl, cyclohexadienyl, cycloheptyl, cyclooctyl, cyclononyl, cyclodecyl, cycloundecyl, cyclododecyl, 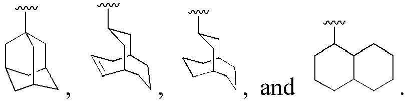 The "carbocyclyl" or "carbocycle" is optionally further substituted with one or more substituents.

"Heterocyclyl" or "heterocycle" refers to a saturated or unsaturated aromatic heterocycle or non-aromatic heterocycle. When it is an aromatic heterocycle, its definition is the same as that of "heteroaryl" above; when it is a non-aromatic heterocycle, it may be a 3- to 10-membered (for example, 3, 4, 5, 6, 7, 8, 9, or 10-membered) monocyclic, 4- to 12-membered (for example, 4, 5, 6, 7, 8, 9, 10, 11, or 12-membered) bicyclic, or 10- to 15-membered (for example, 10, 11, 12, 13, 14, or 15-membered) tricyclic system, and contains 1 to 4 (for example, 1, 2, 3, or 4) heteroatoms selected from the group consisting of N, O, and S, preferably a 3- to 8-membered heterocyclyl group. The ring of "heterocyclyl" or "heterocycle" selectively substituted with 1 to 4 (for example, 1, 2, 3, or 4) N or S can be oxidized to various oxidation states; "heterocyclyl" or "heterocycle" can be attached to a heteroatom or a carbon atom; "heterocyclyl" or "heterocycle" can be a bridged ring or a spiro ring. Non-limiting examples of "heterocyclyl" or "heterocycle" include oxiranyl, oxetanyl, azetidinyl, oxetanyl, azetidinyl, thietanyl, 1,3-dioxolanyl, 1,4-dioxolanyl, 1,3-dioxanyl, azepanyl, oxepanyl, thiepanyl, oxazepinyl, diazepinyl, thiazepinyl, pyridinyl, piperidinyl, homopiperidinyl, furanyl, thienyl, pyranyl, *N*-alkylpyrrolyl, pyrimidinyl, pyrazinyl, pyridazinyl, piperazinyl, homopiperazinyl, imidazolyl, piperidinyl, morpholinyl, thiomorpholinyl, thioxanyl, 1,3-dithianyl, dihydrofuranyl, dithiolanyl, tetrahydrofuranyl, tetrahydrothienyl, tetrahydropyranyl, tetrahydrothiopyranyl, tetrahydropyrrolyl, tetrahydroimidazolyl, tetrahydrothiazolyl, tetrahydropyranyl, benzimidazolyl, benzopyridinyl, pyrrolopyridinyl, benzodihydrofuranyl, 2-pyrrolinyl, 3-pyrrolinyl, dihydroindolyl, 2*H*-pyranyl, 4*H*-pyranyl, dioxanyl, 1,3-dioxolanyl, pyrazolinyl, dithianyl, dithiolanyl, dihydrothienyl, pyrazolidinyl, imidazolinyl, imidazolidinyl, 1,2,3,4-tetrahydroisoquinolinyl, 3-azabicyclo[3.1.0]hexyl, 3-azabicyclo[4.1.0]heptyl, azabicyclo[2.2.2]hexyl, 3*H*-indolylquinolizinyl, *N*-pyridinylurea, 1,1-dioxothiomorpholinyl, azabicyclo[3.2.1]octanyl, azabicyclo[5.2.0]nonanyl, oxatricyclo[5.3.1.1]dodecanyl, azadamantanyl, and oxaspiro[3.3]heptanyl. The "heterocyclyl" or "heterocycle" may optionally be further substituted with one or more substituents.

"Spiro heterocycle" and "bridged heterocycle" respectively refer to structures in which the C atom on a spiro ring or a bridged ring is replaced by a heteroatom (for example, N, O, or S).

"Cycloalkyl" refers to a saturated cyclic hydrocarbon group, the ring of which may be a 3- to 10-membered (for example, 3, 4, 5, 6, 7, 8, 9, or 10-membered) monocyclic, 4- to 12-membered (for example, 4, 5, 6, 7, 8, 9, 10, 11, or 12-membered) bicyclic, or 10- to 20-membered (for example, 10, 11, 12, 13, 14, 15, 16, 17, 18, 19, or 20-membered) polycyclic system (the polycyclic system may, for example, include a bridged ring or a spiro ring), preferably having 3 to 10 ring carbon atoms, and further preferably having 3 to 8 carbon atoms. Non-limiting examples of "cycloalkyl" include cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, 1,5-cyclooctadienyl, 1,4-cyclohexadienyl, and cycloheptatrienyl. When the cycloalkyl is substituted, it may optionally be further substituted with one or more substituents.

"Heterocycloalkyl" refers to substituted or unsubstituted saturated non-aromatic cyclic groups, which may be a 3- to 8-membered (for example, 3, 4, 5, 6, 7, or 8-membered) monocyclic, 4- to 12-membered (for example, 4, 5, 6, 7, 8, 9, 10, 11, or 12-membered) bicyclic, or 10- to 15-membered (for example, 10, 11, 12, 13, 14, or 15-membered) tricyclic system, and contains 1, 2, or 3 heteroatoms selected from the group consisting of N, O, and S, preferably 3- to 8-membered heterocycloalkyl. The ring of "heterocycloalkyl" selectively substituted with 1, 2, or 3 N or S can be oxidized to various oxidation states; "heterocycloalkyl" can be attached to a heteroatom or a carbon atom; "heterocycloalkyl" can be a bridged ring or a spiro ring. Non-limiting examples of "heterocycloalkyl" include oxiranyl, aziridinyl, oxetanyl, azetidinyl, 1,3-dioxolanyl, 1,4-dioxolanyl, 1,3-dioxanyl, azepanyl, piperidinyl, piperidinyl, morpholinyl, thiomorpholinyl, 1,3-dithianyl, tetrahydrofuranyl, tetrahydropyrrolyl, tetrahydroimidazolyl, tetrahydrothiazolyl, tetrahydropyranyl, azabicyclo[3.2.1]octanyl, azabicyclo[5.2.0]nonanyl, oxatricyclo[5.3.1.1]dodecanyl, azadamantanyl, and oxaspiro[3.3]heptanyl.

When the aforementioned "alkyl", "alkoxy", "alkenyl", "alkynyl", "aryl", "heteroaryl", "carbocyclyl", "carbocycle", "heterocyclyl", "heterocycle", "cycloalkyl", "heterocycloalkyl", or "heterocyclyl" is substituted, it may be further substituted with 0, 1, 2, 3, 4, 5, 6, 7, 8, 9, or 10 substituents selected from the group consisting of F, Cl, Br, I, hydroxyl, mercapto, nitro, cyano, amino, C₁₋₆ alkylamino, =O, C₁₋₆ alkyl, C₁₋₆ alkoxy, C₂₋₆ alkenyl, C₂₋₆ alkynyl, -NR_{q4}R_{q5}, =NR_{q6}, -C(=O)OC₁₋₆ alkyl, -OC(=O)C₁₋₆ alkyl, -C(=O)NR_{q4}R_{q5}, C₃₋₈ cycloalkyl, C₃₋₈ heterocycloalkyl, C₆₋₁₀ aryl, C₅₋₁₀ heteroaryl, -C(=O)OC₆₋₁₀ aryl, -OC(=O)C₆₋₁₀ aryl, - OC(=O)C₅₋₁₀ heteroaryl, -C(=O)OC₅₋₁₀ heteroaryl, -OC(=O)C₃₋₈ heterocycloalkyl, -C(=O)OC₃₋₈ heterocycloalkyl, -OC(=O)C₃₋₈ cycloalkyl, -C(=O)OC₃₋₈ cycloalkyl, -NHC(=O)C₃₋₈ heterocycloalkyl, -NHC(=O)C₆₋₁₀ aryl, -NHC(=O)C₅₋₁₀ heteroaryl, -NHC(=O)C₃₋₈ cycloalkyl, -NHC(=O)C₃₋₈ heterocycloalkyl, -NHC(=O)C₂₋₆ alkenyl, and -NHC(=O)C₂₋₆ alkynyl, wherein the substituent C₁₋₆ alkyl, C₁₋₆ alkoxy, C₂₋₆ alkenyl, C₂₋₆ alkynyl, C₃₋₈ cycloalkyl, C₃₋₈ heterocycloalkyl, C₆₋₁₀ aryl, C₅₋₁₀ heteroaryl, -NHC(=O)C₆₋₁₀ aryl, -NHC(=O)C₅₋₁₀ heteroaryl, -NHC(=O)C₃₋₈ heterocycloalkyl, or -NHC(=O)C₃₋₈ cycloalkyl is optionally further substituted with 1 to 3 substituents selected from the group consisting of OH, F, Cl, Br, I, C₁₋₆ alkyl, C₁₋₆ alkoxy, -NR_{q4}R_{q5}, and =O; R_{q1} is C₁₋₆ alkyl, C₁₋₆ alkoxy, or C₆₋₁₀ aryl; R_{q2} and R_{q3} are H or C₁₋₆ alkyl; wherein R_{q4} and R_{q5} are H, C₁₋₆ alkyl, -NH(C=NR_{q1}NR_{q2}R_{q3}, -S(=O)₂NR_{q2}R_{q3}, - C(=O)R_{q1}, or -C(=O)NR_{q2}R_{q3}, wherein the C₁₋₆ alkyl is optionally further substituted with one or more substituents selected from the group consisting of OH, F, Cl, Br, I, C₁₋₆ alkyl, C₁₋₆ alkoxy, C₆₋₁₀ aryl, C₅₋₁₀ heteroaryl, C₃₋₈ cycloalkyl, and C₃₋₈ heterocycloalkyl; or R_{q4} and R_{q5} together with the N atom form a 3- to 8-membered heterocycle, which may contain one or more heteroatoms selected from the group consisting of N, O, and S.

"Pharmaceutically acceptable salt" or "pharmaceutically acceptable salt thereof" refers to a salt of the compound of the present disclosure that retains the biological effectiveness and properties of the free acid or free base and is obtained by reacting the free acid with a non-toxic inorganic or organic base, or the free base with a non-toxic inorganic or organic acid.

"Pharmaceutical composition" refers to a mixture comprising one or more of the compounds of the present disclosure, their pharmaceutically acceptable salts or prodrugs, and other chemical components, wherein "other chemical components" refer to pharmaceutically acceptable carriers, excipients, and/or one or more other therapeutic agents.

"Carrier" refers to a material that does not cause significant irritation to an organism and does not eliminate the biological activity and properties of the administered compound.

"Excipient" refers to an inert substance added to a pharmaceutical composition to facilitate the administration of the compound. Non-limiting examples include calcium carbonate, calcium phosphate, sugar, starch, cellulose derivatives (including microcrystalline cellulose), gelatin, vegetable oil, polyethylene glycols, diluents, granulating agents, lubricants, binders, and disintegrants.

"Prodrug" refers to a compound of the present disclosure that can be metabolized *in vivo* to a biologically active compound. The prodrug of the present disclosure is prepared by modifying the amino or carboxyl group in the compound of the present disclosure, and this modification can be removed by conventional operations or *in vivo* to obtain the parent compound. When the prodrug of the present disclosure is administered to a mammalian subject, the prodrug is cleaved to form a free amino or carboxyl group.

"Cocrystal" refers to a crystal formed by the combination of an active pharmaceutical ingredient (API) and a cocrystal former (CCF) under the action of hydrogen bonds or other non-covalent bonds, wherein the pure forms of both API and CCF are solids at room temperature, and there exists a fixed stoichiometric ratio between the components. A cocrystal is a multicomponent crystal, encompassing both binary cocrystals formed between two neutral solids and multicomponent cocrystals formed between a neutral solid and a salt or solvate.

"Stereoisomer" refers to an isomer resulting from different spatial arrangements of atoms in a molecule, including *cis-trans* isomers, enantiomers, and conformational isomers.

"Optional" or "optionally" or "selective" or "selectively" means that the subsequently described event or circumstance may or may not occur, and the description includes instances where the event or circumstance occurs and instances where it does not. For example, "heterocyclyl optionally substituted with alkyl" means that the alkyl may or may not be present, and the description includes instances where the heterocyclyl is substituted with alkyl and instances where the heterocyclyl is not substituted with alkyl.

### DETAILED DESCRIPTION OF THE PREFERRED EMBODIMENT

The following examples illustrate the technical solutions of the present disclosure in detail, but the scope of protection of the present disclosure includes but is not limited thereto.

### Intermediate A

ethyl 3-(8-cyanoquinolin-5-yl)-5-(trifluoromethyl)-3-azabicyclo[3.1.0]hexane-1-carboxylate
ethyl-3-(8-cyanoquinolin-5-yl)-5-(trifluoromethyl)-3-azabicyclo[3.1.0]hexane-1-carboxylate

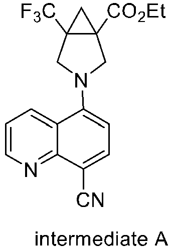

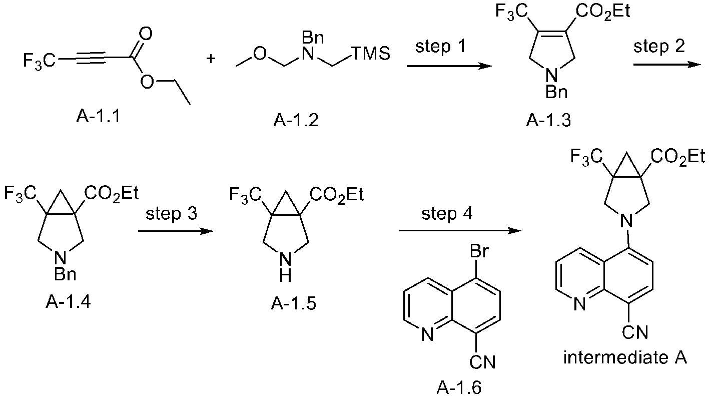

### Step 1:

ethyl 1-benzyl-4-(trifluoromethyl)-2,5-dihydro-1*H*-pyrrole-3-carboxylate A-1.3
ethyl-1-benzyl-4-(trifluoromethyl)-2,5-dihydro-1*H*-pyrrole-3-carboxylate

Under an N₂ atmosphere, A-1.1 (10 g, 60 mmol) was dissolved in 30 mL of dichloromethane (DCM). A solution ofA-1.2 (14.4 g, 60 mmol) in dichloromethane (10 mL) was added dropwise in an ice bath, and then a solution of trifluoroacetic acid (684 mg, 6 mmol) in dichloromethane (10 mL) was slowly added dropwise. The mixture was stirred at room temperature for 2 h. The reaction mixture was added to 30 mL of water and extracted three times with DCM. The organic phase was washed with 30 mL of saturated brine, dried over anhydrous sodium sulfate, and rotary evaporated to dryness. The residue was purified by silica gel column chromatography (ethyl acetate: petroleum ether = 1:10) to obtain the target product ethyl 1-benzyl-4-(trifluoromethyl)-2,5-dihydro-1*H*-pyrrole-3-carboxylate A-1.3 (yellow oil, 15.5 g, yield: 86%), which was directly used in the next step.

¹H NMR (400 MHz, DMSO-*d*₆) δ 7.34-7.24 (m, 5H), 4.19 (q, 2H), 3.79-3.78 (m, 6H), 1.20 (t, 3H).

LC-MS m/z (ESI)= 300.1 [M+1].

### Step 2:

ethyl 3-benzyl-5-(trifluoromethyl)-3-azabicyclo[3.1.0]hexane-1-carboxylate A-1.4
ethyl-3-benzyl-5-(trifluoromethyl)-3-azabicyclo[3.1.0]hexane-1-carboxylate

Under an N₂ atmosphere, trimethylsulfoxonium iodide (3.5 g, 15.8 mmol) was dissolved in 10 mL of dimethyl sulfoxide, and a solution of sodium hydride (633.6 mg, 15.8 mmol) in dimethyl sulfoxide (5 mL) was added portionwise in an ice bath. The mixture was stirred at room temperature for 30 minutes. Subsequently, a solution of A-1.3 (4.3 g, 14.4 mmol) in dimethyl sulfoxide (5 mL) was added dropwise, and the reaction was carried out at 60°C for 5 h. The reaction was quenched with saturated ammonium chloride and extracted with 30 mL of DCM. The organic phase was washed with 30 mL of saturated brine, dried over anhydrous sodium sulfate, and rotary evaporated to dryness. The residue was purified by silica gel column chromatography (ethyl acetate: petroleum ether = 1:10) to obtain the target product ethyl 3-benzyl-5-(trifluoromethyl)-3-azabicyclo[3.1.0]hexane-1-carboxylate A-1.4 (colorless oil, 3.5 g, yield: 78%), which was directly used in the next step.

¹H NMR (400 MHz, DMSO-*d*₆) δ 7.34-7.24 (m, 5 H), 4.11 (q, 2H), 3.66 (s, 2H), 3.08-3.01(m, 2H), 2.86-2.82 (m, 1H), 2.65-2.62 (m, 1H), 1.82-1.79 (m, 2H), 1.15 (t, 3H).

LC-MS m/z (ESI)= 314.1 [M+1].

### Step 3:

ethyl 5-(trifluoromethyl)-3-azabicyclo[3.1.0]hexane-1-carboxylate A-1.5
ethyl-5-(trifluoromethyl)-3-azabicyclo[3.1.0]hexane-1-carboxylate

A-1.4 (1 g, 3.2 mmol) was dissolved in 50 mL of ethanol, followed by the addition of Pd/C (681 mg, 0.64 mmol). The reaction system was purged twice with 1 atm H₂, heated to 60°C, and reacted for 3 h. The mixture was filtered through diatomite and rotary evaporated to remove the solvent until dryness to obtain the target product ethyl 5-(trifluoromethyl)-3-azabicyclo[3.1.0]hexane-1-carboxylate A-1.5 (colorless oil, 1 g, yield: 88%), which was directly used in the next step.

¹H NMR (400 MHz, DMSO-*d*₆) δ 4.11 (q, 2H), 3.20-2.75(m, 5H), 1.75 (d, 1H), 1.48-1.47 (m, 1H), 1.16 (t, 3H).

LC-MS m/z (ESI)= 224.1 [M+1].

### Step 4:

ethyl 3-(8-cyanoquinolin-5-yl)-5-(trifluoromethyl)-3-azabicyclo[3.1.0]hexane-1-carboxylate A
ethyl-3-(8-cyanoquinolin-5-yl)-5-(trifluoromethyl)-3-azabicyclo[3.1.0]hexane-1-carboxylate

Under an N₂ atmosphere, 5-bromoquinoline-8-carbonitrile A-1.6 (654 mg, 2.9 mmol) was dissolved in 30 mL of 1,4-dioxane, followed by the addition of A-1.5 (804 mg, 3.5 mmol). The reaction system was purged with N₂ three times. Subsequently, cesium carbonate (4.3 g, 13.05 mmol) and RuPhosPdG3 (486 mg, 0.58 mmol) were added sequentially, and the reaction system was purged with N₂ three times. The mixture was heated to 90°C and reacted for 2 h. The mixture was rotary evaporated to remove the solvent until dryness and added with ethyl acetate for extraction. The mixture was washed with saturated brine, dried over anhydrous sodium sulfate, filtered, and rotary evaporated to remove the solvent until dryness. The crude product A was directly used in the next step.

LC-MS m/z (ESI)= 376.1 [M+1], 398.1 [M+23].

### Intermediate B-2: (1R,5S)-3-(8-cyanoquinolin-5-yl)-5-(trifluoromethyl)-3-azabicyclo[3.1.0]hexane-1-carboxylic acid

### (1R,5S)-3-(8-cyanoquinolin-5-yl)-5-(trifluoromethyl)-3-azabicyclo[3.1.0]hexane-1-carboxylic acid

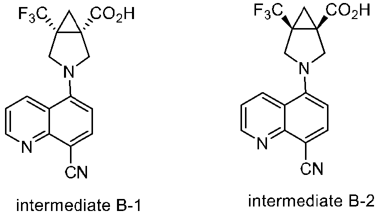

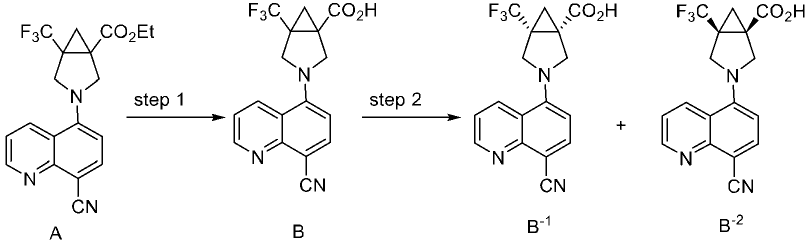

### Step 1:

3-(8-cyanoquinolin-5-yl)-5-(trifluoromethyl)-3-azabicyclo[3.1.0]hexane-1-carboxylic acid B
3-(8-cyanoquinolin-5-yl)-5-(trifluoromethyl)-3-azabicyclo[3.1.0]hexane-1-carboxylic acid

The crude product A (2.02 g, 5.39 mmol) was dissolved in 10 mL of tetrahydrofuran solution, and 10 mL of an aqueous solution of anhydrous lithium hydroxide (1.29 g, 53.9 mmol) was added dropwise to the reaction mixture, which was stirred overnight at room temperature. After the reaction was completed, the mixture was rotary evaporated to remove the tetrahydrofuran. The residue was extracted with ethyl acetate, and the aqueous phase was retained. The pH of the aqueous phase was adjusted to 3-4 with 2 M hydrochloric acid solution, followed by extraction with ethyl acetate. The organic phase was washed with saturated brine, dried over anhydrous sodium sulfate, and the solvent was removed under vacuum. The residue was purified by MPLC (acetonitrile: water = 47:53) to obtain the target product 3-(8-cyanoquinolin-5-yl)-5-(trifluoromethyl)-3-azabicyclo[3.1.0]hexane-1-carboxylic acid compound B (yellow solid, 387 mg, 21%).

¹H NMR (400 MHz, DMSO-*d*₆) δ 13.26 (s, 1H), 9.02-9.00 (m, 1H), 8.64-8.62 (m, 1H), 8.16 (d, *J* = 8.0 Hz, 1H), 7.61 (dd, 1H), 7.25 (d, 1H), 4.05-3.77 (m, 4H), 2.07-1.86 (m, 2H).

LC-MS m/z (ESI)= 348.1 [M+1], 370.1 [M+23].

### Step 6:

(1*S*,5*R*)-3-(8-cyanoquinolin-5-yl)-5-(trifluoromethyl)-3-azabicyclo[3.1.0]hexane-1-carboxylic acid
(1S,5R)-3-(8-cyanoquinolin-5-yl)-5-(trifluoromethyl)-3-azabicyclo[3.1.0]hexane-1-carboxylic acid intermediate B-1
(1*R*,5*S*)-3-(8-cyanoquinolin-5-yl)-5-(trifluoromethyl)-3-azabicyclo[3.1.0]hexane-1-carboxylic acid
(1R,5S)-3-(8-cyanoquinolin-5-yl)-5-(trifluoromethyl)-3-azabicyclo[3.1.0]hexane-1-carboxylic acid intermediate B-2

Compound B-1 and compound B-2 were resolved by chiral prep-HPLC. Analytical method: Chiral column Ig-3, methanol as the mobile phase, flow rate 1 mL/min. The retention time of intermediate B-1 was 3.619 min, and the retention time of intermediate B-2 was 4.741 min.

### Intermediate C

2-(1-methylpiperidin-4-yl)-5-((1*S*,5*R*)-5-(trifluoromethyl)-3-azabicyclo[3.1.0]hexan-1-yl)-1,3,4-oxadiazole intermediate C
2-(1-methylpiperidin-4-yl)-5-((1*S*,5*R*)-5-(trifluoromethyl)-3-azabicyclo[3.1.0]hexan-1-yl)-1,3,4-oxadiazole

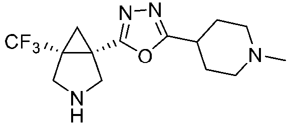

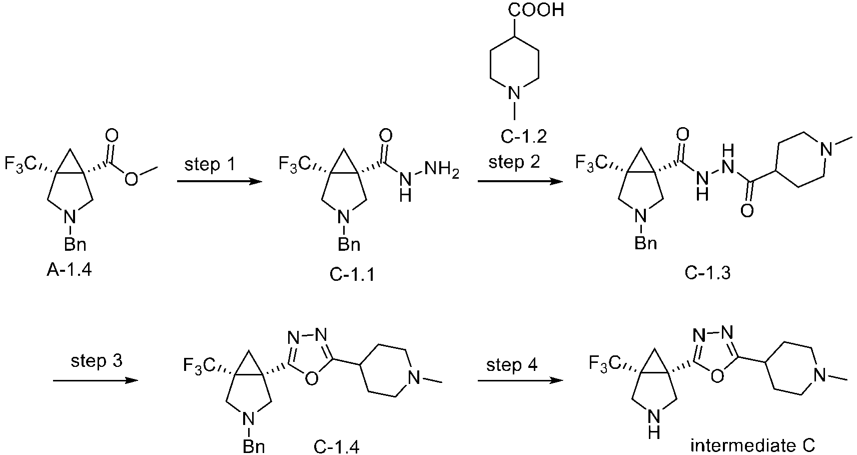

### Step 1:

(1*S*,5*R*)-3-benzyl-5-(trifluoromethyl)-3-azabicyclo[3.1.0]hexane-1-carbohydrazide C-1.1
(1*S*,5*R*)-3-benzyl-5-(trifluoromethyl)-3-azabicyclo[3.1.0]hexane-1-carbohydrazide
(1*S*,5*R*)-Compound A-1.4 (5.0 g, 0.0167 mol) was dissolved in 50 mL of ethanol, followed by the addition of hydrazine hydrate (8.36 g, 0.167 mol). The reaction was carried out at 70°C for 4 h. The mixture was rotary evaporated to remove the solvent until dryness to obtain the target product (1*S*,5*R*)-3-benzyl-5-(trifluoromethyl)-3-azabicyclo[3.1.0]hexane-1-carbohydrazide C-1.1, which was directly used in the next step.

LC-MS m/z (ESI)= 300.3 [M+1].

### Step 2:

(1*S*,5*R*)-3-benzyl-*N*'-(1-methylpiperidine-4-carbonyl)-5-(trifluoromethyl)-3-azabicyclo[3.1.0]hexane-1-carbohydrazide compound C-1.3
(1*S*,5*R*)-3-benzyl-*N*'-(1-methylpiperidine-4-carbonyl)-5-(trifluoromethyl)-3-azabicyclo[3.1.0]hexane-1-carbohydrazide

Compound 1-methylpiperidine-4-carboxylic acid C-1.2 (2.08 g, 0.0147 mol) was dissolved in 20 mL of dichloromethane.

At 0°C, thionyl chloride (4.96 mL, 0.067 mol) was slowly added, and the reaction system was then warmed to 40°C and stirred for 2 h. The mixture was concentrated to remove the solvent, and the residue was dissolved in 10 mL of dichloromethane. Compound (1*S*,5*R*)-3-benzyl-5-(trifluoromethyl)-3-azabicyclo[3.1.0]hexane-1-carbohydrazide compound C-1.1 (4.00 g, 0.0134 mol) was added, and the mixture was stirred at room temperature for 1 h. The reaction was monitored by TLC until completion. Water was added to quench the reaction, and the mixture was extracted with dichloromethane. The combined organic phases were washed with saturated brine, dried over anhydrous sodium sulfate, and concentrated under reduced pressure. The residue was purified by silica gel column chromatography to obtain the target product (1*S*,5*R*)-3-benzyl-*N*'-(1-methylpiperidine-4-carbonyl)-5-(trifluoromethyl)-3-azabicyclo[3.1.0]hexane-1-carbohydrazide C-1.3 (yellow liquid, 5.1 g, yield: 90.0%).

LC-MS m/z (ESI)= 425.2 [M+1].

### Step 3:

2-((1*S*,5*R*)-3-benzyl-5-(trifluoromethyl)-3-azabicyclo[3.1.0]hexan-1-yl)-5-(1-methylpiperidin-4-yl)-1,3,4-oxadiazole
2-((1*S*,5*R*)-3-benzyl-5-(trifluoromethyl)-3-azabicyclo[3.1.0]hexan-1-yl)-5-(1-methylpiperidin-4-yl)-1,3,4-oxadiazole compound C-1.4
(1*S*,5*R*)-3-Benzyl-*N*'-(1-methylpiperidine-4-carbonyl)-5-(trifluoromethyl)-3-azabicyclo[3.1.0]hexane-1-carbohydrazide compound C-1.3 (5.1 g, 0.012 mol) was dissolved in 20 mL of dichloromethane, followed by the addition of triethylamine (12.0 g, 0.12 mol) and *p*-toluenesulfonyl chloride (6.8 g, 0.036 mol). The mixture was stirred at room temperature for 5 hours, and the reaction was monitored by TLC until completion. The reaction mixture was concentrated and purified by silica gel column chromatography to obtain the target product 2-((1*S*,5*R*)-3-benzyl-5-(trifluoromethyl)-3-azabicyclo[3.1.0]hexan-1-yl)-5-(1-methylpiperidin-4-yl)-1,3,4-oxadiazole compound C-1.4 (yellow liquid, 4.2 g, yield: 85%).

LC-MS m/z (ESI)= 407.2 [M+1].

### Step 4:

2-(1-methylpiperidin-4-yl)-5-((1S,5R)-5-(trifluoromethyl)-3-azabicyclo[3.1.0]hexan-1-yl)-1,3,4-oxadiazole intermediate C
2-(1-methylpiperidin-4-yl)-5-((1S,5R)-5-(trifluoromethyl)-3-azabicyclo[3.1.0]hexan-1-yl)-1,3,4-oxadiazole
2-((1*S*,5*R*)-3-Benzyl-5-(trifluoromethyl)-3-azabicyclo[3.1.0]hexan-1-yl)-5-(1-methylpiperidin-4-yl)-1,3,4-oxadiazole compound C-1.4 (1.0 g, 2.5 mmol) was dissolved in 5 mL of methanol. Palladium hydroxide (35 mg, 0.25 mmol) and ammonium formate (464 mg, 3.69 mol) were sequentially added, and the mixture was heated under reflux for 6 h. The reaction was monitored by TLC until completion. The reaction mixture was concentrated and purified by silica gel column chromatography to obtain the target product 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (yellow liquid, 600 mg, yield: 76%).

LC-MS m/z (ESI)= 317.2 [M+1].

### Example 1

5-((1*S*,5*R*)-1-(5-(4-methylpiperazin-1-yl)-1,3,4-oxadiazol-2-yl)-5-(trifluoromethyl)-3-azabicyclo[3.1.0]hexan-3-yl)quinoline-5-carbonitrile compound 1
5-((1S,5R)-1-(5-(4-methylpiperazin-1-yl)-1,3,4-oxadiazol-2-yl)-5(trifluoromethyl)-3-azabicyclo[3.1.0]hexan-3-yl)quinoline-8-carbonitrile

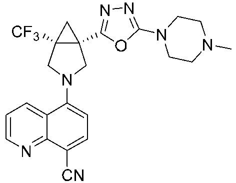

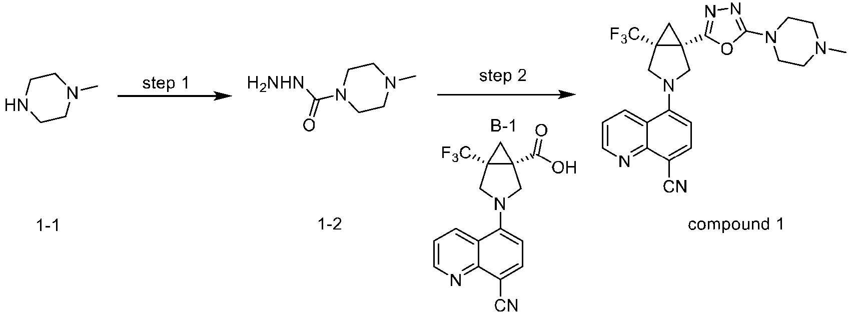

### Step 1:

4-methylpiperazine-1-carbohydrazide 1-2
4-methylpiperazine-1-carbohydrazide

Triphosgene (5.92 g, 20.0 mmol) was dissolved in 10.0 mL of dichloromethane. A mixed solution of methylpiperazine 1-1 (1.00 g, 10.0 mmol) and triethylamine (1.02 g, 10.0 mmol) in 10.0 mL of dichloromethane was slowly added dropwise at 0°C. After reacting for 1 h, hydrazine hydrate (1.28 g, 40.0 mmol) was slowly added dropwise. After reacting for another 1 h, the sample was submitted, and LCMS analysis confirmed the completion of the reaction. The mixture was concentrated to obtain 1.7 g of the crude product 4-methylpiperazine-1-carbohydrazide 1-2 (white solid, 1.72 g).

LC-MS m/z (ESI)= 159.10 [M+1].

### Step 2:

5-((1*S*,5*R*)-1-(5-(4-methylpiperazin-1-yl)-1,3,4-oxadiazol-2-yl)-5-(trifluoromethyl)-3-azabicyclo[3.1.0]hexan-3-yl)quinoline-5-carbonitrile compound 1
5-((1S,5R)-1-(5-(4-methylpiperazin-1-yl)-1,3,4-oxadiazol-2-yl)-5-(trifluoromethyl)-3-azabicyclo[3.1.0]hexan-3-yl)quinoline-8-carbonitrile

The crude product 4-methylpiperazine-1-carbohydrazide was dissolved with (1*S*,5*R*)-3-(8-cyanoquinolin-5-yl)-5-(trifluoromethyl)-3-azabicyclo[3.1.0]hexane-1-carboxylic acid B-1 (1.00 g, 2.87 mmol) in 10.0 mL of phosphorus oxychloride. The mixture was refluxed at 90°C for 3 hours, and then sampled for LCMS analysis. After the reaction was completed, the mixture was quenched by adjusting the pH to 7-8 with saturated sodium hydroxide solution, lyophilized, and added with dichloromethane. The organic phase was filtered, concentrated, and purified by reverse-phase preparative chromatography and then TLC (DCM: MeOH = 10:1) to obtain the target product 5-((1*S*,5*R*)-1-(5-(4-methylpiperazin-1-yl)-1,3,4-oxadiazol-2-yl)-5-(trifluoromethyl)-3-azabicyclo[3.1.0]hexan-3-yl)quinoline-5-carbonitrile compound 1 (pale red solid, 25 mg).

¹H NMR (400 MHz, Chloroform-d) δ 9.12 (dd, 1H), 8.39 (dd, 1H), 8.07 (d, 1H), 7.55 (dd, 1H), 7.17 (d, 1H), 4.04 (d, 1H), 4.02 - 3.91 (m, 2H), 3.82 (d, 1H), 3.59 (d, 4H), 2.57 (s, 4H), 2.40 (s, 3H), 2.26 (d, 1H), 2.17 (d, 1H).

LC-MS m/z (ESI)= 470.10 [M+1].

### Example 2

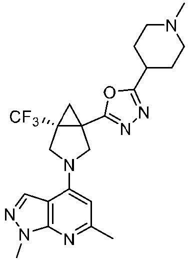

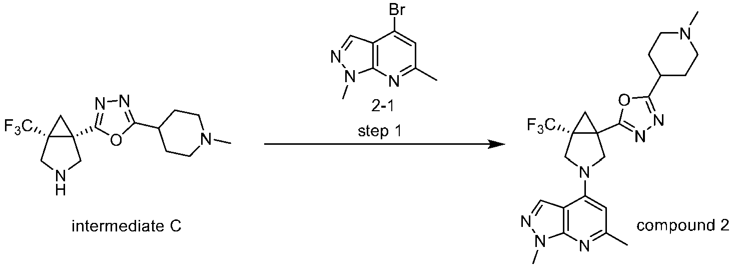

### Step 1:

2-((5*R*)-3-(1,6-dimethyl-1*H*-pyrazolo[3,4-b]pyridin-4-yl)-5-(trifluoromethyl)-3-azabicyclo[3.1.0]hexan-1-yl)-5-(1-methylpiperidin-4-yl)-1,3,4-oxadiazole compound 2
2-((5R)-3-(1,6-dimethyl-1H-pyrazolo[3,4-b]pyridin-4-yl)-5-(trifluoromethyl)-3-azabicyclo[3.1.0]hexan-1-yl)-5-(1-methylpiperidin-4-yl)-1,3,4-oxadiazole

Under an N₂ atmosphere, 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (200 mg, 0.63 mmol) was dissolved in 1,4-dioxane (3.00 mL). Subsequently, 4-bromo-1,6-dimethyl-1*H*-pyrazolo[3,4-*b*]pyridine compound 2-1 (150 mg, 0.67 mmol), cesium carbonate (1.40 g, 2.68 mmol), and RuPhosPdG3 (48.5 mg, 0.067 mmol) were added sequentially. The reaction system was purged with nitrogen three times, and the mixture was heated to 90°C and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The residue was purified by TLC (DCM: MeOH = 10:1) to obtain the target product 2-((5R)-3-(1,6-dimethyl-1*H*-pyrazolo[3,4-*b*]pyridin-4-yl)-5-(trifluoromethyl)-3-azabicyclo[3.1.0]hexan-1-yl)-5-(1-methylpiperidin-4-yl)-1,3,4-oxadiazole compound 2 (yellowish-white solid, 10 mg).

LC-MS m/z (ESI)= 462.10 [M+1].

¹H NMR (400 MHz, DMSO-*d*₆) δ 8.10 (s, 1H), 6.16 (s, 1H), 4.46 - 3.98 (m, 4H), 3.89 (s, 3H), 2.94 (td, 1H), 2.73 (d, *J* = 11.3 Hz, 2H), 2.43 (s, 3H), 2.34 (d, 1H), 2.17 (s, 3H), 2.06 (d, 1H), 2.00 - 1.92 (m, 3H), 1.87 (d, 1H), 1.79 - 1.67 (m, 2H).

### Example 3

2-(4-fluoropiperidin-4-yl)-5-((1S,5R)-3-(8-(trifluoromethoxy)quinolin-5-yl)-5-(trifluoromethyl)-3-azabicyclo[3.1.0]hexan-1-yl)-1,3,4-oxadiazole compound 3
2-(4-fluoropiperidin-4-yl)-5-((1S,5R)-3-(8-(trifluoromethoxy)quinolin-5-yl)-5-(trifluoromethyl)-3-azabicyclo[3.1.0]hexan-1-yl)-1,3,4-oxadiazole

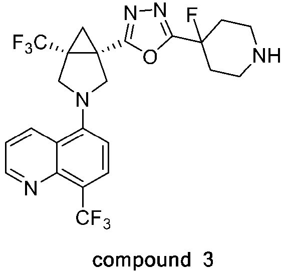

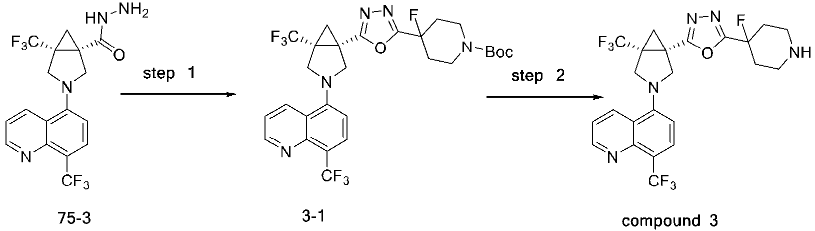

### Step 1:

tert-butyl 4-fluoro-4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)piperidine-1-carboxylate compound 3-1
4-fluoro-4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)piperidine-1-carboxylate
1-(*tert*-Butoxycarbonyl)-4-fluoropiperidine-4-carboxylic acid (230 mg, 0.93 mmol) was dissolved in anhydrous dichloromethane (7.5 mL). DIPEA (230 mg, 1.77 mmol) and HATU (506 mg, 1.33 mmol) were added, and the mixture was stirred at 35°C for 30 minutes and then cooled to room temperature. (1*S*,5*R*)-3-(8-(Trifluoromethoxy)quinolin-5-yl)-5-(trifluoromethyl)-3-azabicyclo[3.1.0]hexane-1-carbohydrazide (373 mg, 0.88 mmol) was added. After completion, the reaction mixture was purged with nitrogen three times and stirred at room temperature for 1 hour. After LCMS confirmed the completion of the reaction, DIPEA (344 mg, 2.66 mmol) and Burgess reagent (634 mg, 2.66 mmol) were added. The reaction mixture was purged with nitrogen three times and stirred at room temperature for 1 hour. After LCMS confirmed the completion of the reaction, the mixture was extracted three times with ethyl acetate and water. The organic phase was dried over anhydrous sodium sulfate and directly concentrated to dryness. The crude product was purified by reverse-phase column chromatography to obtain *tert*-butyl 4-fluoro-4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidine-1-carboxylate compound 3-1 (300 mg, yellow solid, yield: 52%).

LCMS m/z (ESI) = 616.21 [M+1].

### Step 2:

2-(4-fluoropiperidin-4-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 3
2-(4-fluoropiperidin-4-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole
*tert*-Butyl 4-fluoro-4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidine-1-carboxylate 3-1 (300 mg, 0.48 mmol) was dissolved in anhydrous dichloromethane (6 mL), followed by the dropwise addition of trifluoroacetic acid (1.5 mL). The reaction mixture was purged with nitrogen and stirred at room temperature for 2 hours. After the reaction was completed, ammonia water was added to adjust the pH to alkaline. The mixture was extracted three times with dichloromethane and water. The organic phase was dried over anhydrous sodium sulfate, concentrated to dryness, and purified by column chromatography to obtain the target product 2-(4-fluoropiperidin-4-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 3 (130 mg, yellow solid, yield: 51%).

LCMS m/z (ESI) = 516.21 [M+1].

¹H NMR (400 MHz, Chloroform-*d*) *δ* 9.07 (dd, , 1H), 8.36 (dd, 1H), 8.00 (d, 1H), 7.51 (dd, 1H), 7.21 (d, 1H), 4.08 (d, 1H), 3.97 - 3.86 (m, 2H), 3.74 (d ,1H), 3.19 (q, 3H), 3.15 - 3.04 (m, 4H), 2.35 (m, 3H).

### Example 4

2-(1-methylazetidin-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 4
2-(1-methylazetidin-3-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole

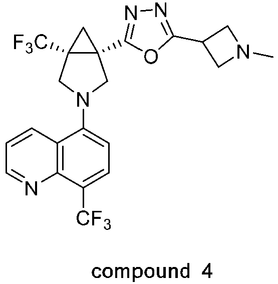

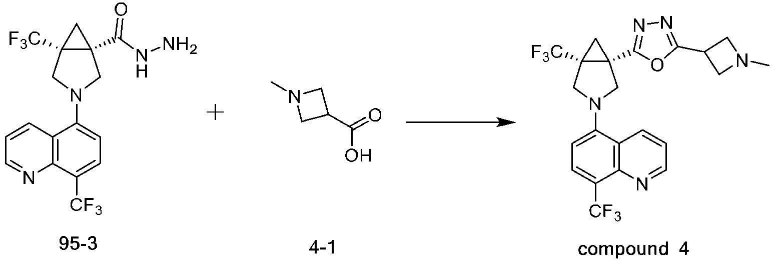

### Step 1:

2-(1-methylazetidin-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazolecompound 4
2-(1-methylazetidin-3-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole
1-(*tert*-Butoxycarbonyl)azetidine-3-carboxylic acid 4-1 (111 mg, 0.75 mmol) was dissolved in 20 mL of phosphorus oxychloride, followed by the addition of (1*S*,5*R*)-5-(trifluoromethyl)-3-(8-(trifluoromethyl)quinolin-5-yl)-3-azabicyclo[3.1.0]hexane-1-carbohydrazide 95-3 (300 mg, 0.75 mmol). The mixture was stirred at 80°C for 2 hours until LCMS indicated completion of the reaction. The reaction mixture was poured into an aqueous sodium carbonate solution to maintain pH = 8, then extracted three times with dichloromethane. The combined organic phases were directly concentrated and purified by reverse-phase C18 column chromatography (acidic method) to obtain the crude target product 2-(1-methylazetidin-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 4 (pale yellow solid, 30 mg, 46.2%).

LC-MS m/z (ESI)= 484.10 [M+1].

¹H NMR (400 MHz, Chloroform-*d*) δ 9.10 - 9.04 (m, 1H), 8.37 (dd, 1H), 8.01 (d, 1H), 7.51 (dd, 1H), 7.20 (d, 1H), 4.07 (d, 1H), 3.96 (m, 1H),3.90 (ddt, 4H), 3.74 (d, 1H), 3.51 (t, 2H), 2.47 (s, 3H), 2.32 (s, 2H).

### Example 5

5-((1*S*,5*R*)-1-(4-methyl-5-(1-methylpiperazin-4-yl)-4*H*-1,2,4-triazol-3-yl)-5-(trifluoromethyl)-3-azabicyclo[3.1.0]hexan-3-yl)quinoline-8-carbonitrile compound 5
5-((1S,5R)-1-(4-methyl-5-(1-methylpiperidin-4-yl)-4H-1,2,4-triazol-3-yl)-5-(trifluoromethyl)-3-azabicyclo[3.1.0]hexan-3-yl)quinoline-8-carbonitrile

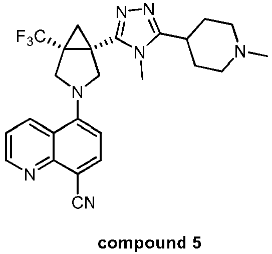

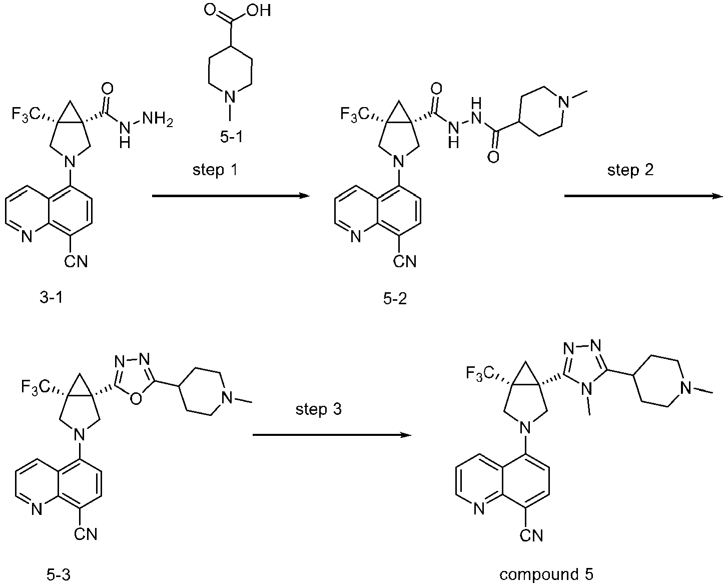

### Step 1:

(1*S*,5*R*)-3-(8-cyanoquinolin-5-yl)-*N*'-(1-methylpiperidine-4-carbonyl)-5-(trifluoromethyl)-3-azabicyclo[3.1.0]hexane-1-carbohydrazide 5-2
(1S,5R)-3-(8-cyanoquinolin-5-yl)-N'-(1-methylpiperidine-4-carbonyl)-5-(trifluoromethyl)-3-azabicyclo[3.1.0]hexane-1-carbohydrazide
1-Methylpiperidine-4-carboxylic acid 5-1 (119 mg, 83 mmol) was dissolved in 10 mL of *N*,*N*-dimethylformamide, followed by the addition of HATU (378 mg, 0.99 mmol) and DIPEA (321 mg, 2.49 mmol) under an ice bath. The mixture was stirred at low temperature for 5 minutes, and then (1S,5R)-3-(8-cyanoquinolin-5-yl)-5-(trifluoromethyl)-3-azabicyclo[3.1.0]hexane-1-carbohydrazide 3-1 (300 mg, 0.83 mmol) was added and stirred for half an hour. The reaction was monitored by TLC until completion. The reaction mixture was directly concentrated and purified by reverse-phase C18 column chromatography (alkaline method) to obtain the target product (1S,5R)-3-(8-cyanoquinolin-5-yl)-*N*'-(1-methylpiperidine-4-carbonyl)-5-(trifluoromethyl)-3-azabicyclo[3.1.0]hexane-1-carbohydrazide 5-2 (pale yellow solid, 380 mg, 89%).

LC-MS m/z (ESI)= 487.20 [M+1].

### Step 2:

5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile 5-3
5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile
(1*S*,5*R*)-3-(8-Cyanoquinolin-5-yl)-*N*'-(1-methylpiperidine-4-carbonyl)-5-(trifluoromethyl)-3-azabicyclo[3.1.0]hexane-1-carbohydrazide 5-2 (115 mg, 0.23 mmol) was dissolved in 6 mL of 1,2-dichloroethane, followed by the addition of triethylamine (47 mg, 0.46 mmol) and *p*-toluenesulfonyl chloride (66 mg, 0.345 mmol). The mixture was stirred at room temperature for 15 hours. After TLC confirmed the completion of the reaction, the reaction mixture was directly concentrated and purified by reverse-phase C18 column chromatography (alkaline method) to obtain the target compound (5-(1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile 5-3 (pale yellow solid, 70 mg, 46%).

¹H NMR (400 MHz, DMSO-*d*₆) δ 9.03 (dd, 1H), 8.69 (dd, 1H), 8.20 (d, 1H), 7.63 (dd, 1H), 7.34 (d, 1H), 4.19 (d, 1H), 4.07 (dd, 2H), 3.92 (d, 1H), 2.97 (td, 1H), 2.77 (d, 2H), 2.24 (d, 2H), 2.21 (*S,* 3H), 2.12 (*S,* 2H), 2.03 - 1.93 (m, 2H), 1.81 - 1.65 (m, 2H).

¹⁹F NMR (376 MHz, DMSO-*d*₆) δ -64.08.

LC-MS m/z (ESI)= 469.20 [M+1].

### Step 3:

5-((1*S*,5*R*)-1-(4-methyl-5-(1-methylpiperazin-4-yl)-4*H*-1,2,4-triazol-3-yl)-5-(trifluoromethyl)-3-azabicyclo[3.1.0]hexan-3-yl)quinoline-8-carbonitrile compound 5
5-((1S,5R)-1-(4-methyl-5-(1-methylpiperidin-4-yl)-4H-1,2,4-triazol-3-yl)-5-(trifluoromethyl)-3-azabicyclo[3.1.0]hexan-3-yl)quinoline-8-carbonitrile

The above compound (5-(1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile 5-3 (150 mg, 0.319 mmol), methylamine hydrochloride (64.1 mg, 0.957 mmol), *p*-toluenesulfonic acid (109 mg, 638 mmol), and DIEA (163 mg, 1.27 mmol) were dissolved in 2.00 mL of mesitylene. The mixture was refluxed at 150°C for 3 hours and sampled for LCMS analysis. After the reaction was completed, the mixture was cooled, and TLC (DCM: MeOH = 10:1) was conducted. The target product 5-((1*S*,5*R*)-1-(4-methyl-5-(1-methylpiperazin-4-yl)-4H-1,2,4-triazol-3-yl)-5-(trifluoromethyl)-3-azabicyclo[3.1.0]hexan-3-yl)quinoline-8-carbonitrile compound 5 (pale yellow solid, 40 mg, yield: 25.9%) was obtained by reverse-phase preparative purification.

¹H NMR (400 MHz, DMSO-*d*₆) δ 9.01 (dd, 1H), 8.73 (dd, 1H), 8.18 (d, 1H), 7.61 (dd, 1H), 7.28 (d, 1H), 4.13 (d, 3H), 3.83 (d, 1H), 3.68 (s, 3H), 3.14 - 2.85 (m, 3H), 2.29 (m, 5H), 2.07 - 1.73 (m, 6H).

LC-MS m/z (ESI)= 482.10 [M+1].

### Example 6

5-((1*S*,5*R*)-1-(5-((1-methylpiperidin-4-yl)amino)-1,3,4-oxadiazol-2-yl)-5-(trifluoromethyl)-3-azabicyclo[3.1.0]hexan-3-yl)quinoline-8-carbonitrile compound 6
5-((1S,5R)-1-(5-((1-methylpiperidin-4-yl)amino)-1,3,4-oxadiazol-2-yl)-5-(trifluoromethyl)-3-azabicyclo[3.1.0]hexan-3-yl)quinoline-8-carbonitrile

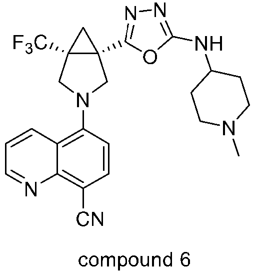

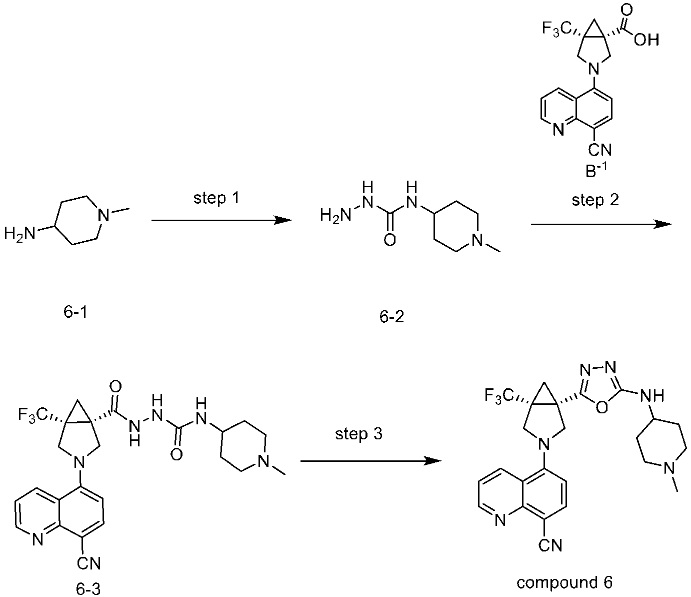

### Step 1:

*N*-(1-methylpiperidin-4-yl)carbohydrazide
N-(1-methylpiperidin-4-yl)hydrazinecarboxamide

Triphosgene (3.90 g, 13.2 mmol) was dissolved in 10.0 mL of dichloromethane. A mixed solution of 4-amino-1-methylpiperidine 6-1 (1.00 g, 8.77 mmol) and triethylamine (1.75 g, 17.5 mmol) in 10.0 mL of dichloromethane was slowly added dropwise at 0°C. After reacting for 1 h, hydrazine hydrate (1.28 g, 40.0 mmol) was slowly added dropwise. The reaction was carried out for 1 h before sampling. LCMS confirmed the completion of the reaction. The mixture was concentrated to obtain 4.3 g of the crude product N-(1-methylpiperidin-4-yl)carbohydrazide 6-2 (yellow oil).

LC-MS m/z (ESI)= 173.10 [M+1].

### Step 2:

2-((1*S*,5*R*)-3-(8-cyanoquinolin-5-yl)-5-(trifluoromethyl)-3-azabicyclo[3.1.0]hexane-1-carbonyl)-*N*-(1-methylpiperidin-4-yl)hydrazine-1-carboxamide compound 6-3
2-((1S,5R)-3-(8-cyanoquinolin-5-yl)-5-(trifluoromethyl)-3-azabicyclo[3.1.0]hexane-1-carbonyl)-N-(1-methylpiperidin-4-yl)hydrazine-1-carboxamide

The crude product *N*-(1-methylpiperidin-4-yl)-carbohydrazide 6-2 and (1*S*,5*R*)-3-(8-cyanoquinolin-5-yl)-5-(trifluoromethyl)-3-azabicyclo[3.1.0]hexane-1-carboxylic acid B-1 (1.00 g, 2.87 mmol) were dissolved in 10.0 mL of phosphorus oxychloride. The mixture was refluxed at 90°C for 3 hours and sampled for LCMS analysis. After the reaction was completed, saturated sodium hydroxide solution was added to adjust the pH to 7-8 for quenching. The mixture was lyophilized, and dichloromethane was added. The mixture was filtered, and the organic phase was concentrated. The residue was purified by reverse-phase preparative chromatography to obtain the target product 2-((1*S*,5*R*)-3-(8-cyanoquinolin-5-yl)-5-(trifluoromethyl)-3-azabicyclo[3.1.0]hexane-1-carbonyl)-*N*-(1-methylpiperidin-4-yl)hydrazine-1-carboxamide compound 6-3 (yellow solid, 100 mg, yield: 2%).

### Step 3

5-((1*S*,5*R*)-1-(5-((1-methylpiperidin-4-yl)amino)-1,3,4-oxadiazol-2-yl)-5-(trifluoromethyl)-3-azabicyclo[3.1.0]hexan-3-yl)quinoline-8-carbonitrile compound 6
5-((1S,5R)-1-(5-((1-methylpiperidin-4-yl)amino)-1,3,4-oxadiazol-2-yl)-5-(trifluoromethyl)-3-azabicyclo[3.1.0]hexan-3-yl)quinoline-8-carbonitrile
2-((1*S*,5*R*)-3-(8-Cyanoquinolin-5-yl)-5-(trifluoromethyl)-3-azabicyclo[3.1.0]hexane-1-carbonyl)-*N*-(1-methylpiperidin-4-yl)hydrazine-1-carboxamide 6-3 (100 mg, 0.19 mmol) was dissolved in 2.00 mL of dichloromethane, followed by the addition of triethylamine (58.1 mg, 0.57 mmol) and Burgess reagent (417 mg, 0.57 mmol). The mixture was stirred at room temperature for 5 hours. After TLC confirmed the completion of the reaction, the reaction mixture was concentrated and purified by MPLC, followed by silica gel column chromatography to obtain the target product 5-((1*S*,5*R*)-1-(5-((1-methylpiperidin-4-yl)amino)-1,3,4-oxadiazol-2-yl)-5-(trifluoromethyl)-3-azabicyclo[3.1.0]hexan-3-yl)quinoline-8-carbonitrile compound 6 (yellow solid, 10 mg, yield: 10.4%).

¹H NMR (400 MHz, DMSO-*d*₆) δ 9.02 (s, 1H), 8.68 (d, 1H), 8.19 (d, 1H), 7.72 (d, 1H), 7.62 (s, 1H), 7.30 (d, 1H), 4.15 (d, 1H), 4.07 (d, 1H), 3.92 (d, 2H), 3.50 (s, 1H), 2.77 (s, 2H), 2.21 (s, 3H), 2.07 (s, 3H), 1.89 (d, 2H), 1.51 (d, 2H), 1.36 (d, 1H).

LC-MS m/z (ESI)= 484.10 [M+1].

### Example 7

5-((1*S*,5*R*)-1-(5-amino-1,3,4-oxadiazol-2-yl)-5-(trifluoromethyl)-3-azabicyclo[3.1.0]hexan-3-yl)quinoline-8-carbonitrile compound 7
5-((1S,5R)-1-(5-amino-1,3,4-oxadiazol-2-yl)-5-(trifluoromethyl)-3-azabicyclo[3.1.0]hexan-3-yl)quinoline-8-carbonitrile

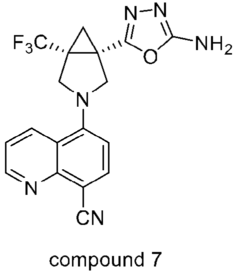

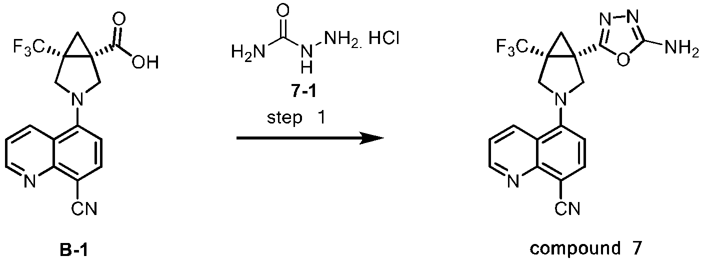

### Step 1:

5-((1*S*,5*R*)-1-(5-amino-1,3,4-oxadiazol-2-yl)-5-(trifluoromethyl)-3-azabicyclo[3.1.0]hexan-3-yl)quinoline-8-carbonitrile compound 7
5-((1S,5R)-1-(5-amino-1,3,4-oxadiazol-2-yl)-5-(trifluoromethyl)-3-azabicyclo[3.1.0]hexan-3-yl)quinoline-8-carbonitrile
(1*S*,5*R*)-3-(8-Cyanoquinolin-5-yl)-5-(trifluoromethyl)-3-azabicyclo[3.1.0]hexane-1-carboxylic acid B-1 (347 mg, 1.00 mmol) was dissolved in 10 mL of phosphorus oxychloride, followed by the addition of hydrazinecarboxamide hydrochloride 7-1 (112 mg, 1.00 mmol). After TLC confirmed the completion of the reaction, the reaction mixture was directly concentrated and then added dropwise to a saturated aqueous sodium carbonate solution. The pH of the aqueous phase was measured to be 9. The mixture was extracted with ethyl acetate (50 mL × 3), and the combined organic phases were concentrated under reduced pressure. The residue was purified by reverse-phase C18 column chromatography (alkaline method) to obtain the target compound 5-((1S,5R)-1-(5-amino-1,3,4-oxadiazol-2-yl)-5-(trifluoromethyl)-3-azabicyclo[3.1.0]hexan-3-yl)quinoline-8-carbonitrile compound 7 (pale yellow solid, 60 mg, 16%).

¹H NMR (400 MHz, DMSO-d6) δ 9.02 (d, 1H), 8.85 - 8.64 (m, 1H), 8.19 (d, 1H), 7.62 (dd, 1H), 7.30 (d, 1H), 7.18 (s, 2H), 4.15 (d, 1H), 4.06 (d, 1H), 3.90 (dd, 2H), 2.07 (q, 2H).

¹⁹F NMR (376 MHz, DMSO-*d*₆) δ -64.07.

LC-MS m/z (ESI)= 387.20 [M+1].

### Example 8

5-((1*S*,5*R*)-1-(5-(1,4-dimethylpiperidin-4-yl)-1,3,4-oxadiazol-2-yl)-5-(trifluoromethyl)-3-azabicyclo[3.1.0]hexan-3-yl)quinoline-8-carbonitrile compound 8
5-((1S,5R)-1-(5-(1,4-dimethylpiperidin-4-yl)-1,3,4-oxadiazol-2-yl)-5-(trifluoromethyl)-3-azabicyclo[3.1.0]hexan-3-yl)quinoline-8-carbonitrile

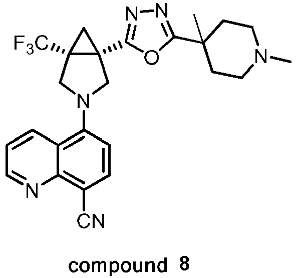

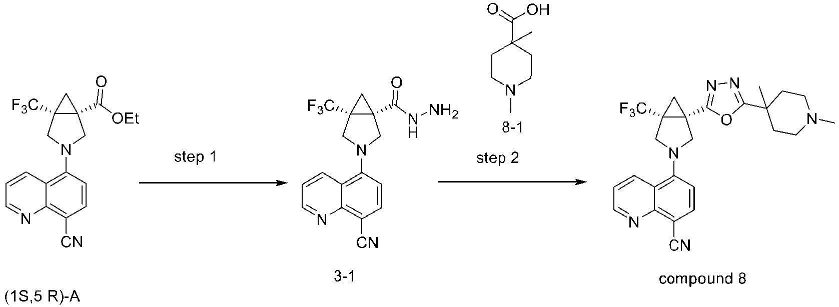

### Step 1:

(1*S*,5*R*)-3-(8-cyanoquinolin-5-yl)-5-(trifluoromethyl)-3-azabicyclo-[3.1.0]hexane-1-carbohydrazide compound 3-1
(1*S*,5*R*)-3-(8-cyanoquinolin-5-yl)-5-(trifluoromethyl)-3-azabicyclo-[3.1.0]hexane-1-carbohydrazide

Ethyl (1*S*,5*R*)-3-(8-cyanoquinolin-5-yl)-5-(trifluoromethyl)-3-azabicyclo[3.1.0]hexane-1-carboxylate (1*S*,5*R*)-A (1 g, 2.6 mmol) was dissolved in 5 mL of ethanol, followed by the addition of 5 mL of hydrazine hydrate. The mixture was heated to 85°C and stirred for 3 hours. After TLC confirmed the completion of the reaction, the reaction mixture was directly concentrated. The crude product was triturated with acetonitrile (5 mL), filtered, and rotary evaporated to dryness to obtain (1*S*,5*R*)-3-(8-cyanoquinolin-5-yl)-5-(trifluoromethyl)-3-azabicyclo-[3.1.0]hexane-1-carbohydrazide 3-1 (pale yellow solid, 460 mg, 49%).

LC-MS m/z (ESI)= 362.20 [M+1].

### Step 2:

5-((1*S*,5*R*)-1-(5-(1,4-dimethylpiperidin-4-yl)-1,3,4-oxadiazol-2-yl)-5-(trifluoromethyl)-3-azabicyclo[3.1.0]hexan-3-yl)quinoline-8-carbonitrile compound 8
5-((1S,5R)-1-(5-(1,4-dimethylpiperidin-4-yl)-1,3,4-oxadiazol-2-yl)-5-(trifluoromethyl)-3-azabicyclo[3.1.0]hexan-3-yl)quinoline-8-carbonitrile

(1*S*,5*R*)-3-(8-Cyanoquinolin-5-yl)-*N*'-(1-methylpiperidine-4-carbonyl)-5-(trifluoromethyl)-3-azabicyclo[3.1.0]hexane-1-carbohydrazide 3-1 (460 mg, 1.27 mmol) was dissolved in 10 mL of phosphorus oxychloride, followed by the addition of 1,4-dimethylpiperidine-4-carboxylic acid 8-1 (400 mg, 2.54 mmol). After TLC confirmed the completion of the reaction, the reaction mixture was directly concentrated and added dropwise to a saturated aqueous sodium carbonate solution. The pH of the aqueous phase was measured to be approximately 9. The mixture was extracted with ethyl acetate (50 mL × 3), and the organic phases were combined. After concentration under reduced pressure, the residue was purified by reverse-phase C18 column chromatography (alkaline method) to obtain the target compound 5-((1S,5R)-1-(5-(1,4-dimethylpiperidin-4-yl)-1,3,4-oxadiazol-2-yl)-5-(trifluoromethyl)-3-azabicyclo[3.1.0]hexan-3-yl)quinoline-8-carbonitrile compound 8 (pale yellow solid, 190 mg, 31%).

¹H NMR (400 MHz, DMSO-d6) δ 9.03 (dd, 1H), 8.69 (dd, 1H), 8.21 (d, 1H), 7.63 (dd, 1H), 7.34 (d, 1H), 4.21 (d, 1H), 4.17 - 4.00 (m, 2H), 3.93 (d, 1H), 2.75 (s, 2H), 2.44 - 2.06 (m, 9H), 1.77 (t, 2H), 1.30 (s, 3H).

¹⁹F NMR (376 MHz, DMSO-*d*₆) δ -64.06.

LC-MS m/z (ESI)= 483.20 [M+1].

### Example 9

5-((1*S*,5*R*)-1-(5-(1-methylpiperidin-4-yl)-4*H*-1,2,4-triazol-3-yl)-5-(trifluoromethyl)-3-azabicyclo[3.1.0]hexan-3-yl)quinoline-8-carbonitrile compound 9
5-((1S,5R)-1-(5-(1-methylpiperidin-4-yl)-4H-1,2,4-triazol-3-yl)-5-(trifluoromethyl)-3-azabicyclo[3.1.0]hexan-3-yl)quinoline-8-carbonitrile

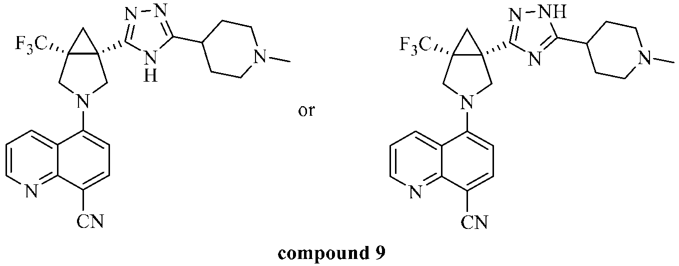

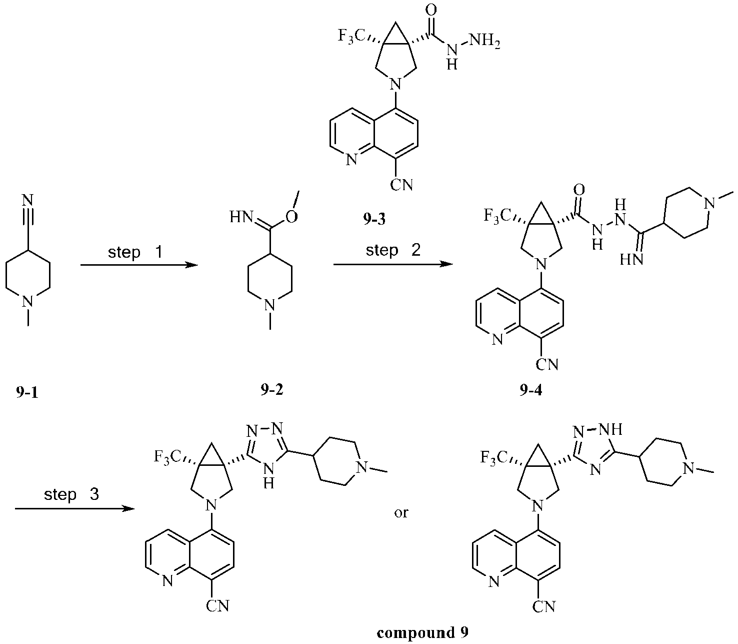

### Step 1:

6-azaspiro[3.4]acetate-2-one 9-2
methyl 1-methylpiperidine-4-carbimidate

The starting material 1-methylpiperidine-4-carbonitrile 9-1 (124 mg, 1.0 mmol) was added to methanol (1.5 mL). After nitrogen protection, the mixture was cooled to 0°C, and then acetyl chloride (1.0 mL) was slowly added dropwise. After the dropwise addition was completed, the mixture was gradually warmed to room temperature and stirred overnight. After the reaction was completed, the mixture was concentrated under reduced pressure at low temperature. The crude product 6-azaspiro[3.4]acetate-2-one 9-2 was directly used in the next step.

### Step 2:

(1*S*,5*R*)-3-(8-cyanoquinolin-5-yl)-*N*'-(imino(1-methylpiperidin-4-yl)methyl)-5-(trifluoromethyl)-3-azabicyclo[3.1.0]hexane-1-carbohydrazide 9-4
(1*S*,5*R*)-3-(8-cyanoquinolin-5-yl)-N'-(imino(1-methylpiperidin-4-yl)methyl)-5-(trifluoromethyl)-3-azabicyclo[3.1.0]hexane-1-carbohydrazide

The starting material 6-azaspiro[3.4]acetate-2-one 2 was added to 20 mL of methanol. After nitrogen protection, the mixture was cooled to 0°C. Sodium ethoxide (1.02 g, 15.0 mmol) was then added, followed by (1*S*,5*R*)-3-(8-cyanoquinolin-5-yl)-5-(trifluoromethyl)-3-azabicyclo[3.1.0]hexane-1-carbohydrazide (361 mg, 1 mmol). The reaction mixture was slowly warmed to room temperature and stirred for 4 hours until the reaction was completed. The reaction mixture was concentrated under reduced pressure and directly purified by column chromatography to obtain (1*S*,5*R*)-3-(8-cyanoquinolin-5-yl)-*N*'-(imino(1-methylpiperidin-4-yl)methyl)-5-(trifluoromethyl)-3-azabicyclo[3.1.0]hexane-1-carbohydrazide 9-4 (150 mg, two-step yield: 30.9%).

LC-MS m/z (ESI)=486.20 [M+1].

### Step 3:

5-((1*S*,5*R*)-1-(5-(1-methylpiperidin-4-yl)-4*H*-1,2,4-triazol-3-yl)-5-(trifluoromethyl)-3-azabicyclo[3.1.0]hexan-3-yl)quinoline-8-carbonitrile compound 9
5-((1S,5R)-1-(5-(1-methylpiperidin-4-yl)-4H-1,2,4-triazol-3-yl)-5-(trifluoromethyl)-3-azabicyclo[3.1.0]hexan-3-yl)quinoline-8-carbonitrile

The starting material (1S,5R)-3-(8-cyanoquinolin-5-yl)-*N*'-(imino(1-methylpiperidin-4-yl)methyl)-5-(trifluoromethyl)-3-azabicyclo[3.1.0]hexane-1-carbohydrazide 9-4 (150 mg, 0.31 mmol) was added to mesitylene (4 mL). The mixture was heated to 150°C and reacted for 4 hours under a nitrogen atmosphere. After the reaction was completed, the mixture was directly purified by column chromatography to obtain 5-((1*S*,5*R*)-1-(5-(1-methylpiperidin-4-yl)-4*H*-1,2,4-triazol-3-yl)-5-(trifluoromethyl)-3-azabicyclo[3.1.0]hexan-3-yl)quinoline-8-carbonitrile compound 9 (90 mg, yield: 62.3%).

LC-MS m/z (ESI)=468.20 [M+1].

¹H NMR (400 MHz, DMSO-*d*₆) δ 13.68 (s, 1H), 9.00 (dd, 1H), 8.68 (dd, 1H), 8.16 (d, 1H), 7.60 (dd, 1H), 7.27 (d, 1H), 4.27 - 3.98 (m, 3H), 3.91 (d, 1H), 2.83 (d, 2H), 2.71 (tt, 1H), 2.22 (s, 3H), 2.16 (d, 1H), 2.07 (t, 2H), 1.90 (dd, 3H), 1.72 (ddt, 2H).

¹⁹F NMR (377 MHz, DMSO-*d*₆) δ -63.40.

### Example 10

5-((1*S*,5*R*)-1-(5-((1*R*,3*S*,5*S*)-8-methyl-8-azabicyclo[3.2.1]octan-3-yl)-1,3,4-oxadiazol-2-yl)-5-(trifluoromethyl)-3-azabicyclo[3.1.0]hexan-3-yl)quinoline-8-carbonitrile compound 10
5-((1S,5R)-1-(5-((1R,3S,5S)-8-methyl-8-azabicyclo[3.2.1]octan-3-yl)-1,3,4-oxadiazol-2-yl)-5-(trifluoromethyl)-3-azabicyclo[3.1.0]hexan-3-yl)quinoline-8-carbonitrile

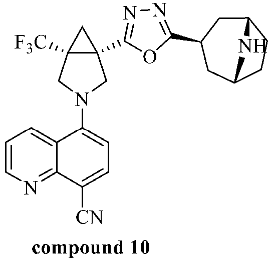

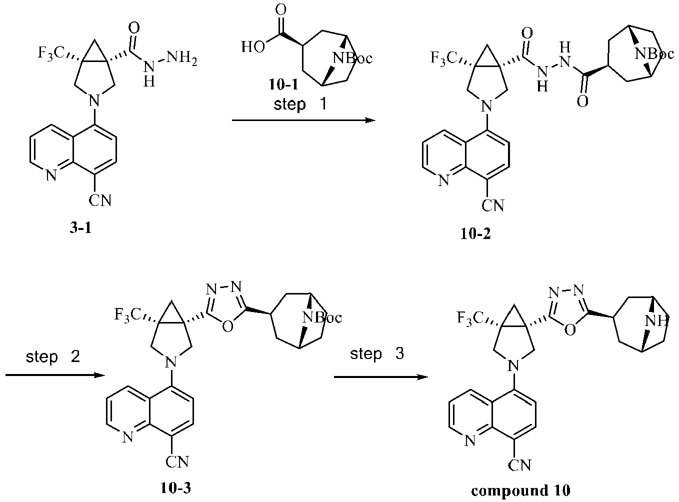

### Step 1:

*tert*-butyl (1*R*,3*S*,5*S*)-3-(2-((1*S*,5*R*)-3-(8-cyanoquinolin-5-yl)-5-(trifluoromethyl)-3-azabicyclo[3.1.0]hexane-1-carbonyl)hydrazine-1-carbonyl)-8-azabicyclo[3.2.1]octane-8-carboxylate 10-2
tert-butyl (1R,3S,5S)-3-(2-((1S,5R)-3-(8-cyanoquinolin-5-yl)-5-(trifluoromethyl)-3-azabicyclo[3.1.0]hexane-1-carbonyl)hydrazine-1-carbonyl)-8-azabicyclo[3.2.1]octane-8-carboxylate

The starting material (1*R*,3*S*,5*S*)-8-(*tert*-butoxycarbonyl)-8-azabicyclo[3.2.1]octane-3-carboxylic acid 10-1 (255 mg, 1.0 mmol) was added to *N*,*N*-dimethylformamide (5 mL), followed by the addition of *N,N,N',N*'-tetramethyl-*O*-(7-azabenzotriazol-1-yl)uronium hexafluorophosphate (570 mg, 1.5 mmol) and *N*,*N*-diisopropylethylamine (258 mg, 2.0 mmol). The reaction mixture was stirred at room temperature for 0.5 hours, and then (1*S*,5*R*)-3-(8-cyanoquinolin-5-yl)-5-(trifluoromethyl)-3-azabicyclo[3.1.0]hexane-1-carbohydrazide 3-1 (361 mg, 1.0 mmol) was added. The reaction mixture was stirred at room temperature for 6 hours and then purified by reverse-phase column chromatography to obtain tert-butyl (1*R*,3*S*,5*S*)-3-(2-((1*S*,5*R*)-3-(8-cyanoquinolin-5-yl)-5-(trifluoromethyl)-3-azabicyclo[3.1.0]hexane-1-carbonyl)hydrazine-1-carbonyl)-8-azabicyclo[3.2.1]octane-8-carboxylate 10-2 (398 mg, yield: 66.4%).

LCMS m/z(ESI)=599.2[M+1].

### Step 2:

*tert*-butyl (1*R*,3*S*,5*S*)-3-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-8-azabicyclo[3.2.1]octane-8-carboxylate 10-3
tert-butyl (1R,3S,5S)-3-(5-((1S,5R)-3-(8-cyanoquinolin-5-yl)-5-(trifluoromethyl)-3-azabicyclo[3.1.0]hexan-1-yl)-1,3,4-oxadiazol-2-yl)-8-azabicyclo[3.2.1]octane-8-carboxylate
*tert*-Butyl (1*R*,3*S*,5*S*)-3-(2-((1*S*,5*R*)-3-(8-cyanoquinolin-5-yl)-5-(trifluoromethyl)-3-azabicyclo[3.1.0]hexane-1-carbonyl)hydrazine-1-carbonyl)-8-azabicyclo[3.2.1]octane-8-carboxylate 10-2 (398 mg, 0.67 mmol) was dissolved in 10 mL of dichloromethane, followed by the addition of triethylamine (338 mg, 3.35 mmol) and *p*-toluenesulfonyl chloride (382 mg, 2.01 mmol). The mixture was stirred at room temperature for 5 hours. After TLC confirmed the completion of the reaction, the reaction mixture was concentrated and purified by silica gel column chromatography to obtain the target product *tert*-butyl (1*R*,*3S*,5*S*)-3-(5-((1S,5R)-3-(8-cyanoquinolin-5-yl)-5-(trifluoromethyl)-3-azabicyclo[3.1.0]hexan-1-yl)-1,3,4-oxadiazol-2-yl)-8-azabicyclo[3.2.1]octane-8-carboxylate 10-3 (yellow solid, 300 mg, yield: 77.1%).

LC-MS m/z (ESI)= 581.2 [M+1].

### Step 3:

5-((1*S*,5*R*)-1-(5-((1*R*,3*S*,5*S*)-8-azabicyclo[3.2.1]octan-3-yl)-1,3,4-oxadiazol-2-yl)-5-(trifluoromethyl)-3-azabicyclo[3.1.0]hexan-3-yl)quinoline-8-carbonitrile compound 10
5-((1S,5R)-1-(5-((1R,3S,5S)-8-azabicyclo[3.2.1]octan-3-yl)-1,3,4-oxadiazol-2-yl)-5-(trifluoromethyl)-3-azabicyclo[3.1.0]hexan-3-yl)quinoline-8-carbonitrile

The starting material *tert*-butyl (1*R*,3*S*,5*S*)-3-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-8-azabicyclo[2.2.1]octane-8-carboxylate 10-3 (300 mg, 0.5 mmol) was added to dichloromethane (6 mL), followed by the addition of trifluoroacetic acid (3 mL). The reaction mixture was stirred at room temperature for 2 hours, concentrated under reduced pressure, and then purified by reverse-phase column chromatography to obtain 5-((1*S*,5*R*)-1-(5-((1*R*,3*S*,5*S*)-8-azabicyclo[3.2.1]octan-3-yl)-1,3,4-oxadiazol-2-yl)-5-(trifluoromethyl)-3-azabicyclo[3.1.0]hexan-3-yl)quinoline-8-carbonitrile compound 10 (215 mg, yield: 89.4%).

LC-MS m/z (ESI)= 481.2 [M+1].

### Example 11

5-((1*S*,5*R*)-1-(5-((1*R*,3*S*,5*S*)-8-methyl-8-azabicyclo[3.2.1]octan-3-yl)-1,3,4-oxadiazol-2-yl)-5-(trifluoromethyl)-3-azabicyclo[3.1.0]hexan-3-yl)quinoline-8-carbonitrile compound 11
5-((1S,5R)-1-(5-((1R,3S,5S)-8-methyl-8-azabicyclo[3.2.1]octan-3-yl)-1,3,4-oxadiazol-2-yl)-5-(trifluoromethyl)-3-azabicyclo[3.1.0]hexan-3-yl)quinoline-8-carbonitrile 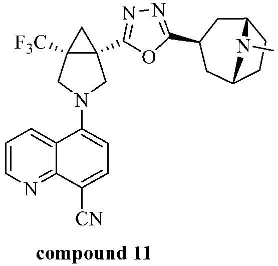 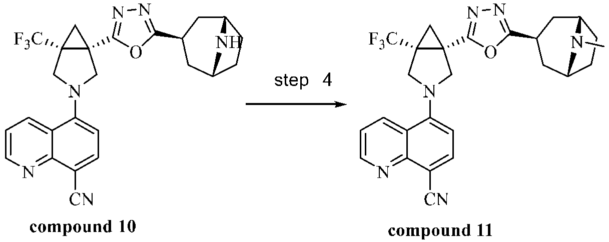
5-((1*S*,5*R*)-1-(5-((1*R*,3*S*,5*S*)-8-methyl-8-azabicyclo[3.2.1]octan-3-yl)-1,3,4-oxadiazol-2-yl)-5-(trifluoromethyl)-3-azabicyclo[3.1.0]hexan-3-yl)quinoline-8-carbonitrile compound 11
5-((1S,5R)-1-(5-((1R,3S,5S)-8-methyl-8-azabicyclo[3.2.1]octan-3-yl)-1,3,4-oxadiazol-2-yl)-5-(trifluoromethyl)-3-azabicyclo[3.1.0]hexan-3-yl)quinoline-8-carbonitrile
5-((1*S*,5*R*)-1-(5-((1*R*,3S,5*S*)-8-Azabicyclo[3.2.1]octan-3-yl)-1,3,4-oxadiazol-2-yl)-5-(trifluoromethyl)-3-azabicyclo[3.1.0]hexan-3-yl)quinoline-8-carbonitrile 10 (215 mg, 0.45 mmol) was dissolved in 5 mL of methanol, followed by the addition of paraformaldehyde (187 mg, 2.08 mmol) and sodium cyanoborohydride (158 mg, 4.16 mmol). The mixture was stirred at room temperature for 3 hours. After TLC confirmed the completion of the reaction, the reaction mixture was directly concentrated and purified by reverse-phase column chromatography to obtain the target product 5-((1*S*,5*R*)-1-(5-((1*R*,3*S*,5*S*)-8-methyl-8-azabicyclo[3.2.1]octan-3-yl)-1,3,4-oxadiazol-2-yl)-5-(trifluoromethyl)-3-azabicyclo[3.1.0]hexan-3-yl)quinoline-8-carbonitrile compound 11 (pale yellow solid, 41 mg, 18.4%).

LC-MS m/z (ESI)= 495.2 [M+1].

¹H NMR (400 MHz, DMSO-*d*₆) δ 9.02 (d, 1H), 8.69 (dd, 1H), 8.21 (d, 1H), 7.63 (dd, 1H), 7.34 (d, 1H), 4.20 (d, 1H), 4.07 (t, 2H), 3.93 (d, 1H), 3.64 (s, 3H), 3.50 - 3.34 (m, 2H), 2.29 (ddd, 4H), 2.15 - 1.93 (m, 2H), 1.68 - 1.15 (m, 5H).

¹⁹F NMR (377 MHz, DMSO-d6) δ -63.82 (d).

### Example 12

5-((1*S*,5*R*)-1-(5-(tetrahydro-2*H*-pyran-4-yl)-1,3,4-oxadiazol-2-yl)-5-(trifluoromethyl)-3-azabicyclo[3.1.0]hexan-3-yl)quinoline-8-carbonitrile compound 12
5-((1S,5R)-1-(5-(tetrahydro-2H-pyran-4-yl)-1,3,4-oxadiazol-2-yl)-5-(trifluoromethyl)-3-azabicyclo[3.1.0]hexan-3-yl)quinoline-8-carbonitrile

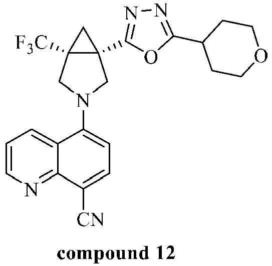

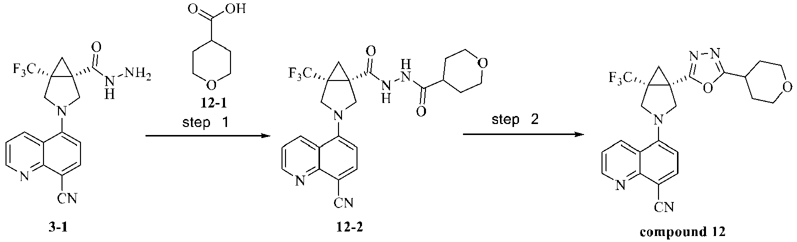

### Step 1:

(1*S*,5*R*)-3-(8-cyanoquinolin-5-yl)-*N*'-(tetrahydro-2*H*-pyran-4-carbonyl)-5-(trifluoromethyl)-3-azabicyclo[3.1.0]hexane-1-carbohydrazide 12-2
(1S,5R)-3-(8-cyanoquinolin-5-yl)-N'-(tetrahydro-2H-pyran-4-carbonyl)-5-(trifluoromethyl)-3-azabicyclo[3.1.0]hexane-1-carbohydrazide

The starting material tetrahydro-2*H*-pyran-4-carboxylic acid 12-1 (195 mg, 1.5 mmol) was added to *N*,*N*-dimethylformamide (5 mL), followed by the addition of *N,N,N',N'-*tetramethyl-*O*-(7-azabenzotriazol-1-yl)uronium hexafluorophosphate (570 mg, 1.5 mmol) and *N*,*N*-diisopropylethylamine (258 mg, 2.0 mmol). The reaction mixture was stirred at room temperature for 0.5 hours, and then (1*S*,5*R*)-3-(8-cyanoquinolin-5-yl)-5-(trifluoromethyl)-3-azabicyclo[3.1.0]hexane-1-carbohydrazide 3-1 (361 mg, 1.0 mmol) was added. The reaction mixture was stirred at room temperature for 6 hours, and then purified by reverse-phase column chromatography to obtain -(1*S*,5*R*)-3-(8-cyanoquinolin-5-yl)-*N'*-(tetrahydro-2*H*-pyran-4 carbonyl)-5-(trifluoromethyl)-3-azabicyclo[3.1.0]hexane-1-carbohydrazide 12-2 (320 mg, yield: 67.5%).

LCMS m/z(ESI)= 474.2 [M+1].

### Step 2:

5-((1*S*,5*R*)-1-(5-(tetrahydro-2*H*-pyran-4-yl)-1,3,4-oxadiazol-2-yl)-5-(trifluoromethyl)-3-azabicyclo[3.1.0]hexan-3-yl)quinoline-8-carbonitrile compound 12
5-((1S,5R)-1-(5-(tetrahydro-2H-pyran-4-yl)-1,3,4-oxadiazol-2-yl)-5-(trifluoromethyl)-3-azabicyclo[3.1.0]hexan-3-yl)quinoline-8-carbonitrile
(1*S*,5*R)*-3-(8-Cyanoquinolin-5-yl)-*N'*-(tetrahydro-2*H*-pyran-4-carbonyl)-5-(trifluoromethyl)-3-azabicyclo[3.1.0]hexane-1-carbohydrazide 12-2 (320 mg, 0.68 mmol) was dissolved in 10 mL of dichloromethane, followed by the addition of triethylamine (338 mg, 3.35 mmol) and *p*-toluenesulfonyl chloride (382 mg, 2.01 mmol). The mixture was stirred at room temperature for 5 hours. After TLC confirmed the completion of the reaction, the reaction mixture was concentrated and purified by silica gel column chromatography to obtain the target product 5-((1*S*,5*R*)-1-(5-(tetrahydro-2*H*-pyran-4-yl)-1,3,4-oxadiazol-2-yl)-5-(trifluoromethyl)-3-azabicyclo[3.1.0]hexan-3-yl)quinoline-8-carbonitrile compound 12 (yellow solid, 185 mg, yield: 60.0%).

LC-MS m/z (ESI) = 456.2 [M+1].

### Example 13

5-((1*S*,5*R*)-1-(5-(2-methyl-2-azaspiro[3.5]nonan-7-yl)-1,3,4-oxadiazol-2-yl)-5-(trifluoromethyl)-3-azabicyclo[3.1.0]hexan-3-yl)quinoline-8-carbonitrile compound 13
5-((1*S*,5*R*)-1-(5-(2-methyl-2-azaspiro[3.5]nonan-7-yl)-1,3,4-oxadiazol-2-yl)-5-(trifluoromethyl)-3-azabicyclo[3.1.0]hexan-3-yl)quinoline-8-carbonitrile

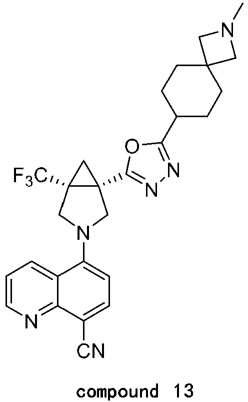

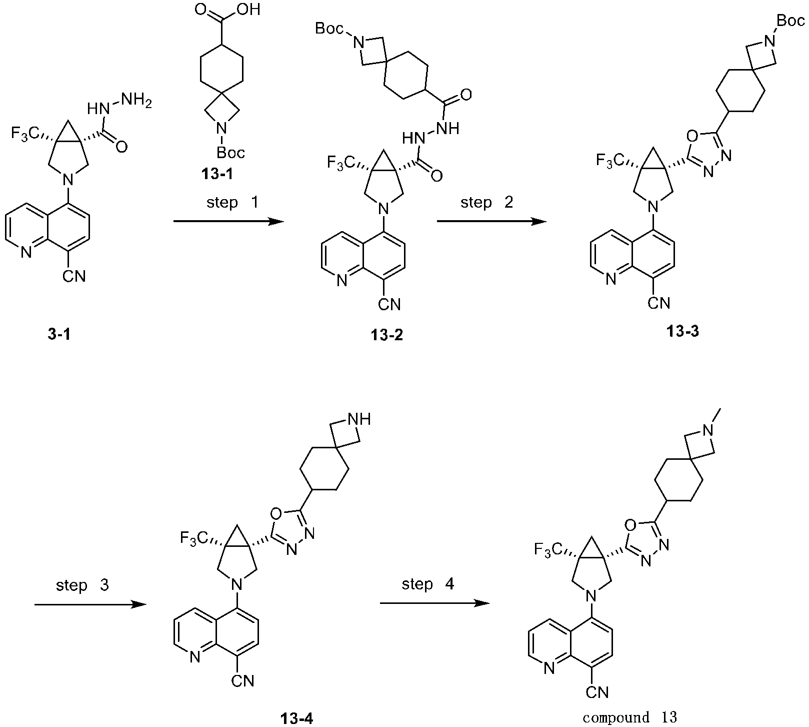

### Step 1:

*tert*-butyl 7-(2-((1*S*,5*R*)-3-(8-cyanoquinolin-5-yl)-5-(trifluoromethyl)-3-azabicyclo[3.1.0]hexane-1-carbonyl)hydrazine-1-carbonyl)-2-azaspiro[3.5]nonane-2-carboxylate 13-2
tert-butyl 7-(2-((1S,5R)-3-(8-cyanoquinolin-5-yl)-5-(trifluoromethyl)-3-azabicyclo[3.1.0]hexane-1-carbonyl)hydrazine-1-carbonyl)-2-azaspiro[3.5]nonane-2-carboxylate
2-(*tert*-Butylcarbonyl)-2-azaspiro[3.5]nonane-7-carboxylic acid 13-1 (150 mg, 0.55 mmol) was dissolved in 5 mL of dichloromethane, followed by the addition of HATU (315 mg, 0.83 mmol) and DIEA (145 mg, 1.1 mmol). The mixture was stirred at room temperature for 15 minutes, and then (1*S*,5*R*)-3-(8-cyanoquinolin-5-yl)-5-(trifluoromethyl)-3-azabicyclo[3.1.0]hexane-1-carbohydrazide 3-1 (200 mg, 0.55 mmol) was added. After LCMS confirmed the completion of the reaction, the reaction mixture was directly concentrated and purified by reverse-phase C18 column chromatography (acidic method) to obtain tert-butyl 7-(2-((1*S*,5*R*)-3-(8-cyanoquinolin-5-yl)-5-(trifluoromethyl)-3-azabicyclo[3.1.0]hexane-1-carbonyl)hydrazine-1-carbonyl)-2-azaspiro[3.5]nonane-2-carboxylate 13-2 (pale yellow solid, 250 mg, 41%).

LC-MS m/z (ESI) = 613.20 [M+H]⁺.

### Step 2:

*tert*-butyl 7-(5-((1*S*,5*R*)-3-(8-cyanoquinolin-5-yl)-5-(trifluoromethyl)-3-azabicyclo[3.1.0]hexen-1-yl)-1,3,4-oxadiazol-2-yl)-2-azaspiro[3.5]nonane-2-carboxylate 13-3
tert-butyl 7-(5-((1S,5R)-3-(8-cyanoquinolin-5-yl)-5-(trifluoromethyl)-3-azabicyclo[3.1.0]hexan-1-yl)-1,3,4-oxadiazol-2-yl)-2-azaspiro[3.5]nonane-2-carboxylate
*tert*-Butyl 7-(2-((1*S*,5*R*)-3-(8-cyanoquinolin-5-yl)-5-(trifluoromethyl)-3-azabicyclo[3.1.0]hexane-1-carbonyl)hydrazine-1-carbonyl)-2-azaspiro[3.5]nonane-2-carboxylate 13-2 (250 mg, 0.4 mmol) was dissolved in 5 mL of 1,4-dioxane, followed by the addition of TsCl (155 mg, 0.8 mmol) and Cs₂CO₃ (400 mg, 1.2 mmol). The mixture was stirred under nitrogen for 2 hours. After LCMS confirmed the completion of the reaction, ethyl acetate and water were added for extraction. The organic phase was rotary evaporated to dryness to obtain the crude target product *tert*-butyl 7-(5-((1*S*,5*R*)-3-(8-cyanoquinolin-5-yl)-5-(trifluoromethyl)-3-azabicyclo[3.1.0]hexen-1-yl)-1,3,4-oxadiazol-2-yl)-2-azaspiro[3.5]nonane-2-carboxylate 13-3 (yellow oil, 200 mg).

LC-MS m/z (ESI) = 595.30 [M+H]⁺.

### Step 3:

5-((1*S*,5*R*)-1-(5-(2-azaspiro[3.5]nonanyl)-1,3,4-oxadiazol-2-yl)-5-(trifluoromethyl)-3-azabicyclo[3.1.0]hexan-3-yl)quinoline-8-carbonitrile 13-4
5-((1S,5R)-1-(5-(2-azaspiro[3.5]nonan-7-yl)-1,3,4-oxadiazol-2-yl)-5-(trifluoromethyl)-3-azabicyclo[3.1.0]hexan-3-yl)quinoline-8-carbonitrile
*tert*-Butyl 7-(5-((1*S*,5*R*)-3-(8-cyanoquinolin-5-yl)-5-(trifluoromethyl)-3-azabicyclo[3.1.0]hexen-1-yl)-1,3,4-oxadiazol-2-yl)-2-azaspiro[3.5]nonane-2-carboxylate 13-3 (200 mg, 0.33 mmol) was dissolved in 10 mL of dichloromethane, followed by the addition of 5 mL of trifluoroacetic acid. The mixture was stirred at room temperature for 1 hour. After LCMS confirmed the completion of the reaction, the reaction mixture was directly concentrated. The residue was purified by reverse-phase C18 column chromatography (acidic method) to obtain the target compound 5-((1*S*,5*R*)-1-(5-(2-azaspiro[3.5]nonanyl)-1,3,4-oxadiazol-2-yl)-5-(trifluoromethyl)-3-azabicyclo[3.1.0]hexan-3-yl)quinoline-8-carbonitrile 13-4 (yellow oil, 170 mg, 100%).

LC-MS m/z (ESI) = 495.20 [M+H]⁺.

### Step 4:

5-((1*S*,5*R*)-1-(5-(2-methyl-2-azaspiro[3.5]nonan-7-yl)-1,3,4-oxadiazol-2-yl)-5-(trifluoromethyl)-3-azabicyclo[3.1.0]hexan-3-yl)quinoline-8-carbonitrile compound 13
5-((1S,5R)-1-(5-(2-methyl-2-azaspiro[3.5]nonan-7-yl)-1,3,4-oxadiazol-2-yl)-5-(trifluoromethyl)-3-azabicyclo[3.1.0]hexan-3-yl)quinoline-8-carbonitrile
5-((1*S*,5*R*)-1-(5-(2-Azaspiro[3.5]nonanyl)-1,3,4-oxadiazol-2-yl)-5-(trifluoromethyl)-3-azabicyclo[3.1.0]hexan-3-yl)quinoline-8-carbonitrile 13-4 (170 mg, 0.35 mmol) was dissolved in 10 mL of dichloromethane, followed by the addition of paraformaldehyde (210 mg, 7 mmol) and sodium cyanoborohydride (220 mg, 3.5 mmol). The mixture was stirred at room temperature for 6 hours. After LCMS confirmed the completion of the reaction, the reaction mixture was directly concentrated. The residue was purified by reverse-phase C18 column chromatography (alkaline method) to obtain the target product *5-((1S,5R)-1-(5-(2-*methyl-2-azaspiro[3.5]nonan-7-yl)-1,3,4-oxadiazol-2-yl)-5-(trifluoromethyl)-3-azabicyclo[3.1.0]hexan-3-yl)quinoline-8-carbonitrile compound 13 (white solid, 50 mg, 28%).

¹H NMR (400 MHz, DMSO-*d₆*) δ 9.03 (dd, 1H), 8.69 (dd, 1H), 8.20 (d, 1H), 7.63 (dd, 1H), 7.34 (d, 1H), 4.19 (d, 1H), 4.06 (dd, 2H), 3.91 (s, 1H), 2.97 (s, 4H), 2.25 (s, 3H), 2.21 (d, 1H), 1.93 - 1.78 (m, 4H), 1.53 (d, 4H), 1.16 (s, 2H).

LC-MS m/z (ESI) = 509.20 [M+H]⁺.

### Example 14

5-((1*S*,5*R*)-1-(5-((*S*)-4-methylmorpholin-2-yl)-1,3,4-oxadiazol-2-yl)-5-(trifluoromethyl)-3-azabicyclo[3.1.0]hexan-3-yl)quinoline-8-carbonitrile compound 14
5-((1*S*,5*R*)-1-(5-((*S*)-4-methylmorpholin-2-yl)-1,3,4-oxadiazol-2-yl)-5-(trifluoromethyl)-3-azabicyclo[3.1.0]hexan-3-yl)quinoline-8-carbonitrile

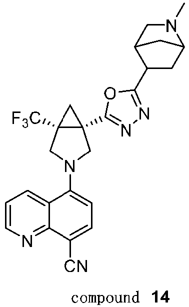

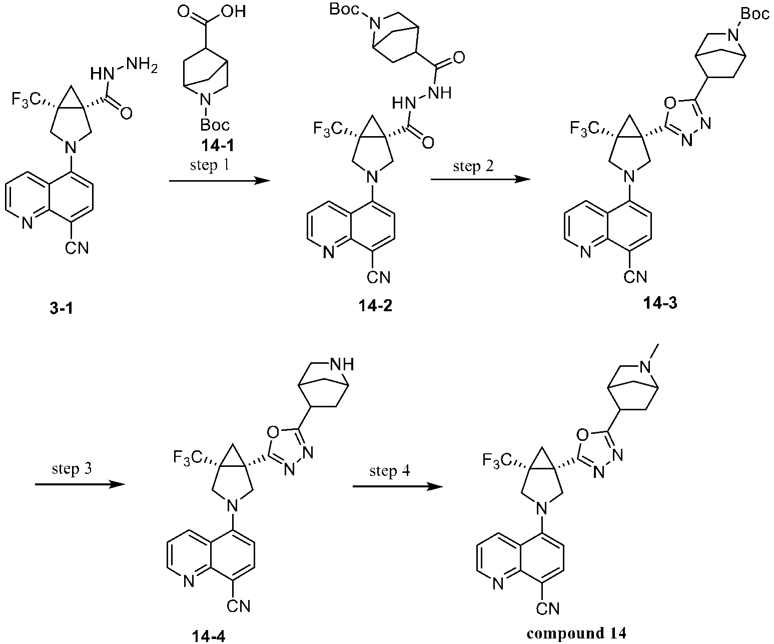

### Step 1:

*tert*-butyl 5-(2-((1*S*,5*R*)-3-(8-cyanoquinolin-5-yl)-5-(trifluoromethyl)-3-azabicyclo[3.1.0]hexane-1-carbonyl)hydrazine-1-carbonyl)-2-azabicyclo[2.2.1]heptane-2-carboxylate 14-2
tert-butyl 5-(2-((1S,5R)-3-(8-cyanoquinolin-5-yl)-5-(trifluoromethyl)-3-azabicyclo[3.1.0]hexane-1-carbonyl)hydrazine-1-carbonyl)-2-azabicyclo[2.2.1]heptane-2-carboxylate
2-(*tert*-Butylcarbonyl)-2-azabicyclo[2.2.1]heptane-5-carboxylic acid 14-1 (135 mg, 0.55 mmol) was dissolved in 5 mL of dichloromethane, followed by the addition of HATU (320 mg, 0.83 mmol) and DIPEA (145 mg, 1.1 mmol) under an ice bath. The mixture was stirred at low temperature for 15 minutes, and then (1S,5R)-3-(8-cyanoquinolin-5-yl)-5-(trifluoromethyl)-3-azabicyclo[3.1.0]hexane-1-carbohydrazide 3-1 (200 mg, 0.55 mmol) was added and stirred for 1 hour. After LCMS confirmed the completion of the reaction, the reaction mixture was directly concentrated and purified by reverse-phase C18 column chromatography (acidic method) to obtain the target product tert-butyl 5-(2-((1S,5R)-3-(8-cyanoquinolin-5-yl)-5-(trifluoromethyl)-3-azabicyclo[3.1.0]hexane-1-carbonyl)hydrazine-1-carbonyl)-2-azabicyclo[2.2.1]heptane-2-carboxylate 14-2 (pale yellow solid, 130 mg, 45%).

LC-MS m/z (ESI) = 585.30 [M+H]⁺.

### Step 2:

tert-butyl 5-(5-((1S,5R)-3-(8-cyanoquinolin-5-yl)-5-(trifluoromethyl)-3-azabicyclo[3.1.0]hexen-1-yl)-1,3,4-oxadiazol-2-yl)-2-azabicyclo[2.2.1]heptane-2-carboxylate 14-3
tert-butyl 5-(5-((1S,5R)-3-(8-cyanoquinolin-5-yl)-5-(trifluoromethyl)-3-azabicyclo[3.1.0]hexan-1-yl)-1,3,4-oxadiazol-2-yl)-2-azabicyclo[2.2.1]heptane-2-carboxylate
*tert-Butyl* 5-(2-((1S,5R)-3-(8-cyanoquinolin-5-yl)-5-(trifluoromethyl)-3-azabicyclo[3.1.0]hexane-1-carbonyl)hydrazine-1-carbonyl)-2-azabicyclo[2.2.1]heptane-2-carboxylate 14-2 (110 mg, 0.2 mmol) was dissolved in 3 mL of 1,4-dioxane, followed by the addition of TsCl (76 mg, 0.4 mmol) and Cs₂CO₃ (200 mg, 0.6 mmol). The mixture was stirred at room temperature for 5 hours. After LCMS confirmed the completion of the reaction, the mixture was extracted with ethyl acetate and water. The organic phase was rotary evaporated to dryness to obtain the crude target compound tert-butyl 5-(5-((1S,5R)-3-(8-cyanoquinolin-5-yl)-5-(trifluoromethyl)-3-azabicyclo[3.1.0]hexen-1-yl)-1,3,4-oxadiazol-2-yl)-2-azabicyclo[2.2.1]heptane-2-carboxylate 14-3 (yellow oil, 100 mg).

LC-MS m/z (ESI) = 567.30 [M+H]⁺.

### Step 3:

5-((1*S*,5*R*)-1-(5-(2-azabicyclo[2.2.1]heptan-5-yl)-1,3,4-oxadiazol-2-yl)-5-(trifluoromethyl)-3-azabicyclo[3.1.0]hexan-3-yl)quinoline-8-carbonitrile 14-4
5-((1S,5R)-1-(5-(2-azabicyclo[2.2.1]heptan-5-yl)-1,3,4-oxadiazol-2-yl)-5-(trifluoromethyl)-3-azabicyclo[3.1.0]hexan-3-yl)quinoline-8-carbonitrile
*tert*-Butyl 5-(5-((1*S*,5*R*)-3-(8-cyanoquinolin-5-yl)-5-(trifluoromethyl)-3-azabicyclo[3.1.0]hexen-1-yl)-1,3,4-oxadiazol-2-yl)-2-azabicyclo[2.2.1]heptane-2-carboxylate 14-3 (100 mg, 0.18 mmol) was dissolved in 10 mL of dichloromethane, followed by the addition of 5 mL of trifluoroacetic acid. The mixture was stirred at room temperature for 1 hour. After LCMS confirmed the completion of the reaction, the reaction mixture was directly concentrated. The residue was purified by reverse-phase C18 column chromatography (acidic method) to obtain the target compound *5-*((*1S,5R*)-1-(5-(2-azabicyclo[2.2.1]heptan-5-yl)-1,3,4-oxadiazol-2-yl)-5-(trifluoromethyl)-3-azabicyclo[3.1.0]hexan-3-yl)quinoline-8-carbonitrile 14-4 (yellow solid, 90 mg, 97%).

LC-MS m/z (ESI) = 467.20 [M+H]⁺.

### Step 4:

5-((1*S*,5*R*)-1-(5-(2-methyl-2-azabicyclo[2.2.1]heptan-5-yl)-1,3,4-oxadiazol-2-yl)-5-(trifluoromethyl)-3-azabicyclo[3.1.0]hexan-3-yl)quinoline-8-carbonitrile compound 14
5-((1S,5R)-1-(5-(2-methyl-2-azabicyclo[2.2.1]heptan-5-yl)-1,3,4-oxadiazol-2-yl)-5-(trifluoromethyl)-3-azabicyclo[3.1.0]hexan-3-yl)quinoline-8-carbonitrile
5-((1*S*,5*R*)-1-(5-(2-Azabicyclo[2.2.1]heptan-5-yl)-1,3,4-oxadiazol-2-yl)-5-(trifluoromethyl)-3-azabicyclo[3.1.0]hexan-3-yl)quinoline-8-carbonitrile 14-4 (90 mg, 0.2 mmol) was dissolved in 10 mL of dichloromethane, followed by the addition of paraformaldehyde (120 mg, 4 mmol) and sodium cyanoborohydride (130 mg, 2 mmol). The mixture was stirred at room temperature for 3 hours. After LCMS confirmed the completion of the reaction, the reaction mixture was directly concentrated. The residue was purified by reverse-phase C18 column chromatography (alkaline method) to obtain the target product 5-((1S,5R)-1-(5-(2-methyl-2-azabicyclo[2.2.1]heptan-5-yl)-1,3,4-oxadiazol-2-yl)-5-(trifluoromethyl)-3-azabicyclo[3.1.0]hexan-3-yl)quinoline-8-carbonitrile compound 14 (yellow solid, 90 mg, 97%).

¹H NMR (400 MHz, DMSO-*d₆*) δ 9.03 (d, 1H), 8.69 (d, 1H), 8.20 (d, 1H), 7.63 (dd, 1H), 7.34 (dd, 1H), 4.22 (d, 1H), 4.12 - 4.00 (m, 2H), 3.94 (dd, 1H), 3.11 (s, 1H), 2.66 (s, 1H), 2.24 - 2.21 (m, 3H), 2.18 (s, 2H), 2.08 (s, 3H), 1.94 - 1.82 (m, 2H), 1.76 (d, 1H), 1.53 (d, 1H).

LC-MS m/z (ESI) = 481.20[M+H]⁺.

### Example 15

5-((1*S*,5*R*)-1-(5-((1-methylpiperidin-4-yl)oxy)-1,3,4-oxadiazol-2-yl)-5-(trifluoromethyl)-3-azabicyclo[3.1.0]hexan-3-yl)quinoline-8-carbonitrile compound 15
5-((1S,5R)-1-(5-((1-methylpiperidin-4-yl)oxy)-1,3,4-oxadiazol-2-yl)-5-(trifluoromethyl)-3-azabicyclo[3.1.0]hexan-3-yl)quinoline-8-carbonitrile

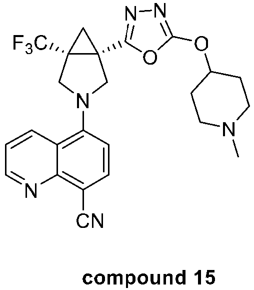

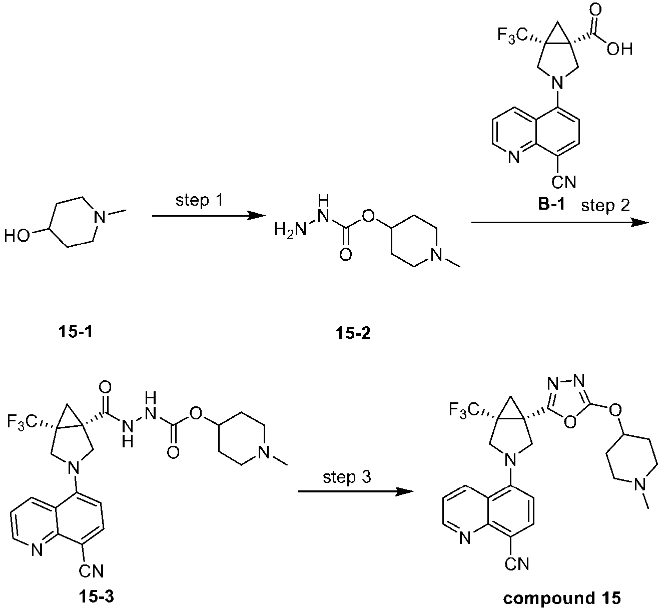

### Step 1:

benzyl 1-methylpiperazine-4-hydrazinecarboxylate 15-2
methylpiperidin-4-yl hydrazinecarboxylate

Triphosgene (3.90 g, 13.2 mmol) was dissolved in 10.0 mL of dichloromethane. A mixed solution of 4-oxo-1-methylpiperidine (1.00 g, 8.77 mmol) and triethylamine (1.75 g, 17.5 mmol) in 10.0 mL of dichloromethane was slowly added dropwise at 0°C. After reacting for 1 h, hydrazine hydrate (1.28 g, 40.0 mmol) was slowly added dropwise. After reacting for another 1 h, the mixture was sampled. After LCMS confirmed the completion of the reaction, the mixture was concentrated to obtain 4.3 g of the crude product benzyl 1-methylpiperazine-4-hydrazinecarboxylate 15-2 (yellow oil).

### Step 2:

benzyl 1-methylpiperidin-4-yl-2-((1*S*,5*R*)-3-(8-cyanoquinolin-5-yl)-5-(trifluoromethyl)-3-azabicyclo[3.1.0]hexane-1-carbonyl)hydrazine-1-carboxylate 15-3
1-methylpiperidin-4-yl-2-((1S,5R)-3-(8-cyanoquinolin-5-yl)-5-(trifluoromethyl)-3-azabicyclo[3.1.0]hexane-1-carbonyl)hydrazine-1-carboxylate

The crude product benzyl 1-methylpiperazine-4-hydrazinecarboxylate (1.00 g, crude) and (1S,5R)-3-(8-cyanoquinolin-5-yl)-5-(trifluoromethyl)-3-azabicyclo[3.1.0]hexane-1-carboxylic acid B-1 (250 mg, 0.72 mmol) were dissolved in 10.0 mL of phosphorus oxychloride. The mixture was refluxed at 90°C for 3 h, and sampled for LCMS analysis. After the reaction was completed, the mixture was quenched by adjusting the pH to 7-8 with saturated sodium hydroxide solution, lyophilized, and then dichloromethane was added. The mixture was filtered, and the organic phase was concentrated. The residue was purified by reverse-phase preparative chromatography to obtain the target product benzyl 1-methylpiperidin-4-yl-2-((1*S*,5*R*)-3-(8-cyanoquinolin-5-yl)-5-(trifluoromethyl)-3-azabicyclo[3.1.0]hexane-1-carbonyl)hydrazine-1-carboxylate 15-3 (yellow solid, 120 mg, yield: 33%).

### Step 3

5-((1*S*,5*R*)-1-(5-((1-methylpiperidin-4-yl)oxy)-1,3,4-oxadiazol-2-yl)-5-(trifluoromethyl)-3-azabicyclo[3.1.0]hexan-3-yl)quinoline-8-carbonitrile compound 15
5-((1S,5R)-1-(5-((1-methylpiperidin-4-yl)oxy)-1,3,4-oxadiazol-2-yl)-5-(trifluoromethyl)-3-azabicyclo[3.1.0]hexan-3-yl)quinoline-8-carbonitrile

Benzyl 1-methylpiperidin-4-yl-2-((1*S*,5*R*)-3-(8-cyanoquinolin-5-yl)-5-(trifluoromethyl)-3-azabicyclo[3.1.0]hexane-1-carbonyl)hydrazine-1-carboxylate 15-3 (90 mg, 0.19 mmol) was dissolved in 2.00 mL of acetonitrile, followed by the addition of cesium carbonate (200 mg, 0.57 mmol) and *p*-toluenesulfonyl chloride (113 mg, 0.57 mmol). The mixture was stirred at 80°C for 5 hours. After TLC confirmed the completion of the reaction, the reaction mixture was concentrated and purified by MPLC, followed by silica gel column chromatography to obtain the target product 55-((1S,5R)-1-(5-((1-methylpiperidin-4-yl)oxy)-1,3,4-oxadiazol-2-yl)-5-(trifluoromethyl)-3-azabicyclo[3.1.0]hexan-3-yl)quinoline-8-carbonitrile compound 15 (yellow solid, 20 mg, yield: 23%).

¹H NMR (400 MHz, DMSO-*d*₆) δ 9.02 (dd, 1H), 8.68 (dd, 1H), 8.20 (d, 1H), 7.62 (dd, 1H), 7.32 (d, 1H), 4.90 - 4.83 (m, 1H), 4.14 (d, 1H), 4.08 (d, 1H), 3.99 (d, 1H), 3.90 (d, 1H), 3.30 (s, 1H), 2.54 (s, 2H), 2.22 (s, 2H), 2.16 (d, 4H), 2.03 (s, 2H), 1.80 (d, 2H).

LC-MS m/z (ESI)= 485.10 [M+1].

### Example 16

2-(6-methyl-4-(trifluoromethyl)-pyridin-2-yl)-3-(4-(m-tolyl)-4H-1,2,4-triazol-3-yl)hexahydrocyclopenta[c]pyrrol-1(2H)-one compound 16
2-(6-methyl-4-(trifluoromethyl)-pyridin-2-yl)-3-(4-(m-tolyl)-4H-1,2,4-triazol-3-yl)hexahydrocyclopenta[c]pyrrol-1(2H)-one

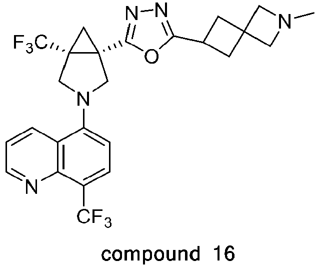

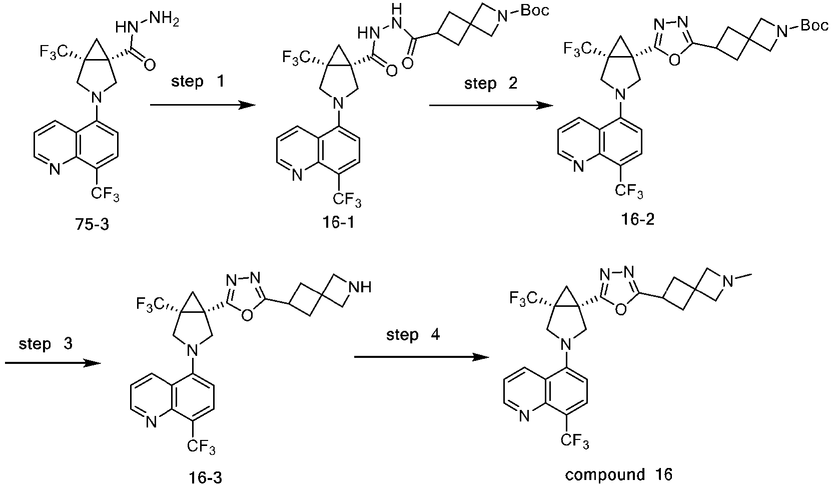

### Step 1:

*tert-*butyl 6-(2-((1*S*,5*R*)-5-(trifluoromethyl)-3-(8-(trifluoromethyl)quinolin-5-yl)-3-azabicyclo[3.1.0]hexane-1-carbonyl)hydrazine-1-carbonyl)-2-azaspiro[3.3]heptane-2-carboxylate compound 16-1
*tert*-butyl 6-(2-((1S,5R)-5-(trifluoromethyl)-3-(8-(trifluoromethyl)quinolin-5-yl)-3-azabicyclo[3.1.0]hexane-1-carbonyl)hydrazine-1-carbonyl)-2-azaspiro[3.3]heptane-2-carboxylate
2-(*tert*-Butoxycarbonyl)-2-azaspiro[3.3]heptane-6-carboxylic acid (180 mg, 0.75 mmol) was dissolved in anhydrous dichloromethane (10 mL), followed by the addition of DIPEA (193 mg, 1.5 mmol) and HATU (427 mg, 1.12 mmol). After activation at 40°C for 30 minutes, (1S,5R)-5-(trifluoromethyl)-3-(8-(trifluoromethyl)quinolin-5-yl)-3-azabicyclo[3.1.0]hexane-1-carbohydrazide 75-3 (303 mg, 0.75 mmol) was added. The mixture was reacted at room temperature under a nitrogen atmosphere for 1 hour. The mixture was extracted three times with water and dichloromethane. The organic phase was separated, collected, dried, and concentrated to obtain the crude product. The crude product was purified by column chromatography to obtain *tert*-butyl 6-(2-((1*S*,5*R*)-5-(trifluoromethyl)-3-(8-(trifluoromethyl)quinolin-5-yl)-3-azabicyclo[3.1.0]hexane-1-carbonyl)hydrazine-1-carbonyl)-2-azaspiro[3.3]heptane-2-carboxylate compound 16-1 (300 mg, yellow solid, yield: 63%).

LCMS m/z (ESI) = 628.23 [M+1].

### Step 2:

*tert*-butyl 6-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)-2-azaspiro[3.3]heptane-2-carboxylate compound 16-2
*tert*-butyl 6-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)-2-azaspiro[3.3]heptane-2-carboxylate
*tert*-Butyl 6-(2-((1*S*,5*R*)-5-(trifluoromethyl)-3-(8-(trifluoromethyl)quinolin-5-yl)-3-azabicyclo[3.1.0]hexane-1-carbonyl)hydrazine-1-carbonyl)-2-azaspiro[3.3]heptane-2-carboxylate 16-1 (300 mg, 0.47 mmol) was dissolved in anhydrous acetonitrile (6 mL), followed by the addition of cesium carbonate (311 mg, 0.95 mmol) and p-toluenesulfonyl chloride (181 mg, 0.95 mmol). The mixture was reacted at 80°C for 2 hours. The reaction mixture was then directly filtered, and the filter cake was washed twice with methanol. The filtrate was concentrated to dryness and then purified by column chromatography to obtain the target product *tert*-butyl 6-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)-2-azaspiro[3.3]heptane-2-carboxylate compound 16-2 (200 mg, pale yellow solid, yield: 69%).

LCMS m/z (ESI) = 610.22 [M+1].

### Step 3:

2-(2-azaspiro[3.3]heptan-6-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 16-3
2-(2-azaspiro[3.3]heptan-6-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole
*tert*-Butyl 6-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)-2-azaspiro[3.3]heptane-2-carboxylate 16-2 (200 mg, 0.32 mmol) was dissolved in anhydrous dichloromethane (4 mL), followed by the addition of trifluoroacetic acid (1 mL). The mixture was reacted at room temperature for 30 minutes. Subsequently, a saturated aqueous sodium carbonate solution was added to the reaction mixture to adjust the pH to alkaline. The mixture was extracted three times with dichloromethane and water. The organic phase was dried and concentrated to obtain the compound 2-(2-azaspiro[3.3]heptan-6-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 16-3 (130 mg, pale yellow solid, yield: 77%), which was directly used in the next step.

LCMS m/z = 510.17 [M+1].

### Step 4:

2-(2-methyl-2-azaspiro[3.3]heptan-6-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole compound 16
2-(2-methyl-2-azaspiro[3.3]heptan-6-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole
2-(2-Azaspiro[3.3]heptan-6-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole 16-3 (130 mg, 0.25 mmol) was dissolved in 5 mL of methanol, followed by the addition of 1 mL of triethylamine, paraformaldehyde (250 mg, 9 mmol), and sodium cyanoborohydride (506 mg, 13.5 mmol). The mixture was heated to 60°C and stirred for 3 hours. After TLC confirmed the completion of the reaction, the reaction mixture was directly filtered, concentrated, and purified by TLC (DCM: MeOH = 10:1) to obtain 2-(2-methyl-2-azaspiro[3.3]heptan-6-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 16 (pale yellow solid, 30 mg, 23.8%).

LC-MS m/z (ESI)= 524.10 [M+1].

¹H NMR (400 MHz, Chloroform-*d*) δ 9.00 (dt, 1H), 8.30 (dt, 1H), 7.93 (d, 1H), 7.44 (dd, 1H), 7.13 (dd, 1H), 4.10 (dd, 3H), 3.97 (d, 1H), 3.87 - 3.78 (m, 2H), 3.67 (t, 2H), 3.61 - 3.54 (m, 1H), 2.90 - 2.79 (m, 4H), 2.77 (s, 1H), 2.66 (dd, 2H), 2.24 (q, 2H).

### Example 17

2-(4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)piperidin-1-yl)acetamide compound 17
2-(4-(5-((1S,5R)-3-(8-cyanoquinolin-5-yl)-5-(trifluoromethyl)-3-azabicyclo[3.1.0]hexan-1-yl)-1,3,4-oxadiazol-2-yl)piperidin-1-yl)acetamide

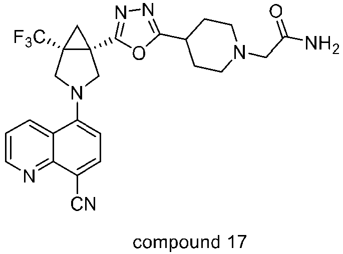

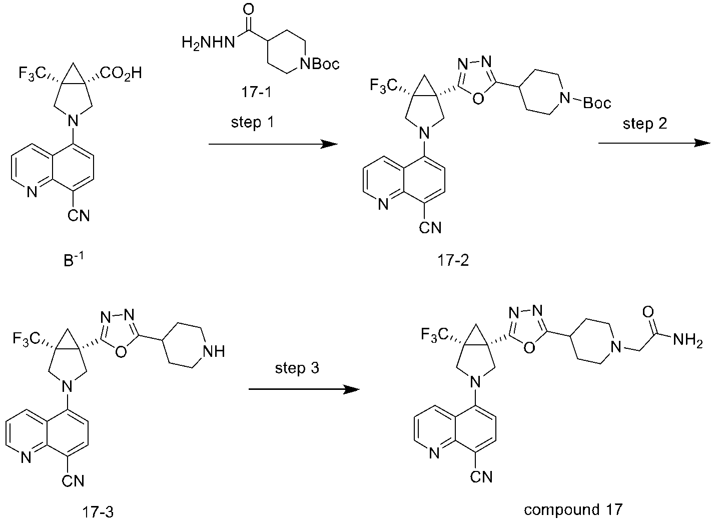

### Step 1:

5-(1*S*,5*R*)-1-(5-(1-(*tert*-butoxycarbonylpiperidin-4-yl)-1,3,4-oxadiazol-2-yl)-5-(trifluoromethyl)-3-azabicyclo[3.1.0]hexan-3-yl)quinoline-8-carbonitrile 17-2
tert-butyl 4-(5-((1S,5R)-3-(8-cyanoquinolin-5-yl)-5-(trifluoromethyl)-3-azabicyclo[3.1.0]hexan-1-yl)-1,3,4-oxadiazol-2-yl)piperidine-1-carboxylate
(1*S*,5*R*)-3-(8-Cyanoquinolin-5-yl)-5-(trifluoromethyl)-3-azabicyclo[3.1.0]hexane-1-carboxylic acid B-1 (250 mg, 0.718 mmol) was dissolved in 10 mL of dichloromethane, followed by the addition of HATU (411 mg, 1.08 mmol) and DIPEA (321 mg, 2.49 mmol). The mixture was stirred at low temperature for 5 minutes, and then 1-Boc-4-piperidinecarbohydrazide 17-1 (210 mg, 0.862 mmol) was added. After stirring for 2 hours, the completion of the reaction was confirmed by TLC. Subsequently, Burgess reagent (1100 mg, 3.9 mmol) was added, and the mixture was stirred for 3 hours. The completion of the reaction was confirmed by LCMS. The reaction mixture was directly concentrated and purified by reverse-phase C18 column chromatography (acidic method) to obtain the target product 5-(1*S*,5*R)*-1-(5-(1-(*tert*-butoxycarbonylpiperidin-4-yl)-1,3,4-oxadiazol-2-yl)-5-(trifluoromethyl)-3-azabicyclo[3.1.0]hexan-3-yl)quinoline-8-carbonitrile 17-2 (pale yellow solid, 380 mg crude).

### Step 2:

5-((1*S*,5*R*)-1-(5-(1-piperidin-4-yl)-1,3,4-oxadiazol-2-yl)-5-(trifluoromethyl)-3-azabicyclo[3.1.0]hexan-3-yl)quinoline-8-carbonitrile 17-3
5-((1S,5R)-1-(5-(piperidin-4-yl)-1,3,4-oxadiazol-2-yl)-5-(trifluoromethyl)-3-azabicyclo[3.1.0]hexan-3-yl)quinoline-8-carbonitrile

The crude product 5-(1*S*,5*R*)-1-(5-(1-(*tert*-butoxycarbonylpiperidin-4-yl)-1,3,4-oxadiazol-2-yl)-5-(trifluoromethyl)-3-azabicyclo[3.1.0]hexan-3-yl)quinoline-8-carbonitrile 17-2 (100 mg) was added to 2.00 mL of hydrogen chloride in ethyl acetate solution and stirred for half an hour. After LCMS confirmed the completion of the reaction, the reaction mixture was concentrated. The pH was adjusted to 7-8 with triethylamine, and the product 17-3 (pale yellow solid, 30 mg, 37%) was obtained by preparative TLC (dichloromethane: methanol = 5:1).

¹H NMR (400 MHz, DMSO-*d*₆) δ 9.02 (dd, 1H), 8.69 (dd, 1H), 8.20 (d, 1H), 7.63 (dd, 1H), 7.33 (d, 1H), 4.19 (d, 1H), 4.07 (dd, 2H), 3.93 (d, 1H), 3.10 (ddd, 1H), 3.04 - 2.94 (m, 2H), 2.65 (td, 2H), 2.22 (s, 2H), 1.93 (dd, 2H), 1.88 (s, 1H), 1.70 - 1.56 (m, 2H).

¹⁹F NMR (376 MHz, DMSO-*d*₆) δ -64.11.

LC-MS m/z (ESI)= 455.10 [M+1].

### Step 3:

2-(4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)piperidin-1-yl)acetamide compound 17
2-(4-(5-((1S,5R)-3-(8-cyanoquinolin-5-yl)-5-(trifluoromethyl)-3-azabicyclo[3.1.0]hexan-1-yl)-1,3,4-oxadiazol-2-yl)piperidin-1-yl)acetamide

The product 5-((1S,5R)-1-(5-(1-piperidin-4-yl)-1,3,4-oxadiazol-2-yl)-5-(trifluoromethyl)-3-azabicyclo[3.1.0]hexan-3-yl)quinoline-8-carbonitrile 17-3 (120 mg, 0.263 mmol) was added to 3.00 mL of dichloroethane, followed by DBU (202 mg, 1.30 mmol) and bromoacetamide (178 mg, 1.30 mmol). The mixture was stirred at 60°C for 3 hours. After LCMS confirmed the completion of the reaction, the reaction mixture was directly concentrated and purified by reverse-phase C18 column chromatography (acid method) to obtain the target product 2-(4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)piperidin-1-yl)acetamide compound 17 (pale yellow solid, 30 mg, 22%).

LC-MS m/z (ESI)= 512.10 [M+1].

¹H NMR (400 MHz, DMSO-*d*₆) δ 9.03 (dd, 1H), 8.69 (dd, 1H), 8.21 (d, 1H), 7.63 (dd, 1H), 7.34 (d, 1H), 7.17 (d, 2H), 4.20 (d, 1H), 4.07 (dd, 2H), 3.93 (d, 1H), 2.97 (s, 1H), 2.85 (s, 2H), 2.79 (d, 2H), 2.30 - 2.16 (m, 3H), 1.97 (d, 2H), 1.80 (d, 2H), 1.23 (s, 1H).

### Example 18

5-((1S,5R)-1-(5-(2-methyl-2-azaspiro[3.3]heptan-6-yl)-1,3,4-oxadiazol-2-yl)-5-(trifluoromethyl)-3-azabicyclo[3.1.0]hexan-3-yl)quinoline-8-carbonitrile compound 18
5-((1S,5R)-1-(5-(2-methyl-2-azaspiro[3.3]heptan-6-yl)-1,3,4-oxadiazol-2-yl)-5-(trifluoromethyl)-3-azabicyclo[3.1.0]hexan-3-yl)quinoline-8-carbonitrile

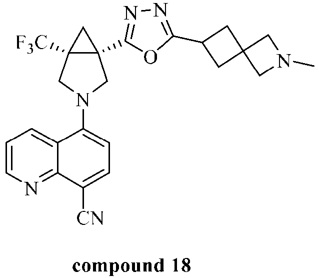

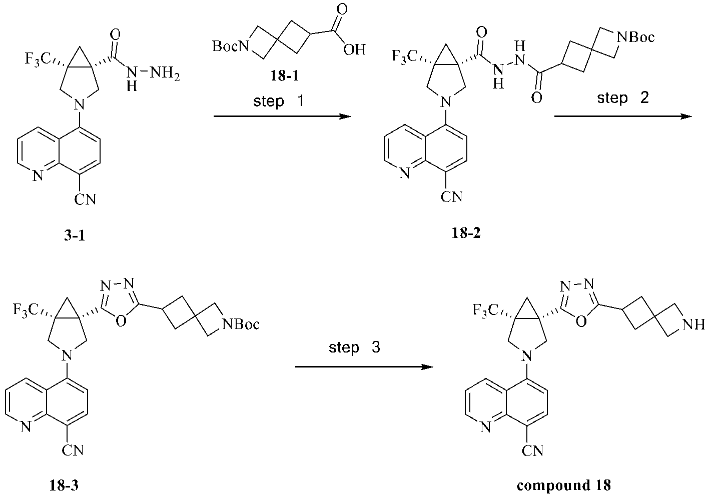

### Step 1:

*tert*-butyl 6-(2-((1S,5R)-3-(8-cyanoquinolin-5-yl)-5-(trifluoromethyl)-3-azabicyclo[3.1.0]hexane-1-carbonyl)hydrazine-1-carbonyl)-2-azaspiro[3.3]heptane-2-carboxylate 18-2
tert-butyl 6-(2-((1S,5R)-3-(8-cyanoquinolin-5-yl)-5-(trifluoromethyl)-3-azabicyclo[3.1.0]hexane-1-carbonyl)hydrazine-1-carbonyl)-2-azaspiro[3.3]heptane-2-carboxylate

The starting material 2-(*tert*-butoxycarbonyl)-2-azaspiro[3.3]heptane-6-carboxylic acid 18-1 (724 mg, 1.5 mmol) was added to *N,N-*dimethylformamide (5 mL), followed by the addition of *N,N,N',N'*-tetramethyl-*O*-(7-azabenzotriazol-1-yl)uronium hexafluorophosphate (1140 mg, 1.5 mmol) and N,N-diisopropylethylamine (516 mg, 2.0 mmol). The reaction mixture was stirred at room temperature for 0.5 hours, and then (1S,5R)-3-(8-cyanoquinolin-5-yl)-5-(trifluoromethyl)-3-azabicyclo[3.1.0]hexane-1-carbohydrazide 3-1 (722 mg, 2.0 mmol) was added. The reaction mixture was stirred at room temperature for 6 hours and then purified by reverse-phase column chromatography to obtain *tert*-butyl 6-(2-((1S,5R)-3-(8-cyanoquinolin-5-yl)-5-(trifluoromethyl)-3-azabicyclo[3.1.0]hexane-1-carbonyl)hydrazine-1-carbonyl)-2-azaspiro[3.3]heptane-2-carboxylate 18-2 (980 mg, yield: 83.8%).

LCMS m/z(ESI)=585.2[M+1].

### Step 2:

*tert*-butyl 6-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-2-azaspiro[3.3]heptane-2-carboxylate 18-3
*tert*-butyl 6-(5-((1S,5R)-3-(8-cyanoquinolin-5-yl)-5-(trifluoromethyl)-3-azabicyclo[3.1.0]hexan-1-yl)-1,3,4-oxadiazol-2-yl)-2-azaspiro[3.3]heptane-2-carboxylate
**tert**-Butyl 6-(2-((1S,5R)-3-(8-cyanoquinolin-5-yl)-5-(trifluoromethyl)-3-azabicyclo[3.1.0]hexane-1-carbonyl)hydrazine-1-carbonyl)-2-azaspiro[3.3]heptane-2-carboxylate 18-2 (980 mg, 1.68 mmol) was dissolved in 10 mL of dichloromethane, followed by the addition of triethylamine (849 mg, 8.40 mmol) and *p*-toluenesulfonyl chloride (962 mg, 5.04 mmol). The mixture was stirred at room temperature for 5 hours. After TLC confirmed the completion of the reaction, the reaction mixture was concentrated and purified by silica gel column chromatography to obtain the target product tert-butyl 6-(5-((1S,5R)-3-(8-cyanoquinolin-5-yl)-5-(trifluoromethyl)-3-azabicyclo[3.1.0]hexan-1-yl)-1,3,4-oxadiazol-2-yl)-2-azaspiro[3.3]heptane-2-carboxylate 18-3 (yellow solid, 675 mg, yield: 70.9%).

LC-MS m/z (ESI)= 567.2 [M+1].

### Step 3:

5-((1*S*,5*R*)-1-(5-(2-azaspiro[3.3]heptan-6-yl)-1,3,4-oxadiazol-2-yl)-5-(trifluoromethyl)-3-azabicyclo[3.1.0]hexan-3-yl)quinoline-8-carbonitrile compound 18
5-((1S,5R)-1-(5-(2-azaspiro[3.3]heptan-6-yl)-1,3,4-oxadiazol-2-yl)-5-(trifluoromethyl)-3-azabicyclo[3.1.0]hexan-3-yl)quinoline-8-carbonitrile

The starting material *tert*-butyl 6-(5-((1S,5R)-3-(8-cyanoquinolin-5-yl)-5-(trifluoromethyl)-3-azabicyclo[3.1.0]hexan-1-yl)-1,3,4-oxadiazol-2-yl)-2-azaspiro[3.3]heptane-2-carboxylate 18-3 (675 mg, 1.19 mmol) was added to dichloromethane (6 mL), followed by the addition of trifluoroacetic acid (3 mL). The reaction mixture was stirred at room temperature for 2 hours, concentrated under reduced pressure, and then purified by reverse-phase column chromatography to obtain 5-((1S,5R)-1-(5-(2-azaspiro[3.3]heptan-6-yl)-1,3,4-oxadiazol-2-yl)-5-(trifluoromethyl)-3-azabicyclo[3.1.0]hexan-3-yl)quinoline-8-carbonitrile compound 18 (430 mg, yield: 77.3%).

LC-MS m/z (ESI)= 467.2 [M+1].

¹H NMR (400 MHz, Chloroform-d) δ 9.09 (dd, 1H), 8.37 (dd, 1H), 8.05 (d, 1H), 7.54 (dd, 1H), 7.17 (d, 1H), 4.39 (s, 1H), 4.11 (d, 1H), 4.03 - 3.69 (m, 7H), 3.59 (m, 1H), 2.80 - 2.67 (m, 2H), 2.60 (h, 2H), 2.32 (d, 1H), 2.23 (d, 1H).

¹⁹F NMR (377 MHz, Chloroform-d) δ -65.21.

### Example 19

5-((1S,5R)-1-(5-(2-methyl-2-azaspiro[3.3]heptan-6-yl)-1,3,4-oxadiazol-2-yl)-5-(trifluoromethyl)-3-azabicyclo[3.1.0]hexan-3-yl)quinoline-8-carbonitrile compound 19
5-((1S,5R)-1-(5-(2-methyl-2-azaspiro[3.3]heptan-6-yl)-1,3,4-oxadiazol-2-yl)-5-(trifluoromethyl)-3-azabicyclo[3.1.0]hexan-3-yl)quinoline-8-carbonitrile 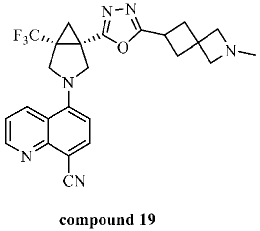 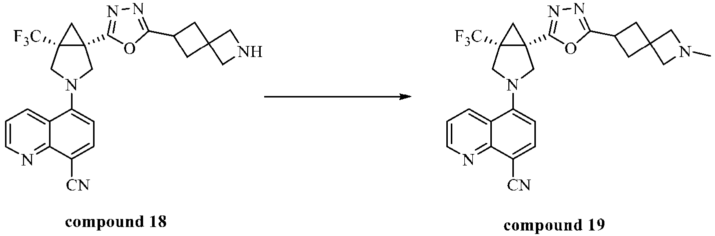
5-((1S,5R)-1-(5-(2-methyl-2-azaspiro[3.3]heptan-6-yl)-1,3,4-oxadiazol-2-yl)-5-(trifluoromethyl)-3-azabicyclo[3.1.0]hexan-3-yl)quinoline-8-carbonitrile compound 19
5-((1S,5R)-1-(5-(2-methyl-2-azaspiro[3.3]heptan-6-yl)-1,3,4-oxadiazol-2-yl)-5-(trifluoromethyl)-3-azabicyclo[3.1.0]hexan-3-yl)quinoline-8-carbonitrile
5-((1*S*,5*R*)-1-(5-(2-Azaspiro[3.3]heptan-6-yl)-1,3,4-oxadiazol-2-yl)-5-(trifluoromethyl)-3-azabicyclo[3.1.0]hexan-3-yl)quinoline-8-carbonitrile compound 18 (380 mg, 0.81 mmol) was dissolved in 5 mL of methanol, followed by the addition of paraformaldehyde (243 mg, 8.1 mmol) and sodium cyanoborohydride (1.02 g, 16.2 mmol). The mixture was stirred at room temperature for 3 hours. After TLC confirmed the completion of the reaction, the reaction mixture was directly concentrated and purified by reverse-phase column chromatography to obtain the target product 5-((1S,5R)-1-(5-(2-methyl-2-azaspiro[3.3]heptan-6-yl)-1,3,4-oxadiazol-2-yl)-5-(trifluoromethyl)-3-azabicyclo[3.1.0]hexan-3-yl)quinoline-8-carbonitrile compound 19 (pale yellow solid, 215 mg, *55.0%).*

LC-MS m/z (ESI)= 481.2 [M+1].

¹H NMR (400 MHz, DMSO-*d*₆) δ 9.02 (s, 1H), 8.67 (d, 1H), 8.19 (d, 1H), 7.62 (s, 1H), 7.32 (d, 1H), 4.05 (ddd, 4H), 3.63 (s, 1H), 3.10 (d, 4H), 2.34 (s, 4H), 2.21 (s, 2H), 2.13 (s, 3H).

¹⁹F NMR (377 MHz, DMSO-d6) δ -64.10.

### Example 20

5-((1*S*,5*R*)-1-(5-((1R,4S)-4-aminocyclohexyl)-1,3,4-oxadiazol-2-yl)-5-(trifluoromethyl)-3-azabicyclo[3.1.0]hexan-3-yl)quinoline-8-carbonitrile compound 20
5-((1S,5R)-1-(5-((1R,4S)-4-aminocyclohexyl)-1,3,4-oxadiazol-2-yl)-5-(trifluoromethyl)-3-azabicyclo[3.1.0]hexan-3-yl)quinoline-8-carbonitrile

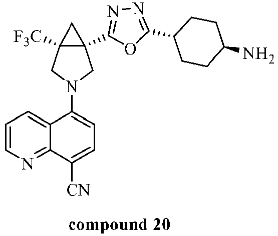

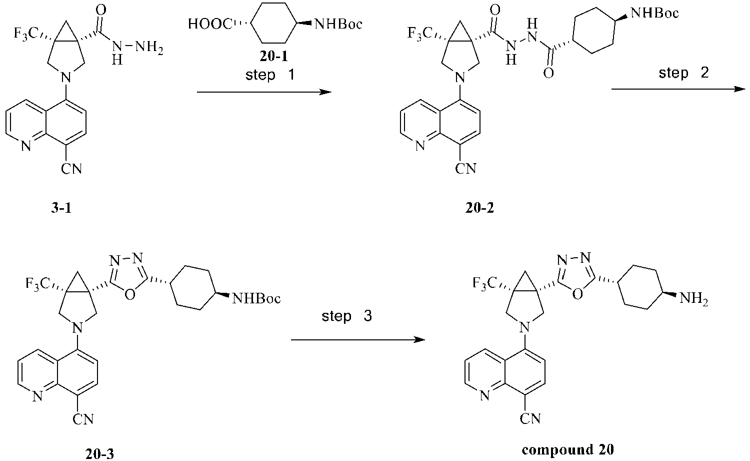

### Step 1:

*ter*t-butyl ((1*S,*4*R*)-4-(2-((1S,5R)-3-(8-cyanoquinolin-5-yl)-5-(trifluoromethyl)-3-azabicyclo[3.1.0]hexane-1-carbonyl)hydrazine-1-carbonyl)cyclohexyl)carbamate 20-2
tert-butyl ((1S,4R)-4-(2-((1S,5R)-3-(8-cyanoquinolin-5-yl)-5-(trifluoromethyl)-3-azabicyclo-[3.1.0]hexane-1-carbonyl)hydrazine-1-carbonyl)cyclohexyl)carbamate

The starting material (1r,4r)-4-((*tert*-butoxycarbonyl)amino)cyclohexane-1-carboxylic acid 20-1 (243 mg, 1.0 mmol) was added to N,N-dimethylformamide (5 mL), followed by the addition of *N,N,N',N'*-tetramethyl-*O*-(7-azabenzotriazol-1-yl)uronium hexafluorophosphate (570 mg, 1.5 mmol) and *N,N-*diisopropylethylamine (258 mg, 2.0 mmol). The reaction mixture was stirred at room temperature for 0.5 hours, and then (1S,5R)-3-(8-cyanoquinolin-5-yl)-5-(trifluoromethyl)-3-azabicyclo[3.1.0]hexane-1-carbohydrazide 3-1 (361 mg, 1.0 mmol) was added. The reaction mixture was stirred at room temperature for 6 hours and then purified by reverse-phase column chromatography to obtain *tert*-butyl *((1S,4R)-*4-(2-((1*S*,5*R*)-3-(8-cyanoquinolin-5-yl)-5-(trifluoromethyl)-3-azabicyclo[3.1.0]hexane-1-carbonyl)hydrazine-1-carbonyl)cyclohexyl)carbamate 20-2 (415 mg, yield: 70.7%).

LCMS m/z(ESI)=587.2[M+1].

### Step 2:

*tert*-butyl ((1*S*,4*R*)-4-(5-((1S,5R)-3-(8-cyanoquinolin-5-yl)-5-(trifluoromethyl)-3-azabicyclo[3.1.0]hexan-1-yl)-1,3,4-oxadiazol-2-yl)cyclohexyl)carbamate 20-3
tert-butyl ((1S,4R)-4-(5-((1S,5R)-3-(8-cyanoquinolin-5-yl)-5-(trifluoromethyl)-3-azabicyclo[3.1.0]hexan-1-yl)-1,3,4-oxadiazol-2-yl)cyclohexyl)carbamate
*tert-Butyl* ((1*S*,4*R*)-4-(2-((1*S*,5*R*)-3-(8-cyanoquinolin-5-yl)-5-(trifluoromethyl)-3-azabicyclo[3.1.0]hexane-1-carbonyl)hydrazine-1-carbonyl)cyclohexyl)carbamate 20-2 (415 mg, 0.71 mmol) was dissolved in 10 mL of dichloromethane, followed by the addition of triethylamine (338 mg, 3.35 mmol) and *p*-toluenesulfonyl chloride (382 mg, 2.01 mmol). The mixture was stirred at room temperature for 5 hours. After TLC confirmed the completion of the reaction, the reaction mixture was concentrated and purified by silica gel column chromatography to obtain the target product tert-butyl *(*(1*S*,4*R*)-4-(5-((1S,5R)-3-(8-cyanoquinolin-5-yl)-5-(trifluoromethyl)-3-azabicyclo[3.1.0]hexan-1-yl)-1,3,4-oxadiazol-2-yl)cyclohexyl)carbamate 20-3 (yellow solid, 323 mg, yield: 80.0%).

LC-MS m/z (ESI)= 569.2 [M+1].

### Step 3:

5-((1*S*,5*R*)-1-(5-((1R,4S)-4-aminocyclohexyl)-1,3,4-oxadiazol-2-yl)-5-(trifluoromethyl)-3-azabicyclo[3.1.0]hexan-3-yl)quinoline-8-carbonitrile compound 20
5-((1S,5R)-1-(5-((1R,4S)-4-aminocyclohexyl)-1,3,4-oxadiazol-2-yl)-5-(trifluoromethyl)-3-azabicyclo[3.1.0]hexan-3-yl)quinoline-8-carbonitrile

The starting material *tert*-butyl ((1S,4R)-4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)cyclohexyl)carbamate 20-3 (300 mg, 0.5 mmol) was added to dichloromethane (6 mL), followed by the addition of trifluoroacetic acid (3 mL). The reaction mixture was stirred at room temperature for 2 hours, concentrated under reduced pressure, and then purified by reverse-phase column chromatography to obtain 5-((1*S*,5*R*)-1-(5-((1*R*,4*S*)-4-aminocyclohexyl)-1,3,4-oxadiazol-2-yl)-5-(trifluoromethyl)-3-azabicyclo[3.1.0]hexan-3-yl)quinoline-8-carbonitrile compound 20 (215 mg, yield: 89.4%).

LC-MS m/z (ESI)= 469.2 [M+1].

¹H NMR (400 MHz, DMSO-*d*₆) δ 9.02 (dd, 1H), 8.68 (dd, 1H), 8.20 (d, 1H), 8.03 (s, 2H), 7.62 (dd, 1H), 7.33 (d, 1H), 4.19 (d, 1H), 4.06 (dd, 2H), 3.91 (d, 1H), 3.13 - 3.02 (m, 1H), 2.99 - 2.94 (m, 1H), 2.22 (s, 2H), 2.16 - 2.07 (m, 2H), 2.06 - 1.98 (m, 2H), 1.67 - 1.40 (m, 4H).

¹⁹F NMR (377 MHz, DMSO-*d*₆) δ -64.11.

### Example 21

5-((1S,5R)-1-(5-((1R,4S)-4-(dimethylamino)cyclohexyl)-1,3,4-oxadiazol-2-yl)-5-(trifluoromethyl)-3-azabicyclo[3.1.0]hexan-3-yl)quinoline-8-carbonitrile compound 21
5-((1S,5R)-1-(5-((1R,4S)-4-(dimethylamino)cyclohexyl)-1,3,4-oxadiazol-2-yl)-5-(trifluoromethyl)-3-azabicyclo[3.1.0]hexan-3-yl)quinoline-8-carbonitrile 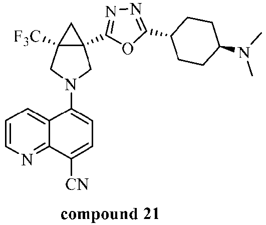 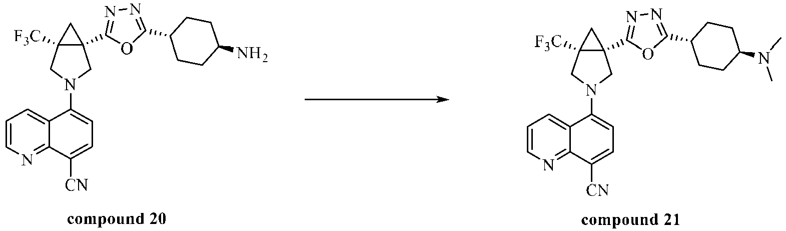
5-((1*S*,5*R*)-1-(5-((1*R*,4*S*)-4-Aminocyclohexyl)-1,3,4-oxadiazol-2-yl)-5-(trifluoromethyl)-3-azabicyclo[3.1.0]hexan-3-yl)quinoline-8-carbonitrile compound 20 (160 mg, 0.34 mmol) was dissolved in 5 mL of methanol, followed by the addition of paraformaldehyde (102 mg, 3.4 mmol) and sodium cyanoborohydride (427 mg, 6.8 mmol). The mixture was stirred at room temperature for 3 hours. After TLC confirmed the completion of the reaction, the reaction mixture was directly concentrated and purified by reverse-phase column chromatography to obtain the target product *5-((1S,5R)-1-(5-((1R,4S)-*4-(dimethylamino)cyclohexyl)-1,3,4-oxadiazol-2-yl)-5-(trifluoromethyl)-3-azabicyclo[3.1.0]hexan-3-yl)quinoline-8-carbonitrile compound 21 (pale yellow solid, 55 mg, 32.5%).

LC-MS m/z (ESI)= 497.2 [M+1].

¹H NMR (400 MHz, DMSO-d6) δ 9.03 (dd, 1H), 8.69 (dd, 1H), 8.21 (d, 1H), 7.63 (dd, 1H), 7.33 (d, 1H), 4.19 (d, 1H), 4.06 (dd, 2H), 3.92 (d, 1H), 3.17 (s, 1H), 2.98 (d, 1H), 2.65 (s, 6H), 2.29 - 2.04 (m, 6H), 1.58 (q, 4H).

¹⁹F NMR (377 MHz, DMSO-d6) δ -64.09.

### Example 22

5-((1S,5R)-1-(5-(2,5-dihydro-1H-pyrrol-3-yl)-1,3,4-oxadiazol-2-yl)-5-(trifluoromethyl)-3-azabicyclo[3.1.0]hexan-3-yl)quinoline-8-carbonitrile compound 22
5-((1S,5R)-1-(5-(2,5-dihydro-1H-pyrrol-3-yl)-1,3,4-oxadiazol-2-yl)-5-(trifluoromethyl)-3-azabicyclo[3.1.0]hexan-3-yl)quinoline-8-carbonitrile

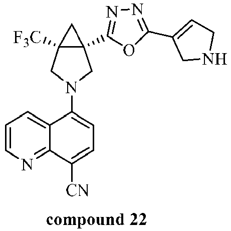

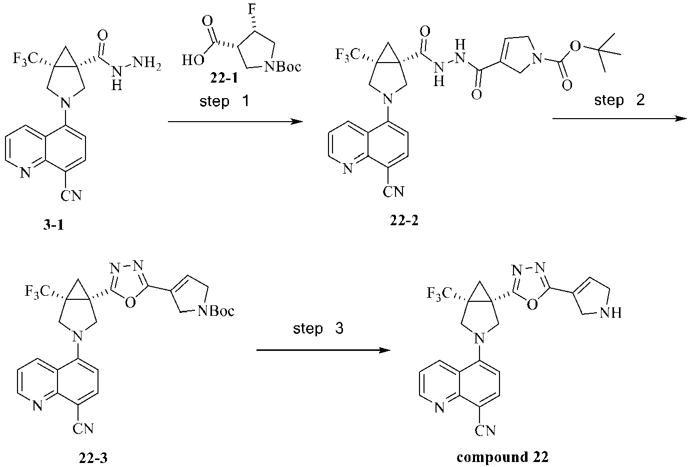

### Step 1:

*tert*-butyl 3-(2-((1*S*,5*R*)-3-(8-cyanoquinolin-5-yl)-5-(trifluoromethyl)-3-azabicyclo[3.1.0]hexane-1-carbonyl)hydrazine-1-carbonyl)-2,5-dihydro-1*H*-pyrrole-1-carboxylate 22-2
tert-butyl 3-(2-((1S,5R)-3-(8-cyanoquinolin-5-yl)-5-(trifluoromethyl)-3-azabicyclo[3.1.0]hexane-1-carbonyl)hydrazine-1-carbonyl)-2,5-dihydro-1H-pyrrole-1-carboxylate

The starting material (3*R*,4*S*)-1-(tert-butoxycarbonyl)-4-fluoropyrrolidine-3-carboxylic acid 22-1 (233 mg, 1.0 mmol) was added to *N,N*-dimethylformamide (3 mL), followed by the addition of *N,N,N',N'*-tetramethyl-*O*-(7-azabenzotriazol-1-yl)uronium hexafluorophosphate (570 mg, 1.5 mmol) and *N,N-*diisopropylethylamine (258 mg, 2.0 mmol). The reaction mixture was stirred at room temperature for 0.5 hours, and then (1*S*,5*R*)-3-(8-cyanoquinolin-5-yl)-5-(trifluoromethyl)-3-azabicyclo[3.1.0]hexane-1-carbohydrazide 3-1 (361 mg, 1.0 mmol) was added. The reaction mixture was stirred at room temperature for 6 hours and then purified by reverse-phase column chromatography to obtain tert-butyl 3-(2-((1S,5R)-3-(8-cyanoquinolin-5-yl)-5-(trifluoromethyl)-3-azabicyclo[3.1.0]hexane-1-carbonyl)hydrazine-1-carbonyl)-2,5-dihydro-1*H*-pyrrole-1-carboxylate 22-2 (375 mg, yield: 67.3%).

LCMS m/z(ESI)=557.2[M+1].

### Step 2:

*tert*-butyl 3-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-2,5-dihydro-1*H*-pyrrole-1-carboxylate 22-3
tert-butyl 3-(5-((1S,5R)-3-(8-cyanoquinolin-5-yl)-5-(trifluoromethyl)-3-azabicyclo[3.1.0]hexan-1-yl)-1,3,4-oxadiazol-2-yl)-2,5-dihydro-1H-pyrrole-1-carboxylate
*tert*-Butyl 3-(2-((1*S*,5*R*)-3-(8-cyanoquinolin-5-yl)-5-(trifluoromethyl)-3-azabicyclo[3.1.0]hexane-1-carbonyl)hydrazine-1-carbonyl)-2,5-dihydro-1*H*-pyrrole-1-carboxylate 22-2 (375 mg, 0.67 mmol) was dissolved in 10 mL of dichloromethane, followed by the addition of triethylamine (338 mg, 3.35 mmol) and *p*-toluenesulfonyl chloride (382 mg, 2.01 mmol). The mixture was stirred at room temperature for 5 hours. After TLC confirmed the completion of the reaction, the reaction mixture was concentrated and purified by silica gel column chromatography to obtain the target product *tert*-butyl *3-(5-((1S,5R)-3-(8-*cyanoquinolin-5-yl)-5-(trifluoromethyl)-3-azabicyclo[3.1.0]hexan-1-yl)-1,3,4-oxadiazol-2-yl)-2,5-dihydro-1*H*-pyrrole-1-carboxylate 22-3 (yellow solid, 215 mg, yield: 60.0%).

LC-MS m/z (ESI)= 539.2 [M+1].

### Step 3

5-((1*S*,5*R*)-1-(5-(2,5-dihydro-1*H*-pyrrol-3-yl)-1,3,4-oxadiazol-2-yl)-5-(trifluoromethyl)-3-azabicyclo[3.1.0]hexan-3-yl)quinoline-8-carbonitrile compound 22
5-((1S,5R)-1-(5-(2,5-dihydro-1H-pyrrol-3-yl)-1,3,4-oxadiazol-2-yl)-5-(trifluoromethyl)-3-azabicyclo[3.1.0]hexan-3-yl)quinoline-8-carbonitrile

The starting material *tert*-butyl 3-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-2,5-dihydro-1*H-*pyrrole-1-carboxylate 22-3 (200 mg, 0.37 mmol) was added to dichloromethane (6 mL), followed by the addition of trifluoroacetic acid (3 mL). The reaction mixture was stirred at room temperature for 2 hours, concentrated under reduced pressure, and then purified by reverse-phase column chromatography to obtain 5-((1*S*,5*R*)-1-(5-(2,5-dihydro-1*H*-pyrrol-3-yl)-1,3,4-oxadiazol-2-yl)-5-(trifluoromethyl)-3-azabicyclo[3.1.0]hexan-3-yl)quinoline-8-carbonitrile compound 22 (115 mg, yield: 71.0%).

LC-MS m/z (ESI)= 439.2 [M+1].

¹H NMR (400 MHz, MSO-*d*₆) δ 9.03 (s, 1H), 8.70 (d, 1H), 8.21 (d, 1H), 7.63 (dd, 1H), 7.35 (d, 1H), 6.89 (s, 1H), 4.27 - 3.80 (m, 8H), 2.40 - 2.15 (m, 2H).

¹⁹F NMR (377 MHz, DMSO-*d*₆) δ -63.92.

### Example 23

5-((1*S*,5*R*)-1-(1-(1-methylpiperidin-4-yl)-1H-1,2,4-triazol-3-yl)-5-(trifluoromethyl)-3-azabicyclo[3.1.0]hexan-3-yl)quinoline-8-carbonitrile compound 23
5-((1*S*,5*R*)-1-(1-(1-methylpiperidin-4-yl)-1H-1,2,4-triazol-3-yl)-5-(trifluoromethyl)-3-azabicyclo[3.1.0]hexan-3-yl)quinoline-8-carbonitrile

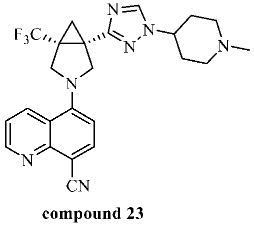

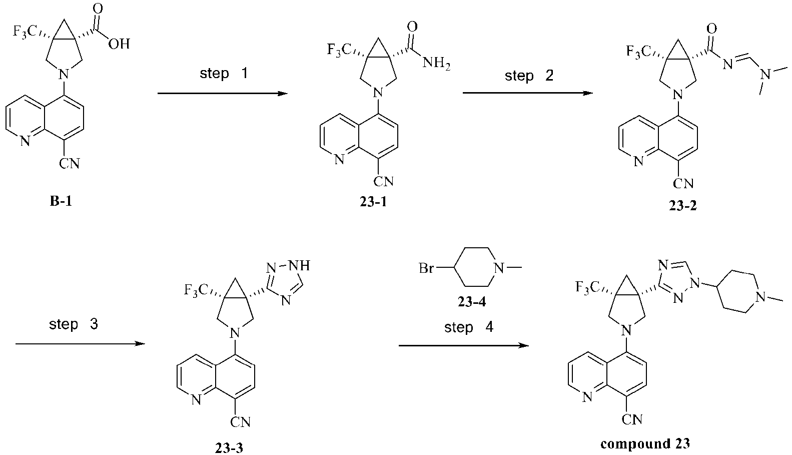

### Step 1:

(1*S*,5*R*)-3-(8-cyanoquinolin-5-yl)-5-(trifluoromethyl)-3-azabicyclo[3.1.0]hexane-1-carboxamide 23-1
(1S,5R)-3-(8-cyanoquinolin-5-yl)-5-(trifluoromethyl)-3-azabicyclo[3.1.0]hexane-1-carboxamide

The starting material (1*S*,5*R*)-3-(8-cyanoquinolin-5-yl)-5-(trifluoromethyl)-3-azabicyclo[3.1.0]hexane-1-carboxylic acid B-1 (1.0 g, 2.88 mmol) was added to dichloromethane (15 mL), followed by the addition of one drop of *N,N-*dimethylformamide. The mixture was cooled to 0°C, and oxalyl chloride (1.10 g, 8.64 mmol) was slowly added dropwise. The reaction was allowed to proceed without exothermic reaction, and the mixture was slowly warmed to room temperature. After stirring for 2 hours, the reaction was completed. The mixture was concentrated under reduced pressure to obtain a yellow solid, which was dissolved in dichloromethane and then added dropwise to a 0.4 mol/L ammonia solution in 1,4-dioxane (20 mL). The mixture was concentrated under reduced pressure, triturated with water, and filtered. The filter cake was concentrated to dryness to obtain the crude product (1*S*,5*R*)-3-(8-cyanoquinolin-5-yl)-5-(trifluoromethyl)-3-azabicyclo[3.1.0]hexane-1-carboxamide 23-1 (950 mg, yield: 95.3%), which was directly used in the next step.

LCMS m/z(ESI)=347.1[M+1].

### Step 2:

(1*S*,5*R*)-3-(8-cyanoquinolin-5-yl)-N-((E)-(dimethylamino)methylene)-5-(trifluoromethyl)-3-azabicyclo[3.1.0]hexane-1-carboxamide 23-2
(1S,5R)-3-(8-cyanoquinolin-5-yl)-N-((E)-(dimethylamino)methylene)-5-(trifluoromethyl)-3-azabicyclo[3.1.0]hexane-1-carboxamide

The starting material (1S,5R)-3-(8-cyanoquinolin-5-yl)-5-(trifluoromethyl)-3-azabicyclo[3.1.0]hexane-1-carboxamide 23-1 from the previous step (950 mg, 2.74 mmol) was added to 1,4-dioxane (10 mL), followed by the addition of *N,N-*dimethylformamide dimethyl acetal (980 mg, 8.22 mmol). The reaction mixture was heated to reflux and stirred for 3 hours. After the reaction was completed, the mixture was directly concentrated under reduced pressure. The crude product (1S,5R)-3-(8-cyanoquinolin-5-yl)-*N*-((*E*)-(dimethylamino)methylene)-5-(trifluoromethyl)-3-azabicyclo[3.1.0]hexane-1-carboxamide 23-2 (1.05 g, yield: 95.5%) was directly used in the next step.

LCMS m/z(ESI)=402.2[M+1].

### Step 3:

5-((1*S*,5*R*)-1-(1*H*-1,2,4-triazol-3-yl)-5-(trifluoromethyl)-3-azabicyclo[3.1.0]hexan-3-yl)quinoline-8-carbonitrile 23-3
5-((1S,5R)-1-(1H-1,2,4-triazol-3-yl)-5-(trifluoromethyl)-3-azabicyclo[3.1.0]hexan-3-yl)quinoline-8-carbonitrile

The starting material (1*S*,5*R*)-3-(8-cyanoquinolin-5-yl)-N-((E)-(dimethylamino)methylene)-5-(trifluoromethyl)-3-azabicyclo[3.1.0]hexane-1-carboxamide 23-2 (1.05 g, 2.61 mmol) from the previous step was added to acetic acid (10 mL), followed by the addition of hydrazine hydrate (262 mg, 5.22 mmol). After nitrogen protection, the temperature was raised to reflux, and the reaction was carried out for 3 hours. After the reaction was completed, the mixture was directly purified by acidic reverse-phase column chromatography to obtain 5-((1*S,5R*)-1-(1H-1,2,4-triazol-3-yl)-5-(trifluoromethyl)-3-azabicyclo[3.1.0]hexan-3-yl)quinoline-8-carbonitrile 23-3 (900 mg, yield: 93.0%).

LCMS m/z(ESI)=371.2[M+1].

### Step 4:

5-((1*S*,5*R*)-1-(1-(1-methylpiperidin-4-yl)-1H-1,2,4-triazol-3-yl)-5-(trifluoromethyl)-3-azabicyclo[3.1.0]hexan-3-yl)quinoline-8-carbonitrile compound 23
5-((1S,5R)-1-(1-(1-methylpiperidin-4-yl)-1H-1,2,4-triazol-3-yl)-5-(trifluoromethyl)-3-azabicyclo[3.1.0]hexan-3-yl)quinoline-8-carbonitrile

The starting material 5-((1S,5R)-1-(1H-1,2,4-triazol-3-yl)-5-(trifluoromethyl)-3-azabicyclo[3.1.0]hexan-3-yl)quinoline-8-carbonitrile compound 23-3 (100 mg, 0.27 mmol) was dissolved in *N,N-*dimethylformamide (2 mL), followed by the addition of cesium carbonate (176 mg, 0.54 mmol) and 23-4 (0.4 mmol). The reaction mixture was protected under nitrogen, heated to 100°C, and reacted for 8 hours. After the reaction was completed, the mixture was directly purified by column chromatography to obtain *5-*((*1S,5R*)-1-(1-(1-methylpiperidin-4-yl)-1*H*-1,2,4-triazol-3-yl)-5-(trifluoromethyl)-3-azabicyclo[3.1.0]hexan-3-yl)quinoline-8-carbonitrile compound 23 (60 mg, yield: 47.6%).

LCMS m/z(ESI)=468.2[M+1].

¹H NMR (400 MHz, DMSO-*d*₆) δ 9.00(d, 1H), 8.69(d, 1H), 8.56(s, 1H), 8.16(d, 1H), 7.60(dd, 1H), 7.27(d, 1H), 4.21(dq, 1H), 4.10(d, 2H), 4.03(d, 1H), 3.92(d, 1H), 2.84(d, 2H), 2.21(s, 3H), 2.18-1.89(m, 8H).

¹⁹F NMR (377 MHz, DMSO-d6) δ -63.33.

### Example 24

5-((1*S*,5*R*)-1-(5-(3-morpholinoprop-1-yn-1-yl)-1,3,4-oxadiazol-2-yl)-5-(trifluoromethyl)-3-azabicyclo[3.1.0]hexan-3-yl)quinoline-8-carbonitrile compound 24
5-((1*S*,5*R*)-1-(5-(3-morpholinoprop-1-yn-1-yl)-1,3,4-oxadiazol-2-yl)-5-(trifluoromethyl)-3-azabicyclo[3.1.0]hexan-3-yl)quinoline-8-carbonitrile

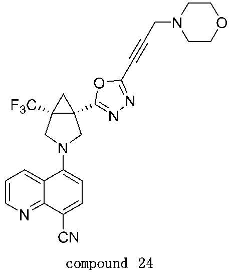

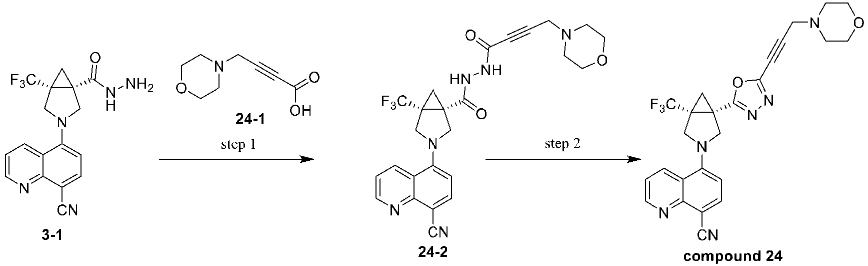

### Step 1:

(1*S*,5*R*)-3-(8-cyanoquinolin-5-yl)-*N'*-(4-morpholinobut-2-ynoyl)-5-(trifluoromethyl)-3-azabicyclo[3.1.0]hexane-1-carbohydrazide 24-2
(1S,5R)-3-(8-cyanoquinolin-5-yl)-N'-(4-morpholinobut-2-ynoyl)-5-(trifluoromethyl)-3-azabicyclo[3.1.0]hexane-1-carbohydrazide
(1*S*,5*R*)-3-(8-Cyanoquinolin-5-yl)-5-(trifluoromethyl)-3-azabicyclo[3.1.0]hexane-1-carbohydrazide 3-1 (200 mg, 0.55 mmol) was dissolved in 4 mL of DMF, followed by the addition of 4-morpholinobut-2-ynoic acid 24-1 (110 mg, 0.66 mmol), T₃P (262 mg, 0.83 mmol), and DIEA (210 mg, 1.65 mmol). The mixture was stirred at room temperature for 2 hours. After LCMS confirmed the completion of the reaction, the mixture was purified by reverse-phase C18 column chromatography (acidic method) to obtain the target compound (*1S,5R*)-3-(8-cyanoquinolin-5-yl)-*N*'-(4-morpholinobut-2-ynoyl)-5-(trifluoromethyl)-3-azabicyclo[3.1.0]hexane-1-carbohydrazide 24-2 (orange solid, 150 mg).

LC-MS m/z (ESI) = 513.10 [M+H]⁺.

### Step 2:

5-((1*S*,5*R*)-1-(5-(3-morpholinoprop-1-yn-1-yl)-1,3,4-oxadiazol-2-yl)-5-(trifluoromethyl)-3-azabicyclo[3.1.0]hexan-3-yl)quinoline-8-carbonitrile compound 24
5-((1S,5R)-1-(5-(3-morpholinoprop-1-yn-1-yl)-1,3,4-oxadiazol-2-yl)-5-(trifluoromethyl)-3-azabicyclo[3.1.0]hexan-3-yl)quinoline-8-carbonitrile
(1*S*,5*R*)-3-(8-Cyanoquinolin-5-yl)-N'-(4-morpholinobut-2-ynoyl)-5-(trifluoromethyl)-3-azabicyclo[3.1.0]hexane-1-carbohydrazide 24-2 (150 mg, 0.3 mmol) was dissolved in 5 mL of 1,4-dioxane, followed by the addition of TsCl (75 mg, 0.4 mmol) and Cs₂CO₃ (260 mg, 0.8 mmol). The mixture was stirred at room temperature under an N₂ atmosphere for 3 hours. After LCMS confirmed the completion of the reaction, the reaction mixture was directly concentrated and purified by reverse-phase C18 column chromatography (alkaline method) to obtain the target compound 5-((1*S*,5*R*)-1-(5-(3-morpholinoprop-1-yn-1-yl)-1,3,4-oxadiazol-2-yl)-5-(trifluoromethyl)-3-azabicyclo[3.1.0]hexan-3-yl)quinoline-8-carbonitrile compound 24 (off-white solid, 50 mg, 41%).

¹H NMR (400 MHz, DMSO-*d₆*) δ 9.03 (dd, 1H), 8.68 (dd, 1H), 8.21 (d, 1H), 7.64 (dd, 1H), 7.34 (d, 1H), 4.19 (d, 1H), 4.09 (t, 2H), 3.94 (d, 1H), 3.75 (s, 2H), 3.61 (t, 4H), 2.52 (d, 4H), 2.32 - 2.23 (m, 2H).

LC-MS m/z (ESI) = 495.10 [M+H]⁺.

### Example 25

5-((1S,5R)-1-(5-(3-(dimethylamino)prop-1-yn-1-yl)-1,3,4-oxadiazol-2-yl)-5-(trifluoromethyl)-3-azabicyclo[3.1.0]hexan-3-yl)quinoline-8-carbonitrile compound 25
5-((1S,5R)-1-(5-(3-(dimethylamino)prop-1-yn-1-yl)-1,3,4-oxadiazol-2-yl)-5-(trifluoromethyl)-3-azabicyclo[3.1.0]hexan-3-yl)quinoline-8-carbonitrile

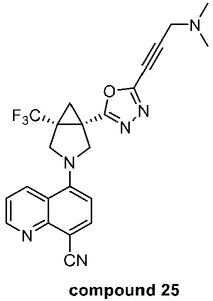

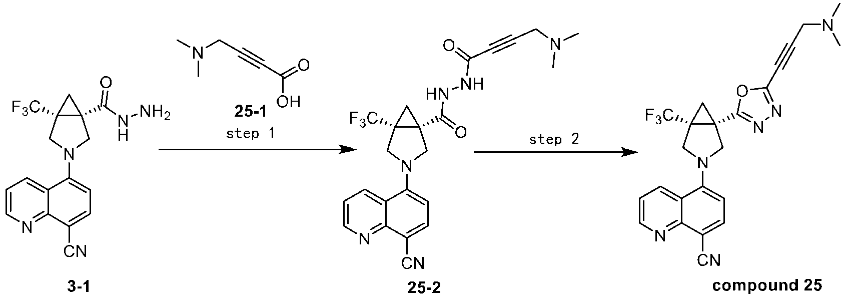

### Step 1:

(1*S*,5*R*)-3-(8-cyanoquinolin-5-yl)-N'-(4-(dimethylamino)but-2-ynoyl)-5-(trifluoromethyl)-3-azabicyclo[3.1.0]hexane-1-carbohydrazide 25-2
(1S,5R)-3-(8-cyanoquinolin-5-yl)-N'-(4-(dimethylamino)but-2-ynoyl)-5-(trifluoromethyl)-3-azabicyclo[3.1.0]hexane-1-carbohydrazide
(1*S*,5*R*)-3-(8-Cyanoquinolin-5-yl)-5-(trifluoromethyl)-3-azabicyclo[3.1.0]hexane-1-carbohydrazide 3-1 (400 mg, 1.1 mmol) was dissolved in 4 mL of DMF, followed by the addition of 4-(dimethylamino)-2-butynoic acid 25-2 (185 mg, 1.44 mmol), T₃P (1.06 mg, 1.66 mmol), and DIEA (430 mg, 3.32 mmol). The mixture was stirred at room temperature for 2 hours. After LCMS confirmed the completion of the reaction, the mixture was purified by reverse-phase C18 column chromatography (acidic method) to obtain the target compound (1*S*,5*R*)-3-(8-cyanoquinolin-5-yl)-*N*'-(4-(dimethylamino)but-2-ynoyl)-5-(trifluoromethyl)-3-azabicyclo[3.1.0]hexane-1-carbohydrazide 25-2 (orange solid, 670 mg).

LC-MS m/z (ESI) = 471.10 [M+H]⁺.

### Step 2:

5-((1*S*,5*R*)-1-(5-(3-(dimethylamino)prop-1-yn-1-yl)-1,3,4-oxadiazol-2-yl)-5-(trifluoromethyl)-3-azabicyclo[3.1.0]hexan-3-yl)quinoline-8-carbonitrile compound 25
5-((1S,5R)-1-(5-(3-(dimethylamino)prop-1-yn-1-yl)-1,3,4-oxadiazol-2-yl)-5-(trifluoromethyl)-3-azabicyclo[3.1.0]hexan-3-yl)quinoline-8-carbonitrile
(1*S*,5*R*)-3-(8-Cyanoquinolin-5-yl)-*N*'-(4-(dimethylamino)but-2-ynoyl)-5-(trifluoromethyl)-3-azabicyclo[3.1.0]hexane-1-carbohydrazide 25-2 (370 mg, 0.8 mmol) was dissolved in 4 mL of dichloromethane, followed by the addition of Burgess reagent (565 mg, 2.36 mmol). The mixture was stirred at room temperature for 3 hours. After LCMS confirmed the completion of the reaction, the reaction mixture was directly concentrated and purified by reverse-phase C18 column chromatography (alkaline method) to obtain the target compound 5-((1*S*,5*R*)-1-(5-(3-(dimethylamino)prop-1-yn-1-yl)-1,3,4-oxadiazol-2-yl)-5-(trifluoromethyl)-3-azabicyclo[3.1.0]hexan-3-yl)quinoline-8-carbonitrile compound 25 (white solid, 13 mg).

¹H NMR (400 MHz, DMSO-*d₆*) δ 9.03 (dd, 1H), 8.68 (dd, 1H), 8.21 (d, 1H), 7.63 (dd, 1H), 7.34 (d, 1H), 4.20 (d, 1H), 4.09 (dd, 2H), 3.94 (d, 1H), 3.67 (s, 2H), 2.30 (d, 1H), 2.25 (s, 7H).

LC-MS m/z (ESI) = 453.10 [M+H]⁺.

### Example 26

5-((1*S*,5*R*)-1-(5-((2,2,6,6-tetramethylpiperidin-4-yl)methyl)-1,3,4-oxadiazol-2-yl)-5-(trifluoromethyl)-3-azabicyclo[3.1.0]hexan-3-yl)quinoline-8-carbonitrile compound 26
5-((1S,5R)-1-(5-((2,2,6,6-tetramethylpiperidin-4-yl)methyl)-1,3,4-oxadiazol-2-yl)-5-(trifluoromethyl)-3-azabicyclo[3.1.0]hexan-3-yl)quinoline-8-carbonitrile

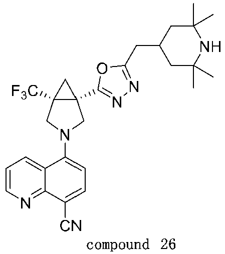

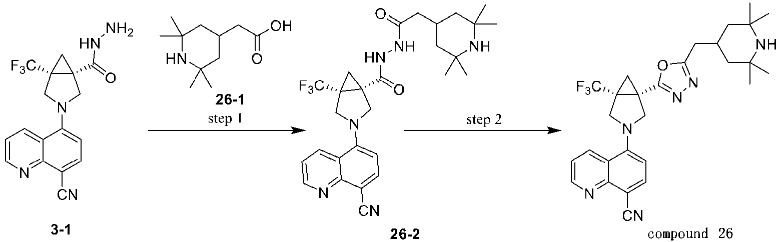

### Step 1:

(1*S*,5*R*)-3-(8-cyanoquinolin-5-yl)-N'-(2-(2,2,6,6-tetramethylpiperidin-4-yl)acetyl)-5-(trifluoromethyl)-3-azabicyclo[3.1.0]hexane-1-carbohydrazide 26-2
(1S,5R)-3-(8-cyanoquinolin-5-yl)-N'-(2-(2,2,6,6-tetramethylpiperidin-4-yl)acetyl)-5-(trifluoromethyl)-3-azabicyclo[3.1.0]hexane-1-carbohydrazide
(1*S*,5*R)*-3-(8-Cyanoquinolin-5-yl)-5-(trifluoromethyl)-3-azabicyclo[3.1.0]hexane-1-carbohydrazide 3-1 (170 mg, 0.5 mmol) was dissolved in 2 mL of DMF, followed by the addition of 2-(2,2,6,6-tetramethylpiperidin-4-yl)acetic acid 26-1 (180 mg, 0.94 mmol), T₃P (600 mg, 0.94 mmol), and DIEA (245 mg, 1.88 mmol). The mixture was stirred at room temperature for 2 hours. After LCMS confirmed the completion of the reaction, the mixture was purified by reverse-phase C18 column chromatography (acidic method) to obtain the target compound (1*S*,5*R*)-3-(8-cyanoquinolin-5-yl)-N'-(4-(dimethylamino)but-2-ynoyl)-5-(trifluoromethyl)-3-azabicyclo[3.1.0]hexane-1-carbohydrazide 26-2 (white solid, 1 g).

LC-MS m/z (ESI) = 543.20 [M+H]⁺.

### Step 2:

5-((1*S*,5*R*)-1-(5-((2,2,6,6-tetramethylpiperidin-4-yl)methyl)-1,3,4-oxadiazol-2-yl)-5-(trifluoromethyl)-3-azabicyclo[3.1.0]hexan-3-yl)quinoline-8-carbonitrile compound 26
5-((1S,5R)-1-(5-((2,2,6,6-tetramethylpiperidin-4-yl)methyl)-1,3,4-oxadiazol-2-yl)-5-(trifluoromethyl)-3-azabicyclo[3.1.0]hexan-3-yl)quinoline-8-carbonitrile
(1*S*,5*R*)-3-(8-Cyanoquinolin-5-yl)-N'-(4-(dimethylamino)but-2-ynoyl)-5-(trifluoromethyl)-3-azabicyclo[3.1.0]hexane-1-carbohydrazide 26-2 (1 g, 1.85 mmol) was dissolved in 10 mL of dichloromethane, followed by the addition of Burgess reagent (565 mg, 2.36 mmol). The mixture was stirred at room temperature for 3 hours. After LCMS confirmed the completion of the reaction, the reaction mixture was directly concentrated and purified by reverse-phase C18 column chromatography (alkaline method) to obtain the target compound 5-((1*S*,5*R*)-1-(5-((2,2,6,6-tetramethylpiperidin-4-yl)methyl)-1,3,4-oxadiazol-2-yl)-5-(trifluoromethyl)-3-azabicyclo[3.1.0]hexan-3-yl)quinoline-8-carbonitrile compound 26 (white solid, 36 mg).

¹H NMR (400 MHz, DMSO-*d₆*) δ 9.03 (dd, 1H), 8.69 (dd, 1H), 8.21 (d, 1H), 7.63 (dd, 1H), 7.33 (d, 1H), 4.20 (d, 1H), 4.07 (dd, 2H), 3.94 (d, 1H), 2.77 (d, 2H), 2.22 (s, 2H), 1.42 (d, 2H), 1.23 (s, 2H), 1.11 (s, 6H), 0.99 (s, 6H), 0.89 - 0.79 (m, 2H).

LC-MS m/z (ESI) = 525.20 [M+H]⁺.

### Example 27

5-((1S,5R)-1-(5-aminoquinoline-1,3,4-oxadiazol-2-yl)-5-(trifluoromethyl)-3-azabicyclo[3.1.0]hexan-3-yl)quinoline-8-carbonitrile compound 27
5-((1S,5R)-1-(5-morpholino-1,3,4-oxadiazol-2-yl)-5-(trifluoromethyl)-3-azabicyclo[3.1.0]hexan-3-yl)quinoline-8-carbonitrile

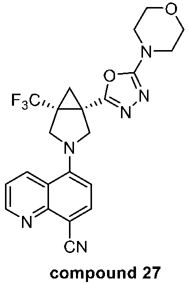

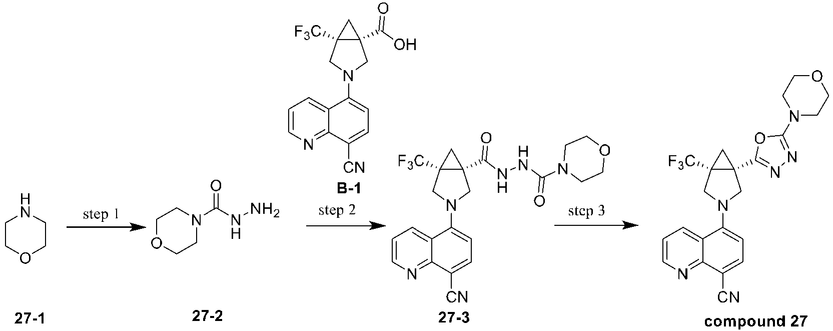

### Step 1:

morpholine-4-carbohydrazide 27-2
morpholine-4-carbohydrazide

Triphosgene (3.4 g, 11.5 mmol) was dissolved in 10 mL of dichloromethane, and the reaction system was purged with nitrogen. The solution was cooled to 0°C, and morpholine 27-1 (500 mg, 5.7 mmol) dissolved in 3 mL of dichloromethane was slowly added dropwise to the system. Triethylamine (3 g, 28.5 mmol) was then added, and the mixture was stirred at room temperature for 5 hours. Hydrazine hydrate (2.8 g, 57 mmol) was then added. After LCMS confirmed the completion of the reaction, the reaction mixture was directly concentrated to obtain the crude product morpholine-4-carbohydrazide 27-2 (pale yellow oil, 1 g).

LC-MS m/z (ESI) = 146.10 [M+H]⁺.

### Step 2:

*N*'-((1*S*,5*R*)-3-(8-cyanoquinolin-5-yl)-5-(trifluoromethyl)-3-azabicyclo[3.1.0]hexane-1-carbonyl)morpholine-4-carbohydrazide 27-3
N'-((1S,5R₎-3-(8-cyanoquinolin-5-yl)-5-(trifluoromethyl)-3-azabicyclo[3.1.0]hexane-1-carbonyl)morpholine-4-carbohydrazide
(1*S*,5*R*)-3-(8-Cyanoquinolin-5-yl)-5-(trifluoromethyl)-3-azabicyclo[3.1.0]hexane-1-carboxylic acid B-1 (200 mg, 0.6 mmol) was dissolved in 10 mL of DMF. HATU (460 mg, 1.2 mmol) and DIPEA (230 mg, 1.8 mmol) were then added under an ice bath, and the mixture was stirred at low temperature for 15 minutes. Morpholine-4-carbohydrazide 27-2 (1 g, 6.9 mmol) was then added, and the reaction was stirred for 1 hour. After LCMS confirmed the completion of the reaction, ethyl acetate and water were added for extraction. The organic phase was rotary evaporated to dryness to obtain the crude target product N'-((1S,5R)-3-(8-cyanoquinolin-5-yl)-5-(trifluoromethyl)-3-azabicyclo[3.1.0]hexane-1-carbonyl)morpholine-4-carbohydrazide 27-3 (pale yellow oil, 320 mg).

LC-MS m/z (ESI) = 475.10 [M+H]⁺.

### Step 3:

5-((1*S*,5*R*)-1-(5-aminoquinoline-1,3,4-oxadiazol-2-yl)-5-(trifluoromethyl)-3-azabicyclo[3.1.0]hexan-3-yl)quinoline-8-carbonitrile compound 27
5-((1*S*,5*R*)-1-(5-morpholino-1,3,4-oxadiazol-2-yl)-5-(trifluoromethyl)-3-azabicyclo[3.1.0]hexan-3-yl)quinoline-8-carbonitrile
*N*'-((1*S*,5*R*)-3-(8-Cyanoquinolin-5-yl)-5-(trifluoromethyl)-3-azabicyclo[3.1.0]hexane-1-carbonyl)morpholine-4-carbohydrazide 27-3 (320 mg, 0.65 mmol) was dissolved in 10 mL of 1,4-dioxane, followed by the addition of TsCl (250 mg, 1.3 mmol) and Cs₂CO₃ (640 mg, 1.95 mmol). The mixture was stirred at room temperature for 5 hours. After LCMS confirmed the completion of the reaction, the mixture was extracted with ethyl acetate and water. The organic phase was rotary evaporated to dryness, and the residue was purified by reverse-phase C18 column chromatography (alkaline method) to obtain the target compound 5-((1*S*,5*R*)-1-(5-aminoquinoline-1,3,4-oxadiazol-2-yl)-5-(trifluoromethyl)-3-azabicyclo[3.1.0]hexan-3-yl)quinoline-8-carbonitrile compound 27 (white solid, 25 mg).

¹H NMR (400 MHz, DMSO-*d₆*) δ 9.02 (dd, 1H), 8.69 (dd, 1H), 8.20 (d, 1H), 7.62 (dd, 1H), 7.32 (d, 1H), 4.13 (d, 1H), 4.07 (d, 1H), 3.99 (d, 1H), 3.89 (d, 1H), 3.73 - 3.65 (m, 4H), 3.45 - 3.38 (m, 4H), 2.20 - 2.11 (m, 2H).

LC-MS m/z (ESI) = 457.10 [M+H]⁺.

### Example 28

5-((1*S*,5*R*)-1-(5-((1-methylpiperidin-4-yl)oxy)-1,3,4-oxadiazol-2-yl)-5-(trifluoromethyl)-3-azabicyclo[3.1.0]hexan-3-yl)quinoline-8-carbonitrile compound 28
5-((1S,5R)-1-(5-((1-methylpiperidin-4-yl)oxy)-1,3,4-oxadiazol-2-yl)-5-(trifluoromethyl)-3-azabicyclo[3.1.0]hexan-3-yl)quinoline-8-carbonitrile

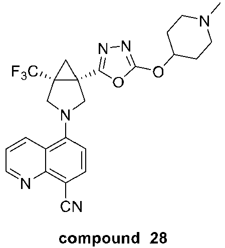

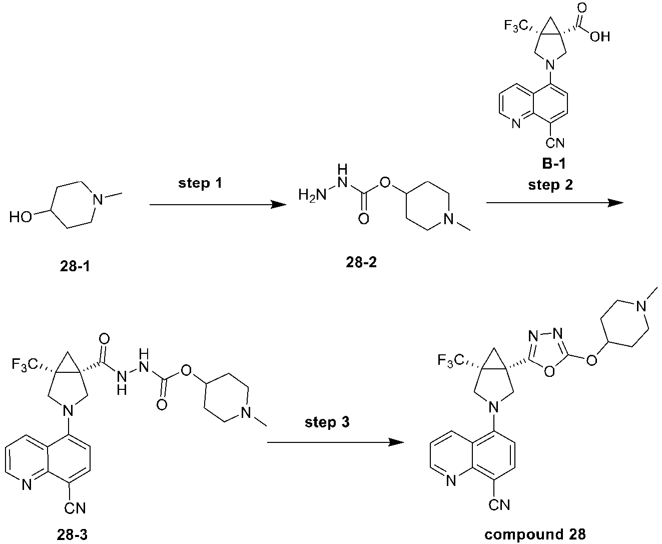

### Step 1:

1-methylpiperidin-4-yl hydrazinecarboxylate 28-2
1-methylpiperidin-4-yl hydrazinecarboxylate

Triphosgene (3.90 g, 13.2 mmol) was dissolved in 10.0 mL of dichloromethane. A mixed solution of 4-oxo-1-methylpiperidine 28-1 (1.00 g, 8.77 mmol) and triethylamine (1.75 g, 17.5 mmol) in 10.0 mL of dichloromethane was slowly added dropwise at 0°C. After reacting for 1 h, hydrazine hydrate (1.28 g, 40.0 mmol) was slowly added dropwise. The reaction was carried out for 1 h and then sampled. LCMS confirmed the completion of the reaction. The mixture was concentrated to obtain 4.3 g of the crude product 28-2 (yellow oil).

### Step 2:

1-methylpiperidin-4-yl-2-((1S,5R)-3-(8-cyanoquinolin-5-yl)-5-(trifluoromethyl)-3-azabicyclo[3.1.0]hexane-1-carbonyl)hydrazine-1-carboxylate 28-3
1-methylpiperidin-4-yl-2-((1S,5R)-3-(8-cyanoquinolin-5-yl)-5-(trifluoromethyl)-3-azabicyclo[3.1.0]hexane-1-carbonyl)hydrazine-1-carboxylate

The crude product 28-2 (1.00 g, crude) and (1S,5R)-3-(8-cyanoquinolin-5-yl)-5-(trifluoromethyl)-3-azabicyclo[3.1.0]hexane-1-carboxylic acid B-1 (250 mg, 0.72 mmol) were dissolved in 10.0 mL of phosphorus oxychloride. The mixture was refluxed at 90°C for 3 hours, and sampled for LCMS analysis. After the reaction was completed, the mixture was quenched by adjusting the pH to 7-8 with saturated sodium hydroxide solution, lyophilized, and then dichloromethane was added. The mixture was filtered, and the organic phase was concentrated. The residue was purified by reverse-phase preparative chromatography to obtain the target product 1-methylpiperidin-4-yl-2-((1S,5R)-3-(8-cyanoquinolin-5-yl)-5-(trifluoromethyl)-3-azabicyclo[3.1.0]hexane-1-carbonyl)hydrazine-1-carboxylate 28-3 (yellow solid, 120 mg, yield: 33%).

### Step 3

5-((1*S*,5*R*)-1-(5-((1-methylpiperidin-4-yl)oxy)-1,3,4-oxadiazol-2-yl)-5-(trifluoromethyl)-3-azabicyclo[3.1.0]hexan-3-yl)quinoline-8-carbonitrile compound 28
5-((1S,5R)-1-(5-((1-methylpiperidin-4-yl)oxy)-1,3,4-oxadiazol-2-yl)-5-(trifluoromethyl)-3-azabicyclo[3.1.0]hexan-3-yl)quinoline-8-carbonitrile
1-Methylpiperidin-4-yl-2-((1*S*,5*R*)-3-(8-cyanoquinolin-5-yl)-5-(trifluoromethyl)-3-azabicyclo[3.1.0]hexane-1-carbonyl)hydrazine-1-carboxylate 28-3 (90 mg, 0.19 mmol) was dissolved in 2.00 mL of acetonitrile, followed by the addition of cesium carbonate (200 mg, 0.57 mmol) and *p*-toluenesulfonyl chloride (113 mg, 0.57 mmol). The mixture was stirred at 80°C for 5 hours. After TLC confirmed the completion of the reaction, the reaction mixture was concentrated and purified by MPLC, followed by silica gel column chromatography to obtain the target product 55-((1S,5R)-1-(5-((1-methylpiperidin-4-yl)oxy)-1,3,4-oxadiazol-2-yl)-5-(trifluoromethyl)-3-azabicyclo[3.1.0]hexan-3-yl)quinoline-8-carbonitrile compound 28 (yellow solid, 20 mg, yield: 23%).

¹H NMR (400 MHz, DMSO-*d*₆) δ 9.02 (dd, 1H), 8.68 (dd, 1H), 8.20 (d, 1H), 7.62 (dd, , 1H), 7.32 (d, 1H), 4.90 - 4.83 (m, 1H), 4.14 (d, 1H), 4.08 (d, 1H), 3.99 (d, 1H), 3.90 (d, 1H), 3.30 (s, 1H), 2.54 (s, 2H), 2.22 (s, 2H), 2.16 (d, 4H), 2.03 (s, 2H), 1.80 (d, 2H).

LC-MS m/z (ESI)= 485.10 [M+1].

### Example 29

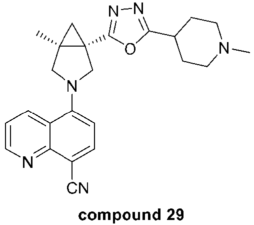

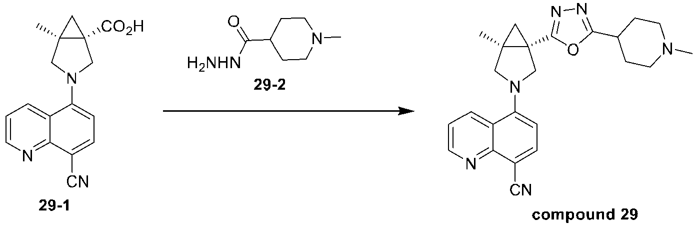

### Step 1:

5-((1*S*,5*S*)-1-methyl-5-(5-(1-methylpiperidin-4-yl)-1,3,4-oxadiazol-2-yl)-3-azabicyclo[3.1.0]hexan-3-yl)quinoline-8-carbonitrile compound 29
5-((1S,5S)-1-methyl-5-(5-(1-methylpiperidin-4-yl)-1,3,4-oxadiazol-2-yl)-3-azabicyclo[3.1.0]hexan-3-yl)quinoline-8-carbonitrile
4-Methylpiperidine-1-carbohydrazide 29-2 (59 mg, 0.375 mmol) and (1S,5R)-3-(8-cyanoquinolin-5-yl)-5-(methyl)-3-azabicyclo[3.1.0]hexane-1-carboxylic acid 29-1 (90 mg, 0.307 mmol) were dissolved in 10.0 mL of phosphorus oxychloride. The mixture was refluxed at 90°C for 3 hrs and sampled for LCMS analysis. After the reaction was completed, a saturated sodium hydroxide solution was added to adjust the pH to 7-8 for quenching. The mixture was lyophilized, and dichloromethane was added. The organic phase was filtered and concentrated. The residue was purified by reverse-phase preparative chromatography and then TLC (DCM: MeOH = 10:1) to obtain the target product *5-((1S,5R)-1-(5-(4-*methylpiperidin-1-yl)-1,3,4-oxadiazol-2-yl)-5-(methyl)-3-azabicyclo[3.1.0]hexan-3-yl)quinoxaline-5-carbonitrile compound 29 (pale yellow solid, 24 mg, 18.9%).

LC-MS m/z (ESI)= 415.10 [M+1].

¹H NMR (400 MHz, Chloroform-d) δ 9.05 (dd, 1H), 8.42 (dd, 1H), 8.00 (d, 1H), 7.47 (dd, 1H), 7.05 (d, 1H), 4.08 (d, 1H), 3.96 (d, 1H), 3.87 (d, 1H), 3.36 (d, 1H), 2.41 (s, 3H), 2.33 (m, 2H), 2.16 (m, 2H), 2.04 (m, 3H), 1.84 (d, 1H), 1.55 (d, 1H), 1.38 (s, 3H), 1.26 (m, 2H).

### Example 30

2-((1-methylazetidin-3-yl)methyl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 30
2-((1-methylazetidin-3-yl)methyl)-5-((1S,5R)-5-(trifluoromethyl)-3-(8-(trifluoromethyl)quinolin-5-yl)-3-azabicyclo[3.1.0]hexan-1-yl)-1,3,4-oxadiazole

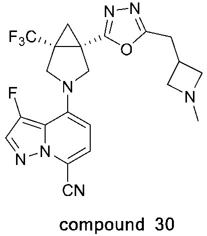

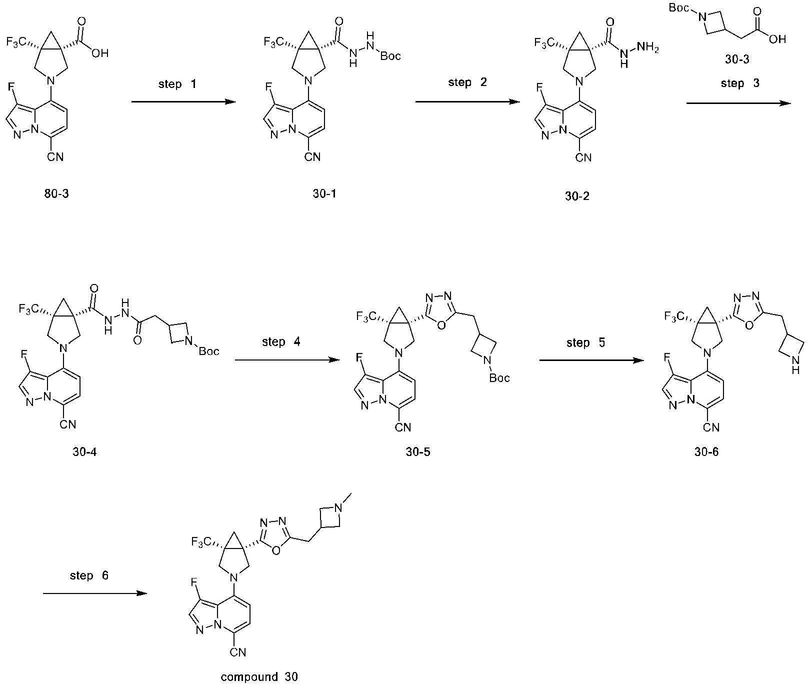

### Step 1:

*tert*-butyl 2-((1*S*,5*R*)-3-(7-cyano-3-fluoropyrazolo[1,5-a]pyridin-4-yl)-5-(trifluoromethyl)-3-azabicyclo[3.1.0]hexane-1-carbonyl)hydrazine-1-carboxylate compound 30-1
tert-butyl 2-((1S,5R)-3-(7-cyano-3-fluoropyrazolo[1,5-a]pyridin-4-yl)-5-(trifluoromethyl)-3-azabicyclo[3.1.0]hexane-1-carbonyl)hydrazine-1-carboxylate

Under an N₂ atmosphere, (1S,5R)-3-(3-fluoro-7-(trifluoromethyl)pyrazolo[1,5-*a*]pyridin-4-yl)-5-(trifluoromethyl)-3-azabicyclo[3.1.0]hexane-1-carboxylic acid 80-3 (1.00 g, 2.52 mmol) was dissolved in 20 mL of dichloromethane. Subsequently, n-butylphosphonic anhydride (3.04 g, 3.78 mmol), DIEA (974 mg, 7.56 mmol), and *tert*-butyl carbazate (368 mg, 2.77 mmol) were added sequentially. The mixture was stirred at room temperature for 2 h. The mixture was rotary evaporated to remove the solvent until dryness, added with ethyl acetate for extraction, and the organic phase was concentrated, washed with saturated brine, dried over anhydrous sodium sulfate, filtered, and rotary evaporated to remove the solvent until dryness. The residue was purified by MPLC to obtain the target product tert-butyl 2-((1S,5R)-3-(7-cyano-3-fluoropyrazolo[1,5-a]pyridin-4-yl)-5-(trifluoromethyl)-3-azabicyclo[3.1.0]hexane-1-carbonyl)hydrazine-1-carboxylate compound 30-1 (yellow solid, 1.23 g, 96%).

LC-MS m/z (ESI)= 469.10 [M+1].

### Step 2:

(1*S*,5*R*)-3-(7-cyano-3-fluoropyrazolo[1,5-a]pyridin-4-yl)-5-(trifluoromethyl)-3-azabicyclo[3.1.0]hexane-1-carbohydrazide compound 30-2
(1S,5R)-3-(7-cyano-3-fluoropyrazolo[1,5-a]pyridin-4-yl)-5-(trifluoromethyl)-3-azabicyclo[3.1.0]hexane-1-carbohydrazide

Under an N₂ atmosphere, -((1S,5R)-3-(7-cyano-3-fluoropyrazolo[1,5-a]pyridin-4-yl)-5-(trifluoromethyl)-3-azabicyclo[3.1.0]hexane-1-carbonyl)hydrazine-1-carboxylate compound 30-1 (yellow solid, 1.23 g) was dissolved in dichloromethane (20.0 mL), followed by the addition of trifluoroacetic acid (4 mL). The mixture was stirred at room temperature for 1 h. After LCMS confirmed the completion of the reaction, the mixture was rotary evaporated to remove the solvent until dryness. The residue was adjusted to pH = 7-8 with an aqueous sodium carbonate solution and extracted with ethyl acetate. The organic phase was concentrated, washed with saturated brine, dried over anhydrous sodium sulfate, filtered, and rotary evaporated to remove the solvent until dryness to obtain the target product *(1S,5R)-*3-(7-cyano-3-fluoropyrazolo[1,5-a]pyridin-4-yl)-5-(trifluoromethyl)-3-azabicyclo[3.1.0]hexane-1-carbohydrazide compound 30-2 (yellow solid, 850 mg, 86.7%).

LC-MS m/z (ESI)= 369.10 [M+1].

### Step 3:

*tert*-butyl 3-(2-(2-((1*S*,5*R*)-3-(7-cyano-3-fluoropyrazolo[1,5-a]pyridin-4-yl)-5-(trifluoromethyl)-3-azabicyclo[3.1.0]hexane-1-carbonyl)hydrazinyl)-2-oxoethyl)azetidine-1-carboxylate compound 30-4
tert-butyl 3-(2-(2-((1S,5R)-3-(7-cyano-3-fluoropyrazolo[1,5-a]pyridin-4-yl)-5-(trifluoromethyl)-3-azabicyclo[3.1.0]hexane-1-carbonyl)hydrazinyl)-2-oxoethyl)azetidine-1-carboxylate

Under an N₂ atmosphere, (1*S*,5*R*)-3-(7-cyano-3-fluoropyrazolo[1,5-a]pyridin-4-yl)-5-(trifluoromethyl)-3-azabicyclo[3.1.0]hexane-1-carbohydrazide 30-2 (500 mg, 1.35 mmol) was dissolved in 10 mL of DMF, followed by sequential addition of n-butylphosphonic anhydride (3.04 g, 3.78 mmol), DIEA (974 mg, 7.56 mmol), and 2-(1-(*tert*-butoxycarbonyl)azetidin-3-yl)acetic acid 30-3 (410 mg, 1.35 mmol). The mixture was stirred at room temperature for 2 h. After LCMS confirmed the completion of the reaction, the mixture was purified by MPLC to obtain the target product *tert*-butyl 3-(2-(2-((1S,5R)-3-(7-cyano-3-fluoropyrazolo[1,5-a]pyridin-4-yl)-5-(trifluoromethyl)-3-azabicyclo[3.1.0]hexane-1-carbonyl)hydrazinyl)-2-oxoethyl)azetidine-1-carboxylate compound 30-4 (yellow solid, 447 mg, 58.4%).

LC-MS m/z (ESI)= 566.2 [M+1].

### Step 4:

tert-butyl 3-((5-((1*S*,5*R*)-3-(7-cyano-3-fluoropyrazolo[1,5-a]pyridin-4-yl)-5-(trifluoromethyl)-3-azabicyclo[3.1.0]hexan-1-yl)-1,3,4-oxadiazol-2-yl)methyl)azetidine-1-carboxylate compound 30-5
tert-butyl 3-((5-((1S,5R)-3-(7-cyano-3-fluoropyrazolo[1,5-a]pyridin-4-yl)-5-(trifluoromethyl)-3-azabicyclo[3.1.0]hexan-1-yl)-1,3,4-oxadiazol-2-yl)methyl)azetidine-1-carboxylate

Under an N₂ atmosphere, 3-(2-(2-((1S,5R)-3-(7-cyano-3-fluoropyrazolo[1,5-a]pyridin-4-yl)-5-(trifluoromethyl)-3-azabicyclo[3.1.0]hexane-1-carbonyl)hydrazinyl)-2-oxoethyl)azetidine-1-carboxylate compound 30-4 (447 mg, 0.789 mmol) was dissolved in 20 mL of dichloromethane. Subsequently, cesium carbonate (772 mg, 2.36 mmol) and p-toluenesulfonyl chloride (302 mg, 1.58 mmol) were added sequentially, and the mixture was stirred at room temperature for 2 h. The mixture was rotary evaporated to remove the solvent until dryness, and the residue was purified by MPLC to obtain the target product *tert*-butyl 3-((5-((1*S*,5*R*)-3-(7-cyano-3-fluoropyrazolo[1,5-a]pyridin-4-yl)-5-(trifluoromethyl)-3-azabicyclo[3.1.0]hexan-1-yl)-1,3,4-oxadiazol-2-yl)methyl)azetidine-1-carboxylate compound 30-5 (yellow solid, 124 mg, 28.7%).

LC-MS m/z (ESI)= 548.10 [M+1].

### Step 5:

4-((1*S,*5*R*₎-1-(5-(azetidin-3-ylmethyl)-1,3,4-oxadiazol-2-yl)-5-(trifluoromethyl)-3-azabicyclo[3.1.0]hexan-3-yl)-3-fluoropyrazolo[1,5-a]pyridine-7-carbonitrile compound 30-6
4-((1S,5R)-1-(5-(azetidin-3-ylmethyl)-1,3,4-oxadiazol-2-yl)-5-(trifluoromethyl)-3-azabicyclo[3.1.0]hexan-3-yl)-3-fluoropyrazolo[1,5-a]pyridine-7-carbonitrile
Under an N₂ atmosphere, tert-butyl 3-((5-((1S,5R)-3-(7-cyano-3-fluoropyrazolo[1,5-a]pyridin-4-yl)-5-(trifluoromethyl)-3-azabicyclo[3.1.0]hexan-1-yl)-1,3,4-oxadiazol-2-yl)methyl)azetidine-1-carboxylate 30-5

124 mg was dissolved in dichloromethane (20.0 mL), followed by the addition of trifluoroacetic acid (4 mL). The mixture was stirred at room temperature for 1 h. After LCMS confirmed the completion of the reaction, the mixture was rotary evaporated to remove the solvent until dryness. The pH was adjusted to 7-8 with an aqueous sodium carbonate solution, and the mixture was extracted with ethyl acetate. The organic phase was concentrated, washed with saturated brine, dried over anhydrous sodium sulfate, filtered, and rotary evaporated to remove the solvent until dryness to obtain the target product 4-((1S,5R)-1-(5-(azetidin-3-ylmethyl)-1,3,4-oxadiazol-2-yl)-5-(trifluoromethyl)-3-azabicyclo[3.1.0]hexan-3-yl)-3-fluoropyrazolo[1,5-a]pyridine-7-carbonitrile compound 30-6 (yellow solid, 110 mg, 99%).

LC-MS m/z (ESI)= 448.10 [M+1].

### Step 6:

3-fluoro-4-((1*S*,5*R*)-1-(5-((1-methylazetidin-3-yl)methyl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 30
3-fluoro-4-((1S,5R)-1-(5-((1-methylazetidin-3-yl)methyl)-1,3,4-oxadiazol-2-yl)-5-(trifluoromethyl)-3-azabicyclo[3.1.0]hexan-3-yl)pyrazolo[1,5-*a*]pyridine-7-carbonitrile
4-((1*S*,5*R*)-1-(5-(Azetidin-3-ylmethyl)-1,3,4-oxadiazol-2-yl)-5-(trifluoromethyl)-3-azabicyclo[3.1.0]hexan-3-yl)-3-fluoropyrazolo[1,5-*a*]pyridine-7-carbonitrile compound 30-6 (110 mg, 0.33 mmol) was dissolved in 5 mL of methanol, followed by the addition of 1 mL of triethylamine, paraformaldehyde (250 mg, 9 mmol), and sodium cyanoborohydride (506 mg, 13.5 mmol). The mixture was heated to 60°C and stirred for 3 hours. After TLC confirmed the completion of the reaction, the reaction mixture was directly filtered, concentrated, and purified by TLC (DCM: MeOH = 10:1) to obtain 3-fluoro-4-((1*S*,5*R*)-1-(5-((1-methylazetidin-3-yl)methyl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 30 (pale yellow solid, 30 mg, 26.5%).

LC-MS m/z (ESI)= 462.10 [M+1].

¹H NMR (400 MHz, Chloroform-*d*) δ 7.86 (d, 1H), 7.15 - 7.13 (m, 1H), 6.16 (d, 1H), 4.08 (s, 2H), 4.04 (dd, 1H), 3.78 (d, 1H), 3.48 (t, 2H), 3.12 - 3.04 (m, 4H), 2.32 (s, 3H), 2.26 (d, 1H). 1.82 (m, 2H).

### Example 31

3-fluoro-4-((1*S*,5*R*)-1-(5-((1-methylpiperidin-4-yl)oxy)-1,3,4-oxadiazol-2-yl)-5-(trifluoromethyl)-3-azabicyclo[3.1.0]hexan-3-yl)pyrazolo[1,5-*a*]pyridine-7-carbonitrile compound 31
3-fluoro-4-((1S,5R)-1-(5-((1-methylpiperidin-4-yl)oxy)-1,3,4-oxadiazol-2-yl)-5-(trifluoromethyl)-3-azabicyclo[3.1.0]hexan-3-yl)pyrazolo[1,5-a]pyridine-7-carbonitrile

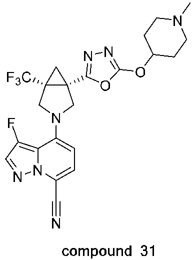

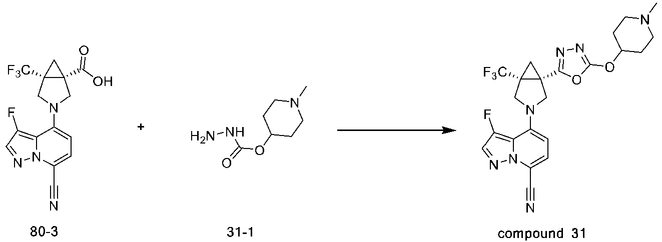

(1*S*,5*R*)-3-(7-Cyano-3-fluoropyrazolo[1,5-*a*]pyridin-4-yl)-5-(trifluoromethyl)-3-azabicyclo[3.1.0]hexane-1-carboxylic acid 80-3 (900 mg, 2.54 mmol) and 1-methylpiperidin-4-yl hydrazinecarboxylate 31-1 (5.5 g, crude) were dissolved in POCl₃ (10 mL) and reacted at 80°C for 2 hours. After LCMS confirmed the completion of the reaction, the pH was adjusted to alkaline with sodium carbonate. The mixture was extracted three times with dichloromethane and rotary evaporated to dryness. The residue was purified by reverse-phase chromatography and further purified by preparative TLC to obtain 3-fluoro-4-((1S,5R)-1-(5-((1-methylpiperidin-4-yl)oxy)-1,3,4-oxadiazol-2-yl)-5-(trifluoromethyl)-3-azabicyclo[3.1.0]hexan-3-yl)pyrazolo[1,5-a]pyridine-7-carbonitrile compound 31 (27 mg, yellow solid).

LCMS m/z (ESI) = 492.17 [M+1].

¹H NMR (400 MHz, Chloroform-*d*) δ 8.26 (s, 1H), 7.75 (d, 1H), 6.08 (d, 1H), 4.95 (s, 1H), 4.85 (d, 1H), 4.76 (d, 1H), 4.30 (d, 1H), 4.05 (d, 1H), 2.73 (s, 2H), 2.45 (s, 2H), 2.33 (d, 4H), 2.22 (s, 2H), 2.01 (s, 2H), 1.74 (d, 1H).

### Example 32:

5-((1*S*,5*R*)-1-(5-(3-fluoroazetidin-3-yl)-1,3,4-oxadiazol-2-yl)-5-(trifluoromethyl)-3-azabicyclo[3.1.0]hexan-3-yl)quinoline-8-carbonitrile compound 32
5-((1*S*,5*R*)-1-(5-(3-fluoroazetidin-3-yl)-1,3,4-oxadiazol-2-yl)-5-(trifluoromethyl)-3-azabicyclo[3.1.0]hexan-3-yl)quinoline-8-carbonitrile

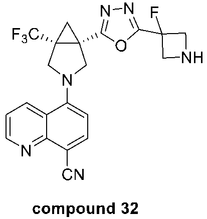

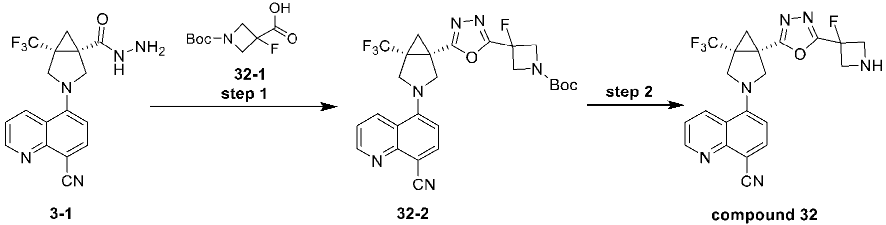

### Step 1:

*tert*-butyl 3-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-3-fluoroazetidine-1-carboxylate 32-2
5-((1S,5R)-1-(5-(3-fluoro-1-methylazetidin-3-yl)-1,3,4-oxadiazol-2-yl)-5-(trifluoromethyl)-3-azabicyclo[3.1.0]hexan-3-yl)quinoline-8-carbonitrile
1-(*tert*-Butoxycarbonyl)-3-fluoroazetidine-3-carboxylic acid 32-1 (479 mg, 2.2 mmol) was dissolved in 20 mL of dichloromethane, followed by the addition of HATU (1.136 g, 2.98 mmol) and DIPEA (766 mg, 5.94 mmol). The mixture was stirred at low temperature for 5 minutes, and then (1S,5R)-3-(8-cyanoquinolin-5-yl)-5-(trifluoromethyl)-3-azabicyclo[3.1.0]hexane-1-carbohydrazide 3-1 (720 mg, 1.98 mmol) was added. The reaction was stirred for 2 hours. After TLC confirmed the completion of the reaction, Burgess reagent (1100 mg, 3.9 mmol) was added, and the mixture was stirred for 3 hours. After LCMS confirmed the completion of the reaction, the reaction mixture was directly concentrated. The residue was purified by reverse-phase C18 column chromatography (acidic method) to obtain the target product *tert*-butyl 3-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-3-fluoroazetidine-1-carboxylate 32-2 (pale yellow solid, 500 mg, 46.2%).

### Step 2:

5-((1*S*,5*R*)-1-(5-(3-fluoroazetidin-3-yl)-1,3,4-oxadiazol-2-yl)-5-(trifluoromethyl)-3-azabicyclo[3.1.0]hexan-3-yl)quinoline-8-carbonitrile compound 32
5-((1*S*,5*R*)-1-(5-(3-fluoroazetidin-3-yl)-1,3,4-oxadiazol-2-yl)-5-(trifluoromethyl)-3-azabicyclo[3.1.0]hexan-3-yl)quinoline-8-carbonitrile

The crude product *tert*-butyl 3-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-3-fluoroazetidine-1-carboxylate (500 mg) was added to 5.00 mL of hydrogen chloride in ethyl acetate solution and stirred for half an hour. After LCMS confirmed the completion of the reaction, the reaction mixture was concentrated to obtain the product compound 32 (pale yellow solid, 400 mg, 97%).

LC-MS m/z (ESI)= 445.10 [M+1].

¹H NMR (400 MHz, Chloroform-d) δ 9.10 (d, 1H), 8.37 (d, 1H), 8.06 (d, 1H), 7.54 (dd, 1H), 7.19 (d, 1H), 4.33 - 4.20 (m, 3H), 4.17 (d, 1H), 3.99 (dd, 2H), 3.81 (d, 1H), 2.46 - 2.25 (m, 4H).

### Example 33:

5-((1*S*,5*R*)-1-(5-(3-fluoroazetidin-3-yl)-1,3,4-oxadiazol-2-yl)-5-(trifluoromethyl)-3-azabicyclo[3.1.0]hexan-3-yl)quinoline-8-carbonitrile compound 33
5-((1*S*,5*R*)-1-(5-(3-fluoroazetidin-3-yl)-1,3,4-oxadiazol-2-yl)-5-(trifluoromethyl)-3-azabicyclo[3.1.0]hexan-3-yl)quinoline-8-carbonitrile

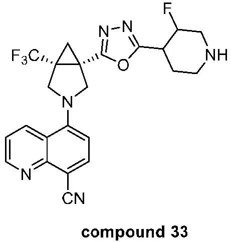

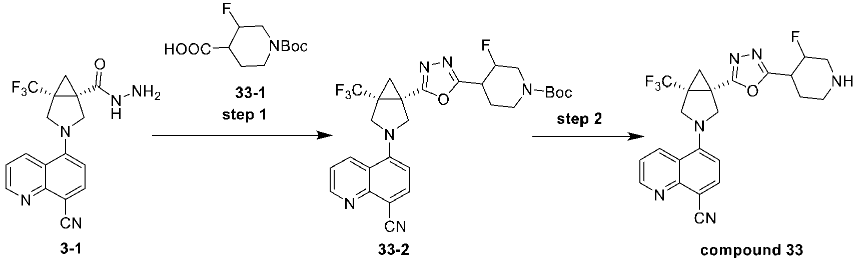

### Step 1:

*ter*t-butyl 4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-3-fluoropiperidine-1-carboxylate 33-2
tert-butyl4-(5-((1S,5R)-3-(8-cyanoquinolin-5-yl)-5-(trifluoromethyl)-3-azabicyclo[3.1.0]hexan-1-yl)-1,3,4-oxadiazol-2-yl)-3-fluoropiperidine-1-carboxylate
1-(*tert*-Butoxycarbonyl)-3-fluoropiperidine-4-carboxylic acid 33-1 (543 mg, 2.2 mmol) was dissolved in 20 mL of dichloromethane, followed by the addition of HATU (789 mg, 2.07 mmol) and DIPEA (534 mg, 4.14 mmol). The mixture was stirred at low temperature for 5 minutes, and then (1S,5R)-3-(8-cyanoquinolin-5-yl)-5-(trifluoromethyl)-3-azabicyclo[3.1.0]hexane-1-carbohydrazide 3-1 (500 mg, 1.98 mmol) was added. The reaction was stirred for 2 hours. After TLC confirmed the completion of the reaction, Burgess reagent (1.53 g, 5.52 mmol) was added, and the mixture was stirred for 3 hours. After LCMS confirmed the completion of the reaction, the reaction mixture was directly concentrated. The residue was purified by reverse-phase C18 column chromatography (acidic method) to obtain the target product *tert*-butyl 3-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-3-fluoroazetidine-1-carboxylate 33-2 (pale yellow solid, 500 mg, 63.2%).

### Step 2:

5-((1*S*,5*R*)-1-(5-(3-fluoroazetidin-3-yl)-1,3,4-oxadiazol-2-yl)-5-(trifluoromethyl)-3-azabicyclo[3.1.0]hexan-3-yl)quinoline-8-carbonitrile compound 33
5-((1S,5R)-1-(5-(3-fluoroazetidin-3-yl)-1,3,4-oxadiazol-2-yl)-5-(trifluoromethyl)-3-azabicyclo[3.1.0]hexan-3-yl)quinoline-8-carbonitrile

The crude product *tert*-butyl 3-(5-((1*S*_{,}5*R*)-3-(8-cyanoquinolin-5-yl)-5-(trifluoromethyl)-3-azabicyclo[3.1.0]hexan-1-yl)-1,3,4-oxadiazol-2-yl)-3-fluoroazetidine-1-carboxylate 33-2 (500 mg) was added to 5.00 mL of hydrogen chloride in ethyl acetate solution and stirred for half an hour. After LCMS confirmed the completion of the reaction, the reaction mixture was concentrated to obtain the product compound 33 (pale yellow solid, 380 mg, 92%).

LC-MS m/z (ESI)= 473.10 [M+1].

¹H NMR (400 MHz, Chloroform-d) δ 9.09 (dd, 1H), 8.37 (dd, 1H), 8.05 (d, 1H), 7.53 (dd, 1H), 7.17 (d, 1H), , 4.13 (dd, 1H), 3.98 (dd, 2H), 3.81 (dd, 1H), 3.44 - 3.20 (m, 3H), 2.91 (dd, 1H), 2.78 (t, 1H), 2.33 (dd, 1H), 2.26 - 2.11 (m, 2H), 2.04 - 1.93 (m, 1H), 1.64 (s, 2H).

### Example 34

2-(6-methyl-4-(trifluoromethyl)-pyridin-2-yl)-3-(4-(m-tolyl)-4H-1,2,4-triazol-3-yl)hexahydrocyclopenta[*c*]pyrrol-1(2*H*)-one compound 34
2-(6-methyl-4-(trifluoromethyl)-pyridin-2-yl)-3-(4-(m-tolyl)-4H-1,2,4-triazol-3-yl)hexahydrocyclopenta[c]pyrrol-1(2H)-one

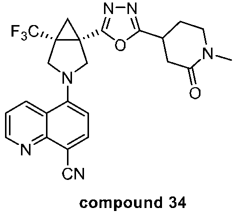

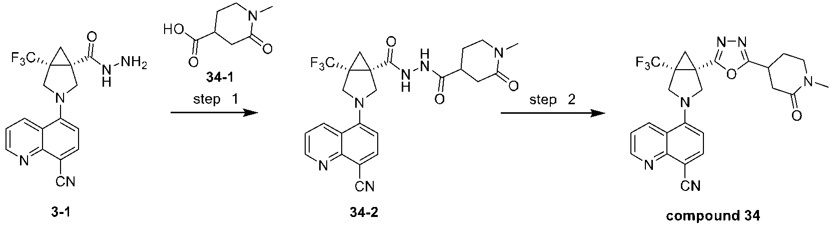

### Step 1:

(1*S*,5iR)-3-(8-cyanoquinolin-5-yl)-N'-(1-methyl-2-oxopiperidine-4-carbonyl)-5-(trifluoromethyl)-3-azabicyclo[3.1.0]hexane-1-carbohydrazide 34-2
(1S,5R)-3-(8-cyanoquinolin-5-yl)-N'-(1-methyl-2-oxopiperidine-4-carbonyl)-5-(trifluoromethyl)-3-azabicyclo[3.1.0]hexane-1-carbohydrazide
1-Methyl-2-oxopiperidine-4-carboxylic acid 34-1 (314 mg, 2 mmol) was dissolved in DMF (3 mL), followed by the addition of DIPEA (517 mg, 4 mmol) and HATU (912 mg, 2.4 mmol). After activation at room temperature for 15 minutes, (1*S*,5*R*)-3-(8-cyanoquinolin-5-yl)-5-(trifluoromethyl)-3-azabicyclo[3.1.0]hexane-1-carbohydrazide 3-1 (722 mg, 2 mmol) was added. The reaction was carried out at room temperature under a nitrogen atmosphere for 1 hour. Subsequently, 20 mL each of water and dichloromethane were added. The phases were separated, and the organic phase was collected, dried, and concentrated to obtain the crude product. The crude product was purified by reverse-phase chromatography to obtain (1S,5R)-3-(8-cyanoquinolin-5-yl)-*N*'-(1-methyl-2-oxopiperidine-4-carbonyl)-5-(trifluoromethyl)-3-azabicyclo[3.1.0]hexane-1-carbohydrazide 34-2 (800 mg, yellow solid, yield: 80%).

LCMS m/z (ESI) = 501.10 [M+1].

### Step 2:

2-(6-methyl-4-(trifluoromethyl)-pyridin-2-yl)-3-(4-(*m*-tolyl)-4*H*-1,2,4-triazol-3-yl)hexahydrocyclopenta[*c*]pyrrol-1(2*H*)-one compound 34
2-(6-methyl-4-(trifluoromethyl)-pyridin-2-yl)-3-(4-(m-tolyl)-4H-1,2,4-triazol-3-yl)hexahydrocyclopenta[*c*]pyrrol-1(2H)-one
(1*S*,5*R*)-3-(8-cyanoquinolin-5-yl)-N'-(1-methyl-2-oxopiperidine-4-carbonyl)-5-(trifluoromethyl)-3-azabicyclo[3.1.0]hexane-1-carbohydrazide 34-2 (125 mg, 0.25 mmol) was dissolved in 1,4-dioxane (2 mL), followed by the addition of TsCl (94 mg, 0.50 mmol) and Cs₂CO₃ (243 mg, 0.75 mmol). The reaction was carried out at 100°C under a nitrogen atmosphere for 2 hours. Subsequently, 20 mL each of water and dichloromethane were added. The phases were separated, and the organic phase was collected, dried, and concentrated to obtain the crude product. The crude product was purified by reverse-phase chromatography to obtain 2-(6-methyl-4-(trifluoromethyl)-pyridin-2-yl)-3-(4-(m-tolyl)-4H-1,2,4-triazol-3-yl)hexahydrocyclopenta[c]pyrrol-1(2*H*)-one compound 34 (30 mg, white solid, yield: 24.8%).

LCMS m/z (ESI) = 483.10 [M+1].

¹H NMR (400 MHz, Chloroform-d) δ 9.09 (dd, 1H), 8.36 (dd, 1H), 8.06 (d, 1H), 7.54 (dd, 1H), 7.17 (d, 1H), 4.12 (dd, 1H), 4.01 - 3.93 (m, 2H), 3.79 (dd, 1H), 3.45 (ddt, 3H), 2.99 (s, 3H), 2.88 (dd, 1H), 2.76 - 2.66 (m, 1H), 2.39 - 2.29 (m, 2H), 2.28 - 2.13 (m, 2H).

### Example 35

5-((1*S*,5*R*)-1-(5-(azetidin-3-ylmethyl)-1,3,4-oxadiazol-2-yl)-5-(trifluoromethyl)-3-azabicyclo[3.1.0]hexan-3-yl)quinoline-8-carbonitrile compound 35
5-((1S,5R)-1-(5-(azetidin-3-ylmethyl)-1,3,4-oxadiazol-2-yl)-5-(trifluoromethyl)-3-azabicyclo[3.1.0]hexan-3-yl)quinoline-8-carbonitrile

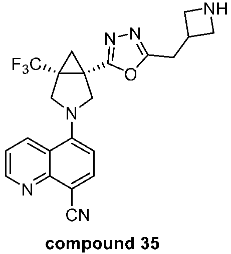

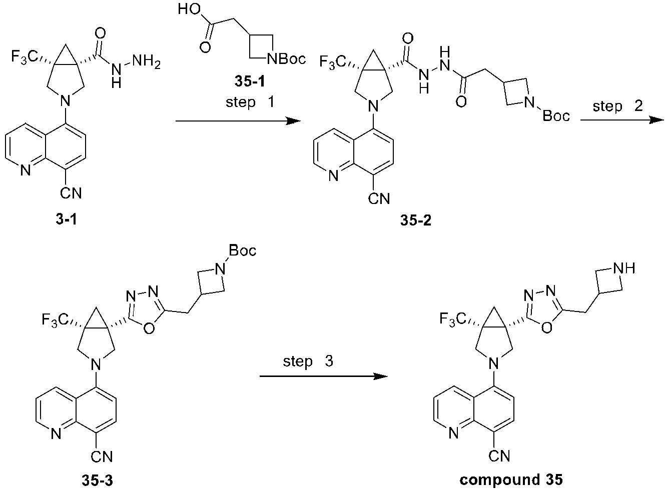

### Step 1:

*tert*-butyl 3-(2-(2-((1*S*,5*R*)-3-(8-cyanoquinolin-5-yl)-5-(trifluoromethyl)-3-azabicyclo[3.1.0]hexane-1-carbonyl)hydrazineyl)-2-oxoethyl)azetidine-1-carboxylate 35-2
tert-butyl 3-(2-(2-((1S,5R)-3-(8-cyanoquinolin-5-yl)-5-(trifluoromethyl)-3-azabicyclo[3.1.0]hexane-1-carbonyl)hydrazineyl)-2-oxoethyl)azetidine-1-carboxylate

2-(1-(*tert*-Butoxycarbonyl)azetidin-3-yl)acetic acid 35-1 (1.07 g, 5 mmol) was dissolved in dichloromethane (10 mL), followed by the addition of DIPEA (1.29 g, 10 mmol) and HATU (2.28 g, 6 mmol). The mixture was activated at room temperature for 15 minutes, and then (1*S*,5*R*)-3-(8-cyanoquinolin-5-yl)-5-(trifluoromethyl)-3-azabicyclo[3.1.0]hexane-1-carbohydrazide 3-1 (1.70 g, 4.7 mmol) was added. The reaction was carried out at room temperature under nitrogen for 2 hours. 200 mL each of water and dichloromethane were added, and the phases were separated. The organic phase was collected, dried, and concentrated to obtain the crude product. The crude product was purified by column chromatography to obtain *tert*-butyl 3-(2-(2-((1*S*,5*R*)-3-(8-cyanoquinolin-5-yl)-5-(trifluoromethyl)-3-azabicyclo[3.1.0]hexane-1-carbonyl)hydrazineyl)-2-oxoethyl)azetidine-1-carboxylate 35-2 (1.93 g, yellow solid, yield: 73%).

LCMS m/z (ESI) = 559.10 [M+1].

### Step 2:

*tert*-butyl 3-((5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)methyl)azetidine-1-carboxylate 35-3
tert-butyl 3-((5-((1S,5R)-3-(8-cyanoquinolin-5-yl)-5-(trifluoromethyl)-3-azabicyclo[3.1.0]hexan-1-yl)-1,3,4-oxadiazol-2-yl)methyl)azetidine-1-carboxylate
*tert*-Butyl 3-(2-(2-((1*S*,5*R*)-3-(8-cyanoquinolin-5-yl)-5-(trifluoromethyl)-3-azabicyclo[3.1.0]hexane-1-carbonyl)hydrazineyl)-2-oxoethyl)azetidine-1-carboxylate 35-2 (1.93 g, 3.45 mmol) was dissolved in anhydrous dichloromethane (20 mL), followed by the addition of DIPEA (1.33 g, 10.3 mmol) and Burgess reagent (2.46 g, 3.45 mmol). The reaction was then carried out at room temperature under a nitrogen atmosphere for 2 hours. Subsequently, 20 mL of water was added, and the phases were separated. The organic phase was collected, dried, and concentrated to obtain the crude product. The crude product was purified by column chromatography to obtain the target product *tert*-butyl 3-((5-((1S,5R)-3-(8-cyanoquinolin-5-yl)-5-(trifluoromethyl)-3-azabicyclo[3.1.0]hexan-1-yl)-1,3,4-oxadiazol-2-yl)methyl)azetidine-1-carboxylate 35-3 (1.86 g, yellow solid, yield: 100%).

LCMS m/z (ESI) = 541.20 [M+1].

### Step 3:

5-((1*S*,5*R*)-1-(5-(azetidin-3-ylmethyl)-1,3,4-oxadiazol-2-yl)-5-(trifluoromethyl)-3-azabicyclo[3.1.0]hexan-3-yl)quinoline-8-carbonitrile compound 35
5-((1S,5R)-1-(5-(azetidin-3-ylmethyl)-1,3,4-oxadiazol-2-yl)-5-(trifluoromethyl)-3-azabicyclo[3.1.0]hexan-3-yl)quinoline-8-carbonitrile
*tert*-Butyl 3-((5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)methyl)azetidine-1-carboxylate 35-3 (1.86 g, 3.45 mmol) was dissolved in dichloromethane (20 mL), and trifluoroacetic acid (10 mL) was added. The reaction was carried out at room temperature under a nitrogen atmosphere for 2 hours. The reaction mixture was then directly concentrated to dryness and purified by reverse-phase chromatography (0.1% TFA in water) to obtain 5-((1S,5R)-1-(5-(azetidin-3-ylmethyl)-1,3,4-oxadiazol-2-yl)-5-(trifluoromethyl)-3-azabicyclo[3.1.0]hexan-3-yl)quinoline-8-carbonitrile compound 35 (1.50 g, yellow solid, yield: 98%).

LCMS m/z = 441.10 [M+1].

¹H NMR (400 MHz, DMSO-*d*₆) δ 9.01 (d, 1H), 8.67 (d, 1H), 8.19 (d,1H), 7.62 (dd, 1H), 7.32 (d, 1H), 4.18 (d, 1H), 4.04 (dd, 2H), 3.91 (d, 1H), 3.26 (t,2H), 3.09 (d,2H), 2.81 (t, 2H), 2.66 (t,1H), 2.20 (s, 2H), 1.50 (s, 1H).

### Example 36

5-((1*S*,5*R*)-1-(5-((1-methylazetidin-3-yl)methyl)-1,3,4-oxadiazol-2-yl)-5-(trifluoromethyl)-3-azabicyclo[3.1.0]hexan-3-yl)quinoline-8-carbonitrile compound 36
5-((1S,5R)-1-(5-((1-methylazetidin-3-yl)methyl)-1,3,4-oxadiazol-2-yl)-5-(trifluoromethyl)-3-azabicyclo[3.1.0]hexan-3-yl)quinoline-8-carbonitrile 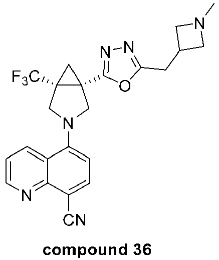 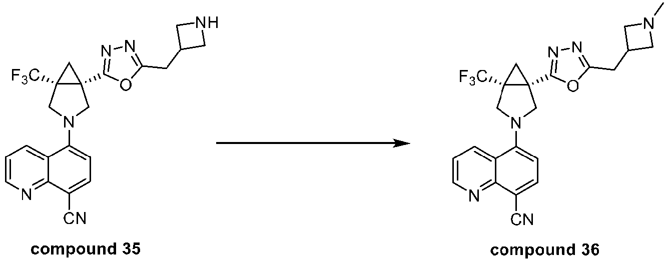
5-((1*S*,5*R*)-1-(5-((1-methylazetidin-3-yl)methyl)-1,3,4-oxadiazol-2-yl)-5-(trifluoromethyl)-3-azabicyclo[3.1.0]hexan-3-yl)quinoline-8-carbonitrile compound 36
5-((1*S*,5*R*)-1-(5-((1-methylazetidin-3-yl)methyl)-1,3,4-oxadiazol-2-yl)-5-(trifluoromethyl)-3-azabicyclo[3.1.0]hexan-3-yl)quinoline-8-carbonitrile
5-((1*S*,5*R*)-1-(5-(Azetidin-3-ylmethyl)-1,3,4-oxadiazol-2-yl)-5-(trifluoromethyl)-3-azabicyclo[3.1.0]hexan-3-yl)quinoline-8-carbonitrile compound 35 (1.30 g, 2.95 mmol) was dissolved in methanol (20 mL). Paraformaldehyde (1.77 g, 59 mmol) and sodium cyanoborohydride (3.70 g, 59 mmol) were added, and the mixture was heated to 80°C and reacted for 2 hours. The reaction mixture was then filtered, and the filtrate was concentrated to dryness and purified by reverse-phase chromatography (0.1% TFA in water) to obtain 5-((1S,5R)-1-(5-((1-methylazetidin-3-yl)methyl)-1,3,4-oxadiazol-2-yl)-5-(trifluoromethyl)-3-azabicyclo[3.1.0]hexan-3-yl)quinoline-8-carbonitrile compound 36 (700 mg, yellow solid, 52%).

LCMS m/z = 455.10 [M+1].

¹H NMR (400 MHz, DMSO-*d*₆) δ 9.01 (d,1H), 8.67 (d, 1H), 8.19 (d, 1H), 7.62 (dd, 1H), 7.32 (d, 1H), 4.18 (d, 1H), 4.04 (dd, 2H), 3.91 (d, 1H), 3.26 (t, 2H), 3.09 (d, 2H), 2.81 (t, 2H), 2.66 (t, 1H), 2.20 (s, 2H), 2.14 (s, 3H).

### Example 37

5-((1*S*,5*R*)-1-(5-((1-methylpiperidin-4-yl)methyl)-1,3,4-oxadiazol-2-yl)-5-(trifluoromethyl)-3-azabicyclo[3.1.0]hexan-3-yl)quinoline-8-carbonitrile compound 37
5-((1S,5R)-1-(5-((1-methylpiperidin-4-yl)methyl)-1,3,4-oxadiazol-2-yl)-5-(trifluoromethyl)-3-azabicyclo[3.1.0]hexan-3-yl)quinoline-8-carbonitrile

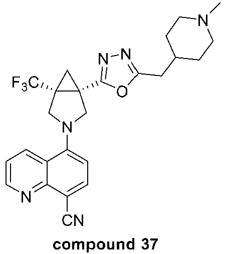

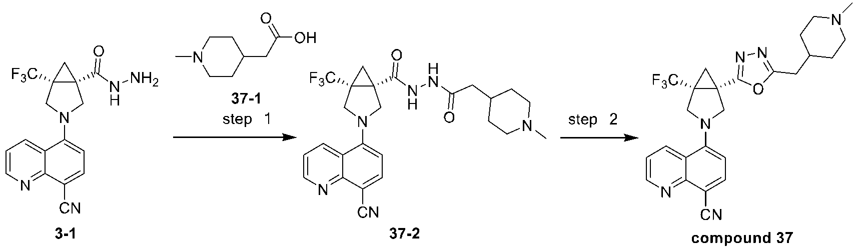

### Step 1:

(1*S*,5*R*)-3-(8-cyanoquinolin-5-yl)-N'-(2-(1-methylpiperidin-4-yl)acetyl)-5-(trifluoromethyl)-3-azabicyclo[3.1.0]hexane-1-carbohydrazide 37-2
(1S,5R)-3-(8-cyanoquinolin-5-yl)-N'-(2-(1-methylpiperidin-4-yl)acetyl)-5-(trifluoromethyl)-3-azabicyclo[3.1.0]hexane-1-carbohydrazide
2-(1-Methylpiperidin-4-yl)acetic acid 37-1 (314 mg, 2 mmol) was dissolved in DMF (3 mL), followed by the addition of DIPEA (517 mg, 4 mmol) and HATU (912 mg, 2.4 mmol). After activation at room temperature for 15 minutes, (1S,5R)-3-(8-cyanoquinolin-5-yl)-5-(trifluoromethyl)-3-azabicyclo[3.1.0]hexane-1-carbohydrazide 3-1 (722 mg, 2 mmol) was added. The reaction was carried out at room temperature under a nitrogen atmosphere for 1 hour. Subsequently, 20 mL each of water and dichloromethane were added. The phases were separated, and the organic phase was collected, dried, and concentrated to obtain the crude product. The crude product was purified by reverse-phase chromatography to obtain (1S,5R)-3-(8-cyanoquinolin-5-yl)-*N*'-(2-(1-methylpiperidin-4-yl)acetyl)-5-(trifluoromethyl)-3-azabicyclo[3.1.0]hexane-1-carbohydrazide 37-2 (800 mg, yellow solid, yield: 80%).

LCMS m/z (ESI) = 501.20 [M+1].

### Step 2:

5-((1S,5R)-1-(5-((1-methylpiperidin-4-yl)methyl)-1,3,4-oxadiazol-2-yl)-5-(trifluoromethyl)-3-azabicyclo[3.1.0]hexan-3-yl)quinoline-8-carbonitrile compound 37
5-((1S,5R)-1-(5-((1-methylpiperidin-4-yl)methyl)-1,3,4-oxadiazol-2-yl)-5-(trifluoromethyl)-3-azabicyclo[3.1.0]hexan-3-yl)quinoline-8-carbonitrile
(1S,5R)-3-(8-Cyanoquinolin-5-yl)-N'-(2-(1-methylpiperidin-4-yl)acetyl)-5-(trifluoromethyl)-3-azabicyclo[3.1.0]hexane-1-carbohydrazide 37-2 (500 mg, 1 mmol) was dissolved in dichloromethane (5 mL), followed by the addition of triethylamine (303 mg, 3 mmol) and Burgess reagent (714 mg, 3 mmol). The reaction was carried out at room temperature under a nitrogen atmosphere for 2 hours. Subsequently, 20 mL each of water and dichloromethane were added. The phases were separated, and the organic phase was collected, dried, and concentrated to obtain the crude product. The crude product was purified by reverse-phase chromatography to obtain 5-((1S,5R)-1-(5-((1-methylpiperidin-4-yl)methyl)-1,3,4-oxadiazol-2-yl)-5-(trifluoromethyl)-3-azabicyclo[3.1.0]hexan-3-yl)quinoline-8-carbonitrile compound 37 (385 mg, yellow solid, yield: 80%).

LCMS m/z (ESI) = 483.20 [M+1].

¹H NMR (400 MHz, DMSO-d6) δ 9.02 (d, 1H), 8.68 (d,1H), 8.20 (d, 1H), 7.71 - 7.58 (m, 1H), 7.33 (d, 1H), 4.19 (d, 1H), 4.05 (dd, 2H), 3.92 (d,1H), 3.08 (d, 3H), 2.91 (q, 3H), 2.71 (s, 3H), 2.22 (s, 2H), 1.99 (s, 1H), 1.82 (d, 2H), 1.52 (d, 2H).

### Example 38

2-(6-methyl-4-(trifluoromethyl)-pyridin-2-yl)-3-(4-(*m*-tolyl)-4H-1,2,4-triazol-3-yl)hexahydrocyclopenta[c]pyrrol-1(2*H*)-one compound 38
2-(6-methyl-4-(trifluoromethyl)-pyridin-2-yl)-3-(4-(m-tolyl)-4H-1,2,4-triazol-3-yl)hexahydrocyclopenta[*c*]pyrrol-1(2H)-one 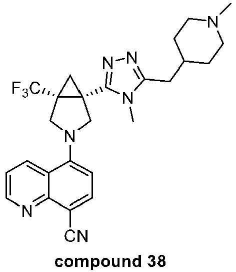 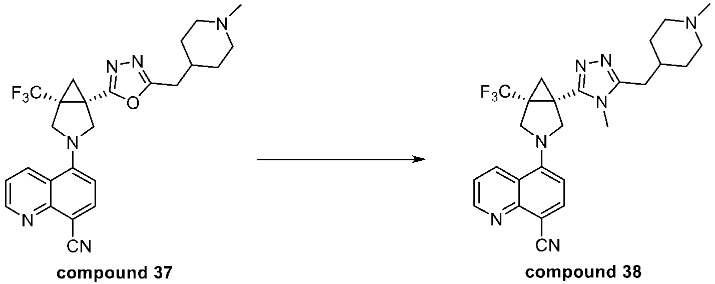
5-((1*S*,5*R*)-1-(5-((1-Methylpiperidin-4-yl)methyl)-1,3,4-oxadiazol-2-yl)-5-(trifluoromethyl)-3-azabicyclo[3.1.0]hexan-3-yl)quinoline-8-carbonitrile compound 37 (145 mg, 0.3 mmol) was dissolved in mesitylene (2 mL), followed by the addition of TsOH (103 mg, 0.6 mmol), methylamine hydrochloride (61 mg, 0.9 mmol), and triethylamine (121 mg, 1.2 mmol). The reaction was carried out at 150°C for 8 hours under a nitrogen atmosphere. The reaction mixture was then purified by silica gel column chromatography to obtain 5-((1S,5R)-1-(4-methyl-5-((1-methylpiperidin-4-yl)methyl)-4*H*-1,2,4-triazol-3-yl)-5-(trifluoromethyl)-3-azabicyclo[3.1.0]hexan-3-yl)quinoline-8-carbonitrile compound 38 (30 mg, yellow solid, yield: 20%).

LCMS m/z (ESI) = 496.20 [M+1].

¹H NMR (400 MHz, DMSO-d6) δ 9.01 (d, 1H), 8.72 (d, 1H), 8.18 (d, 1H), 7.61 (dd, 1H), 7.29 (d, 1H), 4.12 (d, 3H), 3.82 (d, 1H), 3.64 (s, 3H), 2.88 (d, 3H), 2.66 (d, 6H), 2.55 (d, 2H), 2.34 (s, 1H), ,1.85 (d, 2H), 1.74 (d, 2H).

### Example 39

5-((1*S*,5*R*)-1-(5-(1-methylazetidin-3-yl)-1,3,4-oxadiazol-2-yl)-5-(trifluoromethyl)-3-azabicyclo[3.1.0]hexan-3-yl)quinoline-8-carbonitrile compound 39
5-((1S,5R)-1-(5-(1-methylazetidin-3-yl)-1,3,4-oxadiazol-2-yl)-5-(trifluoromethyl)-3-azabicyclo[3.1.0]hexan-3-yl)quinoline-8-carbonitrile 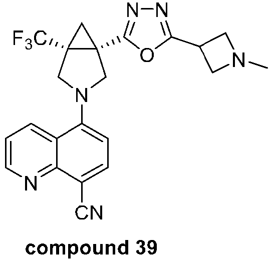 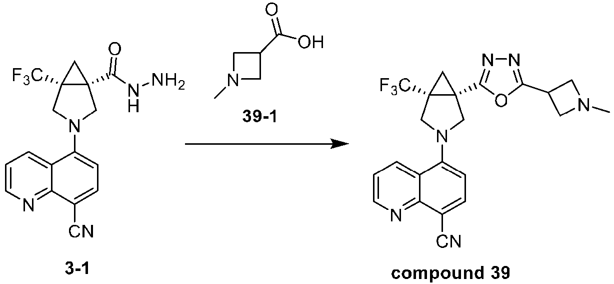
5-((1*S*,5*R*)-1-(5-(1-methylazetidin-3-yl)-1,3,4-oxadiazol-2-yl)-5-(trifluoromethyl)-3-azabicyclo[3.1.0]hexan-3-yl)quinoline-8-carbonitrile compound 39
5-((1*S*,5*R*)-1-(5-(1-methylazetidin-3-yl)-1,3,4-oxadiazol-2-yl)-5-(trifluoromethyl)-3-azabicyclo[3.1.0]hexan-3-yl)quinoline-8-carbonitrile
1-Methylazetidine-3-carboxylic acid 39-1 (345 mg, 3 mmol) and (1*S*,5*R*)-3-(8-cyanoquinolin-5-yl)-5-(trifluoromethyl)-3-azabicyclo[3.1.0]hexane-1-carbohydrazide 3-1 (1.08 g, 3 mmol) were dissolved in phosphorus oxychloride (10 mL). The reaction was carried out at 60°C for 2 hours under a nitrogen atmosphere. The reaction mixture was concentrated to remove most of the phosphorus oxychloride, and triethylamine was added to adjust the pH to alkaline. The crude product was purified by reverse-phase chromatography to obtain 5-((1*S*,5*R*)-1-(5-(1-methylazetidin-3-yl)-1,3,4-oxadiazol-2-yl)-5-(trifluoromethyl)-3-azabicyclo[3.1.0]hexan-3-yl)quinoline-8-carbonitrile compound 39 (200 mg, yellow solid, yield: 15%).

LCMS m/z (ESI) = 441.20 [M+1].

¹H NMR (400 MHz, Chloroform-d) δ 9.14 - 9.01 (m, 1H), 8.36 (d, 1H), 8.05 (d, 1H), 7.53 (dd, 1H), 7.17 (d, 1H), 4.13 (d, 1H), 4.05 - 3.87 (m, 5H), 3.80 (d, 1H), 3.55 (s, 2H), 2.49 (s, 3H), 2.35 (d, 1H), 2.28 - 2.20 (m, 1H).

### Example 40

5-((1*S*,5*R*)-1-(5-(morpholinomethyl)-1,3,4-oxadiazol-2-yl)-5-(trifluoromethyl)-3-azabicyclo[3.1.0]hexan-3-yl)quinoline-8-carbonitrile compound 40
5-((1S,5R)-1-(5-(morpholinomethyl)-1,3,4-oxadiazol-2-yl)-5-(trifluoromethyl)-3-azabicyclo[3.1.0]hexan-3-yl)quinoline-8-carbonitrile

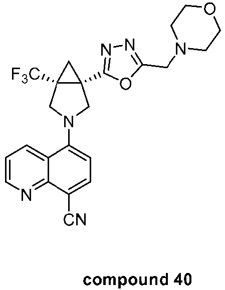

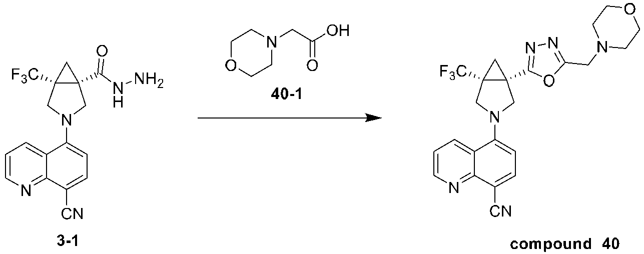

2-Morpholineacetic acid 40-1 (301 mg, 2.07 mmol) was dissolved in 20 mL of dichloromethane, followed by the addition of HATU (791 mg, 2.07 mmol) and DIPEA (534 mg, 4.14 mmol). The mixture was stirred at low temperature for 5 minutes, and then (1*S,*5*R*)-3-(8-cyanoquinolin-5-yl)-5-(trifluoromethyl)-3-azabicyclo[3.1.0]hexane-1-carbohydrazide 3-1 (500 mg, 1.38 mmol) was added. The mixture was stirred for 2 hours. After TLC confirmed the completion of the reaction, Burgess reagent (988 mg, 4.14 mmol) was added, and the mixture was stirred for 3 hours. After LCMS confirmed the completion of the reaction, the reaction mixture was directly concentrated. The residue was purified by reverse-phase C18 column chromatography (acidic method) to obtain 5-((1*S*,5*R*)-1-(5-(morpholinomethyl)-1,3,4-oxadiazol-2-yl)-5-(trifluoromethyl)-3-azabicyclo[3.1.0]hexan-3-yl)quinoline-8-carbonitrile compound 40 (pale yellow solid, 570 mg, 63.2%).

LC-MS m/z (ESI)= 471.10 [M+1].

¹H NMR (400 MHz, Chloroform-*d*) δ 9.10 (dd, 1H), 8.36 (dd, 1H), 8.06 (d, 1H), 7.54 (dd, 1H), 7.17 (d, 1H), 4.15 (d, 1H), 4.03 - 3.92 (m, 2H), 3.87 - 3.78 (m, 3H), 3.74 (t, 4H), 2.57 (s, 4H), 2.38 (d, 1H), 2.25 (d, 1H)

### Example 41

5-((1*S*,5*R*)-1-(5-(pyrrolidin-1-ylmethyl)-1,3,4-oxadiazol-2-yl)-5-(trifluoromethyl)-3-azabicyclo[3.1.0]hexan-3-yl)quinoline-8-carbonitrile compound 41
5-((1S,5R)-1-(5-(pyrrolidin-1-ylmethyl)-1,3,4-oxadiazol-2-yl)-5-(trifluoromethyl)-3-azabicyclo[3.1.0]hexan-3-yl)quinoline-8-carbonitrile

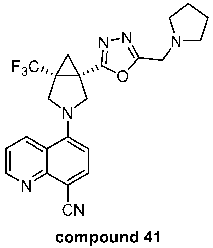

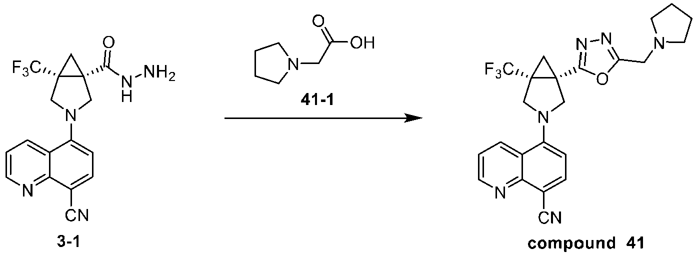

2-(Pyrrolidin-1-yl)acetic acid 41-1 (268 mg, 2.07 mmol) was dissolved in 20 mL of dichloromethane, followed by the addition of HATU (791 mg, 2.07 mmol) and DIPEA (534 mg, 4.14 mmol). The mixture was stirred at low temperature for 5 minutes, then (1*S,*5*R*)-3-(8-cyanoquinolin-5-yl)-5-(trifluoromethyl)-3-azabicyclo[3.1.0]hexane-1-carbohydrazide 3-1 (500 mg, 1.38 mmol) was added and stirred for 2 hours. After TLC confirmed the completion of the reaction, Burgess reagent (988 mg, 4.14 mmol) was added and stirred for 3 hours. After LCMS confirmed the completion of the reaction, the reaction mixture was directly concentrated and purified by reverse-phase C18 column chromatography (acid method) to obtain 5-((1*S*,5*R*)-1-(5-(morpholinomethyl)-1,3,4-oxadiazol-2-yl)-5-(trifluoromethyl)-3-azabicyclo[3.1.0]hexan-3-yl)quinoline-8-carbonitrile compound 41 (pale yellow solid, 400 mg, 63.2%).

LC-MS m/z (ESI)= 455.10 [M+1].

¹H NMR (400 MHz, Chloroform-d) δ 9.09 (dd, 1H), 8.36 (dd, 1H), 8.05 (d, 1H), 7.53 (dd, 1H), 7.17 (d, 1H), 4.16 (d, 1H), 3.97 (dd, 2H), 3.85 - 3.77 (m, 3H), 2.40 (d, 1H), 2.38 (s, 6H), 2.24 (d, 1H), 0.07 (s, 2H).

### Example 42

5-((1*S*,5*R*)-1-(5-((dimethylamino)methyl)-1,3,4-oxadiazol-2-yl)-5-(trifluoromethyl)-3-azabicyclo[3.1.0]hexan-3-yl)quinoline-8-carbonitrile compound 42
5-((1S,5R)-1-(5-((dimethylamino)methyl)-1,3,4-oxadiazol-2-yl)-5-(trifluoromethyl)-3-azabicyclo[3.1.0]hexan-3-yl)quinoline-8-carbonitrile

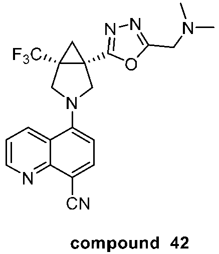

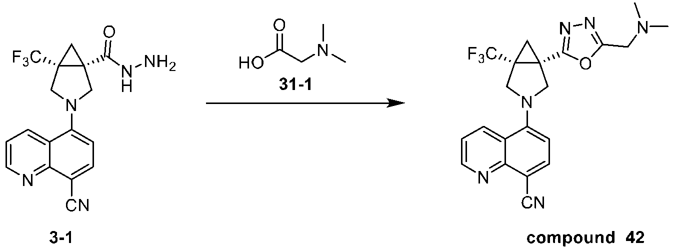

Dimethylglycine 31-1 (213 mg, 2.07 mmol) was dissolved in 20 mL of dichloromethane, followed by the addition of HATU (791 mg, 2.07 mmol) and DIPEA (534 mg, 4.14 mmol). The mixture was stirred at low temperature for 5 minutes, and then (1*S*,5*R*)-3-(8-cyanoquinolin-5-yl)-5-(trifluoromethyl)-3-azabicyclo[3.1.0]hexane-1-carbohydrazide 3-1 (500 mg, 1.38 mmol) was added. The mixture was stirred for 2 hours. After TLC confirmed the completion of the reaction, Burgess reagent (988 mg, 4.14 mmol) was added, and the mixture was stirred for 3 hours. After LCMS confirmed the completion of the reaction, the reaction mixture was directly concentrated. The residue was purified by reverse-phase C18 column chromatography (acidic method) to obtain 5-((1*S*,5*R*)-1-(5-((dimethylamino)methyl)-1,3,4-oxadiazol-2-yl)-5-(trifluoromethyl)-3-azabicyclo[3.1.0]hexan-3-yl)quinoline-8-carbonitrile compound 42 (pale yellow solid, 330 mg, 63.2%).

LC-MS m/z (ESI)= 429.10 [M+1].

¹H NMR (400 MHz, Chloroform-*d*) δ 9.09 (dd, 1H), 8.36 (dd, 1H), 8.05 (d, 1H), 7.53 (dd, 1H), 7.17 (d, 1H), 4.16 (d, 1H), 3.97 (dd, 2H), 3.86 - 3.77 (m, 3H), 2.38 (d, 7H), 2.24 (d, 1H),

### Example 43

5-((1*S*,5*R*)-1-(5-(morpholinoethyl)-1,3,4-oxadiazol-2-yl)-5-(trifluoromethyl)-3-azabicyclo[3.1.0]hexan-3-yl)quinoline-8-carbonitrile compound 43
5-((1S,5R)-1-(5-(2-morpholinoethyl)-1,3,4-oxadiazol-2-yl)-5-(trifluoromethyl)-3-azabicyclo[3.1.0]hexan-3-yl)quinoline-8-carbonitrile

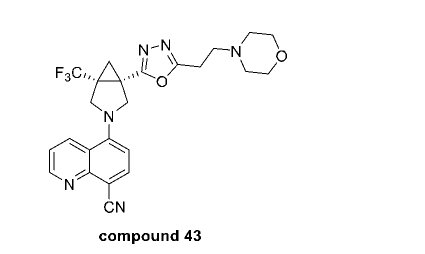

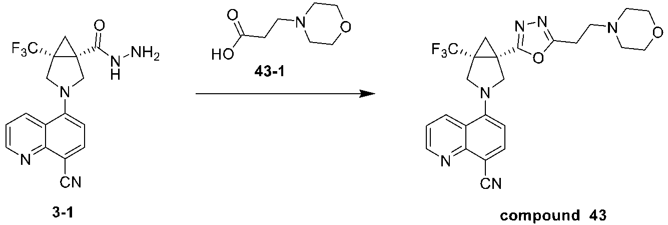

2-Morpholinopropionic acid 43-1 (330 mg, 2.07 mmol) was dissolved in 20 mL of dichloromethane, followed by the addition of HATU (791 mg, 2.07 mmol) and DIPEA (534 mg, 4.14 mmol). The mixture was stirred at low temperature for 5 minutes, and then (1*S*,5*R*)-3-(8-cyanoquinolin-5-yl)-5-(trifluoromethyl)-3-azabicyclo[3.1.0]hexane-1-carbohydrazide 3-1 (500 mg, 1.38 mmol) was added. The mixture was stirred for 2 hours. After TLC confirmed the completion of the reaction, Burgess reagent (988 mg, 4.14 mmol) was added, and the mixture was stirred for 3 hours. After LCMS confirmed the completion of the reaction, the reaction mixture was directly concentrated. The residue was purified by reverse-phase C18 column chromatography (acidic method) to obtain 5-((1S,5R)-1-(5-(morpholinoethyl)-1,3,4-oxadiazol-2-yl)-5-(trifluoromethyl)-3-azabicyclo[3.1.0]hexan-3-yl)quinoline-8-carbonitrile compound 43 (pale yellow solid, 700 mg, 63.2%).

LC-MS m/z (ESI)= 485.10 [M+1].

¹H NMR (400 MHz, Methanol-d4) δ 8.92 - 8.82 (m, 1H), 8.55 (dd, 1H), 8.03 (dd, 1H), 7.53 (dd, 1H), 7.24 (dd, 1H), 4.09 - 3.87 (m, 3H), 3.80 (d, 1H), 3.54 (t, 4H), 3.03 (t, 2H), 2.71 (t, 2H), 2.40 (t, 4H), 2.22 (s, 2H).

### Example 44

5-((1*S*,5*R*)-1-(4-methyl-5-(pyrrolidin-1-ylmethyl)-4*H*-1,2,4-triazol-3-yl)-5-(trifluoromethyl)-3-azabicyclo[3.1.0]hexan-3-yl)quinoline-8-carbonitrile compound 44
5-((1S,5R)-1-(4-methyl-5-(pyrrolidin-1-ylmethyl)-4H-1,2,4-triazol-3-yl)-5-(trifluoromethyl)-3-azabicyclo[3.1.0]hexan-3-yl)quinoline-8-carbonitrile

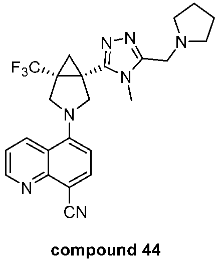

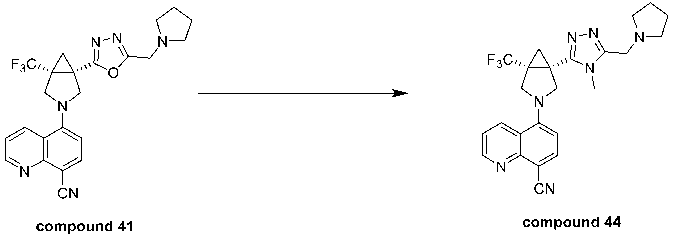

5-((1*S*,5*R*)-1-(5-(Pyrrolidin-1-ylmethyl)-1,3,4-oxadiazol-2-yl)-5-(trifluoromethyl)-3-azabicyclo[3.1.0]hexan-3-yl)quinoline-8-carbonitrile compound 41 (200 mg, 0.44 mmol) was dissolved in 5 mL of mesitylene. Subsequently, methylamine hydrochloride (148 mg, 2.20 mmol), TEA (303 mg, 2.98 mmol), and p-toluenesulfonic acid (151 mg, 0.88 mmol) were added. The mixture was stirred at low temperature for 5 minutes, then stirred at 150°C for hours. After LCMS confirmed the completion of the reaction, the reaction mixture was directly purified by column chromatography to obtain the target product 5-((1*S*,5*R*)-1-(4-methyl-5-(pyrrolidin-1-ylmethyl)-4*H*-1,2,4-triazol-3-yl)-5-(trifluoromethyl)-3-azabicyclo[3.1.0]hexan-3-yl)quinoline-8-carbonitrile compound 44 (pale yellow solid, 80 mg, 38.0%).

LC-MS m/z (ESI)= 468.0 [M+1].

¹H NMR (400 MHz, Methanol-d4) δ 8.94 (dd, 1H), 8.65 (dd, 1H), 8.09 (d, 1H), 7.60 (dd, 1H), 7.30 (d, 1H), 4.08 (d, 3H), 3.86 (d, 6H), 2.57 (qd, 4H), 2.22 (d, 1H), 2.09 - 1.98 (m, 1H), 1.87 - 1.74 (m, 4H).

### Example 45

5-((1*R*,5*S*)-1-((difluoro-3-methyl)-12-fluoraneyl)-5-(5-(5-fluoropiperidin-3-yl)-1,3,4-oxadiazol-2-yl)-3-azabicyclo[3.1.0]hexan-3-yl)quinoline-8-carbonitrile compound 45
5-((1R,5S)-1-((difluoro-13-methyl)-12-fluoraneyl)-5-(5-(5-fluoropiperidin-3-yl)-1,3,4-oxadiazol-2-yl)-3-azabicyclo[3.1.0]hexan-3-yl)quinoline-8-carbonitrile

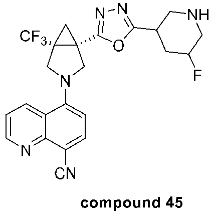

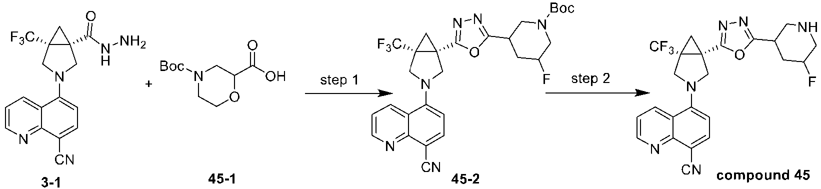

### Step 1:

*tert*-butyl 3-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-5-fluoropiperidine-1-carboxylate 45-2
tert-butyl 3-(5-((1S,5R)-3-(8-cyanoquinolin-5-yl)-5-(trifluoromethyl)-3-azabicyclo[3.1.0]hexan-1-yl)-1,3,4-oxadiazol-2-yl)-5-fluoropiperidine-1-carboxylate

4-(*tert*-Butoxycarbonyl)morpholine-2-carboxylic acid 45-1 (643 mg, 2.6 mmol) was dissolved in 10 mL of dichloromethane, followed by the addition of HATU (1140 mg, 3.00 mmol) and DIPEA (840 mg, 6.00 mmol). The mixture was stirred at low temperature for 5 minutes, and then (1*S*,5*R*)-3-(8-cyanoquinolin-5-yl)-5-(trifluoromethyl)-3-azabicyclo[3.1.0]hexane-1-carbohydrazide 3-1 (724 mg, 2.0 mmol) was added. After stirring for 2 hours, TLC confirmed the completion of the reaction. Subsequently, Burgess reagent (1100 mg, 3.9 mmol) was added, and the mixture was stirred for 3 hours. LCMS confirmed the completion of the reaction. The reaction mixture was directly concentrated and purified by reverse-phase C18 column chromatography (acidic method) to obtain the target product *tert-*butyl 3-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-5-fluoropiperidine-1-carboxylate 45-2 (pale yellow solid, 500 mg crude).

### Step 2:

5-((1*R*,5*S*)-1-((difluoro-3-methyl)-12-fluoraneyl)-5-(5-(5-fluoropiperidin-3-yl)-1,3,4-oxadiazol-2-yl)-3-azabicyclo[3.1.0]hexan-3-yl)quinoline-8-carbonitrile compound 45
5-((1R,5S)-1-((difluoro-13-methyl)-12-fluoraneyl)-5-(5-(5-fluoropiperidin-3-yl)-1,3,4-oxadiazol-2-yl)-3-azabicyclo[3.1.0]hexan-3-yl)quinoline-8-carbonitrile

The crude product 5-((1*S*,5*R*)-1-(5-(1-*tert*-butoxycarbonylpiperidin-4-yl)-1,3,4-oxadiazol-2-yl)-5-(trifluoromethyl)-3-azabicyclo[3.1.0]hexan-3-yl)quinoline-8-carbonitrile 45-2 (500 mg) was added to 2.00 mL of hydrogen chloride in ethyl acetate solution and stirred for half an hour. After LCMS confirmed the completion of the reaction, the mixture was concentrated. The pH was adjusted to 7-8 with triethylamine, and the mixture was purified by TLC (dichloromethane: methanol = 5:1) to obtain the product 5-((1*R*,5*S*)-1-((difluoro-3-methyl)-12-fluoraneyl)-5-(5-(5-fluoropiperidin-3-yl)-1,3,4-oxadiazol-2-yl)-3-azabicyclo[3.1.0]hexan-3-yl)quinoline-8-carbonitrile compound 45 (pale yellow solid, 300 mg, 72.8%).

LC-MS m/z (ESI)= 473.10 [M+1].

¹H NMR (400 MHz, Chloroform-d) δ 9.09 (dd, 1H), 8.36 (dd, 1H), 8.13 - 8.02 (m, 1H), 7.53 (dd, 1H), 7.17 (d, 1H), 4.12 (d, 1H), 4.02 - 3.92 (m, 2H), 3.80 (d, 1H), 3.20 (s, 1H), 3.12 (s, 1H), 2.45 - 2.36 (m, 3H), 2.33 (d, 2H), 2.24 (s, 2H), 1.78 (q, 1H), 1.25 (s, 1H).

### Example 46

5-((1*S*,5*R*)-1-(5-(3-fluoropyrrolidin-3-yl)-1,3,4-oxadiazol-2-yl)-5-(trifluoromethyl)-3-azabicyclo[3.1.0]hexan-3-yl)quinoline-8-carbonitrile compound 46
5-((1S,5R)-1-(5-(3-fluoropyrrolidin-3-yl)-1,3,4-oxadiazol-2-yl)-5-(trifluoromethyl)-3-azabicyclo[3.1.0]hexan-3-yl)quinoline-8-carbonitrile

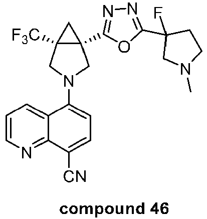

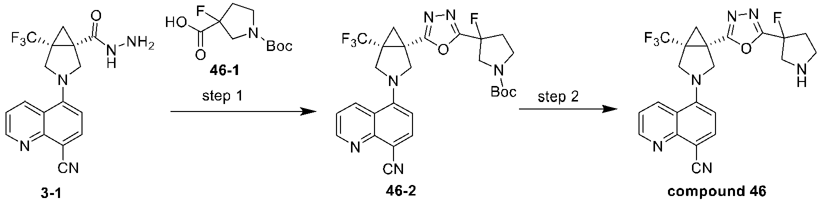

### Step 1:

*tert*-butyl 3-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-3-fluoropyrrolidine-1-carboxylate 46-2
tert-butyl 3-(5-((1S,5R)-3-(8-cyanoquinolin-5-yl)-5-(trifluoromethyl)-3-azabicyclo[3.1.0]hexan-1-yl)-1,3,4-oxadiazol-2-yl)-3-fluoropyrrolidine-1-carboxylate
1-(*tert*-Butoxycarbonyl)-3-fluoropyrrolidine-3-carboxylic acid 46-1 (560 mg, 2.2 mmol) was dissolved in 20 mL of dichloromethane, followed by the addition of HATU (1.136 g, 2.98 mmol) and DIPEA (766 mg, 5.94 mmol). The mixture was stirred at low temperature for 5 minutes, and then (1*S*,5*R*)-3-(8-cyanoquinolin-5-yl)-5-(trifluoromethyl)-3-azabicyclo[3.1.0]hexane-1-carbohydrazide 3-1 (720 mg, 1.98 mmol) was added. The reaction was stirred for 2 hours. After TLC confirmed the completion of the reaction, Burgess reagent (1100 mg, 3.9 mmol) was added, and the mixture was stirred for 3 hours. After LCMS confirmed the completion of the reaction, the reaction mixture was directly concentrated. The residue was purified by reverse-phase C18 column chromatography (acidic method) to obtain the target product *tert*-butyl 3-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-3-fluoropyrrolidine-1-carboxylate 46-2 (pale yellow solid, 780 mg, 85.0%).

LC-MS m/z (ESI)= 559.10 [M+1].

### Step 2:

5-((1*S*,5*R*)-1-(5-(3-fluoropyrrolidin-3-yl)-1,3,4-oxadiazol-2-yl)-5-(trifluoromethyl)-3-azabicyclo[3.1.0]hexan-3-yl)quinoline-8-carbonitrile compound 46
5-((1S,5R)-1-(5-(3-fluoropyrrolidin-3-yl)-1,3,4-oxadiazol-2-yl)-5-(trifluoromethyl)-3-azabicyclo[3.1.0]hexan-3-yl)quinoline-8-carbonitrile

The crude product R *tert*-butyl 3-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-3-fluoropyrrolidine-1-carboxylate 46-2 (500 mg) was dissolved in 5 mL of dichloromethane, and 5.00 mL of TFA was added. After stirring for half an hour, the mixture was sampled for LCMS analysis, which confirmed the completion of the reaction. The reaction mixture was concentrated to obtain the product 5-((1*S*,5*R*)-1-(5-(3*-*fluoropyrrolidin-3-yl)-1,3,4-oxadiazol-2-yl)-5-(trifluoromethyl)-3-azabicyclo[3.1.0]hexan-3-yl)quinoline-8-carbonitrile compound 46 (pale yellow solid, 700 mg, 97%).

LC-MS m/z (ESI)= 459.10 [M+1].

¹H NMR (400 MHz, Methanol-*d*₄) δ 8.87 (dd, 1H), 8.55 (dd, 1H), 8.03 (d, 1H), 7.53 (dd, 1H), 7.25 (d, 1H), 4.10 - 3.98 (m, 2H), 3.97 (d, 1H), 3.82 (d, 1H), 3.43 (ddd, 1H), 3.38 - 3.22 (m, 1H), 3.21 - 3.11 (m, 1H), 3.03 (dt, 1H), 2.53 - 2.31 (m, 2H), 2.27 (s, 2H).

### Example 47

5-((1*S*,5*R*)-1-(5-(3-fluoro-1-methylpyrrolidin-3-yl)-1,3,4-oxadiazol-2-yl)-5-(trifluoromethyl)-3-azabicyclo[3.1.0]hexan-3-yl)quinoline-8-carbonitrile compound 47
5-((1S,5R)-1-(5-(3-fluoro-1-methylpyrrolidin-3-yl)-1,3,4-oxadiazol-2-yl)-5-(trifluoromethyl)-3-azabicyclo[3.1.0]hexan-3-yl)quinoline-8-carbonitrile

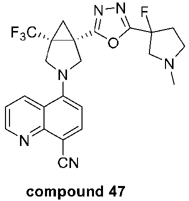

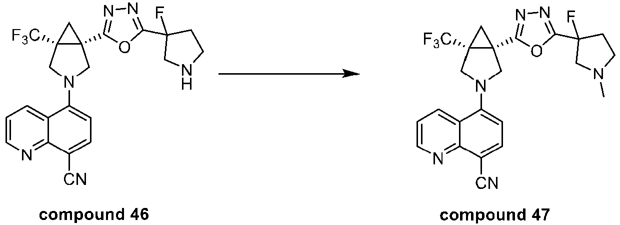

5-((1*S*,5*R*)-1-(5-(3-Fluoropyrrolidin-3-yl)-1,3,4-oxadiazol-2-yl)-5-(trifluoromethyl)-3-azabicyclo[3.1.0]hexan-3-yl)quinoline-8-carbonitrile compound 46 (200 mg, 0.43 mmol) was dissolved in 5 mL of methanol, followed by the addition of 1 mL of triethylamine, paraformaldehyde (129 mg, 4.3 mmol), and sodium cyanoborohydride (270.9 mg, 4.3 mmol). The mixture was heated to 80°C and stirred for 3 hours. After TLC confirmed the completion of the reaction, the reaction mixture was directly concentrated. The residue was purified by reverse-phase C18 column chromatography (alkaline method) to obtain the target product 5 5-((1*S*,5*R*)-1-(5-(3-fluoro-1-methylpyrrolidin-3-yl)-1,3,4-oxadiazol-2-yl)-5-(trifluoromethyl)-3-azabicyclo[3.1.0]hexan-3-yl)quinoline-8-carbonitrile compound 47 (pale yellow solid, 30 mg, 13.7%).

LC-MS m/z (ESI)= 473.10 [M+1].

¹H NMR (400 MHz, Chloroform-d) δ 9.09 (dd, 1H), 8.36 (dd, 1H), 8.06 (d, 1H), 7.54 (dd, 1H), 7.19 (d, 1H), 4.16 (d, 1H), 4.04 - 3.93 (m, 2H), 3.81 (d, 1H), 3.31 (dt, 2H), 3.12 - 3.01 (m, 1H), 2.87 - 2.46 (m, 6H), 2.39 (d, 1H), 2.31 - 2.25 (m, 1H).

### Example 48

5-((1*S*,5*R*)-1-(5-(2-(pyrrolidin-1-yl)ethoxy)-1,3,4-oxadiazol-2-yl)-5-(trifluoromethyl)-3-azabicyclo[3.1.0]hexan-3-yl)quinoline-8-carbonitrile compound 48
5-((1S,5R)-1-(5-(2-(pyrrolidin-1-yl)ethoxy)-1,3,4-oxadiazol-2-yl)-5-(trifluoromethyl)-3-azabicyclo[3.1.0]hexan-3-yl)quinoline-8-carbonitrile

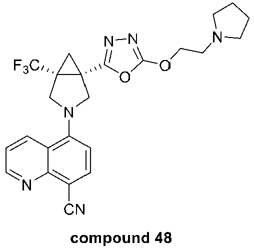

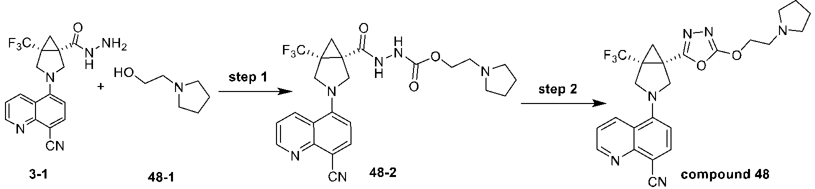

### Step 1:

2-(pyrrolidin-1-yl)ethyl-2-((1*S*,5*R*)-3-(8-cyanoquinolin-5-yl)-5-(trifluoromethyl)-3-azabicyclo[3.1.0]hexane-1-carbonyl)hydrazine-1-carboxylate 48-2
2-(pyrrolidin-1-yl)ethyl-2-((1S,5R)-3-(8-cyanoquinolin-5-yl)-5-(trifluoromethyl)-3-azabicyclo[3.1.0]hexane-1-carbonyl)hydrazine-1-carboxylate
(1*S*,5*R*)-3-(8-Cyanoquinolin-5-yl)-5-(trifluoromethyl)-3-azabicyclo[3.1.0]hexane-1-carbohydrazide 3-1 (724 mg, 2.0 mmol) was dissolved in 10 mL of dichloromethane. Triphosgene (177.6 mg, 0.6 mmol) was dissolved in 10.0 mL of dichloromethane, and a mixed solution of 2-(pyrrolidin-1-yl)ethan-1-ol 48-1 (272 mg, 2.0 mmol) and triethylamine (204 mg, 2.0 mmol) in 10.0 mL of dichloromethane was slowly added dropwise at 0°C. After 1 hour of reaction, LCMS confirmed the completion of the reaction. Methanol was added to quench the reaction, and the reaction mixture was directly concentrated. The residue was purified by reverse-phase C18 column chromatography (acidic method) to obtain the target product 2-(pyrrolidin-1-yl)ethyl-2-((1*S*,5*R*)-3-(8-cyanoquinolin-5-yl)-5-(trifluoromethyl)-3-azabicyclo[3.1.0]hexane-1-carbonyl)hydrazine-1-carboxylate 48-2 (pale yellow solid, 280 mg, 27.8%).

### Step 2:

5-((1*S*,5*R*)-1-(5-(2-(pyrrolidin-1-yl)ethoxy)-1,3,4-oxadiazol-2-yl)-5-(trifluoromethyl)-3-azabicyclo[3.1.0]hexan-3-yl)quinoline-8-carbonitrile compound 48
5-((1S,5R)-1-(5-(2-(pyrrolidin-1-yl)ethoxy)-1,3,4-oxadiazol-2-yl)-5-(trifluoromethyl)-3-azabicyclo[3.1.0]hexan-3-yl)quinoline-8-carbonitrile
2-(Pyrrolidin-1-yl)ethyl-2-((1*S*,5*R*)-3-(8-cyanoquinolin-5-yl)-5-(trifluoromethyl)-3-azabicyclo[3.1.0]hexane-1-carbonyl)hydrazine-1-carboxylate 48-2 (280 mg, 0.55 mmol) was dissolved in 10 mL of dichloromethane. DIEA (204 mg, 2.0 mmol) and Burgess reagent (1100 mg, 3.9 mmol) were added, and the mixture was stirred for 3 hours. After LCMS confirmed the completion of the reaction, the reaction mixture was directly concentrated and purified by reverse-phase C18 column chromatography (acid method) to obtain the target product 5-((1*S*,5*R*)-1-(5-(2-(pyrrolidin-1-yl)ethoxy)-1,3,4-oxadiazol-2-yl)-5-(trifluoromethyl)-3-azabicyclo[3.1.0]hexan-3-yl)quinoline-8-carbonitrile compound 48 (pale yellow solid, 50 mg, 18.5%).

LC-MS m/z (ESI)= 485.2 [M+1].

¹H NMR (400 MHz, Chloroform-*d*) δ 9.09 (dd, 1H), 8.34 (ddd, 1H), 8.04 (dd, 1H), 7.53 (dd, 1H), 7.14 (d, 1H), 4.62 (t, 2H), 4.02 (d, 1H), 3.96 - 3.90 (m, 2H), 3.78 (d, 1H), 2.95 (t, 2H), 2.61 (q, 4H), 2.27 (d, 1H), 2.15 (d, 1H), , 1.86 - 1.79 (m, 4H).

### Example 49

5-((1*S*,5*R*)-1-(5-(3-fluoro-1-methylazetidin-3-yl)-1,3,4-oxadiazol-2-yl)-5-(trifluoromethyl)-3-azabicyclo[3.1.0]hexan-3-yl)quinoline-8-carbonitrile compound 49
5-((1S,5R)-1-(5-(3-fluoro-1-methylazetidin-3-yl)-1,3,4-oxadiazol-2-yl)-5-(trifluoromethyl)-3-azabicyclo[3.1.0]hexan-3-yl)quinoline-8-carbonitrile 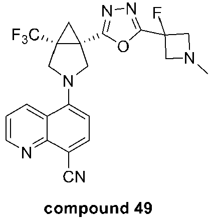 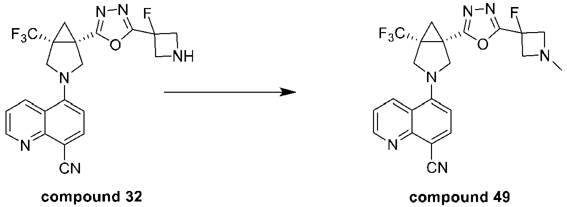
5-((1*S*,5*R*)-1-(5-(3-Fluoroazetidin-3-yl)-1,3,4-oxadiazol-2-yl)-5-(trifluoromethyl)-3-azabicyclo[3.1.0]hexan-3-yl)quinoline-8-carbonitrile compound 32 (400 mg, 0.9 mmol) was dissolved in 5 mL of methanol, followed by the addition of 1 mL of triethylamine, paraformaldehyde (250 mg, 9 mmol), and sodium cyanoborohydride (506 mg, 13.5 mmol). The mixture was heated to 80°C and stirred for 3 hours. After TLC confirmed the completion of the reaction, the reaction mixture was directly concentrated. The residue was purified by reverse-phase C18 column chromatography (alkaline method) to obtain the target product 5-*((1S,5R)-1-(5-((S)-4-methylmorpholin-2-yl)-1,3,4-oxadiazol-2-yl)-5-(trifluoromethyl)-3-*azabicyclo[3.1.0]hexan-3-yl)quinoline-8-carbonitrile compound 49 (pale yellow solid, 110 mg, 26.7%).

LC-MS m/z (ESI)= 459.10 [M+1].

¹H NMR (400 MHz, Chloroform-d) δ 9.10 (dd, 1H), 8.36 (dd, 1H), 8.06 (d, 1H), 7.54 (dd, 1H), 7.19 (d, 1H), 4.17 (d, 1H), 4.14 - 4.02 (m, 2H), 4.02 - 3.94 (m, 2H), 3.81 (d, 1H), 3.68 (dd, 2H), 2.53 (s, 3H), 2.40 (d, 1H), 2.28 (d, 1H).

### Example 50

5-((1*S*,5*R*)-1-(5-(3-fluoro-1-methylpiperidin-4-yl)-1,3,4-oxadiazol-2-yl)-5-(trifluoromethyl)-3-azabicyclo[3.1.0]hexan-3-yl)quinoline-8-carbonitrile compound 50
5-((1S,5R)-1-(5-(3-fluoro-1-methylpiperidin-4-yl)-1,3,4-oxadiazol-2-yl)-5-(trifluoromethyl)-3-azabicyclo[3.1.0]hexan-3-yl)quinoline-8-carbonitrile 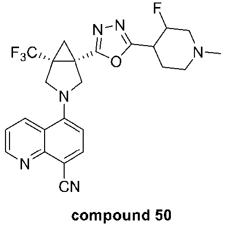 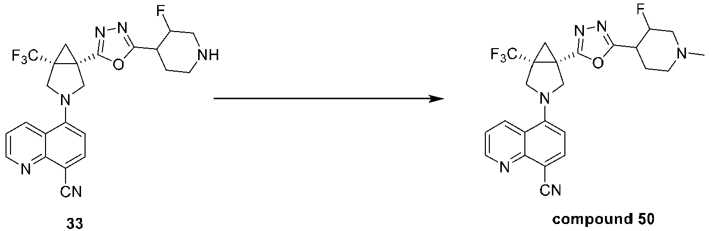
5-((1*S*,5*R*)-1-(5-(3-Fluoroazetidin-3-yl)-1,3,4-oxadiazol-2-yl)-5-(trifluoromethyl)-3-azabicyclo[3.1.0]hexan-3-yl)quinoline-8-carbonitrile compound 33 (100 mg, 0.21 mmol) was dissolved in 5 mL of methanol, followed by the addition of 1 mL of triethylamine, paraformaldehyde (250 mg, 9 mmol), and sodium cyanoborohydride (506 mg, 13.5 mmol). The mixture was heated to 80°C and stirred for 3 hours. After TLC confirmed the completion of the reaction, the reaction mixture was directly concentrated. The residue was purified by reverse-phase C18 column chromatography (alkaline method) to obtain the target product 5-((1*S*,5*R*)-1-(5-((*S*)-4-methylmorpholin-2-yl)-1,3,4-oxadiazol-2-yl)-5-(trifluoromethyl)-3-azabicyclo[3.1.0]hexan-3-yl)quinoline-8-carbonitrile compound 50 (pale yellow solid, 20 mg, 19.6%).

LC-MS m/z (ESI)= 487.10 [M+1].

¹H NMR (400 MHz, Chloroform-d) δ 9.09 (dd, 1H), 8.36 (dd, 1H), 8.05 (d, 1H), 7.53 (dd, 1H), 7.17 (dd, 1H), 5.07 (d, 1H), 4.12 (dd, 1H), 3.98 (dd, 2H), 3.80 (dd, 1H), 3.32 - 3.03 (m, 3H), 2.51 - 2.29 (m, 5H), 2.24 (s, 2H), 2.09 (s, 2H).

### Example 51

5-((1*S*,5*R*)-1-(5-(2-(dimethylamino)ethyl)-1,3,4-oxadiazol-2-yl)-5-(trifluoromethyl)-3-azabicyclo[3.1.0]hexan-3-yl)quinoline-8-carbonitrile compound 51
5-((1S,5R)-1-(5-(2-(dimethylamino)ethyl)-1,3,4-oxadiazol-2-yl)-5-(trifluoromethyl)-3-azabicyclo[3.1.0]hexan-3-yl)quinoline-8-carbonitrile

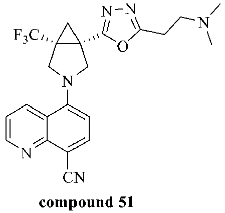

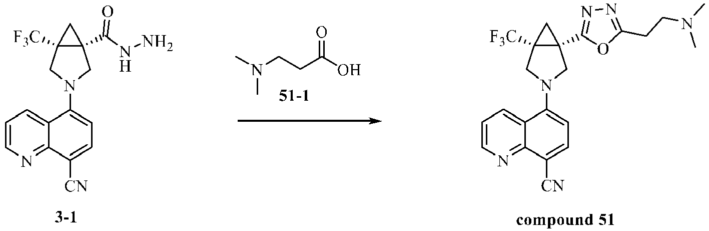

The starting material (1S,5R)-3-(8-cyanoquinolin-5-yl)-5-(trifluoromethyl)-3-azabicyclo[3.1.0]hexane-1-carbohydrazide 3-1 (500 mg, 1.38 mmol) and 3-(dimethylamino)propionic acid 51-1 (161 mg, 1.38 mmol) were added to phosphorus oxychloride (3 mL). The reaction mixture was heated to 100°C and stirred for 3 hours under a nitrogen atmosphere. After the reaction was completed, the mixture was dissolved in dichloromethane and slowly added dropwise to an ice-cold saturated aqueous sodium carbonate solution. After stirring for 0.5 hours, the pH was readjusted to approximately 9. The mixture was extracted three times with ethyl acetate, and the combined organic phases were washed with saturated brine, dried over anhydrous sodium sulfate, and concentrated under reduced pressure. The residue was purified by column chromatography to obtain 5-((1*S*,5*R*)-1-(5-(2-(dimethylamino)ethyl)-1,3,4-oxadiazol-2-yl)-5-(trifluoromethyl)-3-azabicyclo[3.1.0]hexan-3-yl)quinoline-8-carbonitrile compound 51 (220 mg, yield: 36.0%).

LCMS m/z(ESI)=443.2[M+1].

¹H NMR (400 MHz, DMSO-*d*₆) δ 9.02 (dd, 1H), 8.69 (dd, 1H), 8.20 (d, 1H), 7.62 (dd, 1H), 7.33 (d, 1H), 4.23 - 4.16 (m, 1H), 4.06 (dd, 2H), 3.93 (d, 1H), 3.01 (t, 2H), 2.63 (t, 2H), 2.21 (s, 2H), 2.15 (s, 6H).

¹⁹F NMR (377 MHz, DMSO-*d*₆) δ -64.05.

### Example 52

5-((1*S*,5*R*)-1-(5-(4-aminobicyclo[2.2.2]octan-1-yl)-1,3,4-oxadiazol-2-yl)-5-(trifluoromethyl)-3-azabicyclo[3.1.0]hexan-3-yl)quinoline-8-carbonitrile compound 52
5-((1S,5R)-1-(5-(4-aminobicyclo[2.2.2]octan-1-yl)-1,3,4-oxadiazol-2-yl)-5-(trifluoromethyl)-3-azabicyclo[3.1.0]hexan-3-yl)quinoline-8-carbonitrile

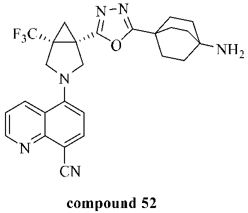

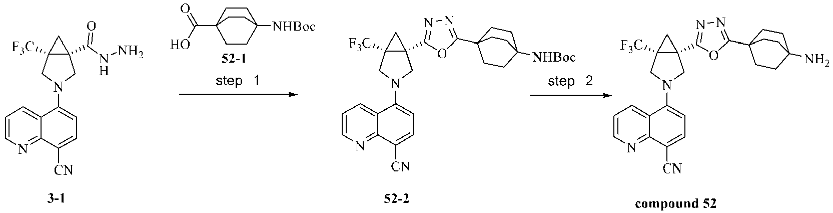

### Step 1:

*tert*-butyl (4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)bicyclo[2.2.2]octan-1-yl)carbamate 52-2
tert-butyl (4-(5-((1S,5R)-3-(8-cyanoquinolin-5-yl)-5-(trifluoromethyl)-3-azabicyclo[3.1.0]hexan-1-yl)-1,3,4-oxadiazol-2-yl)bicyclo[2.2.2]octan-1-yl)carbamate

The starting materials (1*S*,5*R*)-3-(8-cyanoquinolin-5-yl)-5-(trifluoromethyl)-3-azabicyclo[3.1.0]hexane-1-carbohydrazide 3-1 (700 mg, 1.94 mmol) and 4-((*tert-*butoxycarbonyl)amino)bicyclo[2.2.2]octane-1-carboxylic acid 52-1 (522 mg, 1.94 mmol) were added to *N*,*N*-dimethylformamide (10 mL), followed by the addition of 2-(7-azabenzotriazol-1-yl)-*N,N,N',N'*-tetramethyluronium hexafluorophosphate (737 mg, 1.94 mmol) and *N,N-*diisopropylethylamine (752 mg, 5.82 mmol). The reaction mixture was stirred at room temperature for 3 hours. After LCMS confirmed the completion of the reaction, Burgess reagent (1.39 g, 5.82 mmol) was added, and the reaction mixture was stirred at room temperature for 16 hours. After the reaction was completed, the mixture was directly purified by reverse-phase flash column chromatography to obtain *tert*-butyl (4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)bicyclo[2.2.2]octan-1-yl)carbamate 52-2 (560 mg, yield: 48.5%).

LCMS m/z(ESI)=595.3[M+1].

### Step 2:

5-((1*S*,5*R*)-1-(5-(4-aminobicyclo[2.2.2]octan-1-yl)-1,3,4-oxadiazol-2-yl)-5-(trifluoromethyl)-3-azabicyclo[3.1.0]hexan-3-yl)quinoline-8-carbonitrile compound 52
5-((1S,5R)-1-(5-(4-aminobicyclo[2.2.2]octan-1-yl)-1,3,4-oxadiazol-2-yl)-5-(trifluoromethyl)-3-azabicyclo[3.1.0]hexan-3-yl)quinoline-8-carbonitrile

The starting material *tert*-butyl (4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)bicyclo[2.2.2]octan-1-yl)carbamate 52-2 (560 mg, 0.941 mmol) was added to dichloromethane (3 mL), followed by the addition of trifluoroacetic acid (3 mL). The reaction mixture was stirred at room temperature for 1 hour. After the reaction was completed, the mixture was concentrated under reduced pressure. The crude product was purified by column chromatography to obtain 5-*((1S,5R)-1-(5-(4-aminobicyclo[2.2.2]octan-1-yl)-1,3,4-oxadiazol-2-yl)-5-(trifluoromethyl)-3-*azabicyclo[3.1.0]hexan-3-yl)quinoline-8-carbonitrile compound 52 (440 mg, yield: 94.4%).

LCMS m/z(ESI)=495.3[M+1].

¹H NMR (400 MHz, Chloroform-*d*) δ 9.08 (dd, 1H), 8.36 (dd, 1H), 8.04 (d, 1H), 7.53 (dd, 1H), 7.16 (d, 1H), 4.10 (d, 1H), 4.06 - 3.89 (m, 2H), 3.79 (d, 1H), 3.01 (s, 2H), 2.29 (d, 1H), 2.25 - 2.18 (m, 1H), 2.16 - 2.00 (m, 6H), 1.80 - 1.69 (m, 6H).

¹⁹F NMR (377 MHz, Chloroform-d) δ -65.27, -75.64.

### Example 53

5-((1*S*,5*R*)-1-(5-(4-(dimethylamino)bicyclo[2.2.2]octan-1-yl)-1,3,4-oxadiazol-2-yl)-5-(trifluoromethyl)-3-azabicyclo[3.1.0]hexan-3-yl)quinoline-8-carbonitrile compound 53
5-((1S,5R)-1-(5-(4-(dimethylamino)bicyclo[2.2.2]octan-1-yl)-1,3,4-oxadiazol-2-yl)-5-(trifluoromethyl)-3-azabicyclo[3.1.0]hexan-3-yl)quinoline-8-carbonitrile 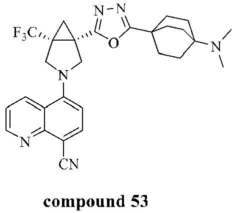 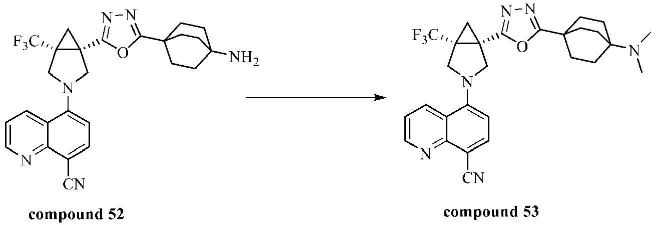
5-((1*S*,5*R*)-1-(5-(4-Aminobicyclo[2.2.2]octan-1-yl)-1,3,4-oxadiazol-2-yl)-5-(trifluoromethyl)-3-azabicyclo[3.1.0]hexan-3-yl)quinoline-8-carbonitrile compound 52 (150 mg, 0.30 mmol) was added to dichloromethane (10 mL), followed by the addition of paraformaldehyde (90 mg, 3.0 mmol) and sodium cyanoborohydride (377 mg, 6.0 mmol). The reaction mixture was stirred at room temperature overnight. After the reaction was completed, the mixture was filtered, and the filtrate was rotary evaporated to dryness. The residue was then purified by flash column chromatography to obtain 5-((1*S*,5*R*)-1-(5-(4-(dimethylamino)bicyclo[2.2.2]octan-1-yl)-1,3,4-oxadiazol-2-yl)-5-(trifluoromethyl)-3-azabicyclo[3.1.0]hexan-3-yl)quinoline-8-carbonitrile compound 53 (56 mg, yield: 35.7%).

LCMS m/z(ESI)=523.2[M+1].

¹H NMR (400 MHz, Chloroform-*d*) δ 9.06 (d, 1H), 8.38 (d, 1H), 8.03 (d, 1H), 7.53 (dd, 1H), 7.17 (d, 1H), 4.11 (d, 1H), 4.04 - 3.88 (m, 3H), 3.77 (dd, 2H), 3.63 - 3.45 (m, 2H), 3.38 (dd, 1H), 2.92 (dt, 2H), 2.30 (d, 1H), 2.05 (s, 12H).

¹⁹F NMR (377 MHz, Chloroform-*d*) δ -65.27.

### Example 54

(*S*)-*N*-(4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)bicyclo[2.2.2]octan-1-yl)morpholine-3-carboxamide compound 54
(S)-N-(4-(5-((1S,5R)-3-(8-cyanoquinolin-5-yl)-5-(trifluoromethyl)-3-azabicyclo[3.1.0]hexan-1-yl)-1,3,4-oxadiazol-2-yl)bicyclo[2.2.2]octan-1-yl)morpholine-3-carboxamide

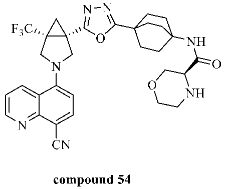

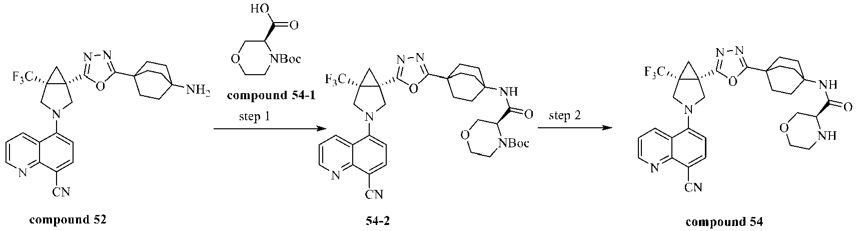

### Step 1:

*tert*-butyl (*S*)-3-((4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)bicyclo[2.2.2]octan-1-yl)carbamoyl)morpholine-4-carboxylate 54-2
tert-butyl (S)-3-((4-(5-((1S,5R)-3-(8-cyanoquinolin-5-yl)-5-(trifluoromethyl)-3-azabicyclo[3.1.0]hexan-1-yl)-1,3,4-oxadiazol-2-yl)bicyclo[2.2.2]octan-1-yl)carbamoyl)morpholine-4-carboxylate

The starting materials 5-((1*S*,5*R*)-1-(5-(4-aminobicyclo[2.2.2]octan-1-yl)-1,3,4-oxadiazol-2-yl)-5-(trifluoromethyl)-3-azabicyclo[3.1.0]hexan-3-yl)quinoline-8-carbonitrile compound 52 (150 mg, 0.30 mmol) and (*S*)-4-(*tert*-butoxycarbonyl)morpholine-3-carboxylic acid 54-1 (69 mg, 0.30 mmol) were added to *N*,*N*-dimethylformamide (2 mL), followed by the addition of 2-(7-azabenzotriazol-1-yl)-*N,N,N',N'*-tetramethyluronium hexafluorophosphate (228 mg, 0.60 mmol) and *N*,*N*-diisopropylethylamine (116 mg, 0.90 mmol). The reaction mixture was stirred at room temperature for 3 hours. After LCMS confirmed the completion of the reaction, the mixture was directly purified by reverse-phase flash column chromatography to obtain *tert*-butyl (*S*)-3-((4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)bicyclo[2.2.2]octan-1-yl)carbamoyl)morpholine-4-carboxylate 54-2 (110 mg, yield: 51.9%).

LCMS m/z(ESI)=708.3[M+1].

### Step 2:

(*S*)-*N*-(4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)bicyclo[2.2.2]octan-1-yl)morpholine-3-carboxamide compound 54
(S)-N-(4-(5-((1S,5R)-3-(8-cyanoquinolin-5-yl)-5-(trifluoromethyl)-3-azabicyclo[3.1.0]hexan-1-yl)-1,3,4-oxadiazol-2-yl)bicyclo[2.2.2]octan-1-yl)morpholine-3-carboxamide

The starting material *tert*-butyl (*S*)-3-((4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)bicyclo[2.2.2]octan-1-yl)carbamoyl)morpholine-4-carboxylate 54-2 (110 mg, 0.156 mmol) was added to dichloromethane (1 mL), followed by the addition of trifluoroacetic acid (1 mL). The reaction mixture was stirred at room temperature for 1 hour. After the reaction was completed, the mixture was concentrated under reduced pressure. The crude product was purified by column chromatography to obtain (*S*)-*N*-(4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)bicyclo[2.2.2]octan-1-yl)morpholine-3-carboxamide compound 54 (62 mg, yield: 65.4%).

LCMS m/z(ESI)=608.3[M+1].

¹H NMR (400 MHz, Chloroform-*d*) δ 9.08 (dd, 1H), 8.37 (dd, 1H), 8.05 (d, 1H), 7.53 (dd, 1H), 7.18 (d, 1H), 4.11 (d, 1H), 3.97 (dd, 2H), 3.79 (d, 1H), 3.41 (br, 2H), 2.60 (s, 6H), 2.30 (d, 1H), 2.23 (d, 1H), 2.19 - 2.05 (m, 7H), 1.95 (dd, 6H).

¹⁹F NMR (377 MHz, DMSO-*d*₆) δ -64.01.

### Example 55

5-((1*S*,5*R*)-1-(1-(piperidin-4-yl)-1*H*-1,2,4-triazol-3-yl)-5-(trifluoromethyl)-3-azabicyclo[3.1.0]hexan-3-yl)quinoline-8-carbonitrile compound 55
5-((1S,5R)-1-(1-(piperidin-4-yl)-1H-1,2,4-triazol-3-yl)-5-(trifluoromethyl)-3-azabicyclo[3.1.0]hexan-3-yl)quinoline-8-carbonitrile

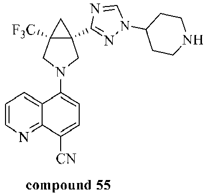

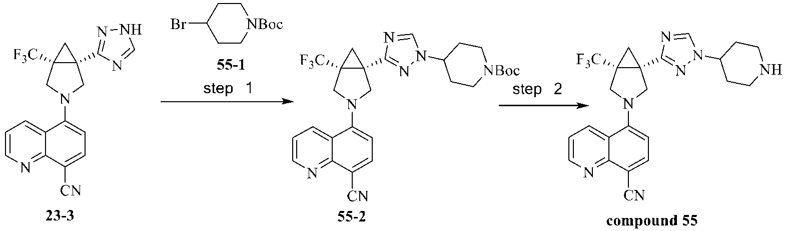

### Step 1:

*tert*-butyl 4-(3-((1*S*,5*R*)-3-(8-cyanoquinolin-5-yl)-5-(trifluoromethyl)-3-azabicyclo[3.1.0]hexan-1-yl)-1H-1,2,4-triazol-1-yl)piperidine-1-carboxylate 55-2
tert-butyl 4-(3-((1S,5R)-3-(8-cyanoquinolin-5-yl)-5-(trifluoromethyl)-3-azabicyclo[3.1.0]hexan-1-yl)-1H-1,2,4-triazol-1-yl)piperidine-1-carboxylate

The starting material 5-((1*S*,5*R*)-1-(1*H*-1,2,4-triazol-3-yl)-5-(trifluoromethyl)-3-azabicyclo[3.1.0]hexan-3-yl)quinoline-8-carbonitrile 23-3 (300 mg, 0.81 mmol) was added to *N*,*N*-dimethylformamide (5 mL), followed by the addition of cesium carbonate (792 mg, 2.43 mmol) and tert-butyl 4-bromopiperidine-1-carboxylate 55-1 (642 mg, 2.43 mmol). The reaction mixture was stirred at 100°C for 6 hours. After the reaction was completed, the mixture was filtered and purified by reverse-phase column chromatography to obtain *tert*-butyl 4-(3-((1*S*,5*R*)-3-(8-cyanoquinolin-5-yl)-5-(trifluoromethyl)-3-azabicyclo[3.1.0]hexan-1-yl)-1*H*-1,2,4-triazol-1-yl)piperidine-1-carboxylate compound 55-2 (178 mg, yield: 39.6%).

LCMS m/z(ESI)=554.2[M+1].

### Step 2:

5-((1*S*,5*R*)-1-(1-(piperidin-4-yl)-1*H*-1,2,4-triazol-3-yl)-5-(trifluoromethyl)-3-azabicyclo[3.1.0]hexan-3-yl)quinoline-8-carbonitrile compound 55
5-((1S,5R)-1-(1-(piperidin-4-yl)-1H-1,2,4-triazol-3-yl)-5-(trifluoromethyl)-3-azabicyclo[3.1.0]hexan-3-yl)quinoline-8-carbonitrile

The starting material *tert*-butyl 4-(3-((1*S*,5*R*)-3-(8-cyanoquinolin-5-yl)-5-(trifluoromethyl)-3-azabicyclo[3.1.0]hexan-1-yl)-1*H*-1,2,4-triazol-1-yl)piperidine-1-carboxylate 55-2 (178 mg, 0.32 mmol) was added to dichloromethane (3 mL), followed by the addition of trifluoroacetic acid (3 mL). The reaction mixture was stirred at room temperature for 1 hour. After the reaction was completed, the mixture was concentrated under reduced pressure. The crude product was purified by reverse-phase column chromatography to obtain 5-((1*S*,5*R*)-1-(1-(piperidin-4-yl)-1*H*-1,2,4-triazol-3-yl)-5-(trifluoromethyl)-3-azabicyclo[3.1.0]hexan-3-yl)quinoline-8-carbonitrile compound 55 (103 mg, yield: 70.7%).

### LCMS m/z(ESI)=454.2[M+1].

¹H NMR (400 MHz, DMSO-*d*₆) δ 9.00 (dd, 1H), 8.69 (dd, 1H), 8.61 (s, 1H), 8.16 (d, 1H), 7.60 (dd, 1H), 7.27 (d, 1H), 4.61 (tt, 1H), 4.10 (s, 2H), 4.04 (d, 1H), 3.92 (d, 1H), 3.11 - 3.01 (m, 3H), 2.29 - 2.03 (m, 5H), 1.93 (d, 1H), 1.19 (t, 2H).

¹⁹F NMR (377 MHz, DMSO-*d*₆) δ -63.32.

### Example 56

2-((*R*)-2-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)morpholino)acetamide compound 56
2-((R)-2-(5-((1S,5R)-3-(8-cyanoquinolin-5-yl)-5-(trifluoromethyl)-3-azabicyclo[3.1.0]hexan-1-yl)-1,3,4-oxadiazol-2-yl)morpholino)acetamide

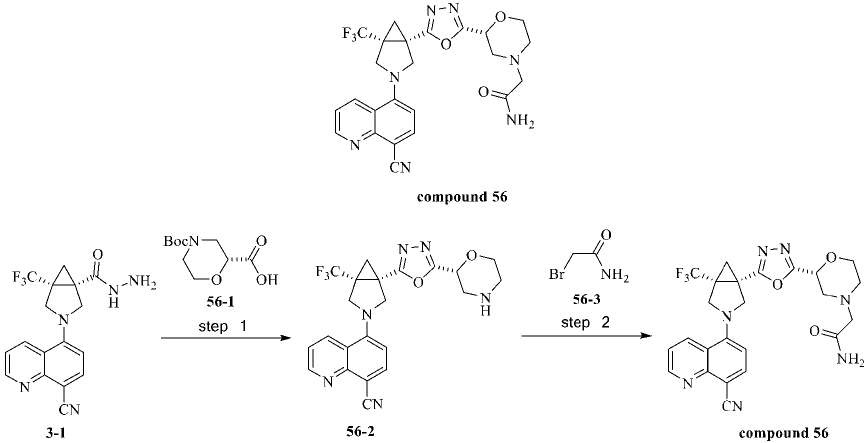

### Step 1:

5-((1*S*,5*R*)-1-(5-((*R*)-morpholin-2-yl)-1,3,4-oxadiazol-2-yl)-5-(trifluoromethyl)-3-azabicyclo[3.1.0]hexan-3-yl)quinoline-8-carbonitrile 56-2
5-((1S,5R)-1-(5-((R)-morpholin-2-yl)-1,3,4-oxadiazol-2-yl)-5-(trifluoromethyl)-3-azabicyclo[3.1.0]hexan-3-yl)quinoline-8-carbonitrile

The starting materials (1*S*,5*R*)-3-(8-cyanoquinolin-5-yl)-5-(trifluoromethyl)-3-azabicyclo[3.1.0]hexane-1-carbohydrazide 3-1 (723 mg, 2.0 mmol) and (*R*)-4-(*tert-*butoxycarbonyl)morpholine-2-carboxylic acid 56-1 (463 mg, 2.0 mmol) were added to phosphorus oxychloride (5 mL). After nitrogen protection, the reaction mixture was heated to 100°C and stirred at 100°C for 4 hours until the reaction was completed. The reaction mixture was cooled to room temperature, diluted with dichloromethane (50 mL), then slowly added to a saturated aqueous sodium carbonate solution, and stirred for half an hour. Ethyl acetate (100 mL × 3) was added for extraction, and the organic phases were combined. The organic phase was washed with saturated brine, dried over anhydrous sodium sulfate, filtered, and rotary evaporated to dryness. The crude product was purified by reverse-phase column chromatography to obtain 5-((1*S*,5*R*)-1-(5-((*R*)-morpholin-2-yl)-1,3,4-oxadiazol-2-yl)-5-(trifluoromethyl)-3-azabicyclo[3.1.0]hexan-3-yl)quinoline-8-carbonitrile 56-2 (138 mg, yield: 14.7%).

LCMS m/z(ESI)=457.2[M+1].

### Step 2:

2-((*R*)-2-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)morpholino)acetamide compound 56
2-((R)-2-(5-((1S,5R)-3-(8-cyanoquinolin-5-yl)-5-(trifluoromethyl)-3-azabicyclo[3.1.0]hexan-1-yl)-1,3,4-oxadiazol-2-yl)morpholino)acetamide

The starting material 5-((1*S*,5*R*)-1-(5-((*R*)-morpholin-2-yl)-1,3,4-oxadiazol-2-yl)-5-(trifluoromethyl)-3-azabicyclo[3.1.0]hexan-3-yl)quinoline-8-carbonitrile 56-2 (150 mg, 0.33 mmol) was added to *N*,*N*-dimethylformamide (3 mL), followed by the addition of cesium carbonate (326 mg, 1.0 mmol) and bromoacetamide (48 mg, 0.35 mmol). The reaction mixture was stirred at room temperature for 4 hours. After the reaction was completed, the mixture was directly purified by reverse-phase column chromatography to obtain 2-((*R*)-2-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)morpholino)acetamide compound 56 (78 mg, yield: 46.3%).

LCMS m/z(ESI)=514.2[M+1].

¹H NMR (400 MHz, DMSO-*d*₆) δ 9.02 (d, 1H), 8.68 (d, 1H), 8.20 (d, 1H), 7.62 (dd, 1H), 7.49 - 7.27 (m, 2H), 7.24 (s, 1H), 5.01 (d, 1H), 4.20 (d, 1H), 4.15 - 4.01 (m, 2H), 3.94 (d, 1H), 3.77 (dt, 2H), 3.09 - 2.83 (m, 3H), 2.69 (dd, 2H), 2.43 (d, 1H), 2.24 (d, 2H).

¹⁹F NMR (377 MHz, DMSO-*d*₆) δ -64.01.

### Example 57

3-fluoro-4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 57
3-fluoro-4-((1S,5R)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile

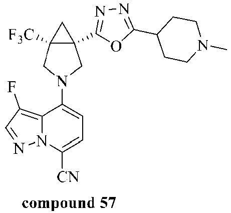

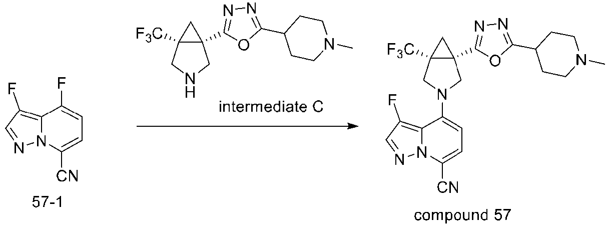

The starting materials 3,4-difluoropyrazolo[1,5-*a*]pyridine-7-carbonitrile 57-1 (358 mg, 2.0 mmol) and 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (949 mg, 3.0 mmol) were added to dimethyl sulfoxide (3 mL), followed by the addition of *N*,*N-*diisopropylethylamine (2.58 g, 20.0 mmol). After nitrogen protection, the reaction mixture was heated to 130°C and reacted for 6 hours. After the reaction was completed, the mixture was directly purified by reverse-phase column chromatography to obtain 3-fluoro-4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 57 (136 mg, yield: 14.3%).

LC-MS m/z (ESI)= 476.20 [M+1].

¹H NMR (400 MHz, DMSO-*d*₆) δ 8.27 (d, 1H), 7.65 (d, 1H), 6.50 (d, 1H), 4.24 (d, 1H), 4.16 (t, 2H), 3.99 (d, 1H), 2.93 (tt, 1H), 2.72 (d, 2H), 2.28 (d, 1H), 2.16 (s, 3H), 2.08 - 1.85 (m, 5H), 1.71 (tdd, 2H).

¹⁹F NMR (377 MHz, DMSO-*d*₆) δ -64.46, -167.61.

### Example 58

2-chloro-5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile compound 58
2-chloro-5-((1S,5R)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile

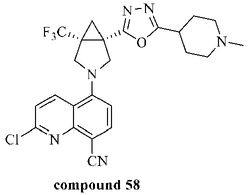

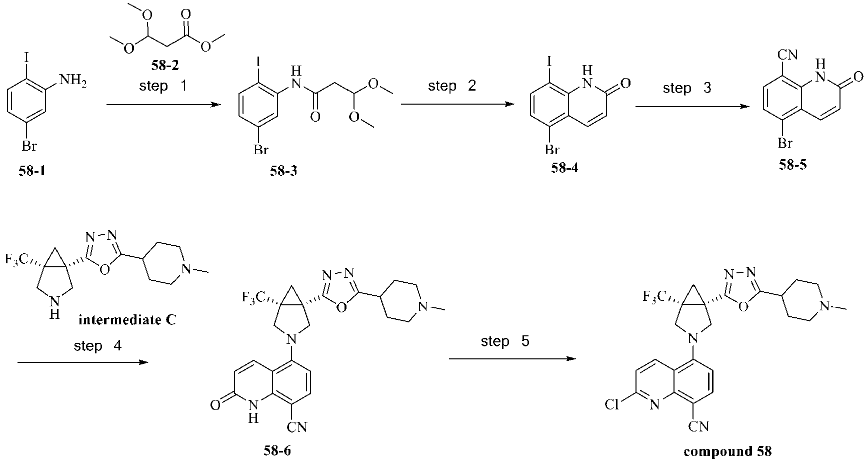

### Step 1:

*N*-(5-bromo-2-iodophenyl)-3,3-dimethoxypropanamide compound 58-3
N-(5-bromo-2-iodophenyl)-3,3-dimethoxypropanamide

The starting materials 5-bromo-2-iodoaniline 58-1 (10.0 g, 33.6 mmol) and methyl 3,3-dimethoxypropionate 58-2 (5.97 g, 40.3 mmol) were added to anhydrous tetrahydrofuran (100 mL). After nitrogen protection, the mixture was cooled to 0°C, and a solution of sodium bis(trimethylsilyl)amide (50 mL, 1 mol/L in tetrahydrofuran) was slowly added dropwise. The reaction mixture was gradually warmed to room temperature and stirred for 6 hours until the reaction was completed. Saturated aqueous ammonium chloride solution and ethyl acetate (100 mL × 3) were added for extraction. The organic phases were combined, washed with saturated brine, dried over anhydrous sodium sulfate, filtered, and concentrated. *N*-(5-Bromo-2-iodophenyl)-3,3-dimethoxypropanamide 58-3 (12.0 g, yield: 86.4%) was obtained.

LC-MS m/z (ESI)= 413.90 [M+1].

### Step 2:

bromo-8-iodoquinolin-2(1*H*)-one 58-4
bromo-8-iodoquinolin-2(1H)-one

The starting material *N*-(5-bromo-2-iodophenyl)-3,3-dimethoxypropanamide 58-3 (12.0 g, 29.0 mmol) was dissolved in dichloromethane (200 mL). The reaction mixture was cooled to 0°C, and then concentrated sulfuric acid (300 mL) was added. The reaction mixture was slowly warmed to room temperature and stirred for 8 hours. After the reaction was completed, the phases were separated. The lower sulfuric acid layer was slowly added dropwise to ice water (1 L), resulting in the precipitation of a large amount of solid. The mixture was filtered, and the filter cake was dried. 5-Bromo-8-iodoquinolin-2(1*H*)-one 58-4 (9.0 g, yield: 88.7%) was obtained.

LC-MS m/z (ESI)= 349.90 [M+1].

### Step 3:

bromo-2-oxo-1,2-dihydroquinoline-8-carbonitrile 58-5
bromo-2-oxo-1,2-dihydroquinoline-8-carbonitrile

The starting material 5-bromo-8-iodoquinolin-2(1*H*)-one 58-4 (9.0 g, 25.7 mmol) was added to *N*,*N*-dimethylformamide (90 mL), followed by the addition of zinc cyanide (1.5 g, 12.9 mmol) and tetrakis(triphenylphosphine)palladium (2.97 g, 2.57 mmol). After nitrogen protection, the reaction mixture was heated to 100°C and reacted for 3 hours. After the reaction was completed, purified water (300 mL) was added, and a large amount of solid precipitated. The mixture was filtered, and the filter cake was purified by column chromatography to obtain 5-bromo-2-oxo-1,2-dihydroquinoline-8-carbonitrile 58-5 (3.5 g, yield: 54.9%).

LC-MS m/z (ESI)= 248.90 [M+1].

### Step 4:

5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)-2-oxo-1,2-dihydroquinoline-8-carbonitrile 58-6
5-((1S,5R)-1-(5-(1-methylpiperidin-4-yl)-1,3,4-oxadiazol-2-yl)-5-(trifluoromethyl)-3-azabicyclo[3.1.0]hexan-3-yl)-2-oxo-1,2-dihydroquinoline-8-carbonitrile

The starting materials 5-bromo-2-oxo-1,2-dihydroquinoline-8-carbonitrile 58-5 (249 mg, 1.0 mmol) and 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (476 mg, 1.5 mmol) were added to 1,4-dioxane (3 mL), followed by the addition of cesium carbonate (977 mg, 3.0 mmol) and methanesulfonato(2-dicyclohexylphosphino-2',6'-di-i-propoxy-1,1'-biphenyl)(2'-amino-1,1'-biphenyl-2-yl)palladium(II) (84 mg, 0.1 mmol). After nitrogen protection, the reaction mixture was heated to 100°C and reacted for 3 hours. After the reaction was completed, the mixture was directly purified by reverse-phase column chromatography to obtain 5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)-2-oxo-1,2-dihydroquinoline-8-carbonitrile 58-6 (89 mg, yield: 18.4%).

LC-MS m/z (ESI)= 485.20 [M+1].

¹H NMR (400 MHz, DMSO-d6) δ 8.17 (d, 1H), 7.87 (d, 1H), 7.07 (d, 1H), 6.59 (d, 1H), 4.10 - 4.01 (m, 2H), 3.96 (d, 1H), 3.88 (d, 1H), 2.93 (ddt, 1H), 2.73 (d, 2H), 2.23 - 2.12 (m, 5H), 2.10 - 2.04 (m, 2H), 2.00 - 1.92 (m, 2H), 1.72 (dddd, 2H).

¹⁹F NMR (377 MHz, DMSO-d6) δ -64.03.

### Step 5:

2-chloro-5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile compound 58
2-chloro-5-((1S,5R)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile

The starting material 5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)-2-oxo-1,2-dihydroquinoline-8-carbonitrile 58-6 (30 mg, 0.062 mmol) was added to phosphorus oxychloride (1 mL). The reaction mixture was heated to 100°C and reacted for 3 hours. After the reaction was completed, saturated aqueous sodium carbonate solution and ethyl acetate (10 mL × 3) were added. The organic phases were combined, washed with saturated brine, dried over anhydrous sodium sulfate, filtered, and concentrated. The crude product was purified by reverse-phase column chromatography to obtain 2-chloro-5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile compound 58 (15 mg, yield: 48.1%).

LC-MS m/z (ESI)= 503.20 [M+1].

¹H NMR (400 MHz, DMSO-*d*₆) δ 8.75 (d, 1H), 8.24 (d, 1H), 7.63 (d, 1H), 7.34 (d, 1H), 4.22 (d, 1H), 4.12 (dd, 2H), 3.98 (d, 1H), 3.64 (d, 1H), 3.21 (dd, 1H), 2.85 (d, 1H), 2.67 (d, 4H), 2.34 - 1.94 (m, 7H).

¹⁹F NMR (377 MHz, DMSO-*d*₆) δ -64.12.

### Example 59

4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 59
4-((1S,5R)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-a]pyridine-7-carbonitrile

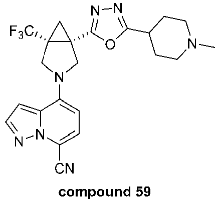

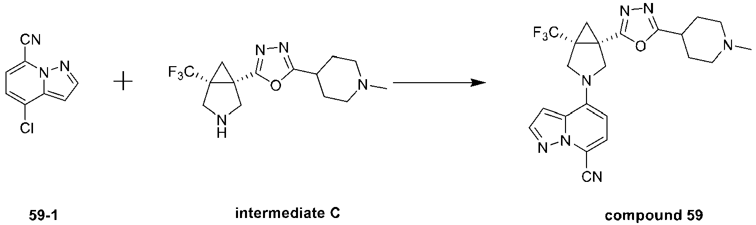

Under an N₂ atmosphere, (2S,6S)-2-methyl-6-(5-(1-methylpiperidin-4-yl)-1,3,4-oxadiazol-2-yl)morpholine intermediate C (500 mg, 1.16 mmol) was dissolved in 1,4-dioxane (4.00 mL), followed by the sequential addition of 4-chloropyrazolo[1,5-*a*]pyridine-7-carbonitrile 59-1 (281 mg, 1.16 mmol), cesium carbonate (3.10 g, 8.12 mmol), RuPhosPdG3 (92.4 mg, 0.11 mmol), and water (1.0 mL). The mixture was purged with nitrogen three times, heated to 80°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated and purified by reverse-phase C18 column chromatography (acidic method) to obtain 450 mg of crude product. TLC (DCM: MeOH = 10:1) was performed to obtain the target product 4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 59 (yellowish-white solid, 90 mg, yield: 12.4%).

LC-MS m/z (ESI)= 458.10 [M+1].

¹H NMR (400 MHz, Chloroform-d) δ 7.94 (d, 1H), 7.16 (d, 1H), 6.78 (d, 1H), 6.01 (d, 1H), 4.38 (d, 1H), 4.27 (d, 1H), 4.19 (d, 1H), 4.04 (d, 1H), 2.85 (d, 3H), 2.35 (d, 1H), 2.28 (s, 3H), 2.19 - 1.99 (m, 4H), 1.91 (q, 2H), 1.69 (dd, 1H).

### Example 60

5-((1*S*,5*R*)-1-(5-(4-fluoropiperidin-4-yl)-1,3,4-oxadiazol-2-yl)-5-(trifluoromethyl)-3-azabicyclo[3.1.0]hexan-3-yl)quinoline-8-carbonitrile compound 60
5-((1S,5R)-1-(5-(4-fluoropiperidin-4-yl)-1,3,4-oxadiazol-2-yl)-5-(trifluoromethyl)-3-azabicyclo[3.1.0]hexan-3-yl)quinoline-8-carbonitrile

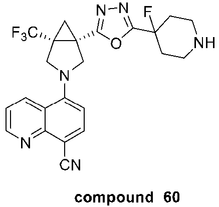

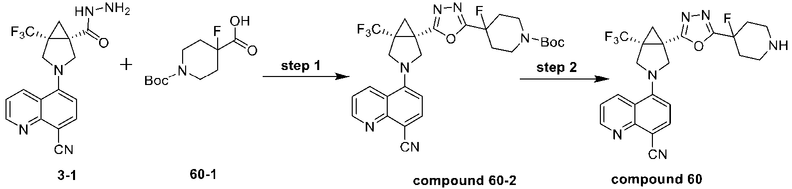

### Step 1:

*tert*-butyl 4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-4-fluoropiperidine-1-carboxylate compound 60-2
4-(5-((1S,5R)-3-(8-cyanoquinolin-5-yl)-5-(trifluoromethyl)-3-azabicyclo[3.1.0]hexan-1-yl)-1,3,4-oxadiazol-2-yl)-4-fluoropiperidine-1-carboxylate
1-(*tert*-Butoxycarbonyl)-4-fluoropiperidine-4-carboxylic acid 60-1 (49 mg, 0.2 mmol) was dissolved in 20 mL of dichloromethane, followed by the addition of HATU (91 mg, 0.24 mmol) and DIPEA (77 mg, 0.6 mmol). The mixture was stirred at low temperature for 5 minutes, and then (1S,5R)-3-(8-cyanoquinolin-5-yl)-5-(trifluoromethyl)-3-azabicyclo[3.1.0]hexane-1-carbohydrazide 3-1 (72 mg, 0.2 mmol) was added. The reaction was stirred for 2 hours. After TLC confirmed the completion of the reaction, Burgess reagent (142 mg, 0.6 mmol) was added, and the mixture was stirred for 3 hours. After LCMS confirmed the completion of the reaction, the reaction mixture was directly concentrated. The residue was purified by reverse-phase C18 column chromatography (acidic method) to obtain the target product *tert*-butyl 4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-4-fluoropiperidine-1-carboxylate 60-2 (pale yellow solid, 70 mg, 61.4%).

LC-MS m/z (ESI)= 573.10 [M+1].

### Step 2:

5-((1*S*,5*R*)-1-(5-(4-fluoropiperidin-4-yl)-1,3,4-oxadiazol-2-yl)-5-(trifluoromethyl)-3-azabicyclo[3.1.0]hexan-3-yl)quinoline-8-carbonitrile compound 60
5-((1S,5R)-1-(5-(4-fluoropiperidin-4-yl)-1,3,4-oxadiazol-2-yl)-5-(trifluoromethyl)-3-azabicyclo[3.1.0]hexan-3-yl)quinoline-8-carbonitrile

The crude product *tert*-butyl 4-(5-((1*S*,5*R*)-3-(8-cyanoquinolin-5-yl)-5-(trifluoromethyl)-3-azabicyclo[3.1.0]hexan-1-yl)-1,3,4-oxadiazol-2-yl)-4-fluoropiperidine-1-carboxylate 60-2 (70 mg) was added to 5.6 mL of dichloromethane and 1.4 mL of trifluoroacetic acid. After stirring for half an hour, LCMS confirmed the completion of the reaction. The reaction mixture was concentrated to obtain the product compound 60 (pale yellow solid, 20 mg, 36.3%).

LC-MS m/z (ESI)= 473.10 [M+1].

¹H NMR (400 MHz, Chloroform-*d*) δ 9.02 (dd, 1H), 8.30 (dd, 1H), 7.98 (d, 1H), 7.47 (dd, 1H), 7.11 (d, 1H), 4.09 (d, 1H), 3.92 (dd, 2H), 3.74 (d, 1H), 3.06 (dt, 2H), 2.91 (ddt, 2H), 2.31 (d, 1H), 2.21 (s, 2H), 2.22 - 2.11 (m, 3H), 1.97 (s, 1H).

### Example 61

2-methoxy-5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile compound 61
2-methoxy-5-((1S,5R)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile 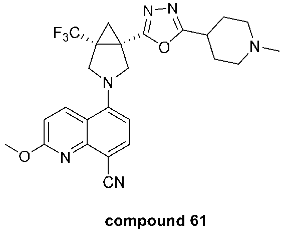 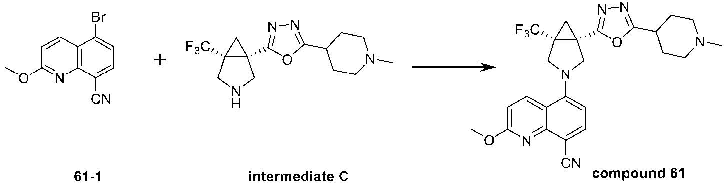
2-methoxy-5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile compound 61
2-methoxy-5-((1S,5R)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile

Under an N₂ atmosphere, 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (442 mg, 1.4 mmol) was dissolved in 1,4-dioxane (28.0 mL), followed by the sequential addition of 5-bromo-2-methoxyquinoline-8-carbonitrile 61-1 (403 mg, 1.54 mmol), cesium carbonate (3.80 g, 9.8 mmol), RuPhosPdG3 (117 mg, 0.14 mmol), and water (2.8 mL). The mixture was purged with nitrogen three times, heated to 80°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated and purified by reverse-phase C18 column chromatography (acidic method) to obtain 450 mg of the crude product. TLC (DCM: MeOH = 10:1) was performed to obtain the target product 2-methoxy-5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile compound 61 (yellowish-white solid, 50 mg, yield: 7.2%).

LC-MS m/z (ESI)= 499.10 [M+1].

¹H NMR (400 MHz, Chloroform-*d*) δ 8.14 (d, 1H), 7.91 (d, 1H), 6.99 (dd, 2H), 4.16 (s, 3H), 4.07 (d, 1H), 3.90 (dd, 3H), 3.75 (d, 1H), 2.91 (d, 2H), 2.34 (s, 3H), 2.27 (d, 1H), 2.18 (d, 2H), 2.10 (d, 3H), 1.97 (q, 2H)

### Example 62

2-(1-methylpiperidin-4-yl)-5-((1*S*,5*R*)-3-(8-nitroquinolin-5-yl)-5-(trifluoromethyl)-3-azabicyclo[3.1.0]hexan-1-yl)-1,3,4-oxadiazole compound 62
2-(1-methylpiperidin-4-yl)-5-((1S,5R)-3-(8-nitroquinolin-5-yl)-5-(trifluoromethyl)-3-azabicyclo[3.1.0]hexan-1-yl)-1,3,4-oxadiazole

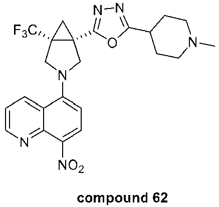

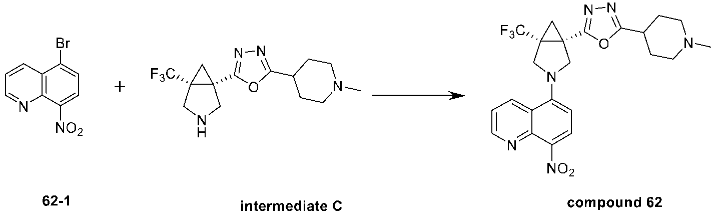

Under an N₂ atmosphere, 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (500 mg, 1.57 mmol) was dissolved in 1,4-dioxane (28.0 mL). Subsequently, 5-bromo-8-nitroquinoline 62-1 (279 mg, 1.57 mmol), cesium carbonate (6.00 g, 11.0 mmol), RuPhosPdG3 (132 mg, 0.16 mmol), and water (2.8 mL) were added sequentially. The mixture was purged with nitrogen three times, heated to 80°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated and purified by reverse-phase C18 column chromatography (acidic method) to obtain 450 mg of the crude product. TLC (DCM: MeOH = 10:1) was performed to obtain the target product 2-methoxy-5-((1S,5R)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile compound 62 (yellowish-white solid, 44 mg, yield: 5.7%).

LC-MS m/z (ESI)= 489.10 [M+1].

¹H NMR (400 MHz, Chloroform-d) δ 9.09 (dd, 1H), 8.38 (dd, 1H), 8.10 (d, 1H), 7.55 (dd, 1H), 7.16 (d, 1H), 4.11 (d, 1H), 4.00 - 3.90 (m, 2H), 3.78 (d, 1H), 2.98 (d, 3H), 2.42 (s, 3H), 2.31 (s, 2H), 2.05 (d, 3H), 1.81 (s, 3H).

### Example 63

3-fluoro-4-((1*S*,5*R*)-1-(5-(4-methylpiperazin-1-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 63
3-fluoro-4-((1S,5R)-1-(5-(4-methylpiperazin-1-yl)-1,3,4-oxadiazol-2-yl)-5-(trifluoromethyl)-3-azabicyclo[3.1.0]hexan-3-yl)pyrazolo[1,5-a]pyridine-7-carbonitrile 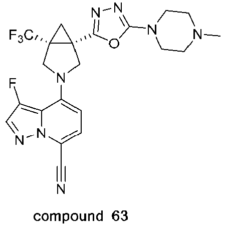 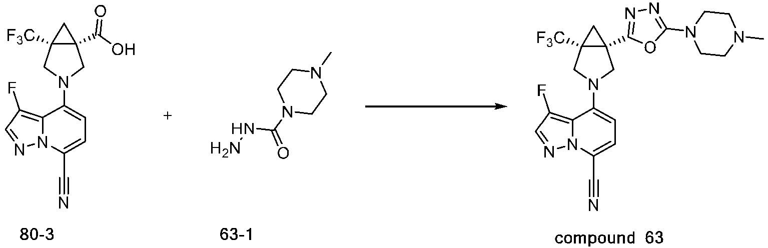
(1*S*,5*R*)-3-(7-Cyano-3-fluoropyrazolo[1,5-*a*]pyridin-4-yl)-5-(trifluoromethyl)-3-azabicyclo[3.1.0]hexane-1-carboxylic acid 80-3 (900 mg, 2.54 mmol) and 4-methylpiperazine-1-carbohydrazide 63-1 (5.5 g, crude) were dissolved in POCl₃ (10 mL) and reacted at 80°C for 2 hours. After LCMS confirmed the completion of the reaction, the pH was adjusted to alkaline with sodium carbonate. The mixture was extracted three times with dichloromethane and rotary evaporated to dryness. The residue was purified by reverse-phase chromatography and further purified by preparative TLC to obtain 3-fluoro-4-((1*S*,5*R*)-1-(5-(4-methylpiperazin-1-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 63 (27 mg, yellow solid).

LCMS m/z (ESI) = 477.16 [M+1].

¹H NMR (400 MHz, Chloroform-d) δ 7.93 (d, 1H), 7.20 (d, 1H), 6.20 (d, 1H), 4.08 (t, 2H), 3.85 (d, 1H), 3.69 (s, 4H), 2.69 (s, 4H), 2.48 (s, 4H), 2.25 (d, 1H), 1.81 (d, 1H).

### Example 64

2-(1-methylpiperidin-4-yl)-5-((1*S*,5*R*)-3-(8-nitroquinolin-5-yl)-5-(trifluoromethoxy)-3-azabicyclo[3.1.0]hexan-1-yl)-1,3,4-oxadiazole compound 64
2-(1-methylpiperidin-4-yl)-5-((1S,5R)-3-(8-nitroquinolin-5-yl)-5-(trifluoromethyl)-3-azabicyclo[3.1.0]hexan-1-yl)-1,3,4-oxadiazole 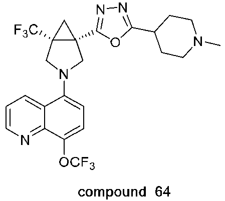 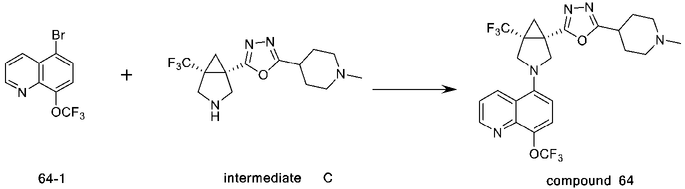
2-(1-methylpiperidin-4-yl)-5-((1*S*,5*R*)-5-(trifluoromethyl)-3-(8-(trifluoromethoxy)quinolin-5-yl)-3-azabicyclo[3.1.0]hexan-1-yl)-1,3,4-oxadiazole compound 64
2-(1-methylpiperidin-4-yl)-5-((1S,5R)-3-(8-(trifluoromethoxy)quinolin-5-yl)-5-(trifluoromethyl)-3-azabicyclo[3.1.0]hexan-1-yl)-1,3,4-oxadiazole

Under an N₂ atmosphere, 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (500 mg, 1.57 mmol) was dissolved in 1,4-dioxane (28.0 mL). Subsequently, 5-bromo-8-trifluoromethylquinoline 64-1 (440 mg, 1.57 mmol), cesium carbonate (6.00 g, 11.0 mmol), RuPhosPdG3 (132 mg, 0.16 mmol), and water (2.8 mL) were added sequentially. The mixture was purged with nitrogen three times, heated to 80°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated and purified by reverse-phase C18 column chromatography (acidic method) to obtain 450 mg of the crude product. TLC (DCM: MeOH = 10:1) was performed to obtain the target product 2-(1-methylpiperidin-4-yl)-5-((1*S*,5*R*)-5-(trifluoromethyl)-3-(8-(trifluoromethoxy)quinolin-5-yl)-3-azabicyclo[3.1.0]hexan-1-yl)-1,3,4-oxadiazole compound 64 (yellowish-white solid, 200 mg, yield: 24.8%).

LC-MS m/z (ESI)= 528.10 [M+1].

¹H NMR (400 MHz, Chloroform-*d*) δ 9.04 (dd, 1H), 8.37 (dd, 1H), 7.63 - 7.45 (m, 2H), 7.22 (d, 1H), 4.00 (d, 1H), 3.85 - 3.72 (m, 2H), 3.67 (d, 1H), 2.95 (d, 3H), 2.37 (d, 4H), 2.27 (d, 2H), 2.17-2.03 (m, 5H).

### Example 65

2-(1-methylpiperidin-4-yl)-5-((1*S*,5*R*)-3-(8-nitroquinolin-5-yl)-5-(trifluoromethoxy)-3-azabicyclo[3.1.0]hexan-1-yl)-1,3,4-oxadiazole compound 65
2-(1-methylpiperidin-4-yl)-5-((1S,5R)-3-(8-nitroquinolin-5-yl)-5-(trifluoromethyl)-3-azabicyclo[3.1.0]hexan-1-yl)-1,3,4-oxadiazole 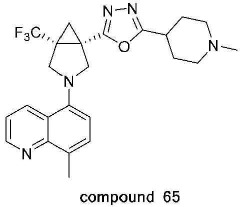 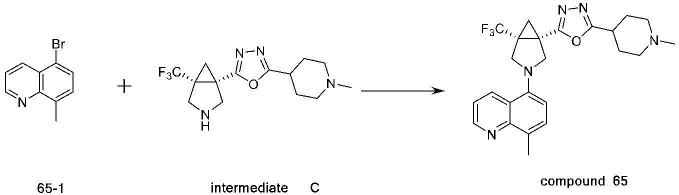
2-(1-methylpiperidin-4-yl)-5-((1*S*,5*R*)-5-(trifluoromethyl)-3-(8-methylquinolin-5-yl)-3-azabicyclo[3.1.0]hexan-1-yl)-1,3,4-oxadiazole compound 65
2-(1-methylpiperidin-4-yl)-5-((1S,5R)-3-(8-methylquinolin-5-yl)-5-(trifluoromethyl)-3-azabicyclo[3.1.0]hexan-1-yl)-1,3,4-oxadiazole

Under an N₂ atmosphere, 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (500 mg, 1.57 mmol) was dissolved in 1,4-dioxane (28.0 mL). Subsequently, 5-bromo-8-methylquinoline 65-1 (386 mg, 1.57 mmol), cesium carbonate (6.00 g, 11.0 mmol), RuPhosPdG3 (132 mg, 0.16 mmol), and water (2.8 mL) were added sequentially. The mixture was purged with nitrogen three times, heated to 80°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated and purified by reverse-phase C18 column chromatography (acidic method) to obtain 450 mg of the crude product. TLC (DCM: MeOH = 10:1) was performed to obtain the target product 2-(1-methylpiperidin-4-yl)-5-((1*S*,5*R*)-5-(trifluoromethyl)-3-(8-methylquinolin-5-yl)-3-azabicyclo[3.1.0]hexan-1-yl)-1,3,4-oxadiazole compound 65 (yellowish-white solid, 210 mg, yield: 24.8%).

LC-MS m/z (ESI)= 458.10 [M+1].

¹H NMR (400 MHz, DMSO-*d*₆) δ 8.91 (dd, 1H), 8.52 (dd, 1H), 7.57 - 7.49 (m, 2H), 7.33 (d, 1H), 3.83 (d, 1H), 3.82 - 3.72 (m, 2H), 3.63 (d, 1H), 2.93 (tt, 1H), 2.72 (d, 2H), 2.65 (s, 3H), 2.47 (d, 1H), 2.18 (d, 1H), 2.16 (s, 3H), 2.08 - 1.91 (m, 4H), 1.72 (dddd, 2H).

### Example 66

2-((1*S*,5*R*)-3-(8-methoxyquinolin-5-yl)-5-(trifluoromethyl)-3-azabicyclo[3.1.0]hexan-1-yl)-5-(1-methylpiperidin-4-yl)-1,3,4-oxadiazole compound 66
2-((1S,5R)-3-(8-methoxyquinolin-5-yl)-5-(trifluoromethyl)-3-azabicyclo[3.1.0]hexan-1-yl)-5-(1-methylpiperidin-4-yl)-1,3,4-oxadiazole

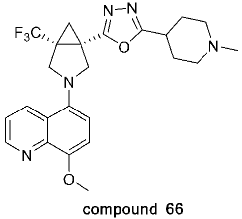

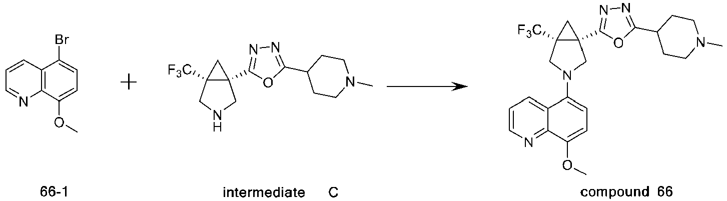

Under an N₂ atmosphere, 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (500 mg, 1.57 mmol) was dissolved in 1,4-dioxane (28.0 mL). Subsequently, 5-bromo-8-methoxyquinoline 66-1 (276 mg, 1.57 mmol), cesium carbonate (6.00 g, 11.0 mmol), RuPhosPdG3 (132 mg, 0.16 mmol), and water (2.8 mL) were added sequentially. The mixture was purged with nitrogen three times, heated to 80°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated and purified by reverse-phase C18 column chromatography (acidic method) to obtain 450 mg of the crude product. TLC (DCM: MeOH = 10:1) was performed to obtain the target product 2-((1*S*,5*R*)-3-(8-methoxyquinolin-5-yl)-5-(trifluoromethyl)-3-azabicyclo[3.1.0]hexan-1-yl)-5-(1-methylpiperidin-4-yl)-1,3,4-oxadiazole compound 66 (yellowish-white solid, 70 mg, yield: 9.3%).

LC-MS m/z (ESI)= 474.10 [M+1].

¹H NMR (400 MHz, Chloroform-*d*) δ 8.95 (dd, 1H), 8.35 (dd, 1H), 7.47 (dd, 1H), 7.27 (d, 1H), 6.99 (d, 1H), 4.09 (s, 3H), 3.93 (d, 1H), 3.74 - 3.65 (m, 2H), 3.59 (d, 1H), 2.92 (d, 3H), 2.37 (d, 4H), 2.30 - 2.07 (m, 5H), 2.07 - 1.94 (m, 2H).

### Example 67

2-((1*S*,5*R*)-3-(8-chloro-5-yl)-5-(trifluoromethyl)-3-azabicyclo[3.1.0]hexan-1-yl)-5-(1-methylpiperidin-4-yl)-1,3,4-oxadiazole compound 67
2-((1S,5R)-3-(8-chloroquinolin-5-yl)-5-(trifluoromethyl)-3-azabicyclo[3.1.0]hexan-1-yl)-5-(1-methylpiperidin-4-yl)-1,3,4-oxadiazole

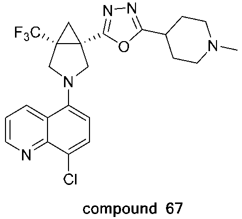

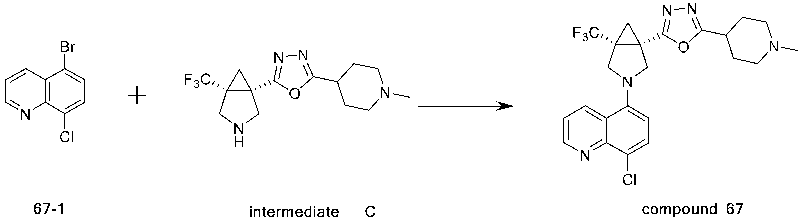

Under an N₂ atmosphere, 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (500 mg, 1.57 mmol) was dissolved in 1,4-dioxane (28.0 mL). Subsequently, 5-bromo-8-chloroquinoline 67-1 (281 mg, 1.57 mmol), cesium carbonate (6.00 g, 11.0 mmol), RuPhosPdG3 (132 mg, 0.16 mmol), and water (2.8 mL) were added sequentially. The mixture was purged with nitrogen three times, heated to 80°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated and purified by reverse-phase C18 column chromatography (acidic method) to obtain 450 mg of the crude product. TLC (DCM: MeOH = 10:1) was performed to obtain the target product 2-((1*S*,5*R*)-3-(8-chloroquinolin-5-yl)-5-(trifluoromethyl)-3-azabicyclo[3.1.0]hexan-1-yl)-5-(1-methylpiperidin-4-yl)-1,3,4-oxadiazole compound 67 (yellowish-white solid, 40 mg, yield: 5.3%).

LC-MS m/z (ESI)= 478.10 [M+1].

¹H NMR (400 MHz, Chloroform-*d*) δ 9.06 (dd, 1H), 8.38 (dd, 1H), 7.78 (d, 1H), 7.50 (dd, 1H), 7.20 (d, 1H), 3.98 (d, 1H), 3.83 - 3.71 (m, 2H), 3.65 (d, 1H), 2.94 (d, 3H), 2.36 (d, 4H), 2.26 (d, 2H), 2.14 (s, 2H), 2.01 (d, 3H).

### Example 68

5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)-1,7-naphthyridine-8-carbonitrile compound 68
5-((1S,5R)-1-(5-(1-methylpiperidin-4-yl)-1,3,4-oxadiazol-2-yl)-5-(trifluoromethyl)-3-azabicyclo[3.1.0]hexan-3-yl)-1,7-naphthyridine-8-carbonitrile

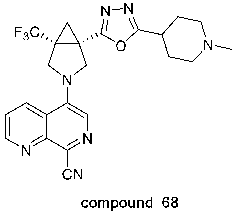

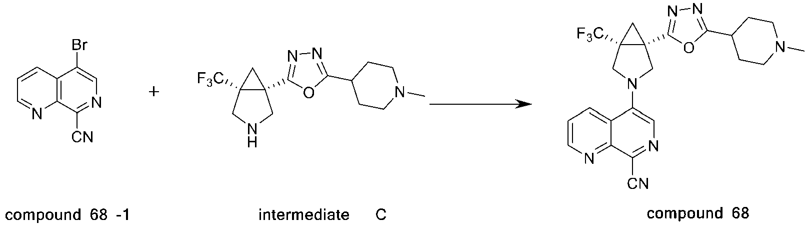

Under an N₂ atmosphere, 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (300 mg, 0.697 mmol) was dissolved in 1,4-dioxane (28.0 mL). Subsequently, 5-bromo-1,7-naphthyridine-8-carbonitrile 68-1 (162 mg, 1.57 mmol), cesium carbonate (1.585 g, 4.88 mmol), RuPhosPdG3 (118 mg, 0.14 mmol), and water (2.8 mL) were added sequentially. The mixture was purged with nitrogen three times, heated to 80°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated and purified by reverse-phase C18 column chromatography (acidic method) to obtain 450 mg of the crude product. TLC (DCM: MeOH = 10:1) was performed to obtain the target product 5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)-1,7-naphthyridine-8-carbonitrile compound 68 (yellowish-white solid, 40 mg, yield: 12.23%).

LC-MS m/z (ESI)= 470.10 [M+1].

¹H NMR (400 MHz, Chloroform-*d*) δ 9.21 - 9.14 (m, 1H), 8.44 - 8.31 (m, 2H), 7.70 (ddd, 1H), 4.31 (dd, 1H), 4.17 - 4.07 (m, 2H), 3.98 (d, 1H), 3.80 - 3.72 (m, 1H), 3.50 (d, 2H), 3.19 (s, 1H), 3.03 (s, 1H), 2.62 (t, 2H), 2.42 - 2.34 (m, 2H), 2.17 (d, 1H) 2.09 (s, 4H)

### Example 69

2-((1*S*,5*R*)-3-(8-fluoroquinolin-5-yl)-5-(trifluoromethyl)-3-azabicyclo[3.1.0]hexan-1-yl)-5-(1-methylpiperidin-4-yl)-1,3,4-oxadiazole compound 69
2-((1S,5R)-3-(8-fluoroquinolin-5-yl)-5-(trifluoromethyl)-3-azabicyclo[3.1.0]hexan-1-yl)-5-(1-methylpiperidin-4-yl)-1,3,4-oxadiazole

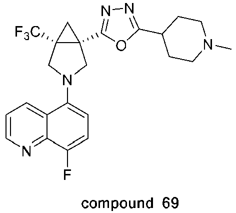

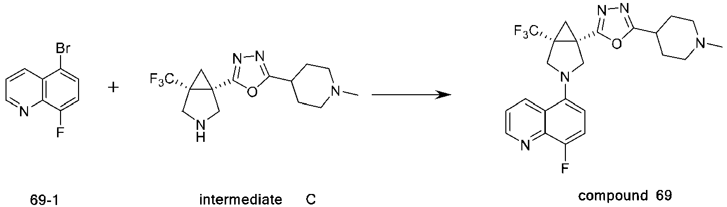

Under an N₂ atmosphere, 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (723 mg, 2.28 mmol) was dissolved in 1,4-dioxane (20.0 mL). Subsequently, 5-bromo-8-fluoroquinoline 69-1 (452 mg, 2.00 mmol), cesium carbonate (2.6 g, 8.00 mmol), RuPhosPdG3 (335 mg, 0.40 mmol), and water (2.0 mL) were added sequentially. The mixture was purged with nitrogen three times, heated to 90°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated and purified by reverse-phase C18 column chromatography (acidic method) to obtain 450 mg of the crude product. TLC (DCM: MeOH = 10:1) was performed to obtain the target product 2-((1*S*,5*R*)-3-(8-fluoroquinolin-5-yl)-5-(trifluoromethyl)-3-azabicyclo[3.1.0]hexan-1-yl)-5-(1-methylpiperidin-4-yl)-1,3,4-oxadiazole compound 69 (yellowish-white solid, 148 mg, yield: 16.8%).

LC-MS m/z (ESI)= 462.18 [M+1].

¹H NMR (400 MHz, Chloroform-d) δ 8.96 (dd, 1H), 8.38 - 8.32 (m, 1H), 7.48 (dd, 1H), 7.33 (dd, 1H), 7.21 (dd, 1H), 3.94 (d, 1H), 3.71 (dd, 2H), 3.61 (d, 1H), 2.89 (dtd, 3H), 2.40 - 2.31 (m, 1H),2.30(s, 3H), 2.23 (d, 1H), 2.16 - 2.03 (m, 4H), 1.95 (ddd, 2H).

### Example 70

2,3-diethyl-8-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoxaline-5-carbonitrile compound 70
2,3-diethyl-8-((1S,5R)-1-(5-(1-methylpiperidin-4-yl)-1,3,4-oxadiazol-2-yl)-5-(trifluoromethyl)-3-azabicyclo[3.1.0]hexan-3-yl)quinoxaline-5-carbonitrile

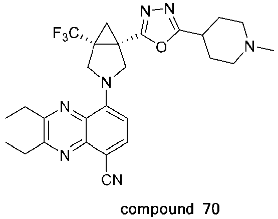

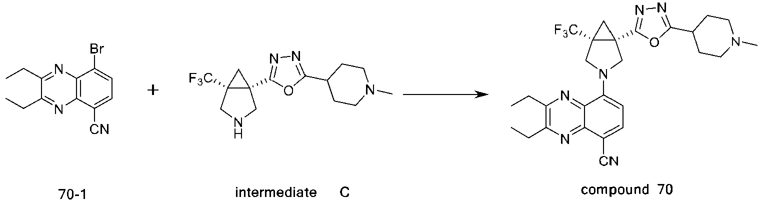

Under an N₂ atmosphere, 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (175 mg, 0.55 mmol) was dissolved in 1,4-dioxane (4.0 mL). Subsequently, 8-bromo-2,3-diethylquinoxaline-5-carbonitrile 70-1 (145 mg, 0.50 mmol), cesium carbonate (975 mg, 3.0 mmol), RuPhosPdG3 (84 mg, 0.1 mmol), and water (0.4 mL) were added sequentially. The mixture was purged with nitrogen three times, heated to 90°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated and purified by reverse-phase C18 column chromatography (acidic method) to obtain 450 mg of the crude product. TLC (DCM: MeOH = 10:1) was performed to obtain the target product 2,3-diethyl-8-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoxaline-5-carbonitrile compound 70 (yellowish-white solid, 10 mg, yield: 3.8%).

LC-MS m/z (ESI)= 526.25 [M+1].

¹H NMR (400 MHz, Chloroform-*d*) δ 7.79 (d, 1H), 6.64 (d, 1H), 4.94 (d, 1H), 4.73 (d, 1H), 4.31 (d, *J* = 1H), 4.06 (d, 1H), 3.42 (s, 3H), 2.98 (m, 5H), 2.38 (s, 2H), 2.30 (d, 1H), 2.03 (s, 2H), 1.75 (d, 1H) 1.55 (m, 4H), 1.39 (t, 3H), 1.33 (t, 3H).

### Example 71

5-((1*S*,5*R*)-1-(1-(1-methylpiperidin-4-yl)-1H-1,2,3-triazol-4-yl)-5-(trifluoromethyl)-3-azabicyclo[3.1.0]hexan-3-yl)quinoline-8-carbonitrile compound 71
5-((1S,5R)-1-(1-(1-methylpiperidin-4-yl)-1H-1,2,3-triazol-4-yl)-5-(trifluoromethyl)-3-azabicyclo[3.1.0]hexan-3-yl)quinoline-8-carbonitrile

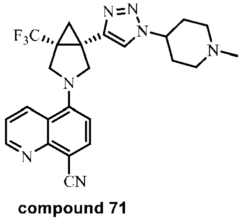

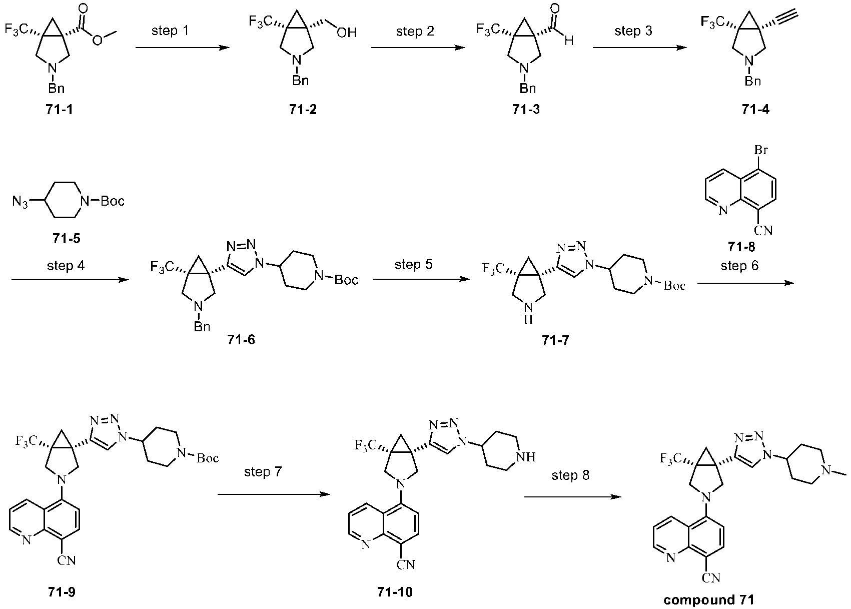

### Step 1:

((1*S*,5*R*)-3-benzyl-5-(trifluoromethyl)-3-azabicyclo[3.1.0]hexan-1-yl)methanol 71-2
((1S,5R)-3-benzyl-5-(trifluoromethyl)-3-azabicyclo[3.1.0]hexan-1-yl)methanol

Under an N₂ atmosphere, compound 71-1 (3.0 g, 10 mmol) was dissolved in 10 mL of tetrahydrofuran, and lithium aluminum hydride (0.38 g, 10 mmol) was added in portions under an ice bath. The mixture was stirred at room temperature for 2 h. After the reaction was completed, the mixture was quenched with saturated ammonium chloride, extracted with DCM, washed with saturated brine, dried over anhydrous sodium sulfate, and rotary evaporated to dryness. The residue was purified by silica gel column chromatography (ethyl acetate: petroleum ether = 1:5) to obtain the target product ((1*S*,5*R*)-3-benzyl-5-(trifluoromethyl)-3-azabicyclo[3.1.0]hexan-1-yl)methanol 71-2 (pale yellow liquid, 2.5 g, yield: 93%).

LC-MS m/z (ESI)= 272.1 [M+1].

### Step 2:

(1*S*,5*R*)-3-benzyl-5-(trifluoromethyl)-3-azabicyclo[3.1.0]hexane-1-carbaldehyde 71-3
(1S,5R)-3-benzyl-5-(trifluoromethyl)-3-azabicyclo[3.1.0]hexane-1-carbaldehyde

Under an N₂ atmosphere, compound 1-2 (1.0 g, 3.7 mmol) was dissolved in 5 mL of dichloromethane, and then DMP (2-iodoxybenzoic acid) oxidant was added in portions. The mixture was stirred at room temperature for 1 h. After the reaction was completed, the mixture was quenched with saturated sodium bicarbonate and extracted with DCM. The organic phase was washed with saturated brine, dried over anhydrous sodium sulfate, and rotary evaporated to dryness. The residue was purified by silica gel column chromatography (ethyl acetate: petroleum ether = 1:10) to obtain the target product (1*S*,5*R*)-3-benzyl-5-(trifluoromethyl)-3-azabicyclo[3.1.0]hexane-1-carbaldehyde 71-3 (colorless liquid, 0.54 g, yield: 54%).

LC-MS m/z (ESI)= 270.1 [M+1].

### Step 3:

(1*S*,5*R*)-3-benzyl-1-ethynyl-5-(trifluoromethyl)-3-azabicyclo[3.1.0]hexane 71-4
(1S,5R)-3-benzyl-1-ethynyl-5-(trifluoromethyl)-3-azabicyclo[3.1.0]hexane

Under an N₂ atmosphere, compound 71-3 (0.54 g, 2.0 mmol) and potassium carbonate (0.96 g, 7.0 mmol) were dissolved in 5 mL of methanol. Then, a methanol solution of dimethyl (1-diazo-2-oxopropyl)phosphonate (0.94 g, 5.0 mmol) was added to the reaction system, and the mixture was stirred at room temperature for 0.5 h. After the reaction was completed, the mixture was rotary evaporated to remove the solvent until dryness. The residue was purified by silica gel column chromatography (ethyl acetate: petroleum ether = 1:50) to obtain the target compound (1*S*,5*R*)-3-benzyl-1-ethynyl-5-(trifluoromethyl)-3-azabicyclo[3.1.0]hexane 71-4 (colorless liquid, 0.50 g, yield: 94%).

LC-MS m/z (ESI) = 266.1 [M+1].

### Step 4:

*tert*-butyl 4-(4-((1*S*,5*R*)-3-benzyl-5-(trifluoromethyl)-3-azabicyclo[3.1.0]hexan-1-yl)-1*H*-1,2,3-triazol-1-yl)piperidine-1-carboxylate 71-6
tert-butyl4-(4-((1S,5R)-3-benzyl-5-(trifluoromethyl)-3-azabicyclo[3.1.0]hexan-1-yl)-1H-1,2,3-triazol-1-yl)piperidine-1-carboxylate

Compound 71-4 (0.50 g, 1.9 mmol) from the previous step was dissolved in 5 mL of dichloromethane, and *tert*-butyl 4-azidopiperidine-1-carboxylate compound 71-5 (0.52 g, 2.1 mmol) was added, followed by the addition of copper(I) iodide (38 mg, 0.2 mmol). The mixture was reacted at room temperature for 1 h. After the reaction was completed, the organic phase was rotary evaporated to dryness. The residue was purified by silica gel column chromatography (ethyl acetate: petroleum ether = 1:20) to obtain the target product *tert*-butyl 4-(4-((1*S*,5*R*)-3-benzyl-5-(trifluoromethyl)-3-azabicyclo[3.1.0]hexan-1-yl)-1*H*-1,2,3-triazol-1-yl)piperidine-1-carboxylate 71-6 (colorless liquid, 0.56 g, yield: 61%).

LC-MS m/z (ESI)= 492.2 [M+1].

### Step 5:

*tert*-butyl 4-(4-((1*S*,5*R*)-5-(trifluoromethyl)-3-azabicyclo[3.1.0]hexan-1-yl)-1*H*-1,2,3-triazol-1-yl)piperidine-1-carboxylate 71-7
tert-butyl 4-(4-((1S,5R)-5-(trifluoromethyl)-3-azabicyclo[3.1.0]hexan-1-yl)-1H-1,2,3-triazol-1-yl)piperidine-1-carboxylate

The starting material *tert*-butyl 4-(4-((1*S*,5*R*)-3-benzyl-5-(trifluoromethyl)-3-azabicyclo[3.1.0]hexan-1-yl)-1*H*-1,2,3-triazol-1-yl)piperidine-1-carboxylate 71-6 (500 mg, 1.02 mmol) was added to methanol (5 mL), followed by the addition of palladium on carbon (250 mg, 10% Pd on carbon slurry) and palladium hydroxide (100 mg). After replacing the gas four times with a hydrogen balloon, the reaction was carried out overnight at room temperature. After the reaction was completed, the mixture was filtered through diatomite. The filtrate was rotary evaporated to dryness, and the crude product (402 mg, yield: 98.5%) was directly used in the next step.

LCMS m/z(ESI)=402.2[M+1].

### Step 6:

*tert*-butyl 4-(4-((1*S*,5*R*)-3-(8-cyanoquinolin-5-yl)-5-(trifluoromethyl)-3-azabicyclo[3.1.0]hexan-1-yl)-1*H*-1,2,3-triazol-1-yl)piperidine-1-carboxylate 71-9
tert-butyl 4-(4-((1S,5R)-3-(8-cyanoquinolin-5-yl)-5-(trifluoromethyl)-3-azabicyclo[3.1.0]hexan-1-yl)-1H-1,2,3-triazol-1-yl)piperidine-1-carboxylate

The starting material *tert*-butyl 4-(4-((1*S*,5*R*)-5-(trifluoromethyl)-3-azabicyclo[3.1.0]hexan-1-yl)-1*H*-1,2,3-triazol-1-yl)piperidine-1-carboxylate 71-7 (402 mg, 1.0 mmol) from the previous step was added to 1,4-dioxane (4 mL), followed by the addition of 5-bromoquinoline-8-carbonitrile 71-8 (233 mg, 1.0 mmol), cesium carbonate (652 mg, 2.0 mmol), and methanesulfonato(2-dicyclohexylphosphino-2',6'-di-i-propoxy-1,1'-biphenyl)(2'-amino-1,1'-biphenyl-2-yl)palladium(II) (84 mg, 0.1 mmol). After nitrogen purging was performed four times, the reaction mixture was heated to 100°C and reacted for 4 hours. After the reaction was completed, the mixture was directly purified by silica gel column chromatography to obtain tert-butyl 4-(4-((1*S*,5*R*)-3-(8-cyanoquinolin-5-yl)-5-(trifluoromethyl)-3-azabicyclo[3.1.0]hexan-1-yl)-1*H*-1,2,3-triazol-1-yl)piperidine-1-carboxylate 71-9 (380 mg, yield: 68.6%).

LCMS m/z(ESI)=554.2[M+1].

### Step 7:

5-((1*S*,5*R*)-1-(1-(piperidin-4-yl)-1*H*-1,2,3-triazol-4-yl)-5-(trifluoromethyl)-3-azabicyclo[3.1.0]hexan-3-yl)quinoline-8-carbonitrile 71-10
5-((1S,5R)-1-(1-(piperidin-4-yl)-1H-1,2,3-triazol-4-yl)-5-(trifluoromethyl)-3-azabicyclo[3.1.0]hexan-3-yl)quinoline-8-carbonitrile

The starting material *tert*-butyl 4-(4-((1*S*,5*R*)-3-(8-cyanoquinolin-5-yl)-5-(trifluoromethyl)-3-azabicyclo[3.1.0]hexan-1-yl)-1*H*-1,2,3-triazol-1-yl)piperidine-1-carboxylate 71-9 (380 mg, 0.686 mmol) was added to dichloromethane (3 mL), followed by the addition of trifluoroacetic acid (3 mL). The reaction mixture was stirred at room temperature for 1 hour. After the reaction was completed, the mixture was concentrated under reduced pressure. The crude product 5-((1*S*,5*R*)-1-(1-(piperidin-4-yl)-1*H*-1,2,3-triazol-4-yl)-5-(trifluoromethyl)-3-azabicyclo[3.1.0]hexan-3-yl)quinoline-8-carbonitrile 71-10 was directly used in the next step.

LCMS m/z(ESI)=454.2[M+1].

### Step 8:

5-((1*S*,5*R*)-1-(1-(1-methylpiperidin-4-yl)-1*H*-1,2,3-triazol-4-yl)-5-(trifluoromethyl)-3-azabicyclo[3.1.0]hexan-3-yl)quinoline-8-carbonitrile compound 71
5-((1S,5R)-1-(1-(1-methylpiperidin-4-yl)-1H-1,2,3-triazol-4-yl)-5-(trifluoromethyl)-3-azabicyclo[3.1.0]hexan-3-yl)quinoline-8-carbonitrile
5-((1*S*,5*R*)-1-(1-(piperidin-4-yl)-1*H*-1,2,3-triazol-4-yl)-5-(trifluoromethyl)-3-azabicyclo[3.1.0]hexan-3-yl)quinoline-8-carbonitrile 71-10 (320 mg, 0.70 mmol) from the previous step was added to dichloromethane (10 mL), followed by the addition of paraformaldehyde (210 mg, 7.0 mmol) and sodium cyanoborohydride (879 mg, 14.0 mmol). The reaction mixture was stirred at room temperature overnight. After the reaction was completed, the mixture was filtered, and the filtrate was rotary evaporated to dryness. The residue was then purified by flash column chromatography to obtain 5-((1*S*,5*R*)-1-(1-(1-methylpiperidin-4-yl)-1*H*-1,2,3-triazol-4-yl)-5-(trifluoromethyl)-3-azabicyclo[3.1.0]hexan-3-yl)quinoline-8-carbonitrile compound 71 (110 mg, yield: 33.6%).

LCMS m/z(ESI)=468.2[M+1].

¹H NMR (400 MHz, DMSO-*d*₆) δ 9.01 (dd, 1H), 8.70 (dd, 1H), 8.38 (s, 1H), 8.19 (d, 1H), 7.61 (dd, 1H), 7.28 (d, 1H), 4.77 (s, 1H), 4.08 (d, 2H), 4.05 (s, 1H), 3.92 (d, 1H), 3.58 (s, 2H), 3.36 (s, 2H), 3.27 - 3.12 (m, 2H), 2.83 (s, 3H), 2.36 (d, 2H), 2.23 - 2.08 (m, 2H).

### Example 72

1-methyl-5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)-2-oxo-1,2-dihydroquinoline-8-carbonitrile compound 72
1-methyl-5-((1S,5R)-1-(5-(1-methylpiperidin-4-yl)-1,3,4-oxadiazol-2-yl)-5-(trifluoromethyl)-3-azabicyclo[3.1.0]hexan-3-yl)-2-oxo-1,2-dihydroquinoline-8-carbonitrile

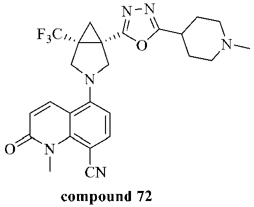

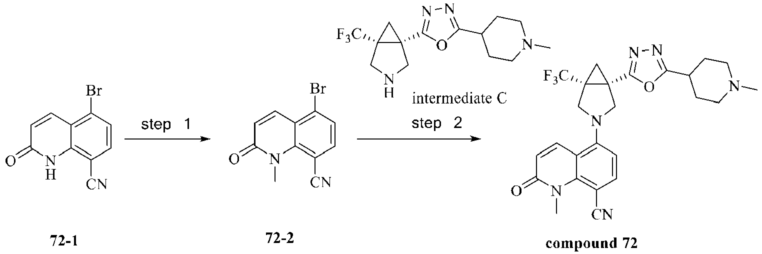

### Step 1:

5-bromo-1-methyl-2-oxo-1,2-dihydroquinoline-8-carbonitrile compound 72-2
5-bromo-1-methyl-2-oxo-1,2-dihydroquinoline-8-carbonitrile

The starting material 5-bromo-2-oxo-1,2-dihydroquinoline-8-carbonitrile 72-1 (800 mg, 3.21 mmol) was added to dimethyl sulfoxide (20 mL), followed by the addition of potassium carbonate (886 mg, 6.42 mmol) and then iodomethane (912 mg, 6.42 mmol). The reaction mixture was stirred at room temperature for 4 hours. After the reaction was completed, purified water and ethyl acetate (100 mL × 3 times) were added. The organic phases were combined, washed with saturated brine, dried over anhydrous sodium sulfate, filtered, and concentrated. The crude product was purified by silica gel column chromatography to obtain 5-bromo-1-methyl-2-oxo-1,2-dihydroquinoline-8-carbonitrile 72-2 (80 mg, yield: 9.4%).

LC-MS m/z (ESI)= 262.90 [M+1].

### Step 2:

1-methyl-5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)-2-oxo-1,2-dihydroquinoline-8-carbonitrile compound 72
1-methyl-5-((1S,5R)-1-(5-(1-methylpiperidin-4-yl)-1,3,4-oxadiazol-2-yl)-5-(trifluoromethyl)-3-azabicyclo[3.1.0]hexan-3-yl)-2-oxo-1,2-dihydroquinoline-8-carbonitrile

The starting materials 5-bromo-2-oxo-1,2-dihydroquinoline-8-carbonitrile 72-2 (249 mg, 1.0 mmol) and 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (476 mg, 1.5 mmol) were added to 1,4-dioxane (3 mL), followed by the addition of cesium carbonate (977 mg, 3.0 mmol) and methanesulfonato(2-dicyclohexylphosphino-2',6'-di-i-propoxy-1,1'-biphenyl)(2'-amino-1,1'-biphenyl-2-yl)palladium(II) (84 mg, 0.1 mmol). After nitrogen protection, the reaction mixture was heated to 100°C and reacted for 3 hours. After the reaction was completed, the mixture was directly purified by reverse-phase column chromatography to obtain 1-methyl-5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)-2-oxo-1,2-dihydroquinoline-8-carbonitrile compound 72 (89 mg, yield: 17.8%).

LC-MS m/z (ESI)= 499.20 [M+1].

¹H NMR (400 MHz, DMSO-*d*₆) δ 8.06 (d, 1H), 7.90 (d, 1H), 7.18 (d, 1H), 6.61 (d, 1H), 4.00 (s, 2H), 3.92 (s, 3H), 3.88 (d, 2H), 2.92 (ddd, 1H), 2.71 (d, 2H), 2.16 (d, 4H), 2.10 - 1.91 (m, 5H), 1.80 - 1.65 (m, 2H).

¹⁹F NMR (376 MHz, DMSO-*d*₆) δ -64.03.

### Example 73

2-methyl-5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile compound 73
2-methyl-5-((1S,5R)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile

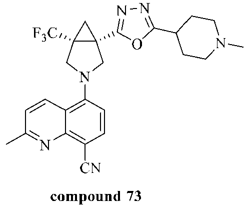

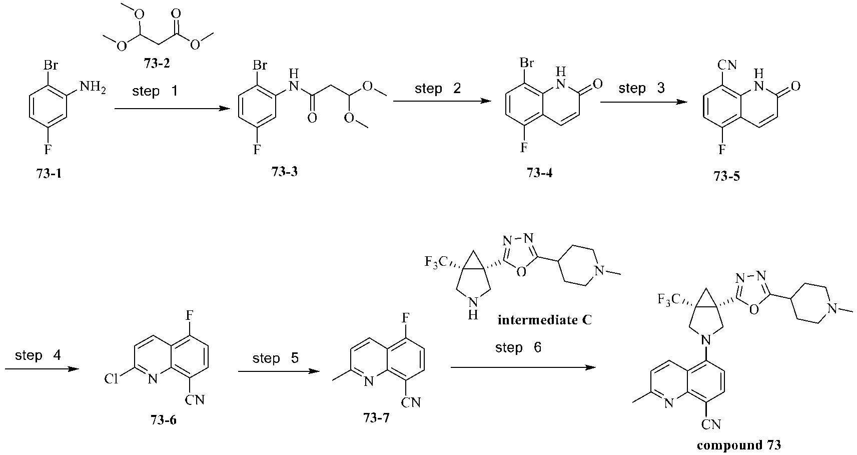

### Step 1:

*N*-(2-bromo-5-fluorophenyl)-3,3-dimethoxypropanamide compound 73-3
N-(2-bromo-5-fluorophenyl)-3,3-dimethoxypropanamide

The starting materials 5-fluoro-2-bromoaniline 73-1 (10.0 g, 52.6 mmol) and methyl 3,3-dimethoxypropionate 73-2 (9.35 g, 63.1 mmol) were added to anhydrous tetrahydrofuran (100 mL). After nitrogen protection, the mixture was cooled to 0°C, and a solution of sodium bis(trimethylsilyl)amide (80 mL, 1 mol/L in tetrahydrofuran) was slowly added dropwise. The reaction mixture was gradually warmed to room temperature and stirred for 6 hours until the reaction was completed. Saturated aqueous ammonium chloride solution and ethyl acetate (100 mL × 3) were added for extraction. The organic phases were combined, washed with saturated brine, dried over anhydrous sodium sulfate, filtered, and concentrated. N-(2-Bromo-5-fluorophenyl)-3,3-dimethoxypropanamide 73-3 (12.0 g, yield: 74.5%) was obtained.

LC-MS m/z (ESI)= 306.00 [M+1].

### Step 2:

8-bromo-5-fluoroquinolin-2(1H)-one compound 73-4
8-bromo-5-fluoroquinolin-2(1H)-one

The starting material *N*-(2-bromo-5-fluorophenyl)-3,3-dimethoxypropanamide 73-3 (12.0 g, 39.2 mmol) was dissolved in dichloromethane (200 mL). The reaction mixture was cooled to 0°C, and then concentrated sulfuric acid (300 mL) was added. The reaction mixture was slowly warmed to room temperature and stirred for 8 hours. After the reaction was completed, the phases were separated. The lower sulfuric acid layer was slowly added dropwise to ice water (1 L), resulting in the precipitation of a large amount of solid. The mixture was filtered, and the filter cake was dried. 8-Bromo-5-fluoroquinolin-2(1H)-one 73-4 (9.0 g, yield: 94.9%) was obtained.

LC-MS m/z (ESI)= 241.90 [M+1].

### Step 3:

5-fluoro-2-oxo-1,2-dihydroquinoline-8-carbonitrile 73-5
5-fluoro-2-oxo-1,2-dihydroquinoline-8-carbonitrile

The starting material 8-bromo-5-fluoroquinolin-2(1H)-one 73-4 (9.0 g, 37.2 mmol) was added to *N*,*N*-dimethylformamide (90 mL), followed by the addition of zinc cyanide (8.73 g, 74.4 mmol) and tetrakis(triphenylphosphine)palladium (4.30 g, 3.72 mmol). After nitrogen protection, the reaction mixture was heated to 100°C and reacted for 6 hours. After the reaction was completed, purified water (300 mL) was added, and a large amount of solid precipitated. The mixture was filtered, and the filter cake was purified by column chromatography to obtain 5-fluoro-2-oxo-1,2-dihydroquinoline-8-carbonitrile 73-5 (3.5 g, yield: 49.8%).

LC-MS m/z (ESI)= 189.00 [M+1].

### Step 4:

2-chloro-5-fluoroquinoline-8-carbonitrile compound 73-6
2-chloro-5-fluoroquinoline-8-carbonitrile

The starting material 5-fluoro-2-oxo-1,2-dihydroquinoline-8-carbonitrile 73-5 (3.5 g, 18.5 mmol) was added to phosphorus oxychloride (30 mL), heated to 110°C, and reacted for 4 hours. After the reaction was completed, the mixture was concentrated under reduced pressure. The crude product was added to a saturated aqueous sodium bicarbonate solution, and the pH was adjusted to 8. Ethyl acetate (50 mL × 3) was added for extraction. The organic phases were combined, washed with saturated brine, dried over anhydrous sodium sulfate, filtered, and concentrated to obtain 2-chloro-5-fluoroquinoline-8-carbonitrile 73-6 (3.4 g, yield: 88.8%).

LC-MS m/z (ESI)= 207.00 [M+1].

### Step 5:

5-fluoro-2-methylquinoline-8-carbonitrile compound 73-7
5-fluoro-2-methylquinoline-8-carbonitrile

The starting material 2-chloro-5-fluoroquinoline-8-carbonitrile 73-6 (3.0 g, 14.5 mmol), methylboronic acid (2.6 g, 43.5 mmol), tetrakis(triphenylphosphine)palladium (1.7 g, 1.45 mmol), and potassium phosphate (9.2 g, 43.5 mmol) were added to 1,4-dioxane (30 mL). After nitrogen replacement was performed four times, the mixture was heated to 100°C and reacted for 4 hours. After the reaction was completed, purified water (100 mL) and ethyl acetate (80 mL × 3) were added. The organic phases were combined, washed with saturated brine, dried over anhydrous sodium sulfate, filtered, and rotary evaporated to dryness. The crude product was purified by column chromatography to obtain 5-fluoro-2-methylquinoline-8-carbonitrile 73-7 (850 mg, yield: 31.4%).

LC-MS m/z (ESI)= 187.10 [M+1].

### Step 6:

2-methyl-5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile compound 73
2-methyl-5-((1S,5R)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile

The starting material 5-fluoro-2-methylquinoline-8-carbonitrile 73-7 (374 mg, 2.0 mmol) and 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (316 mg, 1.0 mmol) were added to dimethyl sulfoxide (1 mL) and *N*,*N*-diisopropylethylamine (1 mL). The reaction mixture was heated to 130°C and reacted for 24 hours. After the reaction was completed, reverse-phase column chromatography purification was performed to obtain 2-methyl-5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)quinoline-8-carbonitrile compound 73 (22 mg, yield: 4.6%) as a white solid.

LC-MS m/z (ESI)= 483.20 [M+1].

¹H NMR (400 MHz, DMSO-*d*₆) δ 8.55 (d, 1H), 8.13 (d, 1H), 7.50 (d, 1H), 7.25 (d, 1H), 4.16 (d, 1H), 4.04 (dd, 2H), 3.89 (d, 1H), 3.25 (d, 2H), 2.70 (s, 7H), 2.22 (d, 5H), 2.13 - 1.92 (m, 3H).

### Example 74

3-fluoro-4-((1*S*,5*R*)-1-(1-(1-methylpiperidin-4-yl)-1*H-*1,2,4-triazol-3-yl)-5-(trifluoromethyl)-3-azabicyclo[3.1.0]hexan-3-yl)pyrazolo[1,5-*a*]pyridine-7-carbonitrile compound 74
3-fluoro-4-((1S,5R)-1-(1-(1-methylpiperidin-4-yl)-1H-1,2,4-triazol-3-yl)-5-(trifluoromethyl)-3-azabicyclo[3.1.0]hexan-3-yl)pyrazolo[1,5-a]pyridine-7-carbonitrile

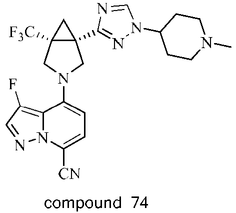

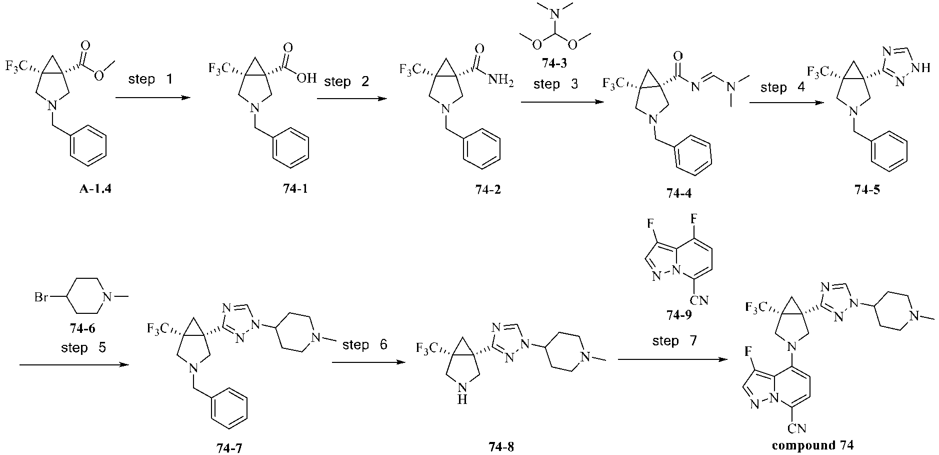

### Step 1:

(1*S*,5*R*)-3-benzyl-5-(trifluoromethyl)-3-azabicyclo[3.1.0]hexane-1-carboxylic acid compound 74-1
(1S,5R)-3-benzyl-5-(trifluoromethyl)-3-azabicyclo[3.1.0]hexane-1-carboxylic acid

The starting material methyl (1*S*,5*R*)-3-benzyl-5-(trifluoromethyl)-3-azabicyclo[3.1.0]hexane-1-carboxylate A-1.4 (40 g, 133.6 mmol) was added to a mixed solution of methanol/water (400 mL/100 mL), followed by the addition of solid sodium hydroxide (16.0 g, 400.8 mmol). The reaction mixture was heated to 50°C and reacted for 2 hours. After the reaction was completed, the mixture was concentrated under reduced pressure. The pH was adjusted to 7-8 with dilute hydrochloric acid, and the mixture was extracted with ethyl acetate (400 mL × 3). The organic phases were combined, washed with saturated brine, dried over anhydrous sodium sulfate, filtered, and concentrated to obtain (1*S*,5*R*)-3-benzyl-5-(trifluoromethyl)-3-azabicyclo[3.1.0]hexane-1-carboxylic acid compound 74-1 as an off-white solid (35.0 g, yield: 91.8%).

LC-MS m/z (ESI)= 286.10 [M+1].

### Step 2:

(1*S*,5*R*)-3-benzyl-5-(trifluoromethyl)-3-azabicyclo[3.1.0]hexane-1-carboxamide compound 74-2
(1S,5R)-3-benzyl-5-(trifluoromethyl)-3-azabicyclo[3.1.0]hexane-1-carboxamide

The starting material (1*S*,5*R*)-3-benzyl-5-(trifluoromethyl)-3-azabicyclo[3.1.0]hexane-1-carboxylic acid 74-1 (15.0 g, 52.6 mmol) was added to anhydrous tetrahydrofuran (300 mL), followed by the addition of *N*,*N*-dimethylformamide (0.2 mL). Oxalyl chloride (13.3 g, 105.2 mmol) was then slowly added dropwise, maintaining the temperature at approximately 25°C. The reaction mixture was stirred at room temperature for 2 hours, then concentrated under reduced pressure to remove excess oxalyl chloride. Tetrahydrofuran was added again to dissolve the residue, and the solution was slowly added dropwise to ice-cold concentrated ammonia water. The mixture was warmed to room temperature and stirred for 2 hours until the reaction was completed. Ethyl acetate (100 mL × 3) was added for extraction, and the organic phases were combined, washed with saturated brine, dried over anhydrous sodium sulfate, filtered, and concentrated to obtain (1*S*,5*R*)-3-benzyl-5-(trifluoromethyl)-3-azabicyclo[3.1.0]hexane-1-carboxamide 74-2 (13.5 g, yield: 90%) as a yellow viscous liquid.

LC-MS m/z (ESI)= 285.10 [M+1].

### Step 3:

(1*S*,5*R*)-3-benzyl-*N*-((*E*)-(dimethylamino)methylene)-5-(trifluoromethyl)-3-azabicyclo[3.1.0]hexane-1-carboxamide compound 74-4
(1S,5R)-3-benzyl-*N*-((*E*)-(dimethylamino)methylene)-5-(trifluoromethyl)-3-azabicyclo[3.1.0]hexane-1-carboxamide

The starting material (1*S*,5*R*)-3-benzyl-5-(trifluoromethyl)-3-azabicyclo[3.1.0]hexane-1-carboxamide 74-2 (13.5 g, 47.4 mmol) was added to 1,4-dioxane (150 mL), followed by the addition of *N,N*-dimethylformamide dimethyl acetal 74-3 (11.3 g, 94.8 mmol). After nitrogen protection, the reaction mixture was heated to reflux and reacted for 2 hours. After the reaction was completed, the mixture was directly concentrated. (1*S*,5*R*)-3-Benzyl-*N*-((*E*)-(dimethylamino)methylene)-5-(trifluoromethyl)-3-azabicyclo[3.1.0]hexane-1-carboxamide 74-4 (16.1 g, 100.0%) was obtained as a yellow viscous liquid.

LC-MS m/z (ESI)= 340.20 [M+1].

### Step 4:

(1S,5R)-3-benzyl-1-(1*H*-1,2,4-triazol-3-yl)-5-(trifluoromethyl)-3-azabicyclo[3.1.0]hexane compound 74-5
(1S,5R)-3-benzyl-1-(1H-1,2,4-triazol-3-yl)-5-(trifluoromethyl)-3-azabicyclo[3.1.0]hexane

The starting material (1*S*,5*R*)-3-benzyl-*N*-((*E*)-(dimethylamino)methylene)-5-(trifluoromethyl)-3-azabicyclo[3.1.0]hexane-1-carboxamide 74-4 (16.1 g, 47.4 mmol) was added to glacial acetic acid (100 mL), followed by the addition of hydrazine hydrochloride (4.87 g, 71.1 mmol). After nitrogen protection, the reaction mixture was heated to reflux and reacted for 4 hours. After the reaction was completed, the mixture was cooled to room temperature, and a saturated sodium carbonate solution was added to adjust the pH to 8-9. The mixture was then extracted with ethyl acetate (150 mL × 3). The combined organic phases were washed with saturated brine, dried over anhydrous sodium sulfate, filtered, and concentrated. The crude product was purified by silica gel column chromatography to obtain (18,5R)-3-benzyl-1-(1*H*-1,2,4-triazol-3-yl)-5-(trifluoromethyl)-3-azabicyclo[3.1.0]hexane 74-5 (13.0 g, yield: 88.7%).

LC-MS m/z (ESI)= 309.10 [M+1].

¹H NMR (400 MHz, DMSO-*d*₆) δ 13.90 (s, 1H), 8.00 (d,1H), 7.50 - 7.10 (m, 5H), 3.80 - 3.59 (m, 2H), 3.19 - 2.96 (m, 3H), 2.72 (d, 1H), 1.86 (d, 2H).

### Step 5:

(1S,5R)-3-benzyl-1-(1-(1-methylpiperidin-4-yl)-1H-1,2,4-triazol-3-yl)-5-(trifluoromethyl)-3-azabicyclo[3.1.0]hexane compound 74-7
(1S,5R)-3-benzyl-1-(1-(1-methylpiperidin-4-yl)-1H-1,2,4-triazol-3-yl)-5-(trifluoromethyl)-3-azabicyclo[3.1.0]hexane

The starting material (1S,5R)-3-benzyl-1-(1H-1,2,4-triazol-3-yl)-5-(trifluoromethyl)-3-azabicyclo[3.1.0]hexane 74-5 (2.0 g, 6.5 mmol) was added to N,N-dimethylformamide (15 mL) and purified water (5 mL), followed by the addition of 4-bromo-1-methylpiperidine 74-6 (4.63 g, 26.0 mmol) and cesium carbonate (8.48 g, 26.0 mmol). After nitrogen protection, the reaction mixture was heated to 100°C and reacted for 2 days. After the reaction was completed, purified water (50 mL) and ethyl acetate (50 mL × 3) were added for extraction. The organic phases were combined, washed with saturated brine, dried over anhydrous sodium sulfate, filtered, and rotary evaporated to dryness. The crude product was purified by silica gel column chromatography to obtain (1S,5R)-3-benzyl-1-(1-(1-methylpiperidin-4-yl)-1H-1,2,4-triazol-3-yl)-5-(trifluoromethyl)-3-azabicyclo[3.1.0]hexane 74-7 (1.5 g, yield: 57.0%).

LC-MS m/z (ESI)= 406.20 [M+1].

### Step 6:

(1S,5R)-1-(1-(1-methylpiperidin-4-yl)-1H-1,2,4-triazol-3-yl)-5-(trifluoromethyl)-3-azabicyclo[3.1.0]hexane compound 74-8
(1S,5R)-1-(1-(1-methylpiperidin-4-yl)-1H-1,2,4-triazol-3-yl)-5-(trifluoromethyl)-3-azabicyclo[3.1.0]hexane

The starting material (1S,5R)-3-benzyl-1-(1H-1,2,4-triazol-3-yl)-5-(trifluoromethyl)-3-azabicyclo[3.1.0]hexane 74-7 (1.5 g, 3.7 mmol) was added to methanol (15 mL), followed by the addition of 10% palladium on carbon (750 mg) and 20% palladium hydroxide on carbon (150 mg). After hydrogen replacement was performed four times, the mixture was heated to 45°C and reacted for 4 hours. After the reaction was completed, the mixture was filtered through diatomite, and the filtrate was concentrated to obtain (1S,5R)-1-(1-(1-methylpiperidin-4-yl)-1H-1,2,4-triazol-3-yl)-5-(trifluoromethyl)-3-azabicyclo[3.1.0]hexane 74-8 (1.2 g, yield: 102.6%).

LC-MS m/z (ESI)= 316.20 [M+1].

### Step 7:

3-fluoro-4-((1S,5R)-1-(1-(1-methylpiperidin-4-yl)-1H-1,2,4-triazol-3-yl)-5- (trifluoromethyl)-3-
azabicyclo[3.1.0]hexan-3-yl)pyrazolo[1,5-a]pyridine-7-carbonitrile compound 74
3-fluoro-4-((1S,5R)-1-(1-(1-methylpiperidin-4-yl)-1H-1,2,4-triazol-3-yl)-5- (trifluoromethyl)-3-azabicyclo[3.1.0]hexan-3-yl)pyrazolo[1,5-a]pyridine-7-carbonitrile

The starting materials 3,4-difluoropyrazolo[1,5-a]pyridine-7-carbonitrile 74-9 (250 mg, 1.40 mmol) and (15,5R)-1-(1-(1-methylpiperidin-4-yl)-1H-1,2,4-triazol-3-yl)-5-(trifluoromethyl)-3-azabicyclo[3.1.0]hexane 74-8 (300 mg, 0.95 mmol) were added to dimethyl sulfoxide (1 mL), followed by the addition of N,Ndiisopropylethylamine (1 mL). After nitrogen protection, the reaction mixture was heated to 130°C and reacted for 4 hours. After the reaction was completed, the mixture was directly purified by reverse-phase column chromatography to obtain 3-fluoro-4-((1S,5R)-1-(1-(1-methylpiperidin-4-yl)-1H-1,2,4-triazol-3-yl)-5-(trifluoromethyl)-3-azabicyclo[3.1.0]hexan-3-yl)pyrazolo[1,5-*a*]pyridine-7-carbonitrile compound 74 (190 mg, yield: 42.1%) as a white solid.

LC-MS m/z (ESI)= 475.20 [M+1].

¹H NMR (400 MHz, DMSO-*d*₆) δ 8.57 (s, 1H), 8.24 (d, 1H), 7.60 (d, 1H), 6.44 (d, 1H), 4.29 - 4.09 (m, 4H), 3.97 (d, 1H), 2.82 (d, 2H), 2.20 (d, 4H), 2.05 - 1.93 (m, 6H), 1.62 (d, 1H).

### Example 75

2-(piperidin-4-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole compound 75
2-(piperidin-4-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole

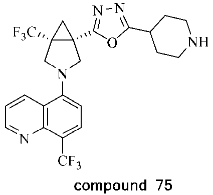

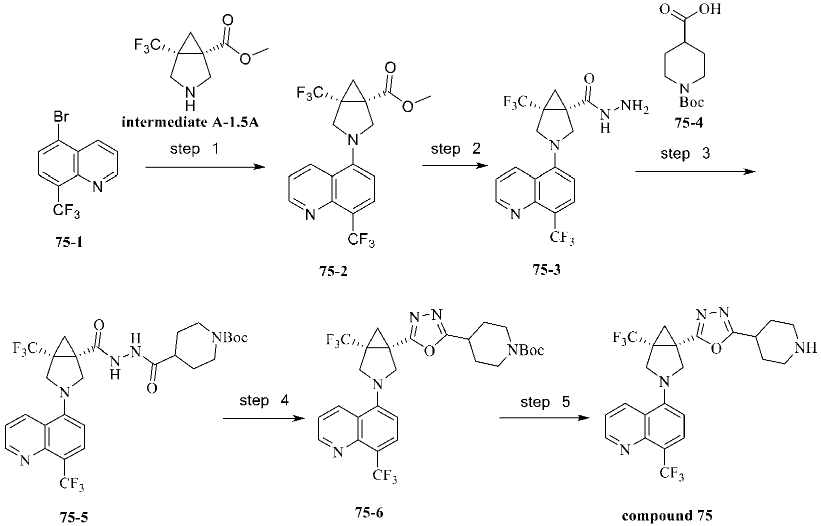

### Step 1:

methyl (1S,5R)-5-(trifluoromethyl)-3-(8-(trifluoromethyl)quinolin-5-yl)-3-azabicyclo[3.1.0]hexane-1-carboxylate compound 75-2
(1S,5R)-5-(trifluoromethyl)-3-(8-(trifluoromethyl)quinolin-5-yl)-3-azabicyclo[3.1.0]hexane-1-carboxylate

Under an N₂ atmosphere, 2-(1-methylpiperidin-4-yl)-5-((1S,5R)-5-(trifluoromethyl)-3-azabicyclo[3.1.0]hexan-1-yl)-1,3,4-oxadiazole intermediate A-1.5A (2.30 g, 10.9 mmol) was dissolved in 1,4-dioxane (45.00 mL). Subsequently, 5-bromo-8-trifluoromethylquinoline 75-1 (3.00 g, 10.9 mmol), cesium carbonate (14.2 g, 43.6 mmol), and RuPhosPdG3 (920 mg, 1.1 mmol) were added sequentially. The reaction system was purged with nitrogen three times, and the mixture was heated to 90°C and reacted for 2 h. The mixture was rotary evaporated to remove the solvent until dryness, added with ethyl acetate for extraction, and the organic phase was washed with saturated brine, dried over anhydrous sodium sulfate, filtered, and rotary evaporated to remove the solvent until dryness. TLC (DCM: MeOH = 10:1) to obtain the target product methyl (1S,5R)-5-(trifluoromethyl)-3-(8-(trifluoromethyl)quinolin-5-yl)-3-azabicyclo[3.1.0]hexane-1-carboxylate 75-2 (yellow solid, 5.00 g).

LC-MS m/z (ESI)= 405.10 [M+1].

### Step 2:

(1S,5R)-5-(trifluoromethyl)-3-(8-(trifluoromethyl)quinolin-5-yl)-3-azabicyclo[3.1.0]hexane-1-carbohydrazide compound 75-3
(1S,5R)-5-(trifluoromethyl)-3-(8-(trifluoromethyl)quinolin-5-yl)-3-azabicyclo[3.1.0]hexane-1-carbohydrazide
Under an N₂ atmosphere, methyl 2(1*S*,5*R*)-5-(trifluoromethyl)-3-(8-(trifluoromethyl)quinolin-5-yl)-3-azabicyclo[3.1.0]hexane-1-carboxylate 75-2 (yellow solid, 5.00 g) was dissolved in 1 methanol (50.0 mL), followed by the sequential addition of DBU (6.67 g, 43.6 mmol) and hydrazine hydrochloride (2.6 g, 43.6 mmol). The mixture was heated to 90°C and reacted for 2 h. The mixture was rotary evaporated to remove the solvent until dryness and added with ethyl acetate for extraction. The organic phase was concentrated, washed with saturated brine, dried over anhydrous sodium sulfate, filtered, and rotary evaporated to remove the solvent until dryness. The crude product was purified by column chromatography (petroleum ether: ethyl acetate = 1:3) to obtain the target product *(1S,5R)-5-*(trifluoromethyl)-3-(8-(trifluoromethyl)quinolin-5-yl)-3-azabicyclo[3.1.0]hexane-1-carbohydrazide compound 75-3 (yellow solid, 2.26 g, 51%).

LC-MS m/z (ESI)= 405.10 [M+1].

### Step 3:

*tert*-butyl 4-(2-((1*S*,5*R*)-5-(trifluoromethyl)-3-(8-(trifluoromethyl)quinolin-5-yl)-3-azabicyclo[3.1.0]hexane-1-carbonyl)hydrazine-1-carbonyl)piperidine-1-carboxylate compound 75-5
tert-butyl 4-(2-((1S,5R)-5-(trifluoromethyl)-3-(8-(trifluoromethyl)quinolin-5-yl)-3-azabicyclo[3.1.0]hexane-1-carbonyl)hydrazine-1-carbonyl)piperidine-1-carboxylate

The starting material 1-(*tert*-butoxycarbonyl)piperidine-4-carboxylic acid 75-4 (688 mg, 3.0 mmol) was added to *N,N*-dimethylformamide (5 mL), followed by the addition of *N*,*N*,*N*',*N*'-tetramethyl-*O*-(7-azabenzotriazol-1-yl)uronium hexafluorophosphate (2.28 g, 3.0 mmol) and *N,N*-diisopropylethylamine (580 mg, 4.5 mmol). The reaction mixture was stirred at room temperature for 0.5 hours, and then (1*S*,5*R*)-5-(trifluoromethyl)-3-(8-(trifluoromethyl)quinolin-5-yl)-3-azabicyclo[3.1.0]hexane-1-carbohydrazide 75-3 (1.21 g, 3.0 mmol) was added. The reaction mixture was stirred at room temperature for 6 hours and then purified by reverse-phase column chromatography to obtain *tert*-butyl 4-(2-((1*S*,5*R*)-5-(trifluoromethyl)-3-(8-(trifluoromethyl)quinolin-5-yl)-3-azabicyclo[3.1.0]hexane-1-carbonyl)hydrazine-1-carbonyl)piperidine-1-carboxylate 75-5 (1.1 g, yield: 59.5%).

LCMS m/z(ESI)=616.2[M+1].

### Step 4:

*tert*-butyl 4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidine-1-carboxylate 75-6
tert-butyl 4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)piperidine-1-carboxylate
*tert*-Butyl 4-(2-((1*S*,5*R*)-5-(trifluoromethyl)-3-(8-(trifluoromethyl)quinolin-5-yl)-3-azabicyclo[3.1.0]hexane-1-carbonyl)hydrazine-1-carbonyl)piperidine-1-carboxylate 75-5 (1.1 g, 1.79 mmol) was dissolved in 20 mL of dichloromethane, followed by the addition of triethylamine (904 mg, 8.95 mmol) and *p*-toluenesulfonyl chloride (1.02 g, 5.37 mmol). The mixture was stirred at room temperature for 5 hours. After TLC confirmed the completion of the reaction, the reaction mixture was concentrated and purified by silica gel column chromatography to obtain the target product *tert*-butyl 4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidine-1-carboxylate 75-6 (yellow solid, 875 mg, yield: 81.8%).

LC-MS m/z (ESI)= 598.2 [M+1].

### Step 5:

2-(piperidin-4-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 75
2-(piperidin-4-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole

The starting material *tert*-butyl 4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidine-1-carboxylate 75-6 (875 mg, 1.46 mmol) was added to dichloromethane (6 mL), followed by the addition of trifluoroacetic acid (3 mL). The reaction mixture was stirred at room temperature for 2 hours, concentrated under reduced pressure, and then purified by reverse-phase column chromatography to obtain 2-(piperidin-4-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 75 (530 mg, yield: 72.9%).

LC-MS m/z (ESI)= 498.2 [M+1].

¹H NMR (400 MHz, DMSO-*d*₆) δ 9.01 (dd, 1H), 8.66 (dd, 1H), 8.04 (d, 1H), 7.63 (dd, 1H), 7.38 (d, 1H), 4.09 (d, 1H), 3.97 (dd, 2H), 3.82 (d, 1H), 3.13 (td, 1H), 3.03 (d, 2H), 2.70 (t, 2H), 2.34 (d, 1H), 2.22 (d, 1H), 1.95 (dd, 2H), 1.65 (q, 2H).

¹⁹F NMR (376 MHz, DMSO-*d*₆) δ -58.08, -64.08, -73.52.

### Example 76

2-(1-(cyclopropylmethyl)piperidin-4-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 76
2-(1-(cyclopropylmethyl)piperidin-4-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole 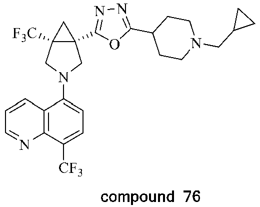 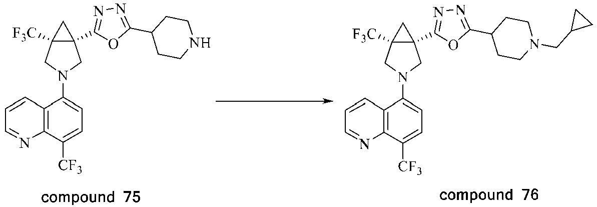
2-(1-(cyclopropylmethyl)piperidin-4-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 76
2-(1-(cyclopropylmethyl)piperidin-4-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole
2-(Piperidin-4-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 75 (100 mg, 0.20 mmol) was dissolved in *N,N*-dimethylformamide (1 mL). Subsequently, cesium carbonate (130 mg, 0.4 mmol) and bromomethylcyclopropane (34 mg, 0.25 mmol) were added, and the mixture was stirred at room temperature overnight. After the reaction was completed, the mixture was directly purified by reverse column chromatography to obtain the target product 2-(1-(cyclopropylmethyl)piperidin-4-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 76 (white solid, 15 mg, 13.6%).

LC-MS m/z (ESI)= 552.2 [M+1].

¹H NMR (400 MHz, DMSO-*d*₆) δ 9.01 (dd, 1H), 8.65 (dd, 1H), 8.04 (d, 1H), 7.63 (dd, 1H), 7.38 (d, 1H), 4.08 (d, 1H), 3.97 (t, 2H), 3.82 (d, 1H), 2.99 - 2.85 (m, 3H), 2.33 (d, 1H), 2.23 (d, 1H), 2.14 (dd, 4H), 1.96 (dd, 2H), 1.87 - 1.67 (m, 2H), 0.96 - 0.63 (m, 1H), 0.57 - 0.34 (m, 2H), 0.14 - 0.01 (m, 2H).

¹⁹F NMR (376 MHz, DMSO-*d*₆) δ -58.07, -64.05.

### Example 77

2-(4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidin-1-yl)acetamide compound 77
2-(4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)piperidin-1-yl)acetamide 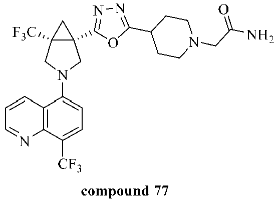 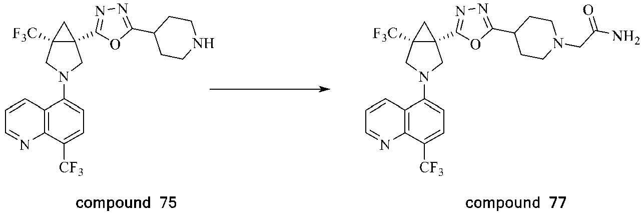
2-(4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidin-1-yl)acetamide compound 77
2-(4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)piperidin-1-yl)acetamide
2-(Piperidin-4-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 75 (100 mg, 0.20 mmol) was dissolved in N,N-dimethylformamide (1 mL). Subsequently, cesium carbonate (130 mg, 0.4 mmol) and bromoacetamide (35 mg, 0.25 mmol) were added, and the mixture was stirred at room temperature overnight. After the reaction was completed, the mixture was directly purified by reverse column chromatography to obtain the target product 2-(1-(cyclopropylmethyl)piperidin-4-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 77 (white solid, 43 mg, 38.7%).

LC-MS m/z (ESI)= 555.2 [M+1].

¹H NMR (400 MHz, DMSO-*d*₆) δ 9.15 - 8.89 (m, 1H), 8.81 - 8.55 (m, 1H), 8.04 (d, 1H), 7.62 (dd, 1H), 7.37 (d, 1H), 7.22 (s, 1H), 7.11 (s, 1H), 4.08 (d, 1H), 4.06 - 3.89 (m, 2H), 3.81 (d, 1H), 2.97 (tt, 1H), 2.85 (s, 2H), 2.79 (d, 2H), 2.34 (d, 1H), 2.22 (q, 3H), 2.05 - 1.90 (m, 2H), 1.82 (dd, 2H).

¹⁹F NMR (376 MHz, DMSO-*d*₆) δ -58.08, -64.06.

### Example 78

2-((1*R*,3*R*,5*S*)-8-methyl-8-azabicyclo[3.2.1]octan-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 78
2-((1R,3r,5S)-8-methyl-8-azabicyclo[3.2.1]octan-3-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole

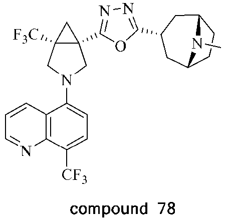

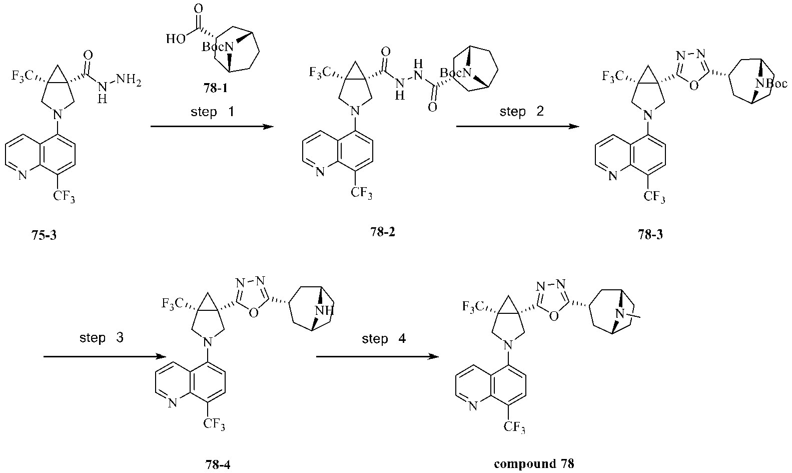

### Step 1:

tert-butyl (1*R*,3*R*,5*S*)-3-(2-((1*S*,5*R*)-5-(trifluoromethyl)-3-(8-(trifluoromethyl)quinolin-5-yl)-3-azabicyclo[3.1.0]hexane-1-carbonyl)hydrazine-1-carbonyl)-8-azabicyclo[3.2.1]octane-8-carboxylate compound 78-2
tert-butyl (1R,3r,5S)-3-(2-((1S,5R)-5-(trifluoromethyl)-3-(8-(trifluoromethyl)quinolin-5-yl)-3-azabicyclo[3.1.0]hexane-1-carbonyl)hydrazine-1-carbonyl)-8-azabicyclo[3.2.1]octane-8-carboxylate

The starting material (1*R*,3*R*,5*S*)-8-(*tert*-butoxycarbonyl)-8-azabicyclo[3.2.1]octane-3-carboxylic acid 78-1 (255 mg, 1.0 mmol) was added to *N,N*-dimethylformamide (1 mL), followed by the addition of *N*,*N*,*N*',*N*'-tetramethyl-*O*-(7-azabenzotriazol-1-yl)uronium hexafluorophosphate (380 mg, 1.0 mmol) and *N,N*-diisopropylethylamine (357 mg, 3.0 mmol). The reaction mixture was stirred at room temperature for 0.5 hours, and then *(1S,5R)-5-*(trifluoromethyl)-3-(8-(trifluoromethyl)quinolin-5-yl)-3-azabicyclo[3.1.0]hexane-1-carbohydrazide 75-3 (316 mg, 1.0 mmol) was added. The reaction mixture was stirred at room temperature for 6 hours and then purified by reverse-phase column chromatography to obtain *tert*-butyl (1*R*,3*R*,5*S*)-3-(2-((1*S*,5*R*)-5-(trifluoromethyl)-3-(8-(trifluoromethyl)quinolin-5-yl)-3-azabicyclo[3.1.0]hexane-1-carbonyl)hydrazine-1-carbonyl)-8-azabicyclo[3.2.1]octane-8-carboxylate 78-2 (380 mg, yield: 59.2%).

LCMS m/z(ESI)=642.2[M+1].

### Step 2:

*tert*-butyl (1*R*,3*R*,5*S*)-3-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)-8-azabicyclo[3.2.1]octane-8-carboxylate compound 78-3
tert-butyl (1R,3r,5S)-3-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)-8-azabicyclo[3.2.1]octane-8-carboxylate
*tert*-Butyl (1*R*,3*R*,5*S*)-3-(2-((1*S*,5*R*)-5-(trifluoromethyl)-3-(8-(trifluoromethyl)quinolin-5-yl)-3-azabicyclo[3.1.0]hexane-1-carbonyl)hydrazine-1-carbonyl)-8-azabicyclo[3.2.1]octane-8-carboxylate 78-2 (380 mg, 0.59 mmol) was dissolved in 5 mL of dichloromethane, followed by the addition of triethylamine (303 mg, 3.0 mmol) and *p-*toluenesulfonyl chloride (338 mg, 1.77 mmol). The mixture was stirred at room temperature for 5 hours. After TLC confirmed the completion of the reaction, the reaction mixture was concentrated and purified by silica gel column chromatography to obtain the target product *tert*-butyl (1*R*,3*R*,5*S*)-3-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)-8-azabicyclo[3.2.1]octane-8-carboxylate 78-3 (yellow solid, 257 mg, yield: 69.8%).

LC-MS m/z (ESI)= 624.2 [M+1].

### Step 3:

2-((1*R*,3*R*,5*S*)-8-azabicyclo[3.2.1]octan-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 78-4
2-((1R,3r,5S)-8-azabicyclo[3.2.1]octan-3-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole

The starting material *tert*-butyl (1*R*,3*R*,5*S*)-3-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)-8-azabicyclo[3.2.1]octane-8-carboxylate 78-3 (257 mg, 0.41 mmol) was added to dichloromethane (4 mL), followed by the addition of trifluoroacetic acid (2 mL). The reaction mixture was stirred at room temperature for 2 hours, concentrated under reduced pressure, and then purified by reverse-phase column chromatography to obtain 2-((1*R*,3*R*,5*S*)-8-azabicyclo[3.2.1]octan-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole 78-4 (110 mg, yield: 51.2%).

LC-MS m/z (ESI)= 524.2 [M+1].

### Step 4:

2-((1*R*,3*R*,5*S*)-8-methyl-8-azabicyclo[3.2.1]octan-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 78
2-((1R,3r,5S)-8-methyl-8-azabicyclo[3.2.1]octan-3-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole

The starting material 2-((1*R*,3*R*,5*S*)-8-azabicyclo[3.2.1]octan-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole 78-4 (110 mg, 0.21 mmol) was added to methanol (5 mL), followed by the addition of paraformaldehyde (63 mg, 2.1 mmol) and sodium cyanoborohydride (264 mg, 4.2 mmol). The reaction mixture was stirred at room temperature overnight. After the reaction was completed, the mixture was directly filtered and purified by reverse-phase column chromatography to obtain 2-((1*R*,3*R*,5*S*)-8-methyl-8-azabicyclo[3.2.1]octan-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 78 (38 mg, yield: 33.6%).

LC-MS m/z (ESI)= 538.2 [M+1].

¹H NMR (400 MHz, DMSO-*d*₆) δ 9.02 (dd, 1H), 8.66 (dd, 1H), 8.05 (d, 1H), 7.63 (dd, 1H), 7.37 (d, 1H), 4.09 (d, 1H), 3.97 (dd, 2H), 3.81 (d, 1H), 3.59 (s, 1H), 3.50 (s, 3H), 2.45 (s, 2H), 2.34 (t, 1H), 2.21 (d, 1H), 2.18 - 2.05 (m, 2H), 1.89 (d, 6H).

¹⁹F NMR (376 MHz, DMSO-*d*₆) δ -58.07, -64.03.

### Example 79

2-((1*R*,3*S*,5*S*)-8-methyl-8-azabicyclo[3.2.1]octan-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 79
2-((1R,3S,5S)-8-methyl-8-azabicyclo[3.2.1]octan-3-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole

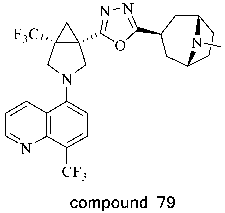

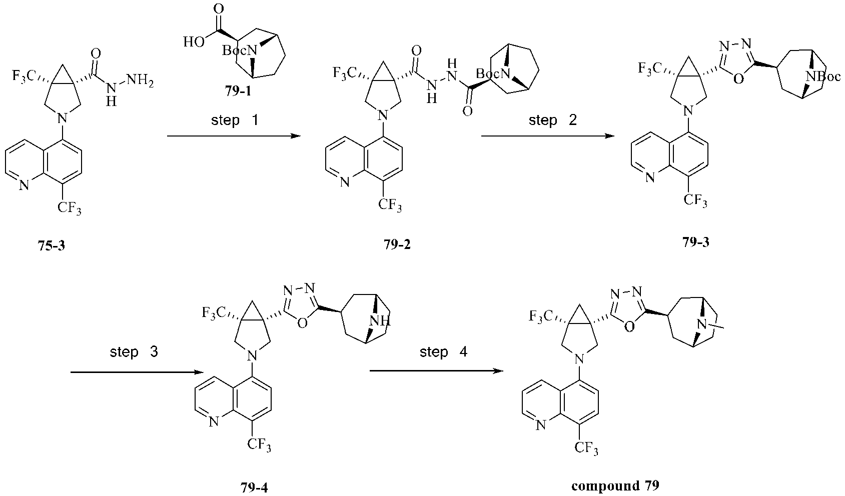

### Step 1:

*tert*-butyl (1*R*,3*S*,5*S*)-3-(2-((1*S*,5*R*)-5-(trifluoromethyl)-3-(8-(trifluoromethyl)quinolin-5-yl)-3-azabicyclo[3.1.0]hexane-1-carbonyl)hydrazine-1-carbonyl)-8-azabicyclo[3.2.1]octane-8-carboxylate compound 79-2
tert-butyl (1R,3S,5S)-3-(2-((1S,5R)-5-(trifluoromethyl)-3-(8-(trifluoromethyl)quinolin-5-yl)-3-azabicyclo[3.1.0]hexane-1-carbonyl)hydrazine-1-carbonyl)-8-azabicyclo[3.2.1]octane-8-carboxylate

The starting material (1*R*,3*S*,5*S*)-8-(*tert*-butoxycarbonyl)-8-azabicyclo[3.2.1]octane-3-carboxylic acid 79-1 (255 mg, 1.0 mmol) was added to *N,N*-dimethylformamide (1 mL), followed by the addition of *N*,*N*,*N*',*N*'-tetramethyl-*O*-(7-azabenzotriazol-1-yl)uronium hexafluorophosphate (380 mg, 1.0 mmol) and *N,N-*diisopropylethylamine (357 mg, 3.0 mmol). The reaction mixture was stirred at room temperature for 0.5 hours, and then *(1S,5R)-5-*(trifluoromethyl)-3-(8-(trifluoromethyl)quinolin-5-yl)-3-azabicyclo[3.1.0]hexane-1-carbohydrazide 75-3 (316 mg, 1.0 mmol) was added. The reaction mixture was stirred at room temperature for 6 hours and then purified by reverse-phase column chromatography to obtain *tert*-butyl (1*R*,3*S*,5*S*)-3-(2-((1*S*,5R)-5-(trifluoromethyl)-3-(8-(trifluoromethyl)quinolin-5-yl)-3-azabicyclo[3.1.0]hexane-1-carbonyl)hydrazine-1-carbonyl)-8-azabicyclo[3.2.1]octane-8-carboxylate compound 79-2 (380 mg, yield: 59.2%).

LCMS m/z(ESI)=642.2[M+1].

### Step 2:

*tert*-butyl (1*R*,3*S*,5*S*)-3-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)-8-azabicyclo[3.2.1]octane-8-carboxylate compound 79-3
tert-butyl (1R,3S,5S)-3-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)-8-azabicyclo[3.2.1]octane-8-carboxylate
*tert*-Butyl (1*R*,3*S*,5*S*)-3-(2-((1*S*,5*R*)-5-(trifluoromethyl)-3-(8-(trifluoromethyl)quinolin-5-yl)-3-azabicyclo[3.1.0]hexane-1-carbonyl)hydrazine-1-carbonyl)-8-azabicyclo[3.2.1]octane-8-carboxylate 79-2 (380 mg, 0.59 mmol) was dissolved in 5 mL of dichloromethane, followed by the addition of triethylamine (303 mg, 3.0 mmol) and *p-*toluenesulfonyl chloride (338 mg, 1.77 mmol). The mixture was stirred at room temperature for 5 hours. After TLC confirmed the completion of the reaction, the reaction mixture was concentrated and purified by silica gel column chromatography to obtain the target product *tert*-butyl (1*R*,3*S*,5*S*)-3-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)-8-azabicyclo[3.2.1]octane-8-carboxylate 79-3 (yellow solid, 257 mg, yield: 69.8%).

LC-MS m/z (ESI)= 624.2 [M+1].

### Step 3:

2-((1*R*,3*S*,5*S*)-8-azabicyclo[3.2.1]octan-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 79-4
2-((1R,3S,5S)-8-azabicyclo[3.2.1]octan-3-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole

The starting material *tert*-butyl (1*R*,3*S*,5*S*)-3-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)-8-azabicyclo[3.2.1]octane-8-carboxylate 79-3 (257 mg, 0.41 mmol) was added to dichloromethane (4 mL), followed by the addition of trifluoroacetic acid (2 mL). The reaction mixture was stirred at room temperature for 2 hours, concentrated under reduced pressure, and then purified by reverse-phase column chromatography to obtain 2-((1*R*,3*S*,5*S*)-8-azabicyclo[3.2.1]octan-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 79-4 (110 mg, yield: 51.2%).

LC-MS m/z (ESI)= 524.2 [M+1].

### Step 4:

2-((1*R*,3*S*,5*S*)-8-methyl-8-azabicyclo[3.2.1]octan-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 79
2-((1R,3S,5S)-8-methyl-8-azabicyclo[3.2.1]octan-3-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole

The starting material 2-((1*R*,3*S*,5*S*)-8-azabicyclo[3.2.1]octan-3-yl)-5-((1S,5R)-5-(trifluoromethyl)-3-(8-(trifluoromethyl)quinolin-5-yl)-3-azabicyclo[3.1.0]hexan-1-yl)-1,3,4-oxadiazole 79-4 (110 mg, 0.21 mmol) was added to methanol (5 mL), followed by the addition of paraformaldehyde (63 mg, 2.1 mmol) and sodium cyanoborohydride (264 mg, 4.2 mmol). The reaction mixture was stirred at room temperature overnight. After the reaction was completed, the mixture was directly filtered and purified by reverse-phase column chromatography to obtain 2-((1*R*,3*S*,5*S*)-8-methyl-8-azabicyclo[3.2.1]octan-3-yl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 79 (38 mg, yield: 33.6%).

LC-MS m/z (ESI)= 538.2 [M+1].

¹H NMR (400 MHz, DMSO-*d*₆) δ 9.01 (dd, 1H), 8.66 (dd, 1H), 8.05 (d, 1H), 7.63 (dd, 1H), 7.38 (d, 1H), 4.09 (d, 1H), 3.98 (t, 2H), 3.83 (d, 1H), 3.50 (s, 2H), 2.43 - 2.20 (m, 9H), 2.01 - 1.84 (m, 2H), 1.50 - 1.29 (m, 3H).

¹⁹F NMR (376 MHz, DMSO-*d*₆) δ -58.07, -63.79.

### Example 80

3-fluoro-4-((1*S*,5*R*)-1-(5-(piperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 80
3-fluoro-4-((1S,5R)-1-(5-(piperidin-4-yl)-1,3,4-oxadiazol-2-yl)-5-(trifluoromethyl)-3-azabicyclo[3.1.0]hexan-3-yl)pyrazolo[1,5-a]pyridine-7-carbonitrile

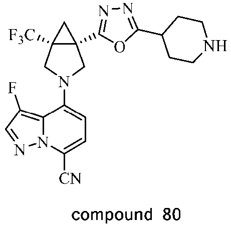

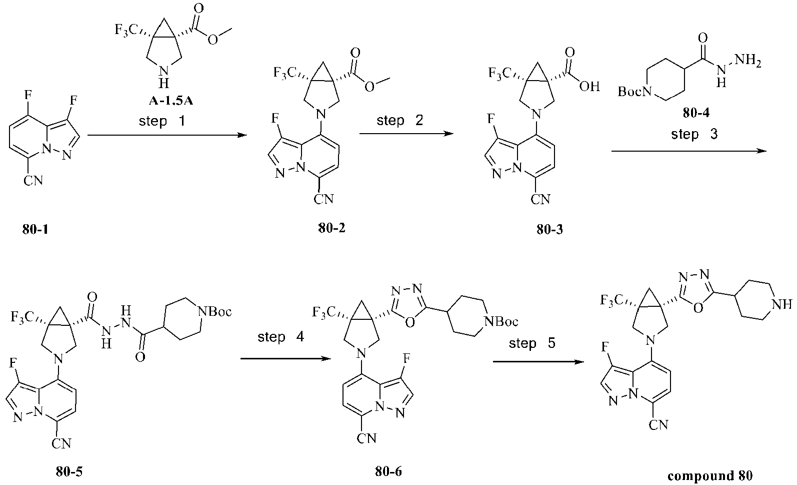

### Step 1:

methyl (1*S*,5*R*)-3-(7-cyano-3-fluoropyrazolo[1,5-*a*]pyridin-4-yl)-5-(trifluoromethyl)-3-azabicyclo[3.1.0]hexane-1-carboxylate compound 80-2
methyl (1S,5R)-3-(7-cyano-3-fluoropyrazolo[1,5-a]pyridin-4-yl)-5-(trifluoromethyl)-3-azabicyclo[3.1.0]hexane-1-carboxylate

The starting materials 3,4-difluoropyrazolo[1,5-*a*]pyridine-7-carbonitrile 80-1 (716 mg, 4.0 mmol) and methyl (1*S*,5*R*)-5-(trifluoromethyl)-3-azabicyclo[3.1.0]hexane-1-carboxylate A-1.5A hydrochloride (982 mg, 4.0 mmol) were added to dimethyl sulfoxide (4 mL), followed by the addition of *N,N-*diisopropylethylamine (4 mL). The reaction mixture was heated to 130°C and reacted for 4 hours. After the reaction was completed, purified water (50 mL) and ethyl acetate (50 mL × 3 times) were added for extraction. The organic phases were combined, washed with saturated brine, dried over anhydrous sodium sulfate, filtered, and rotary evaporated to dryness. The crude product was purified by column chromatography to obtain methyl (1*S*,5*R*)-3-(7-cyano-3-fluoropyrazolo[1,5-*a*]pyridin-4-yl)-5-(trifluoromethyl)-3-azabicyclo[3.1.0]hexane-1-carboxylate 80-2 (1.1 g, yield: 74.5%).

LCMS m/z(ESI)=369.1[M+1].

### Step 2:

(1*S*,5*R*)-3-(7-cyano-3-fluoropyrazolo[1,5-*a*]pyridin-4-yl)-5-(trifluoromethyl)-3-azabicyclo[3.1.0]hexane-1-carboxylic acid compound 80-3
(1*S*,5*R*)-3-(7-cyano-3-fluoropyrazolo[1,5-*a*]pyridin-4-yl)-5-(trifluoromethyl)-3-azabicyclo[3.1.0]hexane-1-carboxylic acid

The starting material methyl (1*S*,5*R*)-3-(7-cyano-3-fluoropyrazolo[1,5-*a*]pyridin-4-yl)-5-(trifluoromethyl)-3-azabicyclo[3.1.0]hexane-1-carboxylate 80-2 (500 mg, 1.36 mmol) was added to a mixed solution of methanol (5 mL) and water (5 mL), followed by the addition of lithium hydroxide (65 mg, 2.72 mmol) solid. The reaction mixture was stirred at room temperature overnight. After the reaction was completed, purified water (50 mL) and ethyl acetate (50 mL × 3 times) were added for extraction. The organic phases were combined, washed with saturated brine, dried over anhydrous sodium sulfate, filtered, and rotary evaporated to dryness. The crude product was purified by column chromatography to obtain (1*S*,5*R*)-3-(7-cyano-3-fluoropyrazolo[1,5-*a*]pyridin-4-yl)-5-(trifluoromethyl)-3-azabicyclo[3.1.0]hexane-1-carboxylic acid 80-3 (380 mg, yield: 78.7%).

LCMS m/z(ESI)=355.1[M+1].

### Step 3:

*tert*-butyl 4-(2-((1*S*,5*R*)-3-(7-cyano-3-fluoropyrazolo[1,5-*a*]pyridin-4-yl)-5-(trifluoromethyl)-3-azabicyclo[3.1.0]hexane-1-carbonyl)hydrazine-1-carbonyl)piperidine-1-carboxylate compound 80-5
tert-butyl 4-(2-((1S,5R)-3-(7-cyano-3-fluoropyrazolo[1,5-a]pyridin-4-yl)-5-(trifluoromethyl)-3-azabicyclo[3.1.0]hexane-1-carbonyl)hydrazine-1-carbonyl)piperidine-1-carboxylate

The starting material (1*S*,5*R*)-3-(7-cyano-3-fluoropyrazolo[1,5-*a*]pyridin-4-yl)-5-(trifluoromethyl)-3-azabicyclo[3.1.0]hexane-1-carboxylic acid 80-3 (355 mg, 1.0 mmol) was added to *N,N-*dimethylformamide (1 mL), followed by the addition of *N*,*N*,*N*',*N*'-tetramethyl-*O*-(7-azabenzotriazol-1-yl)uronium hexafluorophosphate (380 mg, 1.0 mmol) and *N,N-*diisopropylethylamine (357 mg, 3.0 mmol). The reaction mixture was stirred at room temperature for 0.5 hours, and then *tert*-butyl 4-(hydrazinecarbonyl)piperidine-1-carboxylate 80-4 (243 mg, 1.0 mmol) was added. The reaction mixture was stirred at room temperature for 6 hours and then purified by reverse-phase column chromatography to obtain *tert*-butyl 4-(2-((1*S*,5*R*)-3-(7-cyano-3-fluoropyrazolo[1,5-*a*]pyridin-4-yl)-5-(trifluoromethyl)-3-azabicyclo[3.1.0]hexane-1-carbonyl)hydrazine-1-carbonyl)piperidine-1-carboxylate 80-5 (362 mg, yield: 62.4%).

LCMS m/z(ESI)=580.2[M+1].

### Step 4:

*tert*-butyl 4-(5-((1*S*,5*R*)-3-(7-cyano-3-fluoropyrazolo[1,5-*a*]pyridin-4-yl)-5-(trifluoromethyl)-3-azabicyclo[3.1.0]hexan-1-yl)-1,3,4-oxadiazol-2-yl)piperidine-1-carboxylate compound 80-6
tert-butyl 4-(5-((1S,5R)-3-(7-cyano-3-fluoropyrazolo[1,5-a]pyridin-4-yl)-5-(trifluoromethyl)-3-azabicyclo[3.1.0]hexan-1-yl)-1,3,4-oxadiazol-2-yl)piperidine-1-carboxylate
*tert*-Butyl 4-(2-((1*S*,5*R*)-3-(7-cyano-3-fluoropyrazolo[1,5-a]pyridin-4-yl)-5-(trifluoromethyl)-3-azabicyclo[3.1.0]hexane-1-carbonyl)hydrazine-1-carbonyl)piperidine-1-carboxylate 80-5 (362 g, 0.62 mmol) was dissolved in 5 mL of dichloromethane, followed by the addition of triethylamine (303 mg, 3.0 mmol) and *p*-toluenesulfonyl chloride (338 mg, 1.77 mmol). The mixture was stirred at room temperature for 5 hours. After TLC confirmed the completion of the reaction, the reaction mixture was concentrated and purified by silica gel column chromatography to obtain the target product *tert*-butyl 4-(5-((1*S*,5*R*)-3-(7-cyano-3-fluoropyrazolo[1,5-*a*]pyridin-4-yl)-5-(trifluoromethyl)-3-azabicyclo[3.1.0]hexan-1-yl)-1,3,4-oxadiazol-2-yl)piperidine-1-carboxylate compound 80-6 (yellow solid, 251 mg, yield: 72.1%).

LC-MS m/z (ESI)= 562.2 [M+1].

### Step 5:

3-fluoro-4-((1*S*,5*R*)-1-(5-(piperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 80
3-fluoro-4-((1S,5R)-1-(5-(piperidin-4-yl)-1,3,4-oxadiazol-2-yl)-5-(trifluoromethyl)-3-azabicyclo[3.1.0]hexan-3-yl)pyrazolo[1,5-a]pyridine-7-carbonitrile

The starting material *tert*-butyl 4-(5-((1*S*,5*R*)-3-(7-cyano-3-fluoropyrazolo[1,5-*a*]pyridin-4-yl)-5-(trifluoromethyl)-3-azabicyclo[3.1.0]hexan-1-yl)-1,3,4-oxadiazol-2-yl)piperidine-1-carboxylate 80-6 (251 mg, 0.45 mmol) was added to dichloromethane (4 mL), followed by the addition of trifluoroacetic acid (2 mL). The reaction mixture was stirred at room temperature for 2 hours, concentrated under reduced pressure, and then purified by reverse-phase column chromatography to obtain 3-fluoro-4-((1*S*,5*R*)-1-(5-(piperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 80 (110 mg, yield: 52.9%).

LC-MS m/z (ESI)= 462.2 [M+1].

¹H NMR (400 MHz, DMSO-*d*₆) δ 8.25 (s, 1H), 7.62 (d, 1H), 6.48 (d, 1H), 4.40 - 3.94 (m, 4H), 3.04 (dd, 3H), 2.63 (t, 2H), 2.40 - 2.20 (m, 1H), 1.90 (s, 3H), 1.62 (q, 2H).

¹⁹F NMR (376 MHz, DMSO-*d*₆) δ -64.50, -167.63.

### Example 81

4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-amine compound 81
4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-amine

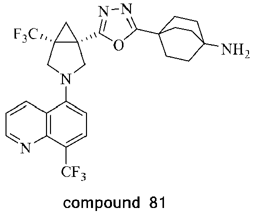

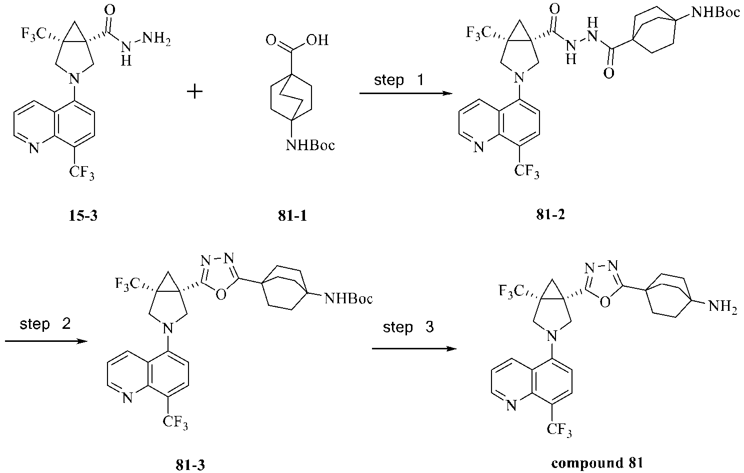

### Step 1:

tert-butyl (4-(2-((1S,5R)-5-(trifluoromethyl)-3-(8-(trifluoromethyl)quinolin-5-yl)-3-azabicyclo[3.1.0]hexane-1-carbonyl)hydrazine-1-carbonyl)bicyclo[2.2.2]octan-1-yl)carbamate compound 81-2
tert-butyl (4-(2-((1S,5R)-5-(trifluoromethyl)-3-(8-(trifluoromethyl)quinolin-5-yl)-3-azabicyclo[3.1.0]hexane-1-carbonyl)hydrazine-1-carbonyl)bicyclo[2.2.2]octan-1-yl)carbamate

The starting material 4-((*tert*-butoxycarbonyl)amino)bicyclo[2.2.2]octane-1-carboxylic acid 81-1 (269 mg, 1.0 mmol) was added to *N,N*-dimethylformamide (1 mL), followed by the addition of *N*,*N*,*N*',*N*'-tetramethyl-*O*-(7-azabenzotriazol-1-yl)uronium hexafluorophosphate (380 mg, 1.0 mmol) and N,N-diisopropylethylamine (357 mg, 3.0 mmol). The reaction mixture was stirred at room temperature for 0.5 hours, and then *(1S,5R)-5-*(trifluoromethyl)-3-(8-(trifluoromethyl)quinolin-5-yl)-3-azabicyclo[3.1.0]hexane-1-carbohydrazide 15-3 (316 mg, 1.0 mmol) was added. The reaction mixture was stirred at room temperature for 6 hours and then purified by reverse-phase column chromatography to obtain *tert*-butyl (4-(2-((1*S*,5*R*)-5-(trifluoromethyl)-3-(8-(trifluoromethyl)quinolin-5-yl)-3-azabicyclo[3.1.0]hexane-1-carbonyl)hydrazine-1-carbonyl)bicyclo[2.2.2]octan-1-yl)carbamate 81-2 (391 mg, yield: 59.6%).

LCMS m/z(ESI)=656.2[M+1].

### Step 2:

*tert*-butyl (4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-yl)carbamate compound 81-3
tert-butyl (4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-yl)carbamate
*tert*-Butyl (4-(2-((1*S*,5*R*)-5-(trifluoromethyl)-3-(8-(trifluoromethyl)quinolin-5-yl)-3-azabicyclo[3.1.0]hexane-1-carbonyl)hydrazine-1-carbonyl)bicyclo[2.2.2]octan-1-yl)carbamate 81-2 (391 g, 0.60 mmol) was dissolved in 5 mL of dichloromethane, followed by the addition of triethylamine (303 mg, 3.0 mmol) and *p*-toluenesulfonyl chloride (340 mg, 1.80 mmol). The mixture was stirred at room temperature for 5 hours. After TLC confirmed the completion of the reaction, the reaction mixture was concentrated and purified by silica gel column chromatography to obtain the target product *tert*-butyl (4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-yl)carbamate 81-3 (yellow solid, 303 mg, yield: 79.3%).

LC-MS m/z (ESI)= 638.2 [M+1].

### Step 3:

4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-amine compound 81
4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-amine

The starting material *tert*-butyl (4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-yl)carbamate 81-3 (303 mg, 0.48 mmol) was added to dichloromethane (4 mL), followed by the addition of trifluoroacetic acid (2 mL). The reaction mixture was stirred at room temperature for 2 hours, concentrated under reduced pressure, and then purified by reverse-phase column chromatography to obtain 4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-amine compound 81 (220 mg, yield: 86.3%).

LC-MS m/z (ESI)= 538.2 [M+1].

¹H NMR (400 MHz, DMSO-*d*₆) δ 9.01 (dd, 1H), 8.65 (dd, 1H), 8.04 (d, 1H), 7.63 (dd, 1H), 7.38 (d, 1H), 5.75 (br, 2H), 4.08 (d, 1H), 3.97 (dd, 2H), 3.81 (d, 1H), 2.34 (d, 1H), 2.22 (d, 1H), 1.95 (dd, 6H), 1.68 (dd, 6H).

¹⁹F NMR (376 MHz, DMSO-*d*₆) δ -58.06, -64.09.

### Example 82

*N,N*-dimethyl-4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-amine compound 82
N,N-dimethyl-4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-amine 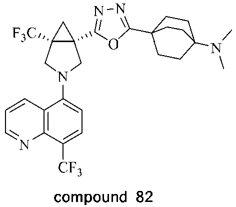 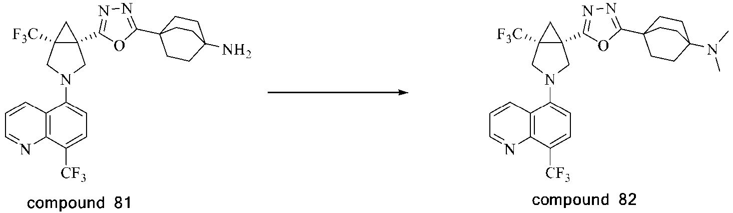
*N,N-dimethyl-4-(5-((1S,5R)-5-(trifluoromethyl)-3-(8-(trifluoromethyl)quinolin-5-yl)-*3-azabicyclo[3.1.0]hexan-1-yl)-1,3,4-oxadiazol-2-yl)bicyclo[2.2.2]octan-1-amine compound 82
N,N-dimethyl-4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-amine

The starting material 4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-amine compound 81 (100 mg, 0.19 mmol) was added to methanol (5 mL), followed by the addition of paraformaldehyde (57 mg, 1.9 mmol) and sodium cyanoborohydride (258 mg, 3.8 mmol). The reaction mixture was stirred at room temperature overnight. After the reaction was completed, the mixture was directly filtered and purified by reverse-phase column chromatography to obtain *N,N*-dimethyl-4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)bicyclo[2.2.2]octan-1-amine compound 82 (56 mg, yield: 52.1%).

LC-MS m/z (ESI) = 566.2[M+1].

¹H NMR (400 MHz, DMSO-*d*₆) δ 9.01 (dd, 1H), 8.65 (dd, 1H), 8.04 (d, 1H), 7.62 (dd, 1H), 7.38 (d, 1H), 4.08 (d, 1H), 3.96 (dd, 2H), 3.81 (d, 1H), 2.33 (d, 1H), 2.30 - 2.12 (m, 7H), 1.99 - 1.88 (m, 6H), 1.67 (dd, 6H).

¹⁹F NMR (376 MHz, DMSO-*d*₆) δ -58.07, -64.10.

### Example 83

3-bromo-4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 83
3-bromo-4-((1S,5R)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-a]pyridine-7-carbonitrile

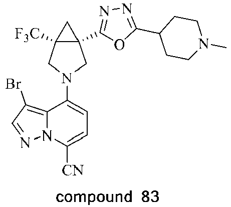

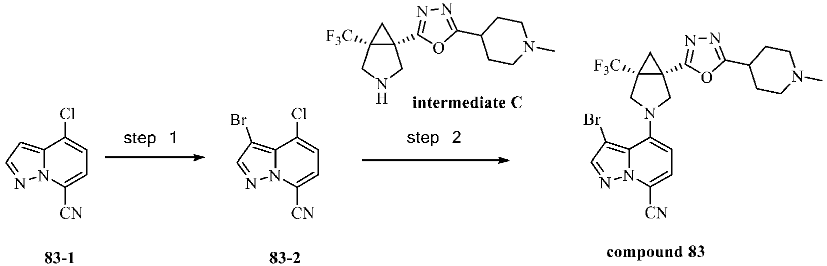

### Step 1:

3-bromo-4-chloropyrazolo[1,5-a]pyridine-7-carbonitrile compound 83-2
3-bromo-4-chloropyrazolo[1,5-a]pyridine-7-carbonitrile

The starting material 4-chloropyrazolo[1,5-*a*]pyridine-7-carbonitrile 83-1 (300 mg, 1.69 mmol) was dissolved in N,N-dimethylformamide (3 mL), followed by the addition of *N-*bromosuccinimide (301 mg, 1.69 mmol). The reaction mixture was stirred at room temperature overnight. After the reaction was completed, purified water (50 mL) and ethyl acetate (50 mL × 3 times) were added. The organic phases were combined. The organic phase was washed with saturated brine, dried over anhydrous sodium sulfate, filtered, and rotary evaporated to dryness. The crude product was purified by silica gel column chromatography to obtain 3-bromo-4-chloropyrazolo[1,5-*a*]pyridine-7-carbonitrile 83-2 (252 mg, yield: 57.8%).

LC-MS m/z (ESI)= 257.9 [M+1].

### Step 2:

3-bromo-4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 83
3-bromo-4-((1S,5R)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile

The starting materials 3-bromo-4-chloropyrazolo[1,5-a]pyridine-7-carbonitrile (252 mg, 0.98 mmol) and 2-(1-methylpiperidin-4-yl)-5-((1*S*,5*R*)-5-(trifluoromethyl)-3-azabicyclo[3.1.0]hexan-1-yl)-1,3,4-oxadiazole compound 82 (476 mg, 1.5 mmol) were added to 1,4-dioxane (3 mL), followed by the addition of cesium carbonate (977 mg, 3.0 mmol) and methanesulfonato(2-dicyclohexylphosphino-2',6'-di-i-propoxy-1,1'-biphenyl)(2'-amino-1,1'-biphenyl-2-yl)palladium(II) (84 mg, 0.1 mmol). After nitrogen protection, the reaction mixture was heated to 100°C and reacted for 3 hours. After the reaction was completed, the mixture was directly purified by reverse-phase column chromatography to obtain 1-methyl-5-((1S,5R)-1-(5-(1-methylpiperidin-4-yl)-1,3,4-oxadiazol-2-yl)-5-(trifluoromethyl)-3-azabicyclo[3.1.0]hexan-3-yl)-2-oxo-1,2-dihydroquinoline-8-carbonitrile compound 83 (110 mg, yield: 20.8%).

LC-MS m/z (ESI)= 538.10 [M+1].

¹H NMR (400 MHz, DMSO-*d*₆) δ 8.30 (s, 1H), 7.78 (d, 1H), 7.04 (d, 1H), 4.09 (d, 1H), 4.02 (d, 1H), 3.88 (d, 1H), 3.74 (d, 1H), 2.94 (ddd, 1H), 2.74 (d, 2H), 2.36 - 2.24 (m, 1H), 2.19 (d, 4H), 2.06 (t, 2H), 2.00 - 1.90 (m, 2H), 1.80 - 1.68 (m, 2H).

¹⁹F NMR (376 MHz, DMSO-*d*₆) δ -63.96.

### Example 84

3-chloro-4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 84
3-chloro-4-((1S,5R)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-a]pyridine-7-carbonitrile

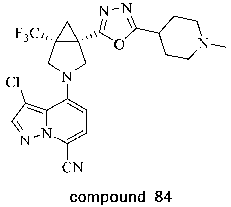

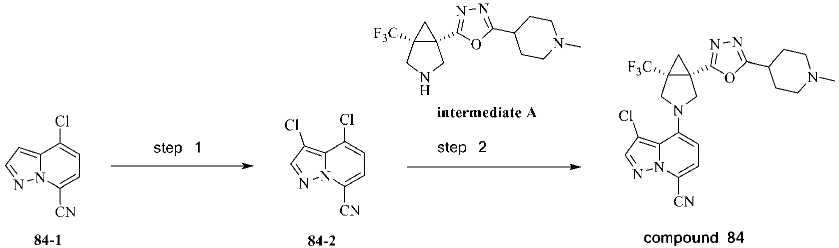

### Step 1:

3,4-dichloropyrazolo[1,5-*a*]pyridine-7-carbonitrile compound 84-2
3,4-dichloropyrazolo[1,5-*a*]pyridine-7-carbonitrile

The starting material 4-chloropyrazolo[1,5-*a*]pyridine-7-carbonitrile 84-1 (300 mg, 1.69 mmol) was dissolved in N,N-dimethylformamide (3 mL), followed by the addition of *N-*chlorosuccinimide (226 mg, 1.69 mmol). The reaction mixture was stirred at room temperature overnight. After the reaction was completed, purified water (50 mL) and ethyl acetate (50 mL × 3 times) were added. The organic phases were combined. The organic phase was washed with saturated brine, dried over anhydrous sodium sulfate, filtered, and rotary evaporated to dryness. The crude product was purified by silica gel column chromatography to obtain 3,4-dichloropyrazolo[1,5-*a*]pyridine-7-carbonitrile compound 84-2 (313 mg, yield: 87.4%).

LC-MS m/z (ESI)= 212.0 [M+1].

### Step 2:

3-chloro-4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 84
3-chloro-4-((1S,5R)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile

The starting materials 3,4-dichloropyrazolo[1,5-*a*]pyridine-7-carbonitrile 84-2 (211 mg, 1.0 mmol) and 2-(1-methylpiperidin-4-yl)-5-((1S,5R)-5-(trifluoromethyl)-3-azabicyclo[3.1.0]hexan-1-yl)-1,3,4-oxadiazole intermediate A (476 mg, 1.5 mmol) were added to 1,4-dioxane (3 mL), followed by the addition of cesium carbonate (977 mg, 3.0 mmol) and methanesulfonato(2-dicyclohexylphosphino-2',6'-di-i-propoxy-1,1'-biphenyl)(2'-amino-1,1'-biphenyl-2-yl)palladium(II) (84 mg, 0.1 mmol). After nitrogen protection, the reaction mixture was heated to 100°C and reacted for 3 hours. After the reaction was completed, the mixture was directly purified by reverse-phase column chromatography to obtain 3-chloro-4-((1S,5R)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 84 (121 mg, yield: 24.6%).

LC-MS m/z (ESI)= 492.1 [M+1].

¹H NMR (400 MHz, Chloroform-*d*) δ 7.99 (s, 1H), 7.29 - 7.25 (m, 1H), 6.61 (d, 1H), 4.11 - 4.03 (m, 2H), 3.97 (d, 1H), 3.62 (d, 1H), 3.01 - 2.83 (m, 3H), 2.33 (s, 3H), 2.21 - 2.06 (m, 5H), 2.03 - 1.95 (m, 3H).

¹⁹F NMR (376 MHz, Chloroform-*d*) δ -65.17.

### Example 85

3,5-dibromo-4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 85
3,5-dibromo-4-((1S,5R)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-a]pyridine-7-carbonitrile

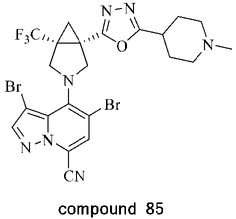

### Example 86

5-bromo-4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 86
5-bromo-4-((1S,5R)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-a]pyridine-7-carbonitrile

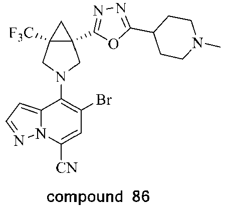

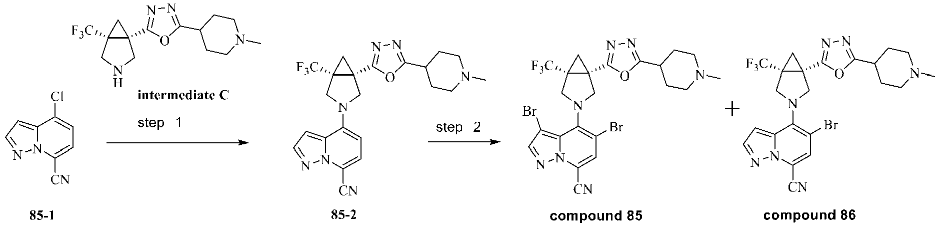

### Step 1:

4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 85-2
4-((1S,5R)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-a]pyridine-7-carbonitrile

The starting materials 4-chloropyrazolo[1,5-*a*]pyridine-7-carbonitrile 85-1 (178 mg, 1.0 mmol) and 2-(1-methylpiperidin-4-yl)-5-((1S,5R)-5-(trifluoromethyl)-3-azabicyclo[3.1.0]hexan-1-yl)-1,3,4-oxadiazole intermediate C (476 mg, 1.5 mmol) were added to 1,4-dioxane (3 mL), followed by the addition of cesium carbonate (977 mg, 3.0 mmol) and methanesulfonato(2-dicyclohexylphosphino-2',6'-di-i-propoxy-1,1'-biphenyl)(2'-amino-1,1'-biphenyl-2-yl)palladium(II) (84 mg, 0.1 mmol). After nitrogen protection, the reaction mixture was heated to 100°C and reacted for 3 hours. After the reaction was completed, the mixture was directly purified by reverse-phase column chromatography to obtain 4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile 85-2 (180 mg, yield: 39.3%).

LC-MS m/z (ESI)= 458.2 [M+1].

### Step 2:

3,5-dibromo-4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 85
3,5-dibromo-4-((1S,5R)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-a]pyridine-7-carbonitrile 5-bromo-4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 86
5-bromo-4-((1S,5R)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-a]pyridine-7-carbonitrile

The starting material 4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile 85-2 (180 mg, 0.39 mmol) was dissolved in *N,N*-dimethylformamide (3 mL), followed by the addition of *N*-chlorosuccinimide (83 mg, 0.47 mmol). The reaction mixture was stirred at room temperature overnight. After the reaction was completed, purified water (50 mL) and ethyl acetate (50 mL × 3 times) were added. The organic phases were combined. The organic phase was washed with saturated brine, dried over anhydrous sodium sulfate, filtered, and rotary evaporated to dryness. The crude product was purified by reverse-phase column chromatography to obtain 3,5-dibromo-4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 85 (30 mg, yield: 12.5%) and 5-bromo-4-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 86 (21 mg, yield: 10.0%).

compound 85: LC-MS m/z (ESI)= 616.0 [M+1].

¹H NMR (400 MHz, Chloroform-*d*) δ 8.07 (s, 1H), 7.50 (s, 1H), 4.54 (d, 1H), 4.31 (d, 1H), 3.60 (dd, 2H), 3.13 - 2.89 (m, 3H), 2.51 - 2.30 (m, 7H), 2.19 (s, 2H), 2.03 (d, 2H).

¹⁹F NMR (376 MHz, Chloroform-*d*) δ -65.51.

compound 86: LC-MS m/z (ESI)= 538.1 [M+1].

¹H NMR (400 MHz, Chloroform-d) δ 8.08 (d, 1H), 7.44 (s, 1H), 6.78 (d, 1H), 4.41 (d, 1H), 4.15 - 4.00 (m, 3H), 2.99 - 2.86 (m, 3H), 2.36 (s, 3H), 2.28 - 2.12 (m, 6H), 2.04 - 1.99 (m, 2H).

¹⁹F NMR (376 MHz, Chloroform-*d*) δ -64.98.

### Example 87

2-(1-methylpiperidin-4-yl)-5-((1*S*,5*R*)-5-(trifluoromethyl)-3-(7-(trifluoromethyl)pyrazolo[1,5-*a*]pyridin-4-yl)-3-azabicyclo[3.1.0]hexan-1-yl)-1,3,4-oxadiazole compound 87
2-(1-methylpiperidin-4-yl)-5-((1S,5R)-5-(trifluoromethyl)-3-(7-(trifluoromethyl)pyrazolo[1,5-a]pyridin-4-yl)-3-azabicyclo[3.1.0]hexan-1-yl)-1,3,4-oxadiazole

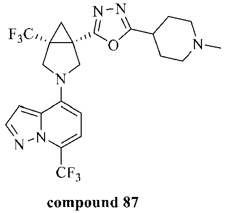

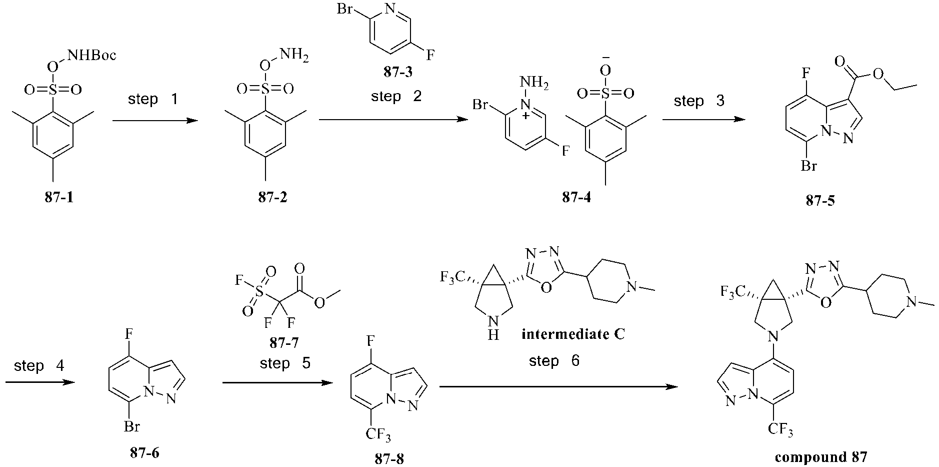

### Step 1:

*O*-(mesitylsulfonyl)hydroxylamine compound 87-2
*O*-(mesitylsulfonyl)hydroxylamine

Trifluoroacetic acid (300 mL) was cooled to 0°C, and then the starting material *tert-*butyl ((methylsulfonyl)oxy)carbamate (100 g, 31.7 mmol) was added. The mixture was stirred at 0°C for 4 hours. After the reaction was completed, the reaction mixture was added dropwise to ice water (1 L), and a large amount of solid precipitated. The mixture was filtered, and the filter cake was rinsed with ice water. The filter cake was then triturated with petroleum ether (150 mL), filtered, and the filter cake was rotary evaporated to dryness and directly used in the next step. *O*-(Mesitylsulfonyl)hydroxylamine 87-2 (65 g, yield: 95.6%) was obtained as a white solid.

LC-MS m/z (ESI) = 216.1 [M+1].

### Step 2:

1-amino-2-bromo-5-fluoropyridin-1-ium, 2,4,6-trimethylbenzenesulfonate compound 87-4
1-amino-2-bromo-5-fluoropyridin-1-ium, 2,4,6-trimethylbenzenesulfonate
*O*-(Mesitylsulfonyl)hydroxylamine 87-2 (60 g, 278 mmol) and 2-bromo-5-fluoropyridine 87-3 (49 g, 278 mmol) were dissolved in dichloromethane (600 mL). The reaction mixture was stirred at 10°C for 18 hours, and then concentrated to obtain the crude product. The crude product was triturated with ethyl acetate (300 mL), filtered, and the solid was concentrated to dryness to obtain 1-amino-2-bromo-5-fluoropyridin-1-ium, 2,4,6-trimethylbenzenesulfonate 87-4 (60 g, yield: 55.6%) as a white solid.

LC-MS m/z (ESI) = 201.0 &190.9.

### Step 3:

ethyl 7-bromo-4-fluoropyrazolo[1,5-*a*]pyridine-3-carboxylate compound 87-5
ethyl 7-bromo-4-fluoropyrazolo[1,5-a]pyridine-3-carboxylate

The starting material 1-amino-2-bromo-5-fluoropyridin-1-ium, 2,4,6-trimethylbenzenesulfonate 87-4 (60 g, 154 mmol) was dissolved in N,N-dimethylformamide (600 mL), and the mixture was cooled to 0°C. Then, methyl propiolate (12.9 g, 154 mmol) was added, followed by the addition of triethylenediamine (34.5 g, 308 mmol) in portions. The reaction exothermically proceeded vigorously. After the addition was completed, the mixture was slowly warmed to room temperature and reacted for 3 hours. After the reaction was completed, purified water (2 L) and ethyl acetate (1 L × 3 times) were added. The organic phases were combined, washed with saturated brine, dried over anhydrous sodium sulfate, filtered, and rotary evaporated to dryness. The crude product was purified by silica gel column chromatography to obtain ethyl 7-bromo-4-fluoropyrazolo[1,5-*a*]pyridine-3-carboxylate 87-5 (14 g, yield: 31.7%).

LC-MS m/z (ESI) = 287.0 [M+1].

### Step 4:

7-bromo-4-fluoropyrazolo[1,5-*a*]pyridine compound 87-6
7-bromo-4-fluoropyrazolo[1,5-a]pyridine

The starting material ethyl 7-bromo-4-fluoropyrazolo[1,5-*a*]pyridine-3-carboxylate 87-5 (2.0 g, 7.0 mmol) was dissolved in glacial acetic acid (12 mL) and purified water (12 mL), and concentrated hydrochloric acid (9 mL, 108 mmol) was added under stirring. After the dropwise addition was completed, the reaction mixture was heated to 100°C and stirred for 18 hours. Then, a saturated aqueous sodium bicarbonate solution was added to adjust the pH to 8, and the mixture was extracted with ethyl acetate. The organic phase was washed with saturated brine, dried over anhydrous sodium sulfate, filtered, and rotary evaporated to dryness. The crude product was purified by silica gel column chromatography to obtain 7-bromo-4-fluoropyrazolo[1,5-*a*]pyridine 87-6 (1.1 g, yield: 72.8%) as a white solid.

LC-MS m/z (ESI) = 215.0 [M+1].

### Step 5:

4-fluoro-7-(trifluoromethyl)pyrazolo[1,5-*a*]pyridine compound 87-8
4-fluoro-7-(trifluoromethyl)pyrazolo[1,5-a]pyridine

The starting material 7-bromo-4-fluoropyrazolo[1,5-*a*]pyridine compound 87-6 (645 mg, 3.0 mmol) was added to *N,N*-dimethylformamide (10 mL), followed by the addition of methyl 2,2-difluoro-2-(fluorosulfonyl)acetate 87-7 (1.16 g, 9 mmol), copper(I) iodide (572 mg, 3.0 mmol), and hexamethylphosphoramide (477 mg, 3.0 mmol). After nitrogen protection, the reaction mixture was heated to 110°C and reacted for 4 hours. After the reaction was completed, purified water and ethyl acetate were added, and the mixture was filtered through diatomite. The phases of the filtrate were separated, and the aqueous phase was extracted again with ethyl acetate. The combined organic phases were washed with saturated brine, dried over anhydrous sodium sulfate, filtered, and rotary evaporated to dryness. The crude product was purified by silica gel column chromatography to obtain 4-fluoro-7-(trifluoromethyl)pyrazolo[1,5-*a*]pyridine 87-8 (430 mg, yield: 69.9%) as a white solid.

LC-MS m/z (ESI) = 205.0 [M+1].

### Step 6:

2-(1-methylpiperidin-4-yl)-5-((1*S*,5*R*)-5-(trifluoromethyl)-3-(7-(trifluoromethyl)pyrazolo[1,5-*a*]pyridin-4-yl)-3-azabicyclo[3.1.08]hexan-1-yl)-1,3,4-oxadiazole compound 87
2-(1-methylpiperidin-4-yl)-5-((1S,5R)-5-(trifluoromethyl)-3-(7-(trifluoromethyl)pyrazolo[1,5-a]pyridin-4-yl)-3-azabicyclo[3.1.0]hexan-1-yl)-1,3,4-oxadiazole

The starting materials 4-fluoro-7-(trifluoromethyl)pyrazolo[1,5-*a*]pyridine 87-8 (205 mg, 1.0 mmol) and (15,5R)-1-(1-(1-methylpiperidin-4-yl)-1*H*-1,2,4-triazol-3-yl)-5-(trifluoromethyl)-3-azabicyclo[3.1.0]hexane intermediate C (300 mg, 0.95 mmol) were added to dimethyl sulfoxide (1 mL), followed by the addition of *N,N*-diisopropylethylamine (1 mL). After nitrogen protection, the reaction mixture was heated to 130°C and reacted for 4 hours. After the reaction was completed, the mixture was directly purified by reverse-phase column chromatography to obtain 2-(1-methylpiperidin-4-yl)-5-((1*S*,5*R*)-5-(trifluoromethyl)-3-(7-(trifluoromethyl)pyrazolo[1,5-*a*]pyridin-4-yl)-3-azabicyclo[3.1.0]hexan-1-yl)-1,3,4-oxadiazole compound 87 (30 mg, yield: 42.1%) as a white solid.

LC-MS m/z (ESI)= 501.20 [M+1].

¹H NMR (400 MHz, DMSO-*d*₆) δ 8.06 (d, 1H), 7.33 (d, 1H), 7.18 (d, 1H), 6.31 (d, 1H), 4.47 (d, 1H), 4.31 (d, 1H), 4.21 (d, 1H), 4.01 (d, 1H), 2.94 (tt, 1H), 2.72 (dt, 2H), 2.36 - 2.27 (m, 1H), 2.16 (s, 3H), 2.09 - 1.90 (m, 5H), 1.72 (dtd, 2H).

¹⁹F NMR (376 MHz, DMSO-*d*₆) δ -64.64, -65.48.

### Example 88

2-(1-methylpiperidin-4-yl)-5-((1*S*,5*R*)-5-(trifluoromethyl)-3-(8-(trifluoromethyl)quinoxalin-5-yl)-3-azabicyclo[3.1.0]hexan-1-yl)-1,3,4-oxadiazole compound 88
2-(1-methylpiperidin-4-yl)-5-((1S,5R)-5-(trifluoromethyl)-3-(8-(trifluoromethyl)quinoxalin-5-yl)-3-azabicyclo[3.1.0]hexan-1-yl)-1,3,4-oxadiazole

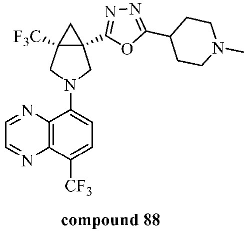

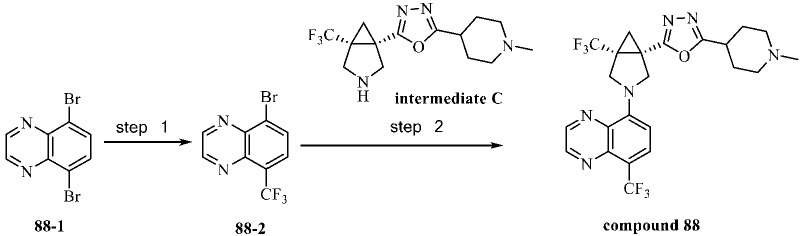

### Step 1:

5-bromo-8-(trifluoromethyl)quinoxaline 88-2
5-bromo-8-(trifluoromethyl)quinoxaline

The starting material 5,8-dibromoquinoxaline 88-1 (864 mg, 3.0 mmol) was added to N,N-dimethylformamide (10 mL), followed by the addition of methyl 2,2-difluoro-2-(fluorosulfonyl)acetate (773 mg, 6.0 mmol), copper(I) iodide (572 mg, 3.0 mmol), and hexamethylphosphoramide (477 mg, 3.0 mmol). The reaction mixture was placed in a microwave reactor, heated to 130°C, and reacted for 2 hours. After the reaction was completed, purified water and ethyl acetate were added. The mixture was filtered through diatomite, and the phases of the filtrate were separated. The aqueous phase was extracted again with ethyl acetate. The combined organic phases were washed with saturated brine, dried over anhydrous sodium sulfate, filtered, and rotary evaporated to dryness. The crude product was purified by silica gel column chromatography to obtain 5-bromo-8-(trifluoromethyl)quinoxaline 88-2 (310 mg, yield: 37.3%) as a white solid.

LC-MS m/z (ESI) = 276.90 [M+1].

### Step 2:

2-(1-methylpiperidin-4-yl)-5-((1*S*,5*R*)-5-(trifluoromethyl)-3-(8-(trifluoromethyl)quinoxalin-5-yl)-3-azabicyclo[3.1.0]hexan-1-yl)-1,3,4-oxadiazole compound 88
2-(1-methylpiperidin-4-yl)-5-((1S,5R)-5-(trifluoromethyl)-3-(8-(trifluoromethyl)quinoxalin-5-yl)-3-azabicyclo[3.1.0]hexan-1-yl)-1,3,4-oxadiazole

The starting materials 5-bromo-8-(trifluoromethyl)quinoxaline 88-2 (277 mg, 1.0 mmol) and 2-(1-methylpiperidin-4-yl)-5-((1S,5R)-5-(trifluoromethyl)-3-azabicyclo[3.1.0]hexan-1-yl)-1,3,4-oxadiazole intermediate C (476 mg, 1.5 mmol) were added to 1,4-dioxane (3 mL), followed by the addition of cesium carbonate (977 mg, 3.0 mmol) and methanesulfonato(2-dicyclohexylphosphino-2',6'-di-i-propoxy-1,1'-biphenyl)(2'-amino-1,1'-biphenyl-2-yl)palladium(II) (84 mg, 0.1 mmol). After nitrogen protection, the reaction mixture was heated to 100°C and reacted for 3 hours. After the reaction was completed, the mixture was directly purified by reverse-phase column chromatography to obtain 2-(1-methylpiperidin-4-yl)-5-((1*S*,5*R*)-5-(trifluoromethyl)-3-(8-(trifluoromethyl)quinoxalin-5-yl)-3-azabicyclo[3.1.0]hexan-1-yl)-1,3,4-oxadiazole compound 88 (156 mg, yield: 30.4%).

LC-MS m/z (ESI) = 513.20 [M+1].

¹H NMR (400 MHz, DMSO-*d*₆) δ 9.02 (d, 1H), 8.93 (d, 1H), 8.03 (d, 1H), 7.04 (d, 1H), 4.84 (d, 2H), 4.27 (d, 1H), 4.11 (d, 1H), 2.95 (ddt, 1H), 2.73 (d, 2H), 2.29 (d, 1H), 2.17 (s, 3H), 2.12 - 1.89 (m, 5H), 1.80 - 1.66 (m, 2H).

¹⁹F NMR (376 MHz, DMSO-*d*₆) δ -57.56, -64.56.

### Example 89

4-((1*S*,5*R*)-1-(5-((1-methylpiperidin-4-yl)oxy)-1,3,4-oxadiazol-2-yl)-5-(trifluoromethyl)-3-azabicyclo[3.1.0]hexan-3-yl)pyrazolo[1,5-*a*]pyridine-7-carbonitrile compound 89
4-((1S,5R)-1-(5-((1-methylpiperidin-4-yl)oxy)-1,3,4-oxadiazol-2-yl)-5-(trifluoromethyl)-3-azabicyclo[3.1.0]hexan-3-yl)pyrazolo[1,5-*a*]pyridine-7-carbonitrile

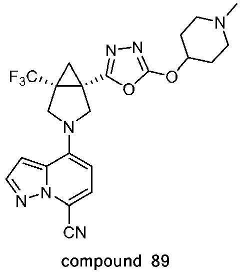

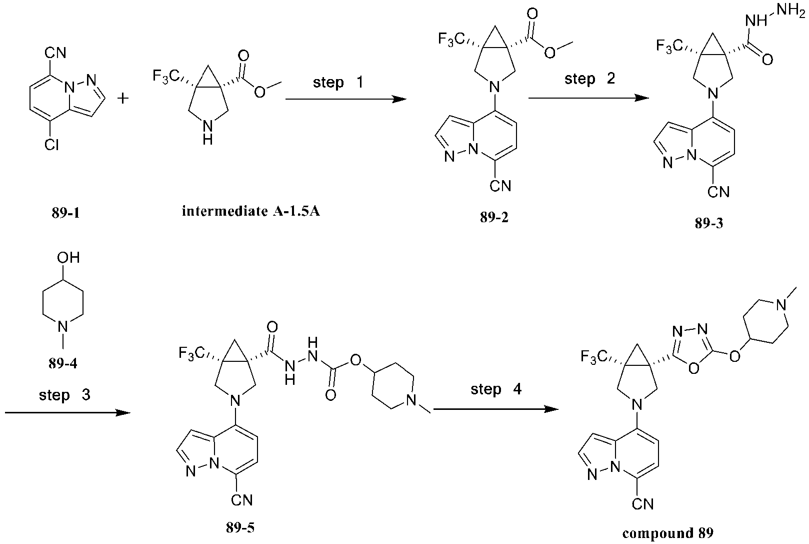

### Step 1: methyl (1S,5R)-3-(7-cyanopyrazolo[1,5-a]pyridin-4-yl)-5-(trifluoromethyl)-3-azabicyclo[3.1.0]hexane-1-carboxylate compound 89-2

(1S,5R)-3-(7-cyanopyrazolo[1,5-a]pyridin-4-yl)-5-(trifluoromethyl)-3-azabicyclo[3.1.0]hexane-1-carboxylate
Under an N₂ atmosphere, methyl (1S,5R)-5-(trifluoromethyl)-3-azabicyclo[3.1.0]hexane-1-carboxylate intermediate A-1.5A hydrochloride (1.00 g, 4.08 mmol) was dissolved in 1,4-dioxane (20.0 mL) and water (2.00 mL), followed by the sequential addition of 84-chloropyrazolo[1,5-*a*]pyridine-7-carbonitrile 89-1 (830 mg, 4.68 mmol), cesium carbonate (7.98 g, 24.5 mmol), and RuPhosPdG3 (341 mg, 0.4 mmol). The mixture was purged with nitrogen three times, heated to 90°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated and purified by reverse-phase C18 column chromatography (acidic method) to obtain 1.10 g of the crude target product methyl *(1S,5R)-*3-(7-cyanopyrazolo[1,5-*a*]pyridin-4-yl)-5-(trifluoromethyl)-3-azabicyclo[3.1.0]hexane-1-carboxylate compound 89-2 (yellow solid, 1.1 g, yield: 3.8%).

LC-MS m/z (ESI)= 351.25 [M+1].

### Step 2: (1S,5R)-3-(7-cyanopyrazolo[1,5-a]pyridin-4-yl)-5-(trifluoromethyl)-3-azabicyclo[3.1.0]hexane-1-carbohydrazide compound 89-3

(1S,5R)-3-(7-cyanopyrazolo[1,5-a]pyridin-4-yl)-5-(trifluoromethyl)-3-azabicyclo[3.1.0]hexane-1-carbohydrazide
Under an N₂ atmosphere, methyl (1*S*,5*R*)-3-(7-cyanopyrazolo[1,5-*a*]pyridin-4-yl)-5-(trifluoromethyl)-3-azabicyclo[3.1.0]hexane-1-carboxylate 89-2 (1.00 g, 4.08 mmol) was dissolved in methanol (20.0 mL), followed by the sequential addition of DBU (2.888 g, 18.7 mmol), hydrazine hydrochloride (963 mg, 14.2 mmol), and water (0.4 mL). The mixture was purged with nitrogen three times, heated to 80°C, and reacted for 3 h. The mixture was rotary evaporated to remove the solvent until dryness, and the residue was purified by reverse-phase C18 column chromatography (acidic method) to obtain the target product (1S,5R)-3-(7-cyanopyrazolo[1,5-*a*]pyridin-4-yl)-5-(trifluoromethyl)-3-azabicyclo[3.1.0]hexane-1-carbohydrazide compound 89-3 (yellowish-white solid, 300 mg, yield: 30%).

LC-MS m/z (ESI)= 351.2 [M+1].

### Step 3: 1-methylpiperidin-4-yl 2-((1S,5R)-3-(7-cyanopyrazolo[1,5-a]pyridin-4-yl)-5-(trifluoromethyl)-3-azabicyclo[3.1.0]hexane-1-carbonyl)hydrazine-1-carboxylatecompound 89-5

1-methylpiperidin-4-yl 2-((1S,5R)-3-(7-cyanopyrazolo[1,5-a]pyridin-4-yl)-5-(trifluoromethyl)-3-azabicyclo[3.1.0]hexane-1-carbonyl)hydrazine-1-carboxylate
(1*S*,5*R*)-3-(7-Cyanopyrazolo[1,5-*a*]pyridin-4-yl)-5-(trifluoromethyl)-3-azabicyclo[3.1.0]hexane-1-carbohydrazide 89-3 (300 mg, 0.85 mmol), 4-oxo-1-methylpiperidine 89-4 (98 mg, 0.85 mmol), and triethylamine (156 mg, 1.53 mmol) were dissolved in 10.0 mL of dichloromethane. A mixed solution of triphosgene (75 mg, 0.255 mmol) in 10.0 mL of dichloromethane was slowly added dropwise at 0°C. After reacting for 1 h, the reaction mixture was sampled, and LCMS confirmed the completion of the reaction. The mixture was concentrated and purified by reverse-phase C18 column chromatography (acidic method) to obtain the target product 1-methylpiperidin-4-yl 2-((1*S*,5*R*)-3-(7-cyanopyrazolo[1,5-*a*]pyridin-4-yl)-5-(trifluoromethyl)-3-azabicyclo[3.1.0]hexane-1-carbonyl)hydrazine-1-carboxylate 89-5 (yellow oil, 100 mg).

LC-MS m/z (ESI)= 492.1 [M+1].

### Step 4: 4-((1S,5R)-1-(5-((1-methylpiperidin-4-yl)oxy)-1,3,4-oxadiazol-2-yl)-5-(trifluoromethyl)-3-azabicyclo[3.1.0]hexan-3-yl)pyrazolo[1,5-a]pyridine-7-carbonitrile compound 89

4-((1S,5R)-1-(5-((1-methylpiperidin-4-yl)oxy)-1,3,4-oxadiazol-2-yl)-5-(trifluoromethyl)-3-azabicyclo[3.1.0]hexan-3-yl)pyrazolo[1,5-a]pyridine-7-carbonitrile
1-Methylpiperidin-4-yl 2-((1*S*,5*R*)-3-(7-cyanopyrazolo[1,5-*a*]pyridin-4-yl)-5-(trifluoromethyl)-3-azabicyclo[3.1.0]hexane-1-carbonyl)hydrazine-1-carboxylate 89-5 (100 mg, 0.23 mmol) was dissolved in 20 mL of acetonitrile, followed by the addition of cesium carbonate (150 mg, 0.46 mmol) and *p*-toluenesulfonyl chloride (88 mg, 0.46 mmol). The mixture was stirred for 3 hours. After LCMS confirmed the completion of the reaction, the reaction mixture was directly concentrated and purified by TLC (acidic method) to obtain the target product *tert*-butyl 3-(5-((1*S*,5*R*)-3-(8-cyanoquinolin-5-yl)-5-(trifluoromethyl)-3-azabicyclo[3.1.0]hex-1-yl)-1,3,4-oxadiazol-2-yl)-3-fluoroazetidine-1-carboxylate compound 89 (pale yellow solid, 15 mg, 15.6%).

LC-MS m/z (ESI)= 474.2 [M+1].

¹H NMR (400 MHz, Chloroform-*d*) δ 8.01 (d, 1H), 7.23 (d, 1H), 6.83 (d, 1H), 6.06 (d, 1H), 5.01 (s, 1H), 4.39 - 4.19 (m, 3H), 4.10 (d, 1H), 2.77 (s, 2H), 2.40 (m, 6H), 2.24 (s, 2H), 2.05 (s, 3H)

### Example 90

4-((1*S*,5*R*)-1-(5-(4-fluoro-1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 90
4-((1S,5R)-1-(5-(4-fluoro-1-methylpiperidin-4-yl)-1,3,4-oxadiazol-2-yl)-5-(trifluoromethyl)-3-azabicyclo[3.1.0]hexan-3-yl)pyrazolo[1,5-a]pyridine-7-carbonitrile

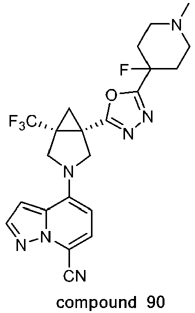

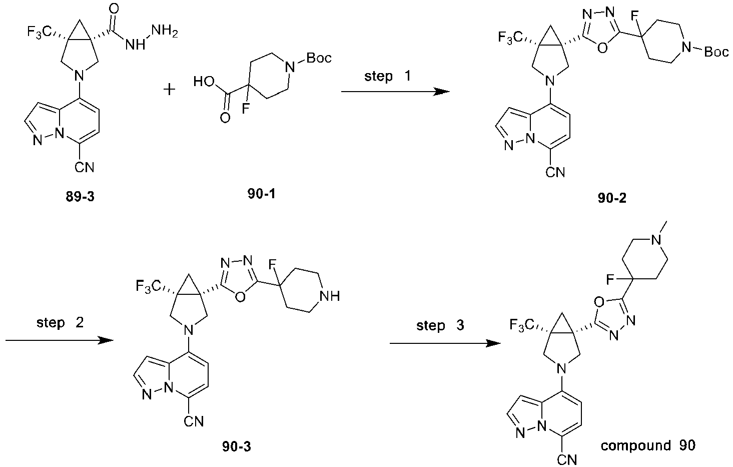

### Step 1:

*tert*-butyl 4-(5-((1*S*,5*R*)-3-(7-cyanopyrazolo[1,5-*a*]pyridin-4-yl)-5-(trifluoromethyl)-3-azabicyclo[3.1.0]hexan-1-yl)-1,3,4-oxadiazol-2-yl)-4-fluoropiperidine-1-carboxylate compound 90-2
tert-butyl 4-(5-((1S,5R)-3-(7-cyanopyrazolo[1,5-a]pyridin-4-yl)-5-(trifluoromethyl)-3-azabicyclo[3.1.0]hexan-1-yl)-1,3,4-oxadiazol-2-yl)-4-fluoropiperidine-1-carboxylate
1-(*tert*-Butoxycarbonyl)-4-fluoropiperidine-4-carboxylic acid 90-1 (197 mg, 0.79 mmol) was dissolved in 20 mL of dichloromethane, followed by the addition of HATU (403 mg, 1.03 mmol) and DIPEA (203 mg, 1.58 mmol). The mixture was stirred at low temperature for 5 minutes, and then (1*S*,5*R*)-3-(7-cyanopyrazolo[1,5-*a*]pyridin-4-yl)-5-(trifluoromethyl)-3-azabicyclo[3.1.0]hexane-1-carbohydrazide 89-3 (280 mg, 0.79 mmol) was added. The reaction was stirred for 2 hours, and after TLC confirmed the completion of the reaction, Burgess reagent (1100 mg, 3.9 mmol) was added and stirred for 3 hours. After LCMS confirmed the completion of the reaction, the mixture was directly concentrated and purified by reverse-phase C18 column chromatography (acidic method) to obtain the target product *tert*-butyl 4-(5-((1*S*,5*R*)-3-(7-cyanopyrazolo[1,5-*a*]pyridin-4-yl)-5-(trifluoromethyl)-3-azabicyclo[3.1.0]hexan-1-yl)-1,3,4-oxadiazol-2-yl)-4-fluoropiperidine-1-carboxylate 90-2 (pale yellow solid, 330 mg, yield: 73.8%).

LC-MS m/z (ESI)= 562.2 [M+1].

### Step 2:

4-((1*S*,5*R*)-1-(5-(4-fluoropiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 90-3
4-((1S,5R)-1-(5-(4-fluoropiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile

The crude product 4-((1*S*,5*R*)-1-(5-(4-fluoropiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile 90-2 (330 mg) was added to 0.60 mL of trifluoroacetic acid and 3.00 mL of dichloromethane. After stirring for half an hour, the sample was submitted for LCMS analysis, which confirmed the completion of the reaction. The reaction mixture was concentrated to obtain the product 90-3 (pale yellow solid, 210 mg, 77%).

LC-MS m/z (ESI)= 462.10 [M+1].

### Step 3:

4-((1*S*,5*R*)-1-(5-(4-fluoro-1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 90
4-((1S,5R)-1-(5-(4-fluoro-1-methylpiperidin-4-yl)-1,3,4-oxadiazol-2-yl)-5-(trifluoromethyl)-3-azabicyclo[3.1.0]hexan-3-yl)pyrazolo[1,5-a]pyridine-7-carbonitrile
5-((1*S*,5*R*)-1-(5-(3-Fluoroazetidin-3-yl)-1,3,4-oxadiazol-2-yl)-5-(trifluoromethyl)-3-azabicyclo[3.1.0]hexan-3-yl)quinoline-8-carbonitrile 90-3 (130 mg, 0.28 mmol) was dissolved in 5 mL of methanol, followed by the addition of 1 mL of triethylamine, paraformaldehyde (250 mg, 9 mmol), and sodium cyanoborohydride (506 mg, 13.5 mmol). The mixture was heated to 80°C and stirred for 3 hours. After TLC confirmed the completion of the reaction, the reaction mixture was directly concentrated. The residue was purified by reverse-phase C18 column chromatography (alkaline method) to obtain the target product 4-((1*S*,5*R*)-1-(5-(4-fluoro-1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 90 (pale yellow solid, 110 mg, 26.7%).

LC-MS m/z (ESI)= 476.10 [M+1].

¹H NMR (400 MHz, Chloroform-d) δ 8.03 (d, 1H), 7.24 (s, 1H), 6.85 (d, 1H), 6.12 (d, 1H), 4.49 - 4.35 (m, 2H), 4.28 (d, 1H), 4.12 (d, 1H), 3.46 (d, 4H), 2.67 (s, 4H), 2.48 (d, 1H), 1.86 (d, 4H).

### Example 91

5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)-[1,2,4]triazolo[1,5-*a*]pyridine-8-carbonitrile compound 91
5-((1S,5R)-1-(5-(1-methylpiperidin-4-yl)-1,3,4-oxadiazol-2-yl)-5-(trifluoromethyl)-3-azabicyclo[3.1.0]hexan-3-yl)-[1,2,4]triazolo[1,5-a]pyridine-8-carbonitrile

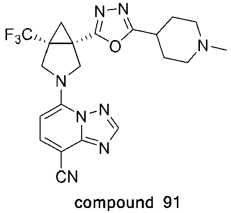

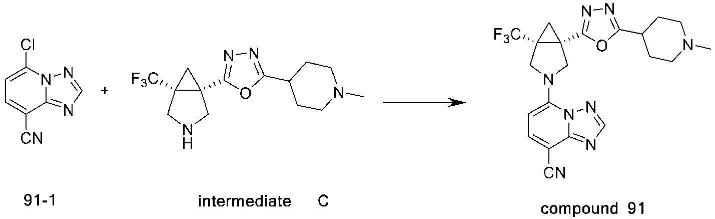

### Step 1:

5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)-[1,2,4]triazolo[1,5-*a*]pyridine-8-carbonitrile compound 91
5-((1S,5R)-1-(5-(1-methylpiperidin-4-yl)-1,3,4-oxadiazol-2-yl)-5-(trifluoromethyl)-3-azabicyclo[3.1.0]hexan-3-yl)-[1,2,4]triazolo[1,5-a]pyridine-8-carbonitrile

Under an N₂ atmosphere, 2-(1-methylpiperidin-4-yl)-5-((1*S*,5*R*)-5-(trifluoromethyl)-3-azabicyclo[3.1.0]hexan-1-yl)-1,3,4-oxadiazole intermediate C (176 mg, 0.55 mmol) was dissolved in 1,4-dioxane (3.50 mL). Subsequently, 5-chloro-[1,2,4]triazolo[1,5-*a*]pyridine-8-carbonitrile 91-1 (100 mg, 0.55 mmol), cesium carbonate (717 mg, 2.20 mmol), RuPhosPdG3 (46 mg, 0.055 mmol), and water (0.60 mL) were added sequentially. The mixture was purged with nitrogen three times, heated to 80°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated. The residue was purified by TLC (DCM: MeOH = 10:1) to obtain the target product 5-((1*S*,5*R*)-1-(5-(1-methylpiperidin-4-yl)-1,3,4-oxadiazol-2-yl)-5-(trifluoromethyl)-3-azabicyclo[3.1.0]hexan-3-yl)-[1,2,4]triazolo[1,5-*a*]pyridine-8-carbonitrile compound 91 (yellow-white solid, 25 mg, 9.7%).

LC-MS m/z (ESI)= 459.10 [M+1].

¹H NMR (400 MHz, DMSO-*d*₆) δ 8.58 (s, 1H), 8.13 (d, 1H), 6.51 (d, 1H), 4.89 (t, 2H), 4.44 (d, 1H), 4.29 (d, 1H), 2.94 (tt, 1H), 2.72 (d, 2H), 2.32 (d, 1H), 2.17 (s, 3H), 2.00 (ddt, 5H), 1.79 - 1.66 (m, 2H).

### Example 92

2-((1-methylpiperidin-4-yl)oxy)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 92
2-((1-methylpiperidin-4-yl)oxy)-5-((1S,5R)-5-(trifluoromethyl)-3-(8-(trifluoromethyl)quinolin-5-yl)-3-azabicyclo[3.1.0]hexan-1-yl)-1,3,4-oxadiazole

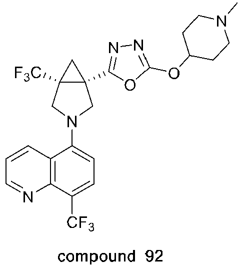

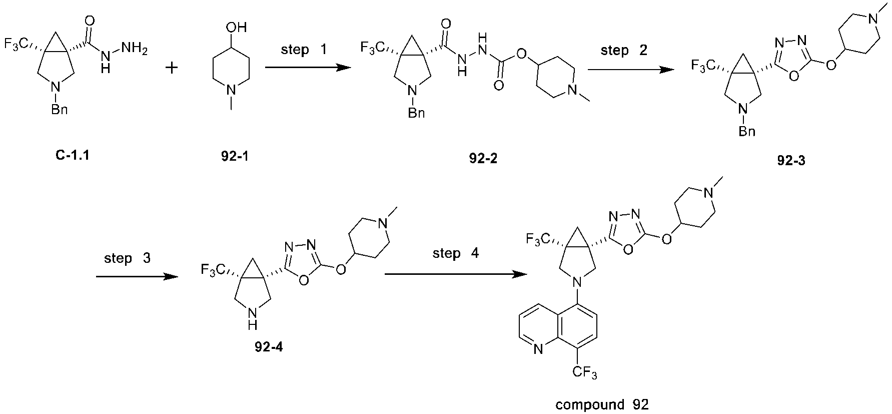

### Step 1: 1-methylpiperidin-4-yl 2-((1S,5R)-3-(7-cyanopyrazolo[1,5-a]pyridin-4-yl)-5-(trifluoromethyl)-3-azabicyclo[3.1.0]hexane-1-carbonyl)hydrazine-1-carboxylate compound 92-2

1-methylpiperidin-4-yl 2-((1S,5R)-3-benzyl-5-(trifluoromethyl)-3-azabicyclo[3.1.0]hexane-1-carbonyl)hydrazine-1-carboxylate
(1*S*,5*R*)-3-Benzyl-5-(trifluoromethyl)-3-azabicyclo[3.1.0]hexane-1-carbohydrazide C-1.1 (4.00 8g, 8.5 mmol), 4-oxo-1-methylpiperidine 92-1 (0.98 g, 8.5 mmol), and triethylamine (5.2 g, 51 mmol) were dissolved in 10.0 mL of dichloromethane. A solution of triphosgene (1.00 g, 3.4 mmol) in 10.0 mL of dichloromethane was slowly added dropwise at 0°C. After reacting for 1 hour, the reaction mixture was sampled, and LCMS confirmed the completion of the reaction. The mixture was concentrated and purified by column chromatography (DCM: EA = 10:1) to obtain the target product 11-methylpiperidin-4-yl 2-((1*S*,5*R*)-3-(7-cyanopyrazolo[1,5-*a*]pyridin-4-yl)-5-(trifluoromethyl)-3-azabicyclo[3.1.0]hexane-1-carbonyl)hydrazine-1-carboxylatecompound 92-2 (yellow oil, 1.20 g).

LC-MS m/z (ESI)= 441.1 [M+1].

### Step 2: 2-((1S,5R)-3-benzyl-5-(trifluoromethyl)-3-azabicyclo[3.1.0]hexan-1-yl)-5-((1-methylpiperidin-4-yl)oxy)-1,3,4-oxadiazole compound 92-3

(1S,5R)-3-(7-cyanopyrazolo[1,5-a]pyridin-4-yl)-5-(trifluoromethyl)-3-azabicyclo[3.1.0]hexane-1-carbohydrazide
Under an N₂ atmosphere, 11-methylpiperidin-4-yl 2-((1*S*,5*R*)-3-(7-cyanopyrazolo[1,5-*a*]pyridin-4-yl)-5-(trifluoromethyl)-3-azabicyclo[3.1.0]hexane-1-carbonyl)hydrazine-1-carboxylate 92-2 (1.20 g, 2.72 mmol) was dissolved in acetonitrile (20.0 mL), followed by the sequential addition of cesium carbonate (1.77 g, 5.44 mmol) and *p*-toluenesulfonyl chloride (1.04 g, 5.44 mmol). The mixture was stirred for 3 hours, then heated to 80°C, and reacted for 3 h. The mixture was rotary evaporated to remove the solvent until dryness, and purified by reverse-phase C18 column chromatography (acidic method) to obtain the target product 2-((1*S*,5*R*)-3-benzyl-5-(trifluoromethyl)-3-azabicyclo[3.1.0]hexan-1-yl)-5-((1-methylpiperidin-4-yl)oxy)-1,3,4-oxadiazole compound 92-3 (yellowish-white solid, 1.0 g, yield: 86.9%).

LC-MS m/z (ESI)= 423.2 [M+1].

### Step 3: 2-2-((1-methylpiperidin-4-yl)oxy)-5-((1S,5R)-5-(trifluoromethyl)-3-azabicyclo[3.1.0]hexan-1-yl)-1,3,4-oxadiazole compound 92-4

2-((1-methylpiperidin-4-yl)oxy)-5-((1S,5R)-5-(trifluoromethyl)-3-azabicyclo[3.1.0]hexan-1-yl)-1,3,4-oxadiazole
Under an N₂ atmosphere, 12-((1*S*,5*R*)-3-benzyl-5-(trifluoromethyl)-3-azabicyclo[3.1.0]hexan-1-yl)-5-((1-methylpiperidin-4-yl)oxy)-1,3,4-oxadiazole 92-3 (1.0 g, 2.36 mmol) was dissolved in methanol (20.0 mL), followed by the sequential addition of Pd/C (1.77 g, 5.44 mmol) and p-toluenesulfonyl chloride (1.04 g, 5.44 mmol). The mixture was stirred for 3 hours, then heated to 80°C, and reacted for 3 h. The mixture was rotary evaporated to remove the solvent until dryness, and the residue was purified by reverse-phase C18 column chromatography (acid method) to obtain the target product 2-2-((1-methylpiperidin-4-yl)oxy)-5-((1*S*,5*R*)-5-(trifluoromethyl)-3-azabicyclo[3.1.0]hexan-1-yl)-1,3,4-oxadiazole compound 92-4 (yellow-white solid, 500 mg, yield: 86.9%).

LC-MS m/z (ESI)= 333.2 [M+1].

### Step 4:

2-((1-methylpiperidin-4-yl)oxy)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 92
2-((1-methylpiperidin-4-yl)oxy)-5-((1S,5R)-5-(trifluoromethyl)-3-(8-(trifluoromethyl)quinolin-5-yl)-3-azabicyclo[3.1.0]hexan-1-yl)-1,3,4-oxadiazole

Under an N₂ atmosphere, 2-((1-methylpiperidin-4-yl)oxy)-5-((1*S*,5*R*)-5-(trifluoromethyl)-3-azabicyclo[3.1.0]hexan-1-yl)-1,3,4-oxadiazole 92-4 (100 mg, 0.24 mmol) was dissolved in 1,4-dioxane (3.50 mL). Subsequently, 5-bromo-8-trifluoromethylquinoline (65.7 mg, 0.24 mmol), cesium carbonate (313 mg, 0.96 mmol), and RuPhosPdG3 (20 mg, 0.024 mmol) were added sequentially. The mixture was purged with nitrogen three times, heated to 80°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated and purified by TLC (DCM: MeOH = 10:1) to obtain the target product 2-((1-methylpiperidin-4-yl)oxy)-5-((1S,5R)-5-(trifluoromethyl)-3-(8-(trifluoromethyl)quinolin-5-yl)-3-azabicyclo[3.1.0]hexan-1-yl)-1,3,4-oxadiazole compound 92 (yellowish-white solid, 40 mg).

LC-MS m/z (ESI)= 528.10 [M+1]

¹H NMR (400 MHz, Chloroform-d) δ 9.07 (dd, 1H), 8.35 (dd, 1H), 8.00 (d, 1H), 7.50 (dd, 1H), 7.17 (d, 1H), 4.98 (s, 1H), 3.97 (d, 1H), 3.86 (dd, 2H), 3.71 (d, 1H), 2.74 (s, 2H), 2.36 - 2.43 (m, 5H) 2.25 (d, 4H), 2.03 (s, 2H)

### Example 93

2-((1-methylpiperidin-4-yl)oxy)-5-((1*S*,5*R*)-5-(trifluoromethyl)-3-(8-(trifluoromethoxy)quinolin-5-yl)-3-azabicyclo[3.1.0]hexan-1-yl)-1,3,4-oxadiazole compound 93
2-((1-methylpiperidin-4-yl)oxy)-5-((1S,5R)-5-(trifluoromethyl)-3-(8 (trifluoromethyl)quinolin-5-yl)-3-azabicyclo[3.1.0]hexan-1-yl)-1,3,4-oxadiazole

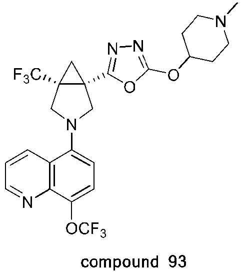

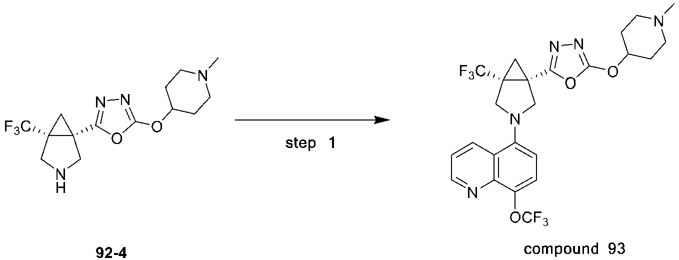

Under an N₂ atmosphere, 2-((1-methylpiperidin-4-yl)oxy)-5-((1*S*,5*R*)-5-(trifluoromethyl)-3-azabicyclo[3.1.0]hexan-1-yl)-1,3,4-oxadiazole 92-4 (100 mg, 0.24 mmol) was dissolved in 1,4-dioxane (3.50 mL). Subsequently, 5-bromo-8-trifluoromethoxyquinoline (65.7 mg, 0.24 mmol), cesium carbonate (313 mg, 0.96 mmol), and RuPhosPdG3 (20 mg, 0.024 mmol) were added sequentially. The mixture was purged with nitrogen three times, heated to 80°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated and purified by TLC (DCM: MeOH = 10:1) to obtain the target product 2-((1-methylpiperidin-4-yl)oxy)-5-((1*S*,5*R*)-5-(trifluoromethyl)-3-(8-(trifluoromethoxy)quinolin-5-yl)-3-azabicyclo[3.1.0]hexan-1-yl)-1,3,4-oxadiazole compound 93 (yellowish-white solid, 30 mg).

LC-MS m/z (ESI)= 544.10 [M+1]

¹H NMR (400 MHz, Chloroform-d) δ 9.04 (dd, 1H), 8.36 (dd, 1H), 7.57 (dd, 1H), 7.51 (dd, 1H), 7.20 (d, 1H), 4.99 (s, 1H), 3.92 (d, 1H), 3.80 - 3.72 (m, 2H), 3.65 (d, 1H), 2.76 (s, 2H), 2.39 - 2.45 (m, 5H), 2.32 - 2.21 (m, 4H), 2.05 (s, 2H)

### Example 94

4-((1*S*,5*R*)-1-(5-(4-fluoro-1-methylpiperidin-4-yl)-1,3,4-oxadiazol-2-yl)-5-(trifluoromethyl)-3-azabicyclo[3.1.0]hexan-3-yl)pyrazolo[1,5-*a*]pyridine-7-carbonitrile compound 94
4-((1S,5R)-1-(5-(4-fluoro-1-methylpiperidin-4-yl)-1,3,4-oxadiazol-2-yl)-5-(trifluoromethyl)-3-azabicyclo[3.1.0]hexan-3-yl)pyrazolo[1,5-a]pyridine-7-carbonitrile

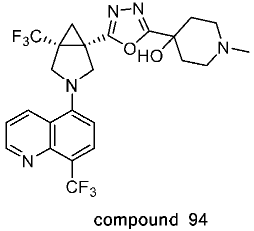

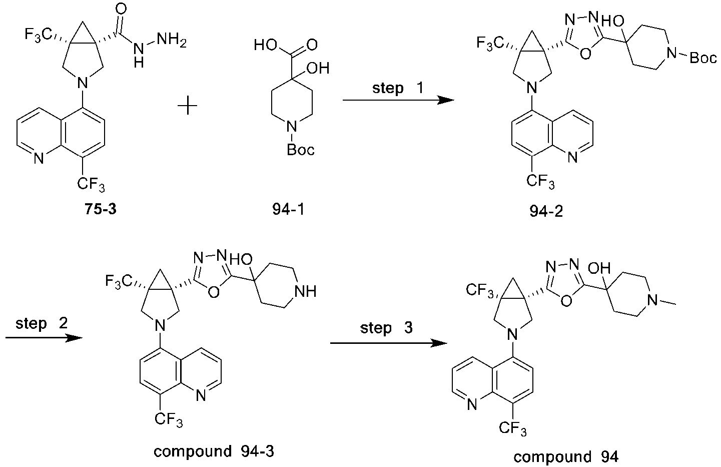

### Step 1:

*tert*-butyl 4-hydroxy-4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidine-1-carboxylate compound 94-2
tert-butyl 4-hydroxy-4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)piperidine-1-carboxylate

1-(*tert*-Butoxycarbonyl)-4-hydroxypiperidine-4-carboxylic acid 94-1 (218 mg, 0.79 mmol) was dissolved in 20 mL of dichloromethane, followed by the addition of HATU (428.7 mg, 1.125 mmol) and DIPEA (390 mg, 3.00 mmol). The mixture was stirred at 40°C for 5 minutes, and then (1*S*,5*R*)-5-(trifluoromethyl)-3-(8-(trifluoromethyl)quinolin-5-yl)-3-azabicyclo[3.1.0]hexane-1-carbohydrazide 75-3 (300 mg, 0.75 mmol) was added. After stirring for 2 hours, TLC confirmed the completion of the reaction. Burgess reagent (636 mg, 2.25 mmol) was then added, and the mixture was stirred for an additional 3 hours. After LCMS confirmed the completion of the reaction, the mixture was directly concentrated. The residue was purified by reverse-phase C18 column chromatography (acidic method) to obtain the target product *tert*-butyl 4-4-hydroxy-4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidine-1-carboxylate 94-2 (pale yellow solid, 170 mg, 37.3%).

LC-MS m/z (ESI)= 614.2 [M+1].

### Step 2:

4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidin-4-ol compound 94-3
4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)piperidin-4-ol

The crude product *tert*-butyl 4-hydroxy-4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidine-1-carboxylate 94-2 (170 mg) was added to 0.60 mL of trifluoroacetic acid and 3.00 mL of dichloromethane. After stirring for half an hour, LCMS analysis confirmed the completion of the reaction. The reaction mixture was concentrated to obtain the product 4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidin-4-ol 94-3 (pale yellow solid, 130 mg, 91.5%).

LC-MS m/z (ESI)= 514.10 [M+1].

### Step 3:

1-methyl-4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidin-4-ol compound 94
1-methyl-4-(5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)piperidin-4-ol

4-(5-((1*S*,5*R*)-5-(Trifluoromethyl)-3-(8-(trifluoromethyl)quinolin-5-yl)-3-azabicyclo[3.1.0]hexan-1-yl)-1,3,4-oxadiazol-2-yl)piperidin-4-ol 94-3 (130 mg, 0.25 mmol) was dissolved in 5 mL of methanol, followed by the addition of 1 mL of triethylamine, paraformaldehyde (250 mg, 9 mmol), and sodium cyanoborohydride (506 mg, 13.5 mmol). The mixture was heated to 80°C and stirred for 3 hours. After TLC confirmed the completion of the reaction, the reaction mixture was directly concentrated. After TLC confirmed the completion of the reaction, the reaction mixture was directly filtered and concentrated. The residue was purified by TLC (DCM: MeOH = 10:1) to obtain the target product 1-methyl-4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidin-4-ol compound 94 (pale yellow solid, 30 mg, 26.7%).

LC-MS m/z (ESI)= 528.10 [M+1].

¹H NMR (400 MHz, Chloroform-d) δ 9.07 (td 1H), 8.36 (ddd, 1H), 8.00 (dd, 1H), 7.55 - 7.45 (m, 1H), 7.19 (s, 1H), 4.06 (t, 1H), 3.94 - 3.84 (m, 2H), 3.75 - 3.68 (m, 1H), 3.41 (s, 1H), 3.26 (s, 1H), 2.83 (s, 3H), 2.48 (s, 4H), 2.32 (s, 2H), 2.22 (d, 1H), 2.13 (d, 1H). 2.04 (s, 1H)

### Example 95

2-((1-methylazetidin-3-yl)methyl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 95
2-((1-methylazetidin-3-yl)methyl)-5-((1S,5R)-5-(trifluoromethyl)-3-(8-(trifluoromethyl)quinolin-5-yl)-3-azabicyclo[3.1.0]hexan-1-yl)-1,3,4-oxadiazole

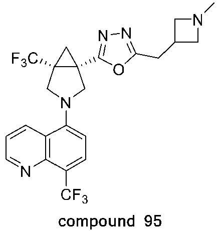

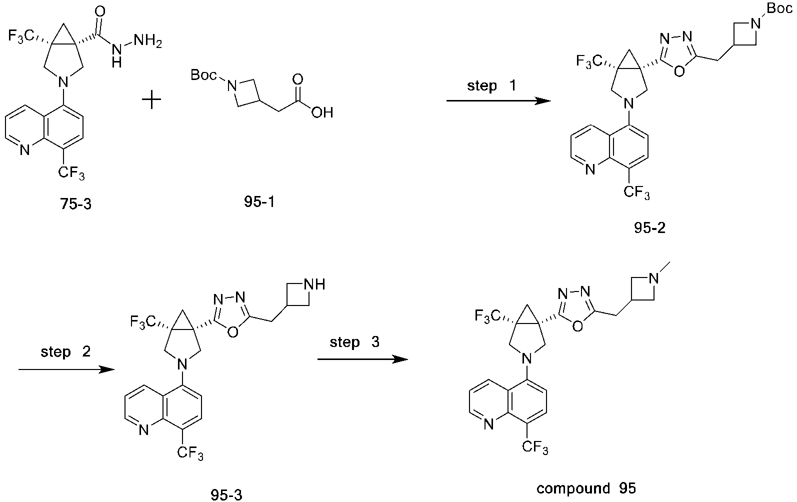

### Step 1:

tert-butyl 3-((5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)methyl)azetidine-1-carboxylate compound 95-2
tert-butyl 3-((5-((1S,5R)-5-(trifluoromethyl)-3-(8-(trifluoromethyl)quinolin-5-yl)-3-azabicyclo[3.1.0]hexan-1-yl)-1,3,4-oxadiazol-2-yl)methyl)azetidine-1-carboxylate
2-(1-(*tert*-Butoxycarbonyl)azetidin-3-yl)acetic acid 95-1 (191 mg, 0.88 mmol) was dissolved in 20 mL of dichloromethane, followed by the addition of HATU (428.7 mg, 1.125 mmol) and DIPEA (390 mg, 3.00 mmol). The mixture was stirred at 40°C for 5 minutes, and then (1*S*,5*R*)-5-(trifluoromethyl)-3-(8-(trifluoromethyl)quinolin-5-yl)-3-azabicyclo[3.1.0]hexane-1-carbohydrazide 75-3 (300 mg, 0.75 mmol) was added. After stirring for 2 hours, TLC confirmed the completion of the reaction. Burgess reagent (636 mg, 2.25 mmol) was then added, and the mixture was stirred for an additional 3 hours. After LCMS confirmed the completion of the reaction, the mixture was directly concentrated. The residue was purified by reverse-phase C18 column chromatography (acidic method) to obtain the target product *tert*-butyl 3-((5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)methyl)azetidine-1-carboxylate 95-2 (pale yellow solid, 200 mg, 46.2%).

LC-MS m/z (ESI)= 584.2 [M+1].

### Step 2:

2-(azetidin-3-ylmethyl)-5-((1S,5R)-5-(trifluoromethyl)-3-(8-(trifluoromethyl)quinolin-5-yl)-3-azabicyclo[3.1.0]hexan-1-yl)-1,3,4-oxadiazole compound 95-3
2-(azetidin-3-ylmethyl)-5-((1S,5R)-5-(trifluoromethyl)-3-(8-(trifluoromethyl)quinolin-5-yl)-3-azabicyclo[3.1.0]hexan-1-yl)-1,3,4-oxadiazole

The crude product *tert*-butyl 4-hydroxy-4-(5-((1*S*,5*R*)-5-(trifluoromethyl)-3-(8-(trifluoromethyl)quinolin-5-yl)-3-azabicyclo[3.1.0]hexan-1-yl)-1,3,4-oxadiazol-2-yl)piperidine-1-carboxylate 95-2 (200 mg) was added to 0.40 mL of trifluoroacetic acid and 2.00 mL of dichloromethane. After stirring for half an hour, LCMS analysis confirmed the completion of the reaction. The reaction mixture was concentrated to obtain the product 2-(azetidin-3-ylmethyl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole 95-3 (pale yellow solid, 160 mg, 96.9%).

LC-MS m/z (ESI)= 484.10 [M+1].

### Step 3:

2-((1-methylazetidin-3-yl)methyl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 95
2-((1-methylazetidin-3-yl)methyl)-5-((1S,5R)-5-(trifluoromethyl)-3-(8-(trifluoromethyl)quinolin-5-yl)-3-azabicyclo[3.1.0]hexan-1-yl)-1,3,4-oxadiazole
2-(Azetidin-3-ylmethyl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole 95-3 (160 mg, 0.33 mmol) was dissolved in 5 mL of methanol, followed by the addition of 1 mL of triethylamine, paraformaldehyde (250 mg, 9 mmol), and sodium cyanoborohydride (506 mg, 13.5 mmol). The mixture was heated to 60°C and stirred for 3 hours. After TLC confirmed the completion of the reaction, the reaction mixture was directly filtered, concentrated, and purified by TLC (DCM: MeOH = 10:1) to obtain 2-((1-methylazetidin-3-yl)methyl)-5-((1*S*,5*R*)-5-(trifluoromethyl)-3-(8-(trifluoromethyl)quinolin-5-yl)-3-azabicyclo[3.1.0]hexan-1-yl)-1,3,4-oxadiazole compound 95 (pale yellow solid, 50 mg, 30.7%).

LC-MS m/z (ESI)= 498.10 [M+1].

¹H NMR (400 MHz, Chloroform-d) δ 9.07 (dd, 1H), 8.36 (dd, 1H), 8.01 (d, 1H), 7.51 (dd, 1H), 7.20 (d, 1H), 4.04 (d, 1H), 3.96 - 3.83 (m, 4H), 3.72 (d, J = 9.6 Hz, 2H), 3.31 (s, 2H), 3.21 (s, 1H), 2.64 (s, 3H), 2.31 (q, 3H).

### Example 96

2-(4-fluoropiperidin-4-yl)-5-((1*S*,5*R*)-3-(8-(trifluoromethoxy)quinolin-5-yl)-5-(trifluoromethyl)-3-azabicyclo[3.1.0]hexan-1-yl)-1,3,4-oxadiazole compound 96
2-(4-fluoropiperidin-4-yl)-5-((1S,5R)-3-(8-(trifluoromethoxy)quinolin-5-yl)-5-(trifluoromethyl)-3-azabicyclo[3.1.0]hexan-1-yl)-1,3,4-oxadiazole

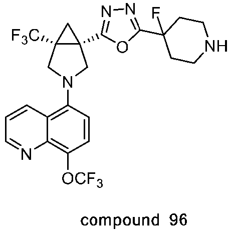

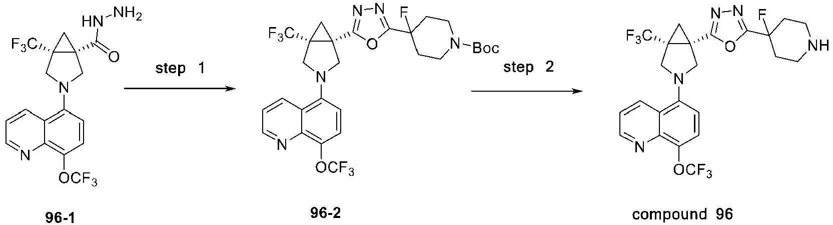

### Step 1:

*tert*-butyl 4-fluoro-4-(5-((1*S*,5*R*)-3-(8-(trifluoromethoxy)quinolin-5-yl)-5-(trifluoromethyl)-3-azabicyclo[3.1.0]hexan-1-yl)-1,3,4-oxadiazol-2-yl)piperidine-1-carboxylate compound 96-2
tert-butyl 4-fluoro-4-(5-((1S,5R)-3-(8-(trifluoromethoxy)quinolin-5-yl)-5-(trifluoromethyl)-3-azabicyclo[3.1.0]hexan-1-yl)-1,3,4-oxadiazol-2-yl)piperidine-1-carboxylate
1-(*tert*-Butoxycarbonyl)-4-fluoropiperidine-4-carboxylic acid (230 mg, 0.93 mmol) was dissolved in anhydrous dichloromethane (7.5 mL). DIPEA (230 mg, 1.77 mmol) and HATU (506 mg, 1.33 mmol) were added, and the mixture was stirred at 35°C for 30 minutes and then cooled to room temperature. (1*S*,5*R*)-3-(8-(Trifluoromethoxy)quinolin-5-yl)-5-(trifluoromethyl)-3-azabicyclo[3.1.0]hexane-1-carbohydrazide 96-1 (373 mg, 0.88 mmol) was added. After completion, the reaction mixture was purged with nitrogen three times and stirred at room temperature for 1 hour. After LCMS confirmed the completion of the reaction, DIPEA (344 mg, 2.66 mmol) and Burgess reagent (634 mg, 2.66 mmol) were added. The reaction mixture was purged with nitrogen three times and stirred at room temperature for 1 hour. After LCMS confirmed the completion of the reaction, the mixture was extracted three times with ethyl acetate and water. The organic phase was dried over anhydrous sodium sulfate and directly concentrated to dryness. The crude product was purified by reverse-phase column chromatography to obtain *tert*-butyl 4-fluoro-4-(5-((1*S*,5*R*)-3-(8-(trifluoromethoxy)quinolin-5-yl)-5-(trifluoromethyl)-3-azabicyclo[3.1.0]hexan-1-yl)-1,3,4-oxadiazol-2-yl)piperidine-1-carboxylate compound 96-2 (373 mg, yellow solid, yield: 67%).

LCMS m/z (ESI) = 632.20 [M+1].

### Step 2:

2-(4-fluoropiperidin-4-yl)-5-((1*S*,5*R*)-3-(8-(trifluoromethoxy)quinolin-5-yl)-5-(trifluoromethyl)-3-azabicyclo[3.1.0]hexan-1-yl)-1,3,4-oxadiazole compound 96
2-(4-fluoropiperidin-4-yl)-5-((1S,5R)-3-(8-(trifluoromethoxy)quinolin-5-yl)-5-(trifluoromethyl)-3-azabicyclo[3.1.0]hexan-1-yl)-1,3,4-oxadiazole
*tert*-Butyl 4-fluoro-4-(5-((1*S*,5*R*)-3-(8-(trifluoromethoxy)quinolin-5-yl)-5-(trifluoromethyl)-3-azabicyclo[3.1.0]hexan-1-yl)-1,3,4-oxadiazol-2-yl)piperidine-1-carboxylate 96-2 (373 mg, 0.59 mmol) was dissolved in anhydrous dichloromethane (6 mL), followed by the dropwise addition of trifluoroacetic acid (1.5 mL). The reaction mixture was purged with nitrogen and stirred at room temperature for 2 hours. After the reaction was completed, ammonia water was added to the reaction mixture to adjust the pH to alkaline. The mixture was extracted three times with dichloromethane and water. The organic phase was dried over anhydrous sodium sulfate, concentrated to dryness, and purified by column chromatography to obtain the target product 2-(4-fluoropiperidin-4-yl)-5-((1*S*,5*R*)-3-(8-(trifluoromethoxy)quinolin-5-yl)-5-(trifluoromethyl)-3-azabicyclo[3.1.0]hexan-1-yl)-1,3,4-oxadiazole compound 96 (280 mg, yellow solid, yield: 89%).

LCMS m/z (ESI) = 532.20 [M+1].

¹H NMR (400 MHz, Chloroform-d) *δ* 9.02 (dd, 1H), 8.36 (dd, 1H), 7.56 (dd, 1H), 7.51 (dd, 1H), 7.23 (d, 1H), 6.94 (s, 1H), 4.01 (d, 1H), 3.80 (dd, 2H), 3.67 (d, 1H), 3.49 - 3.24 (m, 4H), 2.54 (dq, 4H), 2.41 (d, 1H), 2.31 (d,1H).

### Example 97

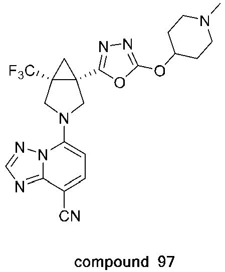

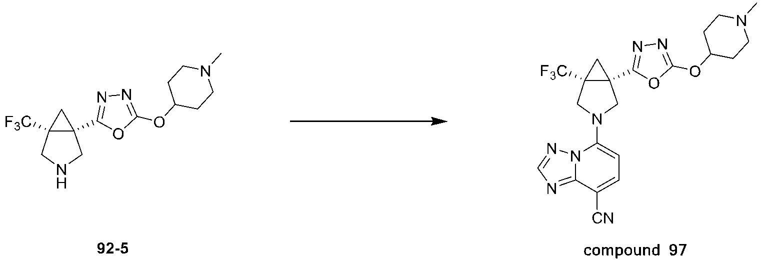

5-((1*S*,5*R*)-1-(5-((1-methylpiperidin-4-yl)oxy)-1,3,4-oxadiazol-2-yl)-5-(trifluoromethyl)-3-azabicyclo[3.1.0]hexan-3-yl)-[1,2,4]triazolo[1,5-*a*]pyridine-8-carbonitrile compound 97
5-((1S,5R)-1-(5-((1-methylpiperidin-4-yl)oxy)-1,3,4-oxadiazol-2-yl)-5-(trifluoromethyl)-3-azabicyclo[3.1.0]hexan-3-yl)-[1,2,4]triazolo[1,5-a]pyridine-8-carbonitrile

Under an N₂ atmosphere, 2-((1-methylpiperidin-4-yl)oxy)-5-((1*S*,5*R*)-5-(trifluoromethyl)-3-azabicyclo[3.1.0]hexan-1-yl)-1,3,4-oxadiazole 92-5 (200 mg, 0.56 mmol) was dissolved in 1,4-dioxane (3.50 mL). Subsequently, 5-chloro-[1,2,4]triazolo[1,5-*a*]pyridine-8-carbonitrile (100 mg, 0.56 mmol), cesium carbonate (730 mg, 2.24 mmol), and RuPhosPdG3 (32 mg, 0.056 mmol) were added sequentially. The mixture was purged with nitrogen three times, heated to 80°C, and reacted for 2 h. The mixture was rotary evaporated to remove the solvent until dryness, and the residue was added with ethyl acetate for extraction. The organic phase was washed with saturated brine, dried over anhydrous sodium sulfate, filtered, and rotary evaporated to remove the solvent until dryness. The reaction mixture was directly concentrated. The residue was purified by TLC (DCM: MeOH = 10:1) to obtain the target product 5-((1*S*,5*R*)-1-(5-((1-methylpiperidin-4-yl)oxy)-1,3,4-oxadiazol-2-yl)-5-(trifluoromethyl)-3-azabicyclo[3.1.0]hexan-3-yl)-[1,2,4]triazolo[1,5-*a*]pyridine-8-carbonitrile compound 97 (yellow-white solid, 10 mg).

LC-MS m/z (ESI)= 474.10 [M+1]

¹H NMR (400 MHz, Chloroform-d) δ 8.26 (s, 1H), 7.75 (d, 1H), 6.08 (d, 1H), 4.95 (s, 1H), 4.85 (d, 1H), 4.76 (d, 1H), 4.30 (d, 1H), 4.05 (d, 1H), 2.73 (s, 2H), 2.44 (s, 2H), 2.33 (d, 4H), 2.22 (s, 2H), 2.01 (s, 2H), 1.74 (d, 1H).

### Biological assays

### HEK-Blue-hTLR7/8/9 cell inhibition assay

1. HEK-Blue-hTLR7/8 cells (1 × 10⁴ cells/well) and HEK-Blue-hTLR9 cells (1.5 × 10⁴ cells/well) were seeded in a 384-well cell culture plate, with a volume of 30 µL per well. The plate was incubated at 37°C in a 5% CO₂ incubator for 4 h.
2. The compound was prepared with DMSO and diluted in DMEM medium to 10 concentrations (1:3 dilution), with final concentrations of 10000, 3333.3, 1111.1, 370.4, 123.5, 41.2, 13.7, 4.6, 1.5, and 0.5 nM; R848 was prepared with DMSO and diluted in DMEM medium, with final concentrations of 0.8 µM (HEK-Blue-hTLR7) and 3 µM (HEK-Blue-hTLR8); ODN2006 was prepared with endotoxin-free water and diluted in DMEM medium, with a final concentration of 1 µM.
3. Cells treated with DMSO were used as the blank control group, while cells treated solely with Resiquimod (R848) were used as the positive control group for HEK-Blue-hTLR7/8, and cells treated solely with ODN2006 were used as the positive control group for HEK-Blue-hTLR9. Cells treated with the test compound together with R848 or ODN2006 were used as the test group. Two parallel wells were set for each group and were placed at 37°C in a 5% CO₂ incubator for incubation.
4. After being incubated for 4 h, the 384-well plate of HEK-Blue-hTLR7/8 was taken out from the incubator, and R848 alone or both R848 and the diluted compound were separately added into each well. The plate was placed at 37°C in a 5% CO₂ incubator for incubation for 16 h; the 384-well plate of HEK-Blue-hTLR9 was taken out from the incubator, and ODN2006 alone or both ODN2006 and the diluted compound were added separately to each well. The plate was incubated at 37°C in a 5% CO₂ incubator for 16 h.
5. After being incubated for 16 h, the 384-well plate was removed from the incubator and centrifuged at 1000 rpm for 1 minute. The optical density value of each well at 620 nm was measured using a multifunctional microplate reader.
6. The cell inhibition rate was calculated as (1-(OD620 test group-OD620 blank group)/(OD620 positive group-OD620 blank group))×100%, and the half maximal inhibitory concentration (IC₅₀) was calculated through curve fitting.

**Table 1: Activity of the compounds of the present disclosure in HEK-Blue-hTLR7/8/9 cellular assays**

| Compound | TLR7 IC₅₀ (nM) | TLR8 IC₅₀ (nM) | TLR9 IC₅₀ (nM) |
|---|---|---|---|
| Compound 1 | <100 | <100 | >10000 |
| Compound 2 | >1000 | >1000 | >10000 |
| Compound 3 | <100 | <100 | >1000 |
| Compound 4 | <100 | <100 | >1000 |
| Compound 5 | <1000 | >1000 | >1000 |
| Compound 6 | <100 | <100 | >1000 |
| Compound 7 | <100 | <100 | >10000 |
| Compound 8 | <100 | <100 | >1000 |
| Compound 9 | <100 | <100 | >1000 |
| Compound 11 | <100 | <100 | >1000 |
| Compound 12 | <100 | <100 | >10000 |
| Compound 13 | <100 | <100 | >1000 |
| Compound 14 | <100 | <100 | >1000 |
| Compound 15 | <100 | <100 | >1000 |
| Compound 16 | <100 | <100 | / |
| Compound 17 | <100 | <100 | >10000 |
| Compound 18 | <100 | <100 | <1000 |
| Compound 19 | <100 | <100 | >1000 |
| Compound 20 | <100 | <100 | >1000 |
| Compound 21 | <100 | <100 | >1000 |
| Compound 22 | <100 | <100 | >10000 |
| Compound 23 | <100 | <100 | >1000 |
| Compound 24 | <1000 | <100 | >10000 |
| Compound 25 | <100 | <100 | <1000 |
| Compound 26 | <100 | <100 | >1000 |
| Compound 27 | <100 | <1000 | >10000 |
| Compound 28 | <100 | <100 | >1000 |
| Compound 30 | <100 | <100 | >1000 |
| Compound 31 | <100 | <100 | >1000 |
| Compound 32 | <100 | <100 | >10000 |
| Compound 33 | <100 | <100 | >1000 |
| Compound 34 | <100 | <1000 | >10000 |
| Compound 35 | <1000 | <1000 | >1000 |
| Compound 36 | <100 | <100 | >1000 |
| Compound 37 | <100 | <100 | >1000 |
| Compound 38 | <1000 | <1000 | >10000 |
| Compound 39 | <100 | <100 | >1000 |
| Compound 40 | <1000 | <1000 | >10000 |
| Compound 41 | <100 | <1000 | >10000 |
| Compound 42 | <100 | <1000 | >10000 |
| Compound 43 | <1000 | <100 | >10000 |
| Compound 44 | <1000 | >1000 | >10000 |
| Compound 45 | <100 | <100 | >10000 |
| Compound 46 | <100 | <100 | >10000 |
| Compound 47 | <100 | <100 | >10000 |
| Compound 48 | <100 | <100 | / |
| Compound 49 | <100 | <100 | >10000 |
| Compound 50 | <100 | <100 | >1000 |
| Compound 51 | <100 | <100 | >10000 |
| Compound 52 | <100 | <100 | <1000 |
| Compound 53 | <100 | <100 | >1000 |
| Compound 54 | <100 | <100 | >1000 |
| Compound 56 | <100 | <100 | >10000 |
| Compound 57 | <100 | <100 | >1000 |
| Compound 58 | >1000 | >1000 | / |
| Compound 59 | <100 | <1000 | >1000 |
| Compound 60 | <100 | <100 | <1000 |
| Compound 61 | >1000 | <1000 | / |
| Compound 62 | <100 | <100 | / |
| Compound 63 | <100 | <100 | / |
| Compound 64 | <100 | <100 | / |
| Compound 65 | <100 | <100 | / |
| Compound 66 | <100 | <100 | >1000 |
| Compound 67 | <100 | <100 | / |
| Compound 68 | <100 | <100 | / |
| Compound 69 | <100 | <100 | / |
| Compound 70 | >1000 | >10000 | / |
| Compound 71 | <100 | <100 | >1000 |
| Compound 72 | >1000 | <1000 | / |
| Compound 73 | >1000 | >1000 | / |
| Compound 74 | <100 | <100 | / |
| Compound 75 | <100 | <100 | >1000 |
| Compound 76 | <100 | <100 | >1000 |
| Compound 77 | <100 | <100 | >10000 |
| Compound 78 | <100 | <100 | / |
| Compound 79 | <100 | <100 | >1000 |
| Compound 80 | <100 | <100 | >1000 |
| Compound 81 | <100 | <100 | >1000 |
| Compound 82 | <100 | <100 | / |
| Compound 83 | <1000 | >1000 | >1000 |
| Compound 84 | <100 | <1000 | >1000 |
| Compound 85 | >1000 | <1000 | >1000 |
| Compound 86 | <1000 | <100 | >1000 |
| Compound 87 | <100 | <1000 | >1000 |
| Compound 88 | <100 | <100 | / |
| Compound 89 | <100 | <100 | / |
| Compound 90 | <100 | <100 | >1000 |
| Compound 91 | <100 | <100 | >10000 |
| Compound 92 | <100 | <100 | >1000 |
| Compound 93 | <1000 | <100 | >10000 |
| Compound 94 | <100 | <100 | >1000 |
| Compound 95 | <100 | <100 | >1000 |
| Compound 96 | <100 | <100 | >1000 |
| Compound 97 | <100 | <100 | / |

| | | | |
|---|---|---|---|
| Conclusion: The compounds of the present disclosure exhibit superior antagonistic activity against HEK-Blue-hTLR7/8 cells with significant antagonistic effects, while showing no obvious antagonistic effects on HEK-Blue-hTLR9 cells. | | | |

*In vivo* inhibition assay of IL-6

Experimental materials: Preparation of compound solutions: R848 was prepared with pure water (sterilized), with a final concentration of 25 µg/100 µL; the test compound was prepared with sodium citrate buffer at pH = 3, with a final concentration of 0.1 mg/mL.

Experimental animals: C57BL/6 female mice were purchased from GemPharmatech, aged 6-8 weeks.

Experimental methods: C57BL/6 female mice were randomly divided into a blank group, control group, and experimental group, with 8 in each group.

Blank group: Blood was collected from the mice after 12 h of fasting.

Control group: The mice were administered sodium citrate buffer at pH = 3 by gavage after 12 hours of fasting, and the remaining procedures were consistent with those of the experimental group.

Experimental group: After fasting for 12 h, the mice were administered with the test compound (1 mg/kg) by gavage, followed by an intraperitoneal injection of 25 µg R848 (purchased from MCE) 1 h later; 2 h later, the eyeballs were removed for blood collection, the serum was allowed to stand at room temperature for 30 min, then centrifuged for 10 min (3000 rpm), the supernatant was collected, and the serum IL-6 concentration was measured using an IL-6 ELISA kit. The IL-6 inhibition rate was calculated based on the serum IL-6 concentrations of the control and experimental groups. Calculation formula: inhibition rate = (C2-C3)/(C2-C1)×100%.
C1: serum IL-6 concentration of the blank group;
C2: serum IL-6 concentration of the control group;
C3: serum IL-6 concentration of the experimental group.

Four additional groups of experiments were performed separately according to the above experimental procedures, and the differences between these four groups and the above experiment were respectively: "25 µg R848 (purchased from MCE) was intraperitoneally injected after 4 h", "25 µg R848 (purchased from MCE) was intraperitoneally injected after 8 h", "25 µg R848 (purchased from MCE) was intraperitoneally injected after 16 h", and "25 µg R848 (purchased from MCE) was intraperitoneally injected after 24 h".

**Table 2: In vivo inhibition assay of IL-6**

| Compound | IL-6 inhibition rate (intraperitoneal injection after 1 h) |
|---|---|
| Compound 30 | >90% |
| Compound 57 | >90% |
| Compound 59 | >80% |
| Compound 64 | >70% |
| Compound 95 | >90% |
| Compound 97 | >90% |

| | |
|---|---|
| Conclusion: The compounds of the present disclosure exhibit significant inhibitory effects on IL-6. | |

### Mouse pharmacokinetics assay

### Experimental protocol:

(1) Healthy male ICR mice (purchased from Beijing Vital River Laboratory Animal Technology Co., Ltd., 18-22g) were prepared. For each compound, 6 mice were divided into 2 groups (iv and po groups), with 3 mice per group. Blood microsampling (50 µL) was performed at each time point.
(2) After overnight fasting (with free access to water), the compounds of the present disclosure were dissolved (or formed into a suspension) using 10% DMSO and 90% of 30% HP-β-CD (v:v) as the iv vehicle and 0.5% MC as the po vehicle. Administration was performed intravenously (iv, 1 mg/kg) and intragastrically (po, 10 mg/kg), respectively.
(3) In the iv group, blood samples were collected via the orbital venous plexus at 5 min, 15 min, 0.5 h, 1 h, 2 h, 4 h, 8 h, 12 h, and 24 h after administration, anticoagulated with EDTA-K₂, centrifuged at 4°C for 5 min to separate plasma, and stored at -20°C until analysis. In the po group, blood samples were collected via the orbital venous plexus before administration and at 15 min, 0.5 h, 1 h, 2 h, 4 h, 6 h, 8 h, 12 h, and 24 h after administration, and the processing method was the same as that of the intravenous administration group.
(4) The concentration of the compound of the present disclosure in plasma was determined by the LC-MS/MS method.
(5) The calculation and fitting of the results were performed using WinNonlin software from Certara.

The results show that the compounds of the present disclosure exhibit good pharmacokinetic properties.

### Rat pharmacokinetics assay

### Experimental protocol:

(1) Healthy male SD rats (purchased from Beijing Vital River Laboratory Animal Technology Co., Ltd., 190-220g) were prepared. For each compound, 6 mice were divided into 2 groups (iv and po groups), with 3 mice per group. Blood microsampling (100 µL) was performed at each time point.
(2) After overnight fasting (with free access to water), the compounds of the present disclosure were dissolved (or formed into a suspension) using 10% DMSO and 90% of 30% HP-β-CD (v:v) as the iv vehicle and 0.5% MC as the po vehicle. Administration was performed intravenously (iv, 1 mg/kg) and intragastrically (po, 5 mg/kg), respectively.
(3) In the iv group, blood samples were collected via the jugular vein at 5 min, 15 min, 0.5 h, 1 h, 2 h, 4 h, 8 h, 12 h, and 24 h after administration, anticoagulated with EDTA-K₂, centrifuged at 4°C for 5 min to separate plasma, and stored at -20°C until analysis. In the po group, blood samples were collected via the jugular vein before administration and at 15 min, 0.5 h, 1 h, 2 h, 4 h, 6 h, 8 h, 12 h, and 24 h after administration, and the processing method was the same as that of the intravenous administration group.
(4) The concentration of the compound of the present disclosure in plasma was determined by the LC-MS/MS method.
(5) The calculation and fitting of the results were performed using WinNonlin software from Certara.

The results show that the compounds of the present disclosure exhibit good pharmacokinetic properties.

### Pharmacokinetics assay in dogs

### Experimental protocol:

(1) Healthy male beagle dogs (purchased from Chengdu Dossy Experimental Animals Co., Ltd., 6-8 kg) were prepared. For each compound, 6 dogs were divided into 2 groups (iv and po groups), with 3 dogs per group.
(2) After overnight fasting (with free access to water), the compounds of the present disclosure were dissolved (or formed into a suspension) using 10% DMSO and 90% of 30% HP-β-CD (v:v) as the iv vehicle and 0.5% MC as the po vehicle. Administration was performed intravenously (iv, 1 mg/kg) and intragastrically (po, 3 mg/kg), respectively.
(3) In the iv group, 0.5 mL blood samples were collected via the forelimb vein at 5 min, 15 min, 0.5 h, 1 h, 2 h, 4 h, 8 h, 12 h, and 24 h after administration, anticoagulated with EDTA-K₂, centrifuged at 4°C for 5 min to separate plasma, and stored at -20°C until analysis. In the po group, 0.5 mL blood samples were collected via the forelimb vein before administration and at 15 min, 0.5 h, 1 h, 2 h, 4 h, 6 h, 8 h, 12 h, and 24 h after administration, and the processing method was the same as that of the intravenous administration group.
(4) The concentration of the compound of the present disclosure in plasma was determined by the LC-MS/MS method.
(5) The calculation and fitting of the results were performed using WinNonlin software from Certara.

The results show that the compounds of the present disclosure exhibit good pharmacokinetic properties.

### Human and monkey hepatocyte stability assay

### Experimental steps:

Human hepatocytes (BioIVT, X008000) or monkey hepatocytes (RILD, HP-SXH-02M) were used for hepatocyte stability assays. Hepatocytes were counted and viable cell density was determined using AO/PI staining, cells were diluted with culture medium to 0.5 × 10⁶ viable cells/mL, 198 µL of hepatocyte suspension was inoculated into a 96-well plate, and transferred to an incubator for 10 minutes of incubation. Subsequently, 2 µL of 100 µM test compound or positive control solution was respectively added to the corresponding 96 wells to start the reaction, and incubation was carried out in the incubator, with two duplicate wells set. After the reaction was started, 25 µL of samples were taken respectively at 0.5, 15, 30, 60, 90, and 120-minute time points, and 300 µL of acetonitrile containing internal standard IS (100 nM alprazolam, 200 nM caffeine, 200 nM labetalol, and 100 nM tolbutamide) was added to terminate the reaction, followed by vortexing for 5 minutes. The supernatant was taken after centrifugation at 3,220 g for 45 minutes. The supernatant was diluted with an equal volume of ultrapure water and then detected by LC/MS/MS.

### Data analysis:

The peak area was determined according to the extracted ion chromatogram. The *in vitro* half-life (t_{1/2}) was determined through regression analysis of the percentage reduction of the test compound versus the time curve, and the calculation formula was t_{1/2} = -0.693/k. The *in vitro* intrinsic clearance rate (*in vitro* Clᵢₙₜ, µL/min/1 × 10⁶ cells) was calculated according to the formula Clᵢₙₜ (*in vitro*) = -kV/N (V = incubation volume (*i.e.* 0.2 mL); N = number of hepatocytes per well (*i.e.* 0.1 × 10⁶ cells).

The results show that the compounds of the present disclosure exhibit good metabolic stability in monkey or human hepatocytes.

### Monkey PK protocol

### Experimental protocol:

(1) Monkey PK experiments were conducted using cynomolgus monkeys (male, provided by Sichuan Green-house Biotech Co., Ltd.), with 6 monkeys used for each compound, divided into 2 groups (iv and po groups), with 3 monkeys in each group.
(2) The animals in the intravenous administration group were not fasted and had free access to water. The animals in the intragastric administration group were fasted for more than 12 h before administration, had free access to water, and were fed 4 h after administration. 10% DMSO and 90% of 30% HP-β-CD (v:v) were used as the iv vehicle, and sodium citrate buffer (pH = 3) was used as the po vehicle; the compound of the present disclosure was dissolved (or formed a suspension). Administration was performed intravenously (iv, 1 mg/kg) and intragastrically (po, 3 mg/kg), respectively.
(3) In the iv group, blood samples were collected at 5 min, 15 min, 0.5, 1, 2, 4, 8, and 24 h after administration; in the po group, blood samples were collected at 15 min, 0.5, 1, 2, 4, 6, 8, and 24 h after administration. At each time point, 1 mL of blood from the forelimb vein was collected (blood samples were placed in an ice bath after collection), anticoagulated with EDTA-K₂, and plasma was obtained after centrifugation at 4°C, 3500 rpm for 10 min.
(4) The concentration of the compound of the present disclosure in plasma was determined by the LC-MS/MS method.
(5) The calculation and fitting of the results were performed using WinNonlin software from Certara.

The results show that the compounds of the present disclosure exhibit good pharmacokinetic properties.

The specification of the present disclosure provides a detailed description of specific embodiments. Those skilled in the art should recognize that the above embodiments are exemplary and should not be understood as limitations of the present disclosure. For those skilled in the art, without departing from the principles of the present disclosure, modifications and adjustments made to the present disclosure, and the technical solutions obtained through such modifications and adjustments, also fall within the scope of protection of the claims of the present disclosure.

## Claims

1. A compound represented by general formula (I), or all stereoisomers, pharmaceutically acceptable salts, or deuterated derivatives thereof: 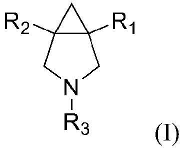 wherein:
R₂ is C₁₋₆ alkyl or C₃₋₁₀ cycloalkyl, wherein the C₁₋₆ alkyl or C₃₋₁₀ cycloalkyl is optionally further substituted with one or more halogens;
R₃ is 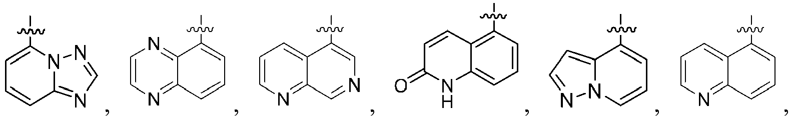 or 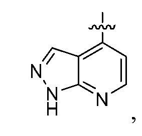 and R₃ is optionally further substituted with one or more substituents selected from the group consisting of C₁₋₃ haloalkyl, D, C₁₋₃ alkyl, halogen, -C₁₋₃ alkoxy, -O-C₁₋₃ haloalkyl, cyano, and -NO₂;
R₁ is 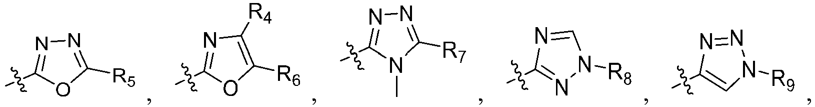 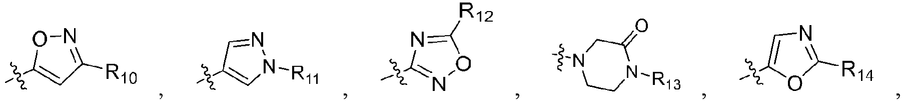 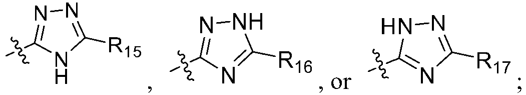
and when R₃ is 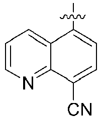 and R₁ is 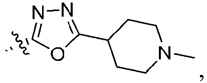 then R₂ is not -CF₃;
R₄ is H, C₁₋₆ alkyl, C₃₋₁₀ cycloalkyl, or halogen;
R₅, R₆, R₇, R₈, R₉, R₁₀, R₁₁, R₁₂, R₁₃, R₁₄, R₁₅, R₁₆, and R₁₇ are each independently H, C₁₋₆ alkyl, halogen, 7- to 10-membered bridged or spiro ring, 3- to 10-membered monocyclic heterocycle, 4- to 7-membered monocyclic carbocycle, 2- to 6-membered heteroalkyl, alkynyl, -NH₂, -C₁₋₃ alkyl-NH₂, -O-3- to 10-membered monocyclic heterocycle, -C₁₋₃ alkoxy-3- to 10-membered monocyclic heterocycle, -NH-3- to 10-membered monocyclic heterocycle, or -C₁₋₃ alkyl-3- to 10-membered monocyclic heterocycle;
R₅, R₆, R₇, R₈, R₉, R₁₀, R₁₁, R₁₂, R₁₃, R₁₄, R₁₅, R₁₆, and R₁₇ are each independently optionally further substituted with one or more substituted or unsubstituted -NH-CHO, C₁₋₆ alkyl, 2- to 6-membered heteroalkyl, -C₁₋₃ alkyl-CO-NH₂, =O, halogen, 3- to 10-membered cycloalkyl, hydroxyl, or -NH₂.

2. The compound, or all stereoisomers, pharmaceutically acceptable salts, or deuterated derivatives thereof according to claim 1, wherein:
the substituted R₅, R₆, R₇, R₈, R₉, R₁₀, R₁₁, R₁₂, R₁₃, R₁₄, R₁₅, R₁₆, or R₁₇ is further substituted with one or more substituents selected from the group consisting of 3- to 10-membered cycloalkyl, 3- to 10-membered heterocycloalkyl, C₁₋₆ alkyl, halogen, and -N-(CH₃)₂.

3. The compound, or all stereoisomers, pharmaceutically acceptable salts, or deuterated derivatives thereof according to claim 1 or 2, wherein:
R₁ is 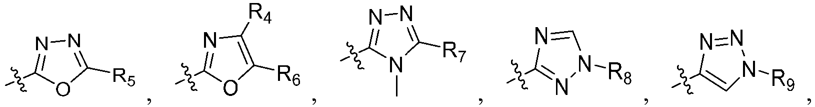 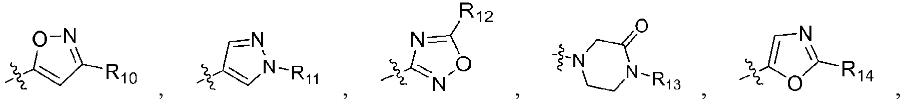 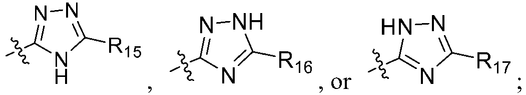
R₄ is H, C₁₋₆ alkyl, or halogen;
R₅, R₆, R₇, R₈, R₉, R₁₀, R₁₁, R₁₂, R₁₃, R₁₄, R₁₅, R₁₆, and R₁₇ are each independently C₁₋₆ alkyl, 2- to 6-membered heteroalkyl, alkynyl, -NH₂, -C₁₋₃ alkyl-NH₂, -O-3- to 10-membered monocyclic heterocycle, -C₁₋₃ alkoxy-3- to 10-membered monocyclic heterocycle, -NH-3- to 10-membered monocyclic heterocycle, or -C₁₋₃ alkyl-3- to 10-membered monocyclic heterocycle;
R₅, R₆, R₇, R₈, R₉, R₁₀, R₁₁, R₁₂, R₁₃, R₁₄, R₁₅, R₁₆, and R₁₇ are each independently optionally further substituted with one or more substituted or unsubstituted -NH-CHO, C₁₋₆ alkyl, 2- to 6-membered heteroalkyl, -C₁₋₃ alkyl-CO-NH₂, =O, halogen, 3- to 10-membered cycloalkyl, hydroxyl, or -NH₂;
the substituted R₅, R₆, R₇, R₈, R₉, R₁₀, R₁₁, R₁₂, R₁₃, R₁₄, R₁₅, R₁₆, or R₁₇ is optionally further substituted with one or more substituents selected from the group consisting of 3- to 10-membered cycloalkyl, 3- to 10-membered heterocycloalkyl, C₁₋₆ alkyl, halogen, and -N-(CH₃)₂.

4. The compound, or all stereoisomers, pharmaceutically acceptable salts, or deuterated derivatives thereof according to claim 3, wherein:
R₅ and R₇ are each independently C₁₋₆ alkyl, 2- to 6-membered heteroalkyl, alkynyl, -NH₂, -C₁₋₃ alkyl-NH₂, -O-3- to 10-membered monocyclic heterocycle, -C₁₋₃ alkoxy-3- to 10-membered monocyclic heterocycle, -NH-3- to 10-membered monocyclic heterocycle, or -C₁₋₃ alkyl-3- to 10-membered monocyclic heterocycle;
R₅ and R₇ are each independently optionally further substituted with one or more substituted or unsubstituted C₁₋₆ alkyl, 2- to 6-membered heteroalkyl, or -NH₂;
the substituted R₅ or R₇ is optionally further substituted with one or more substituents selected from the group consisting of 3- to 10-membered heterocycloalkyl, C₁₋₆ alkyl, and -N-(CH₃)₂.

5. The compound, or all stereoisomers, pharmaceutically acceptable salts, or deuterated derivatives thereof according to claim 1 or 2, wherein:
R₁ is 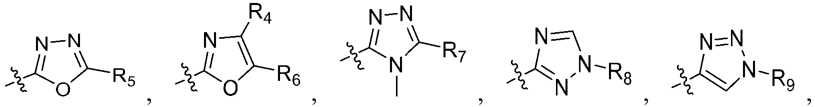 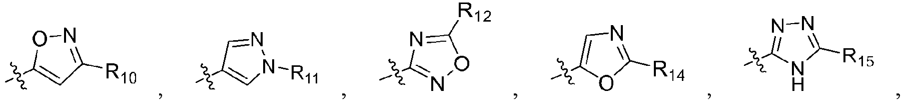 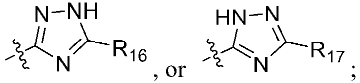
R₄ is H or halogen;
R₅, R₆, R₇, R₈, R₉, R₁₀, R₁₁, R₁₂, R₁₄, R₁₅, R₁₆, and R₁₇ are each independently 7- to 8-membered bridged ring, 7- to 9-membered spiro heterocycle, 4- to 6-membered monocyclic heterocycle, or 6-membered monocyclic carbocycle;
R₅, R₆, R₇, R₈, R₉, R₁₀, R₁₁, R₁₂, R₁₄, R₁₅, R₁₆, and R₁₇ are each independently optionally further substituted with one or more substituted or unsubstituted -NH-CHO, C₁₋₆ alkyl, hydroxyl, -C₁₋₃ alkyl-CO-NH₂, =O, halogen, or -NH₂;
the substituted R₅, R₆, R₇, R₈, R₉, R₁₀, R₁₁, R₁₂, R₁₄, R₁₅, R₁₆, or R₁₇ is further substituted with one or more substituents selected from the group consisting of 3- to 5-membered cycloalkyl, 5- to 6-membered heterocycloalkyl, and C₁₋₆ alkyl.

6. The compound, or all stereoisomers, pharmaceutically acceptable salts, or deuterated derivatives thereof according to claim 5, wherein:
R₅ is 4- to 6-membered non-aromatic nitrogen-containing monocyclic heterocycle, 6-membered non-aromatic monocyclic carbocycle, 8-membered bridged carbocycle, 7- to 8-membered nitrogen-containing bridged heterocycle, or 7- to 9-membered nitrogen-containing spiro heterocycle;
the 4- to 6-membered non-aromatic nitrogen-containing monocyclic heterocycle is optionally further substituted with one or more substituents selected from the group consisting of halogen, C₁₋₃ alkyl, -C₁₋₃ alkyl-CO-NH₂, =O, -NH₂, hydroxyl, and cyclopropyl-substituted C₁₋₃ alkyl;
the 8-membered bridged carbocycle is optionally further substituted with one or more substituents selected from the group consisting of -NH₂ and -NH-CHO, wherein the -NH₂ or - NH-CHO is optionally further substituted with C₁₋₃ alkyl or 6-membered nitrogen-containing heterocyclyl;
the 7- to 8-membered nitrogen-containing bridged heterocycle is optionally further substituted with one or more C₁₋₃ alkyl;
the 7- to 9-membered nitrogen-containing spiro heterocycle is optionally further substituted with one or more substituents selected from the group consisting of -C₁₋₃ alkyl-CO-NH₂, C₁₋₃ alkyl, and cyclopropyl-substituted C₁₋₃ alkyl;
the 6-membered non-aromatic monocyclic carbocycle is optionally further substituted with one or more -NH₂ or N-(CH₃)₂;
R₆, R₇, R₈, R₉, R₁₀, R₁₁, R₁₂, R₁₄, R₁₅, and R₁₆ are 6-membered non-aromatic nitrogen-containing monocyclic heterocycle, wherein the 6-membered non-aromatic nitrogen-containing monocyclic heterocycle is optionally further substituted with one or more C₁₋₃ alkyl.

7. The compound, or all stereoisomers, pharmaceutically acceptable salts, or deuterated derivatives thereof according to any one of claims 1-6, wherein the compound is one of the following structures:

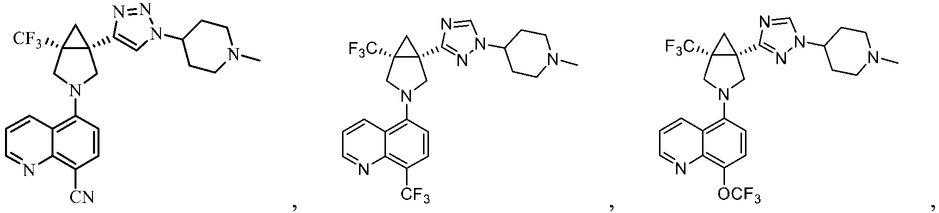

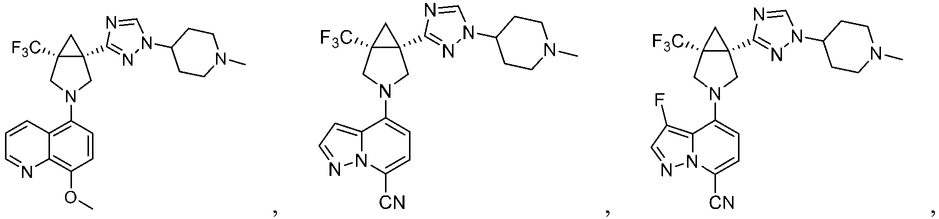

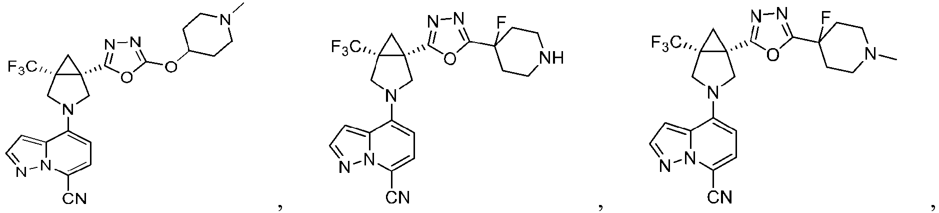

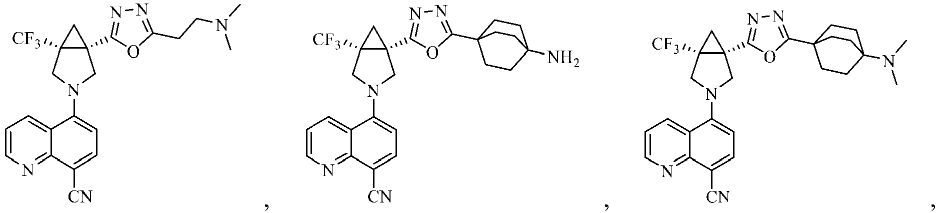

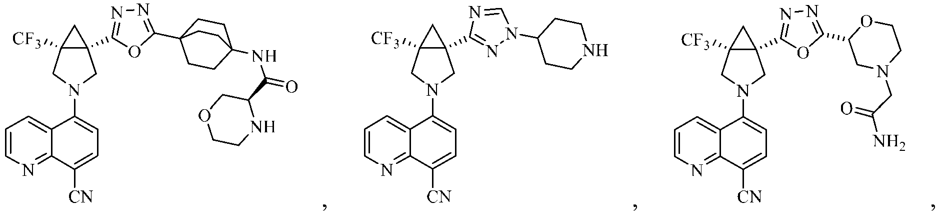

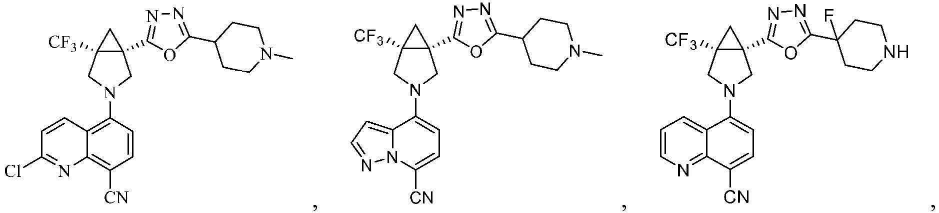

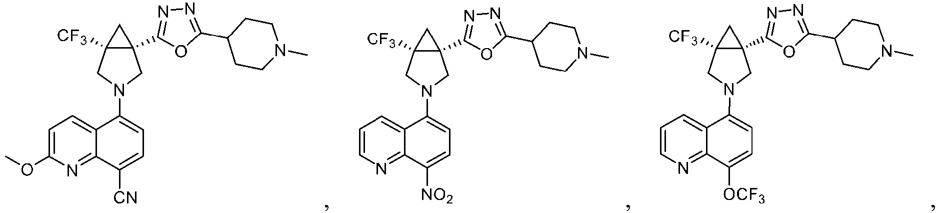

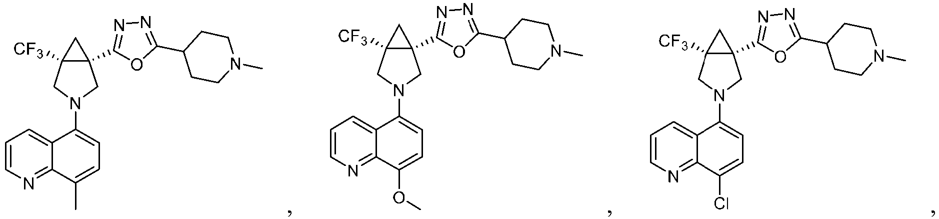

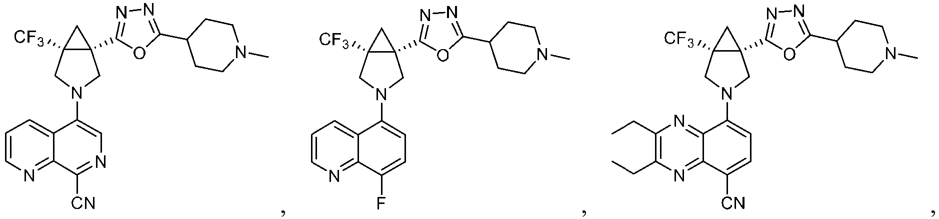

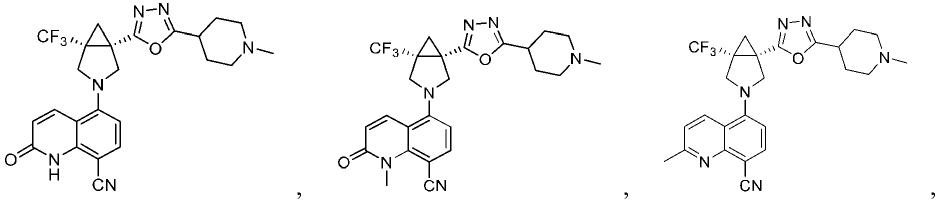

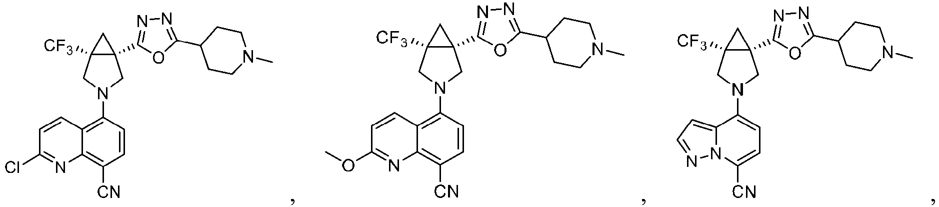

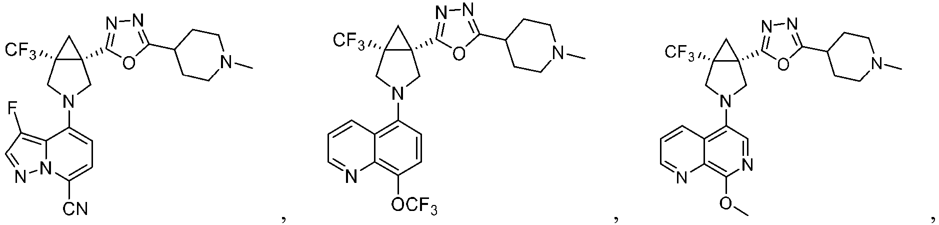

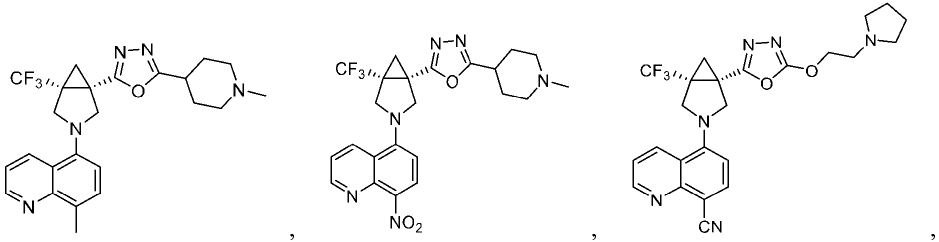

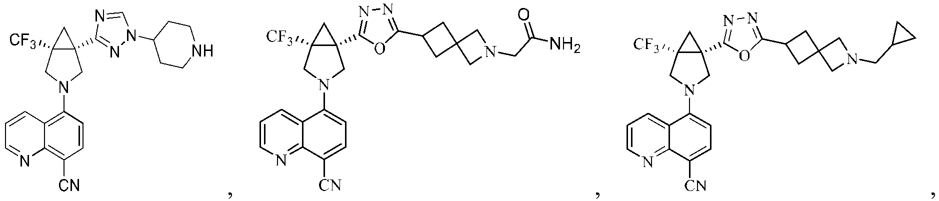

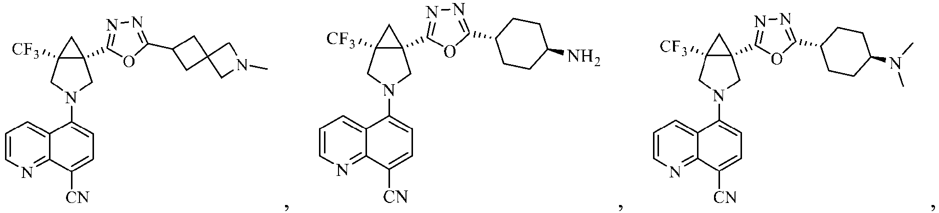

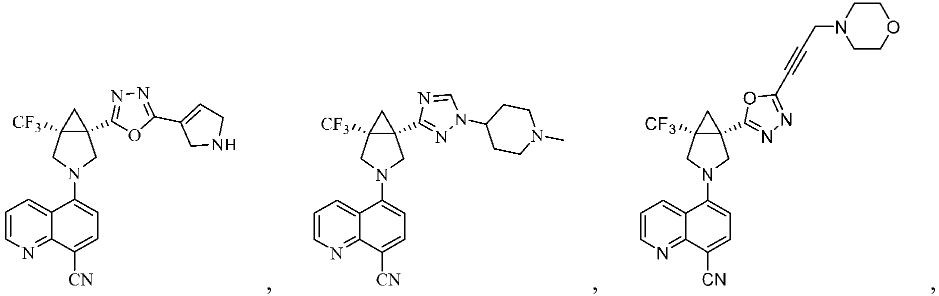

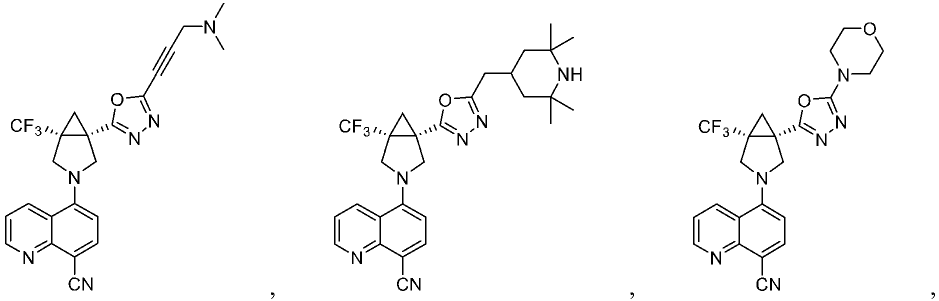

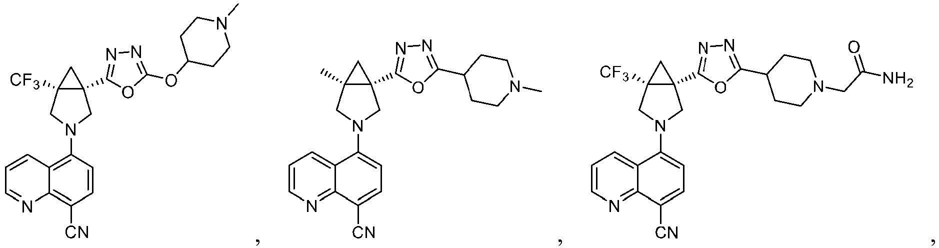

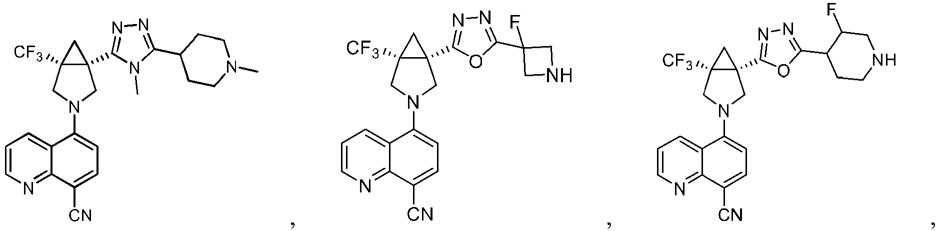

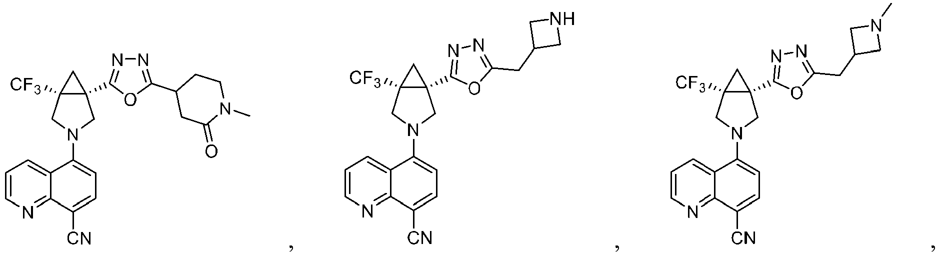

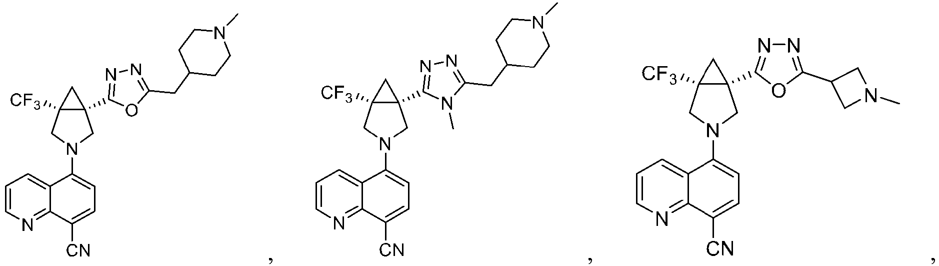

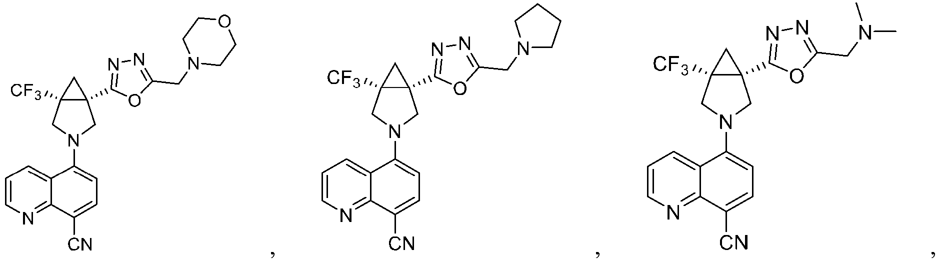

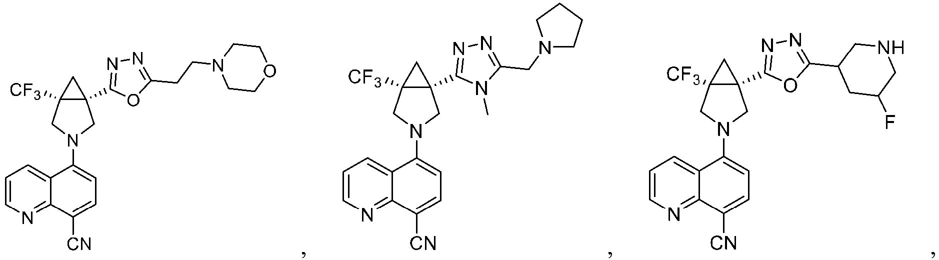

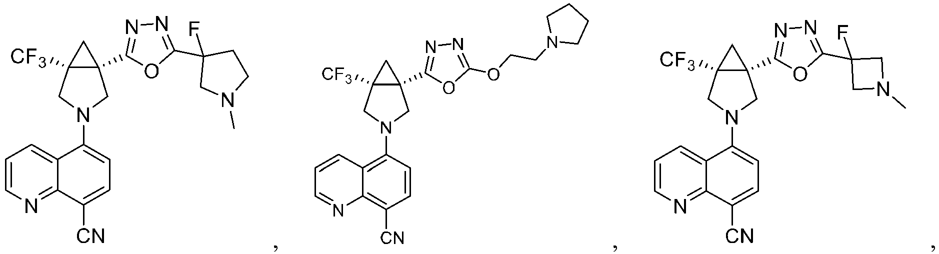

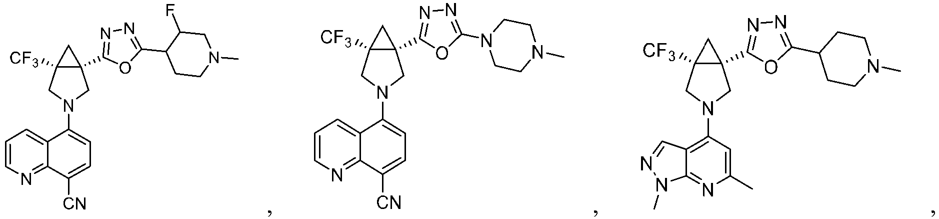

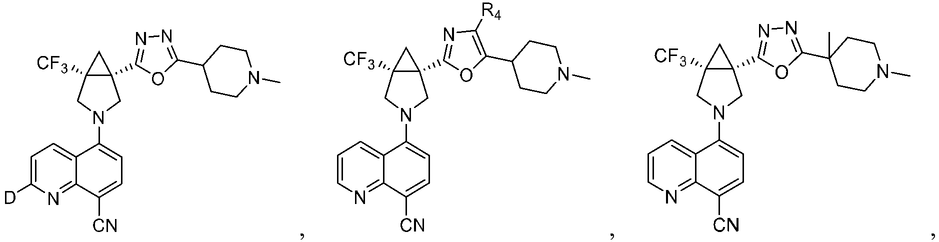

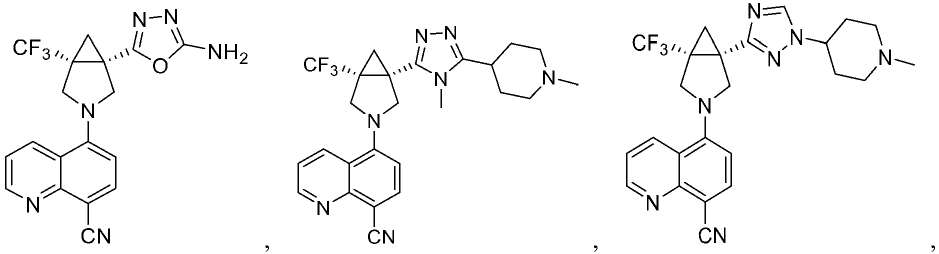

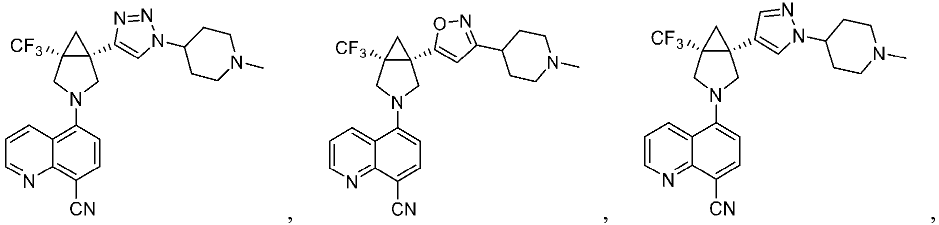

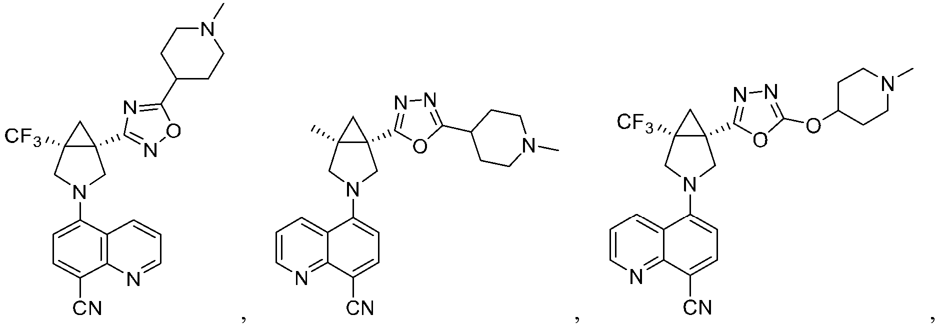

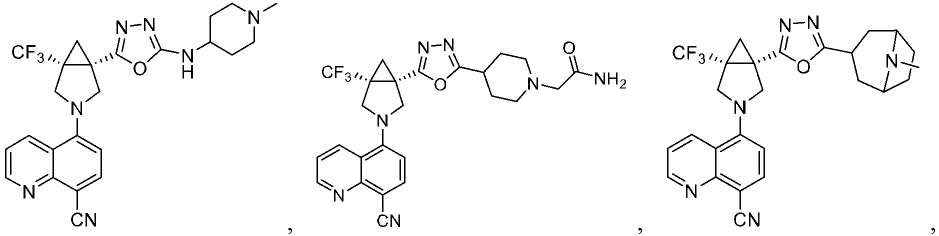

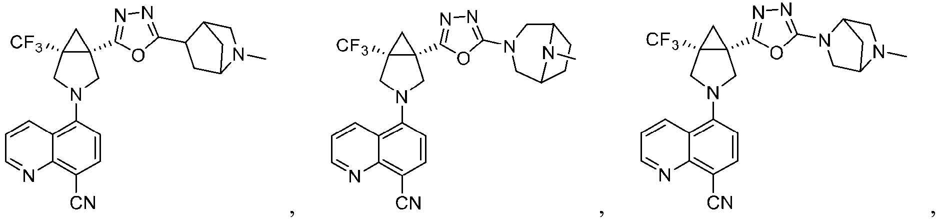

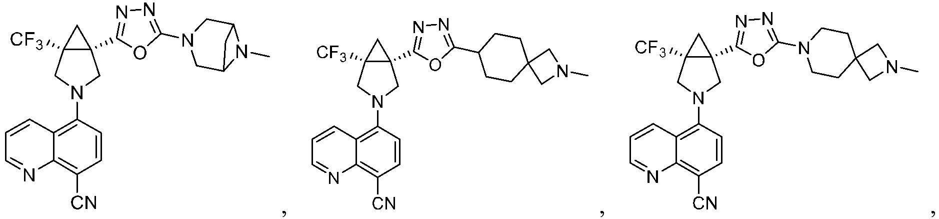

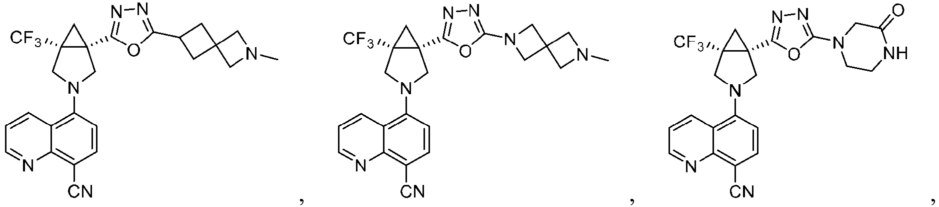

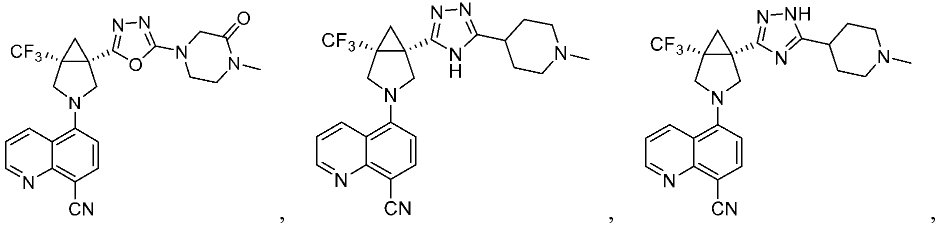

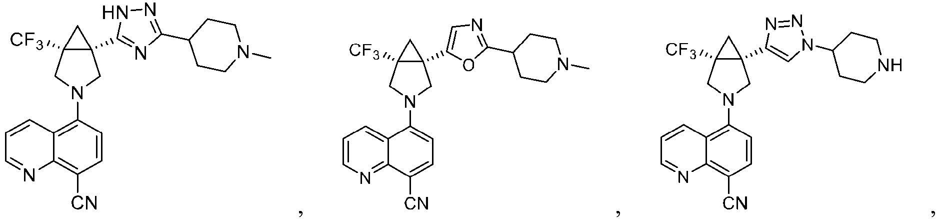

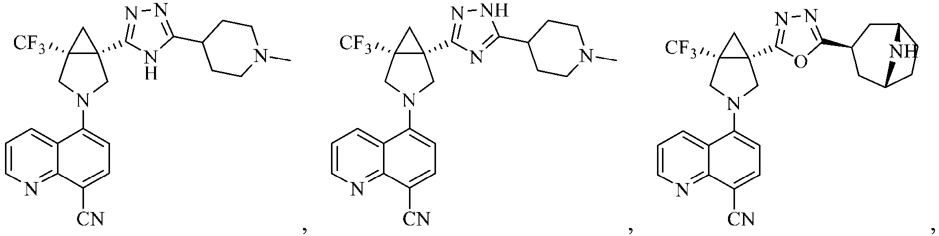

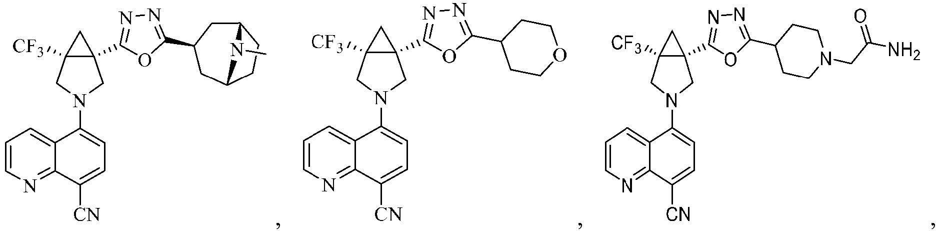

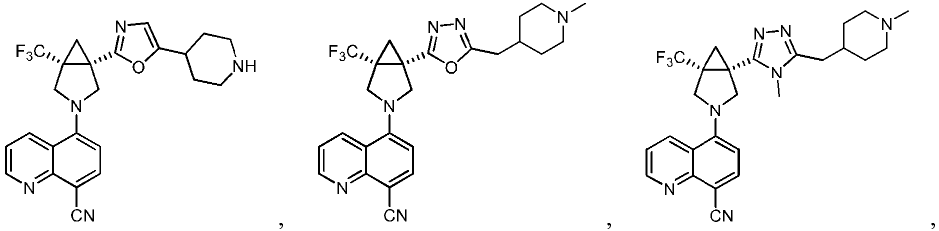

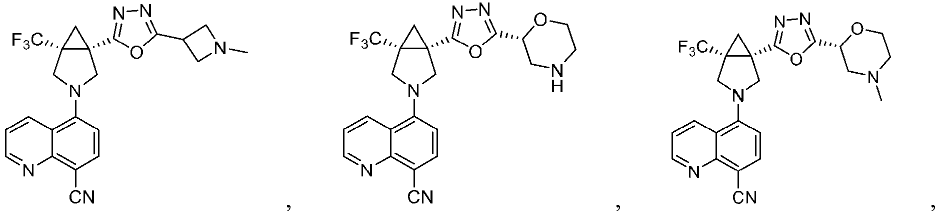

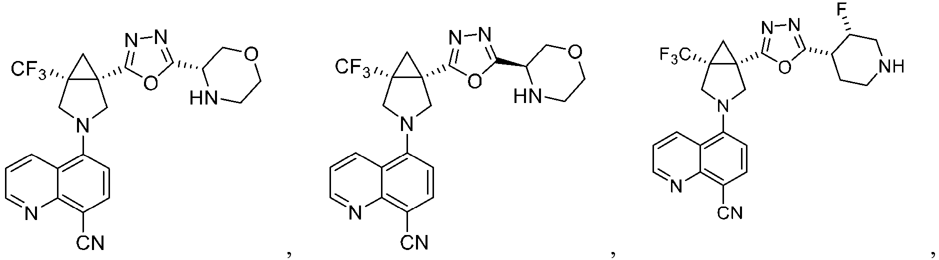

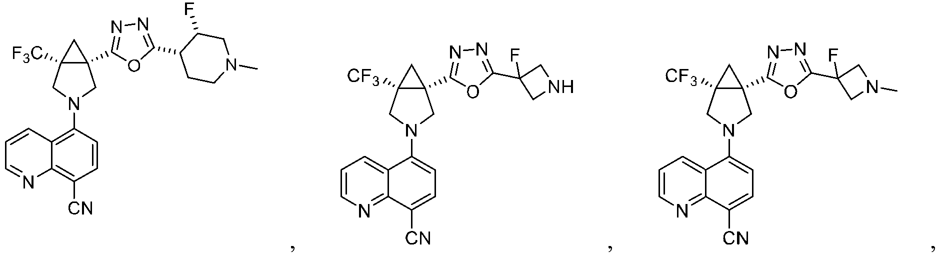

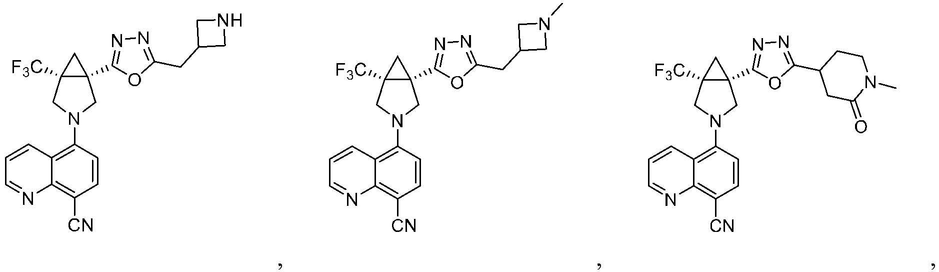

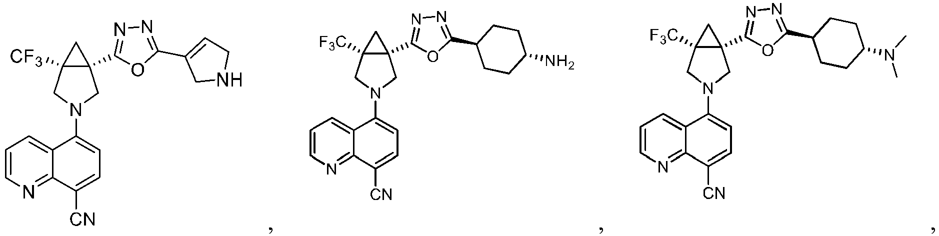

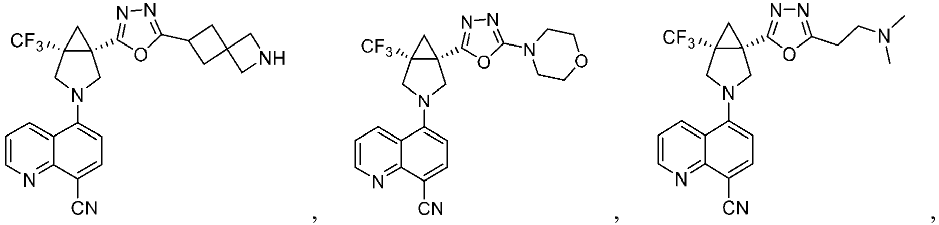

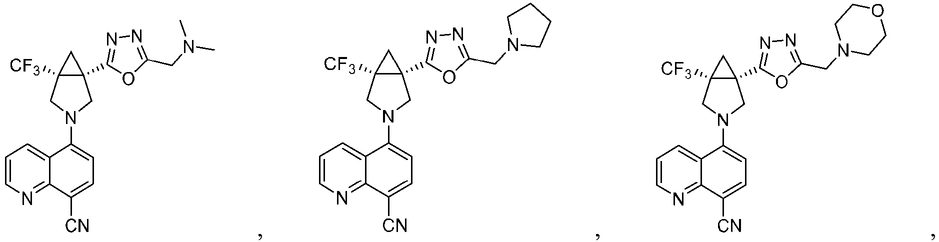

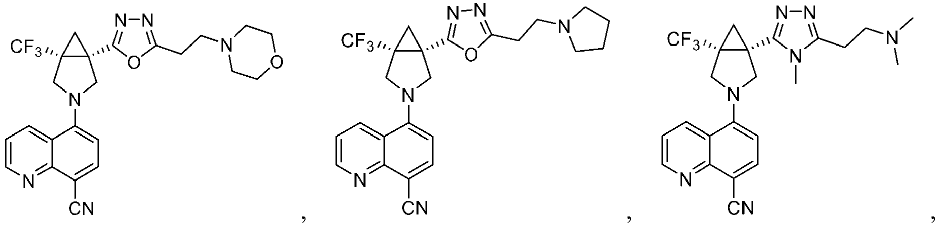

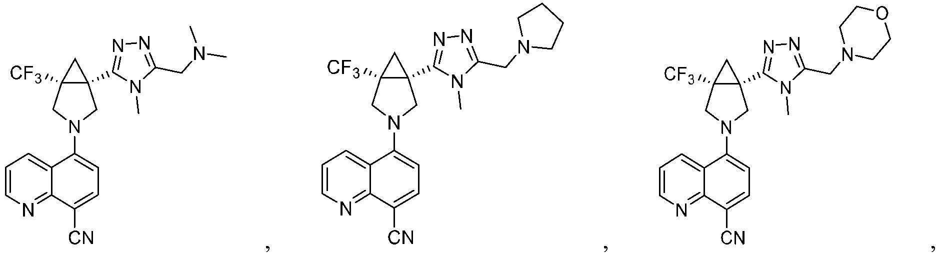

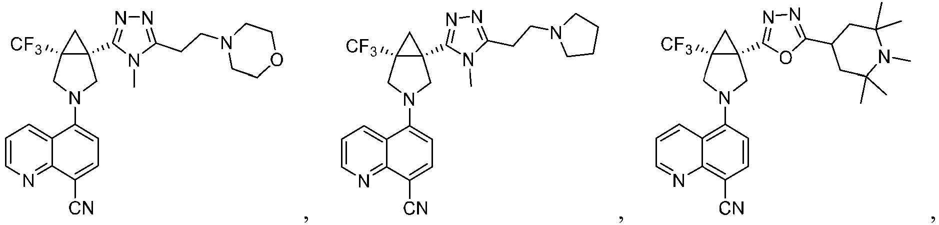

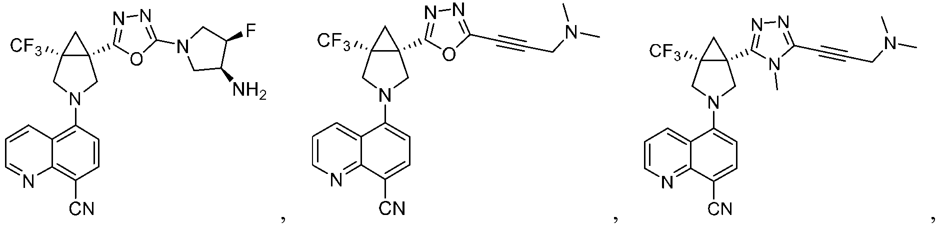

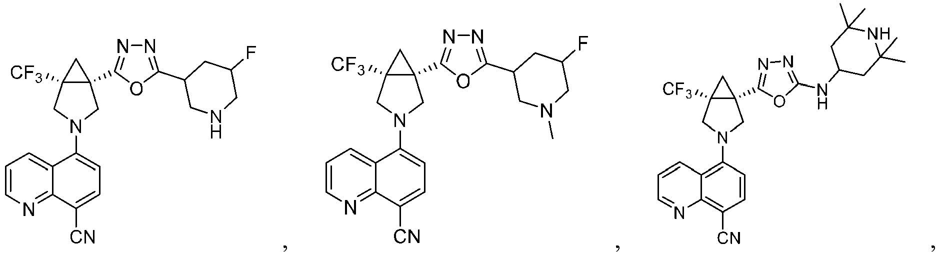

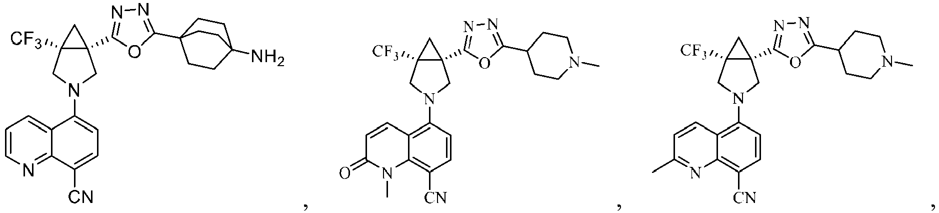

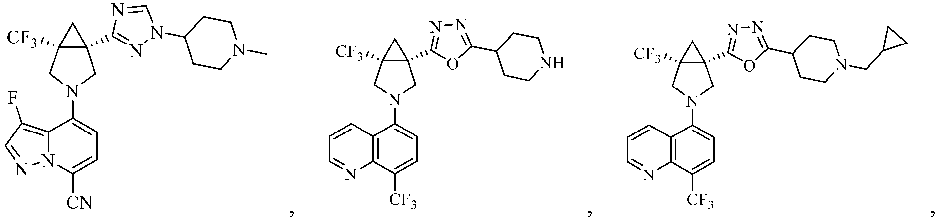

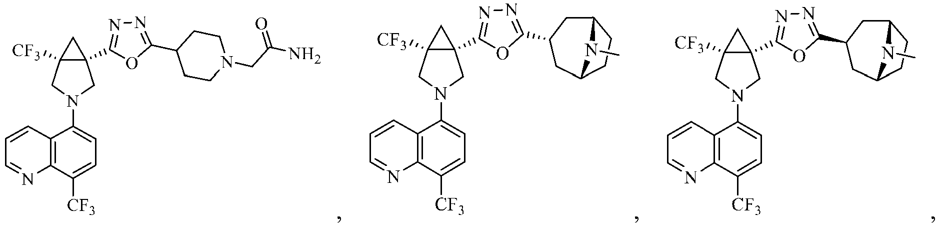

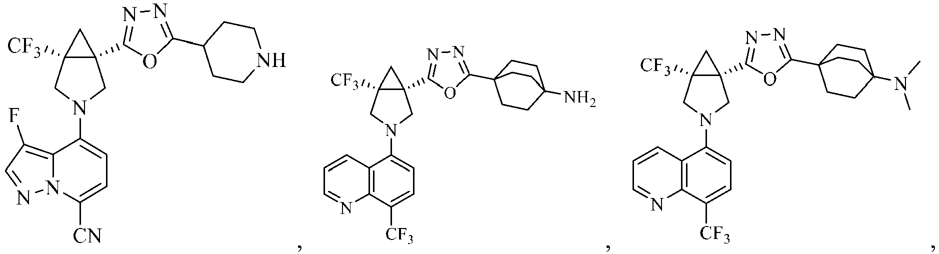

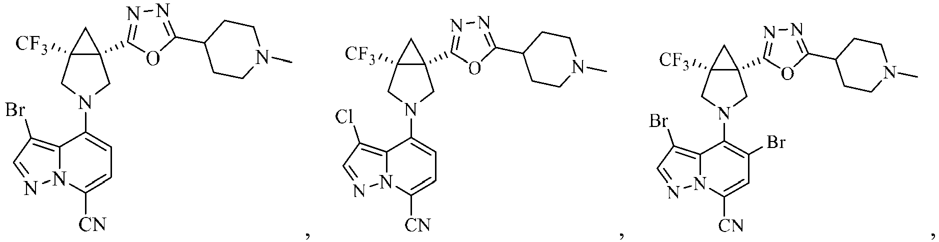

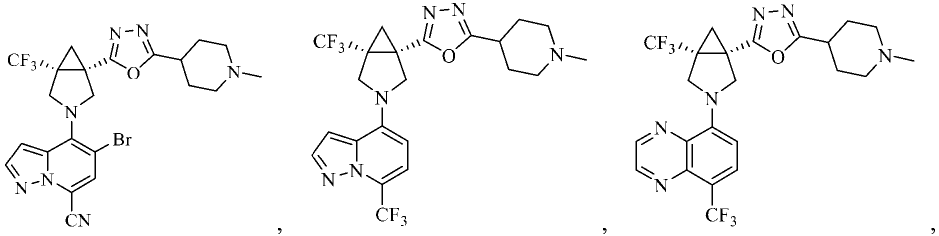

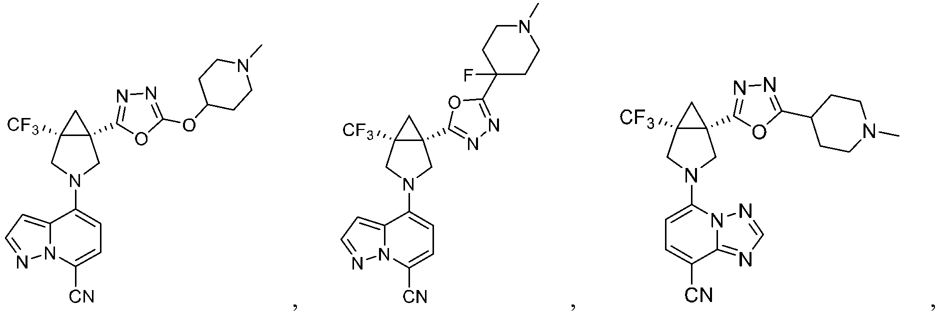

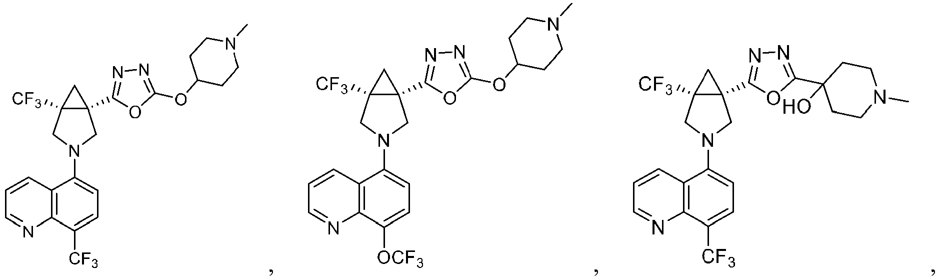

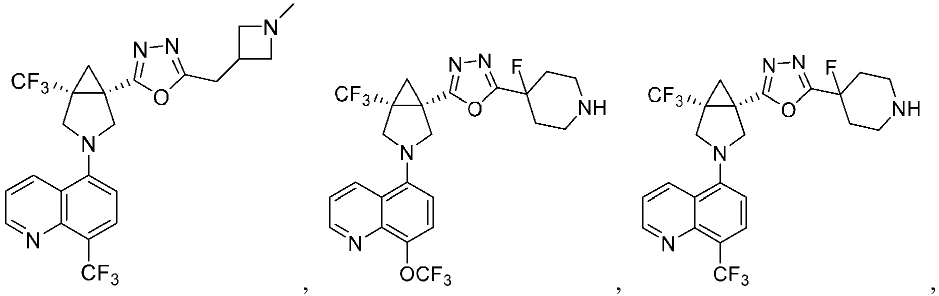

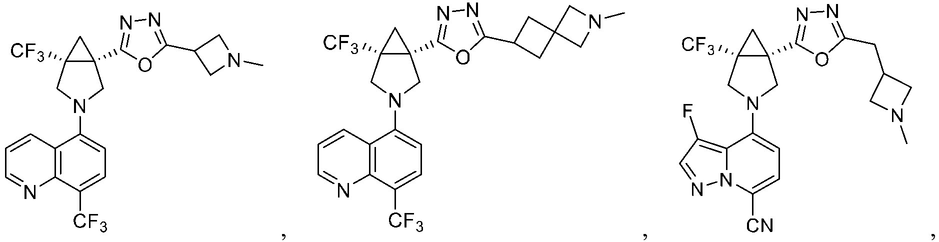

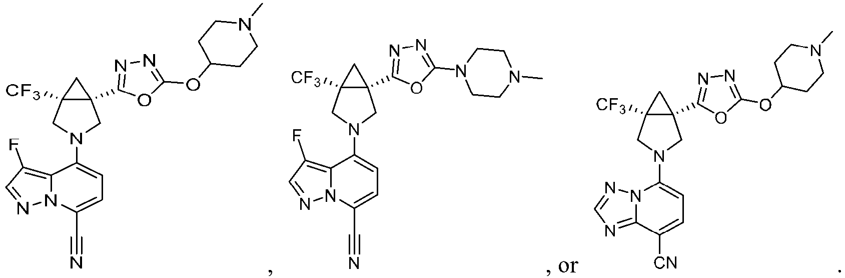

8. A compound represented by general formula (II), or all stereoisomers, pharmaceutically acceptable salts, or deuterated derivatives thereof: 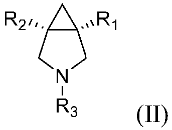 wherein:
R₂ is C₁₋₆ alkyl, wherein the alkyl is optionally further substituted with one or more halogens;
R₃ is 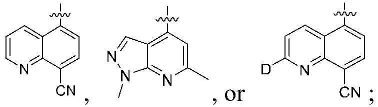
R₁ is 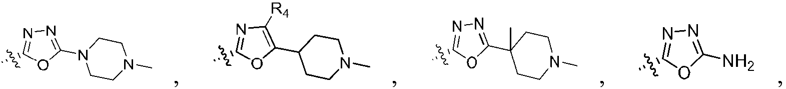 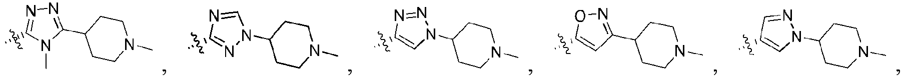 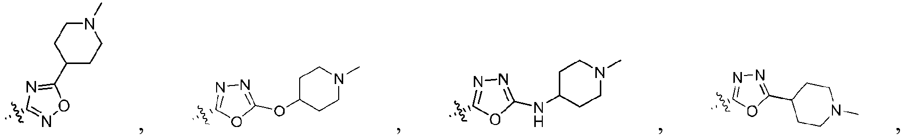 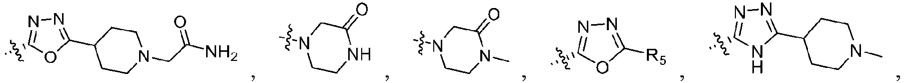 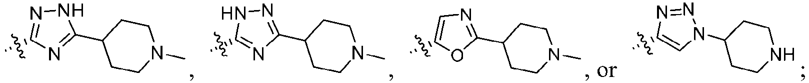
and when R₃ is 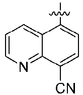 and R₁ is 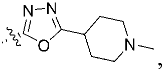 then R₂ is not -CF₃;
R₄ is C₁₋₆ alkyl or halogen;
R₅ is 7- to 10-membered bridged or spiro ring, and R₅ contains at least one N;
R₅ is: 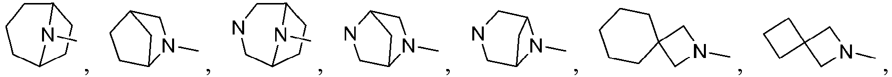 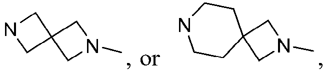 and the connection site of R₅ is at any connectable position thereon.

9. A pharmaceutical composition comprising:
the compound, or the stereoisomer, pharmaceutically acceptable salt, or deuterated derivative thereof as defined in any one of claims 1-8;
optionally one or more other active ingredients; and
a pharmaceutically acceptable carrier and/or excipient.

10. Use of the compound, or the stereoisomer, pharmaceutically acceptable salt, or deuterated derivative thereof as defined in any one of claims 1-8, or the pharmaceutical composition as defined in claim 9, in the manufacture of a medicament for treating and/or preventing an autoimmune disease.
